(19) Europäisches Patentamt
European Patent Office
Office européen des brevets

(11) EP 1 349 843 B1

(12) **EUROPEAN PATENT SPECIFICATION**

(45) Date of publication and mention of the grant of the patent:
**20.04.2005 Bulletin 2005/16**

(21) Application number: **01994514.6**

(22) Date of filing: **19.12.2001**

(51) Int Cl.[7]: **C07D 277/24**, C07D 277/28, C07D 277/26, C07D 263/32, A61K 31/42, A61K 31/425, A61P 3/10, A61P 9/10, A61P 3/04

(86) International application number:
**PCT/US2001/051056**

(87) International publication number:
**WO 2002/059098 (01.08.2002 Gazette 2002/31)**

(54) **THIAZOLE AND OXAZOLE DERIVATIVES AS ACTIVATORS OF HUMAN PEROXISOME PROLIFERATOR ACTIVATED RECEPTORS**

THIAZOL UND OXAZOL-DERIVATE ALS AKTIVATOREN VON MENSCHLICHEN PEROXISOM PROLIFERATOR AKTIVIERTEN REZEPTOREN

DERIVES DE THIAZOLE ET D'OXAZOLE EN TANT QU'ACTIVATEURS DE RECEPTEURS ACTIVES PAR LE PROLIFERATEUR DU PEROXYSOME HUMAIN

(84) Designated Contracting States:
**AT BE CH CY DE DK ES FI FR GB GR IE IT LI LU MC NL PT SE TR**

(30) Priority: **20.12.2000 GB 0031103**

(43) Date of publication of application:
**08.10.2003 Bulletin 2003/41**

(73) Proprietor: **Smithkline Beecham Corporation
Philadelphia, Pennsylvania 19101 (US)**

(72) Inventors:
- **BANKER, Pierette, GlaxoSmithKline
Research Triangle Park, NC 27709 (US)**
- **CADILLA, Rodolfo, GlaxoSmithKline
Research Triangle Park, NC 27709 (US)**
- **LAMBERT, Millard, Hurst III, GlaxoSmithKline
NC 27709 (US)**
- **RAFFERTY, Stephen, W., GlaxoSmithKline
NC 27709 (US)**
- **STERNBACH, Daniel David, GlaxoSmithKline
NC 27709 (US)**
- **SZNAIDMAN, Marcos, Luis
Durham, NC 27713 (US)**

(74) Representative: **Learoyd, Stephanie Anne
GlaxoSmithKline
Corporate Intellectual Property (CN9.25.1)
980 Great West Road
Brentford, Middlesex TW8 9GS (GB)**

(56) References cited:
**WO-A-00/08002**
**WO-A-01/00603**
**WO-A-01/40207**
**US-A- 5 972 881**

- **D. BISHOP-BAILEY: "Peroxisome Proliferator Activated Receptors in the Cardiovascular System" BRITISH JOURNAL OF PHARMACOLOGY, vol. 129, no. 5, March 2000 (2000-03), pages 823-34, XP001079321**
- **J. M. LEHMANN ET. AL.: "An Antidiabetic Thiazolidinedione is a High Affinity Ligand for Peroxisome Proliferator-activated Receptor gamma." JOURNAL OF BIOLOGICAL CHEMISTRY, vol. 270, no. 22, 2 June 1995 (1995-06-02), pages 12953-6, XP000577082**
- **W. R. OLIVER ET. AL.: "A Selective Peroxisome Proliferator -activated Receptor delta Agonist Promotes Reverse Cholesterol Transport." PROCEEDINGS OF THE NATIONAL ACADEMY OF SCIENCES OF THE USA, vol. 98, no. 9, 24 April 2001 (2001-04-24), pages 5306-11, XP001080446**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 349 843 B1

## Description

The present invention relates to certain novel compounds. In particular, the present invention relates to compounds that activate human peroxisome proliferator activated receptors ("hPPARs"). The present invention also relates to methods for preparing the compounds, their use in medicine, pharmaceutical compositions containing them and methods for the prevention or treatment of PPAR mediated diseases or conditions.

Several independent risk factors have been associated with cardiovascular disease. These include hypertension, increased fibrinogen levels, high levels of triglycerides, elevated LDL cholesterol, elevated total cholesterol, and low levels of HDL cholesterol. HMG CoA reductase inhibitors ("statins") are useful for treating conditions characterized by high LDL-c levels. It has been shown that lowering LDL-c is not sufficient for reducing the risk of cardiovascular disease in some patients, particularly those with normal LDL-c levels. This population pool is identified by the independent risk factor of low HDL-c. The increased risk of cardiovascular disease associated with low HDL-c levels has not yet been successfully addressed by drug therapy (i.e. currently there are no drugs on the market that are useful for raising HDL-c). (Bisgaier, C. L.; Pape, M. E. *Curr. Pharm. Des.* 1998, *4*, 53-70).

Syndrome X (including metabolic syndrome) is loosely defined as a collection of abnormalities including hyperinsulinemia, obesity, elevated levels of trigycerides, uric acid, fibrinogen, small dense LDL particles, and plasminogen activator inhibitor 1 (PAI-1), and decreased levels of HDL-c.

NIDDM is described as insulin resistance which in turn causes anomalous glucose output and a decrease in glucose uptake by skeletal muscle. These factors eventually lead to impaired glucose tolerance (IGT) and hyperinsulinemia.

Peroxisome Proliferator Activated Receptors (PPARs) are ophan receptors belonging to the steroid/retinoid receptor superfamily of ligand-activated transcription factors. See, for example Willson T.M. and Wahli, W., Curr. Opin. Chem. Biol. (1997) Vol 1 pp 235-241 and Willson T.M. et. al., J. Med. Chem (2000) Vol 43 p527-549. The binding of agonist ligands to the receptor results in changes in the expression level of mRNA's encoded by PPAR target genes.

Three mammalian Peroxisome Proliferator-Activated Receptors have been isolated and termed PPAR-alpha, PPAR-gamma, and PPAR-delta (also known as NUC1 or PPAR-beta). These PPARs regulate expression of target genes by binding to DNA sequence elements, termed PPAR response elements (PPRE). To date, PPRE's have been identified in the enhancers of a number of genes encoding proteins that regulate lipid metabolism suggesting that PPARs play a pivotal role in the adipogenic signaling cascade and lipid homeostasis (H. Keller and W. Wahli, *Trends Endocrin. Met* 291-296, 4 (1993)).

It has now been reported that thiazolidinediones are potent and selective activators of PPAR-gamma and bind directly to the PPAR-gamma receptor (J. M. Lehmann et. al., *J. Biol. Chem.* 12953-12956, 270 (1995)), providing evidence that PPAR-gamma is a possible target for the therapeutic actions of the thiazolidinediones.

Activators of the nuclear receptor PPARγ, for example troglitazone, have been shown in the clinic to enhance insulin-action, reduce serum glucose and have small but significant effects on reducing serum triglyceride levels in patients with Type 2 diabetes. See, for example, D. E. Kelly et al., *Curr. Opin. Endocrinol.* Diabetes, 90-96, 5 (2), (1998); M. D. Johnson et al., *Ann. Pharmacother., 337-348*, 32 (3), (1997); and M. Leutenegger et al., *Curr. Ther. Res., 403-418*, 58 (7), (1997).

The mechanism for this triglyceride lowering effect appears to be predominantly increased clearance of very low density lipoproteins (VLDL) through induction of liporotein lipase (LPL) gene expression. See, for example, B. Staels et al., *Arterioscler. Thromb., Vasc. Biol., 1756-1764,* 17 (9), (1997).

Fibrates are a class of drugs which may lower serum triglycerides 20-50%, lower LDLc 10-15%, shift the LDL particle size from the more atherogenic small dense to normal dense LDL, and increase HDLc 10-15%. Experimental evidence indicates that the effects of fibrates on serum lipids are mediated through activation of PPARα. See, for example, B. Staels et al., *Curr. Pharm. Des., 1-14*, 3 (1), (1997). Activation of PPARα results in transcription of enzymes that increase fatty acid catabolism and decrease de-novo fatty acid synthesis in the liver resulting in decreased triglyceride synthesis and VLDL production/secretion. In addition. PPARα activation decreases production of apoC-III. Reduction in apoC-III, an inhibitor of LPL activity, increases clearance of VLDL. See, for example, J. Auwerx et al., *Atherosclerosis, (Shannon, Irel.), S29-S37*, 124 (Suppl), (1996).

Certain compounds that activate or otherwise interact with one or more of the PPARs have been implicated in the regulation of triglyceride and cholesterol levels in animal models. See, for example, U.S. Patents 5,847,008 (Doebber et al.) and 5,859,051 (Adams et al.) and PCT publications WO 97/28149 (Leibowitz et al.) and WO99/04815 (Shimokawa et al.). In a recent report (Berger et al., *J. Biol. Chem.* 1999), vol. 274, pp. 6718-6725) it was stated that PPARδ activation does not appear to modulate glucose or triglyceride levels. WO 00/08002 describes oxazole and thiazole derivatives as PPAR activators and Proceedings of the National Academy of Sciences USA 98, 5306 - 5311 (2001) describes the compound 2-(4-trifluoromethylphenyl)-4-((4-carboxymethoxy-3-methyl)phenylthiomethyl)-5-methyl-thiazole as a selective PPAR delta agonist.

In one aspect, the present invention provides compounds of formula (I) and pharmaceutically acceptable

salts, solvates, and hydrolysable esters thereof wherein;

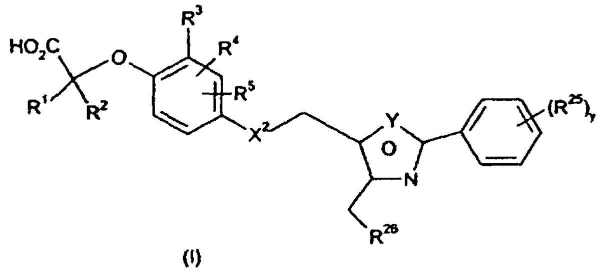

(I)

$R^1$ and $R^2$ are independently hydrogen or $C_{1-3}$ alkyl;
$X^2$ is O, S, or $CH_2$;
$R^3$, $R^4$, and $R^5$ are independently H, $C_{1-3}$alkyl, $OCH_3$, $CF_3$, $OCF_3$, allyl, CN, or halogen;
Y is S or O;
each $R^{25}$ is independently $CH_3$, $OCH_3$, $OCF_3$, $CF_3$, or halogen;
y is 0, 1, 2, 3, 4 or 5; and
$R^{26}$ is selected from the group consisting of the moieties **A** through K depicted below:

**A**

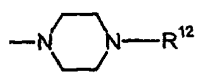

wherein $R^{12}$ is selected from the group consisting of $C_{1-6}$alkyl, $C_{1-6}$alkylenearyl, and the moieties depicted below in Group II,

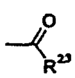
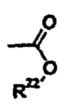
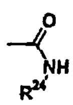
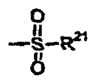
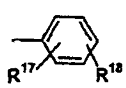

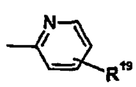
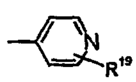
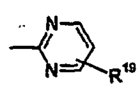
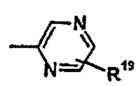

**Group II**

wherein $R^{17}$ and $R^{18}$ are independently hydrogen, halogen, hydroxy, -CN, $C_{1-6}$alkyl, $C_{1-6}$perfluoroalkyl, $C_{1-6}$acyl, -$OC_{1-6}$alkyl, perfluoro$OC_{1-6}$alkyl, or $C_{1-6}$hydroxyalkyl;
$R^{19}$ is hydrogen or $C_{1-6}$alkyl;
$R^{21}$ is $C_{1-6}$alkyl, -$C_{1-6}$alkylenearyl, aryl, or -aryl-heteroaryl;
$R^{22}$ is $C_{1-6}$alkyl, aryl, or -$C_{1-6}$alkylenearyl;
$R^{23}$ is $C_{1-6}$alkyl, $C_{3-6}$cycloalkyl, or aryl;
$R^{24}$ is $C_{1-6}$alkyl, -$C_{1-6}$alkylenearyl, $C_{3-6}$cycloalkyl, or aryl;

B

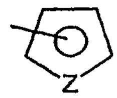

wherein Z is O, N or S (note that when Z is N, the depicted bond can be attached to the nitrogen in the ring as well as any of the carbons in the ring);

C

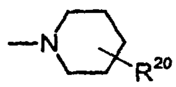

wherein $R^{20}$ is $C_{1-6}$alkyl, aryl, -O$C_{1-6}$alkyl, hydroxy, $C_{1-6}$hydroxyalkyl, or 1-alkoxy$C_{1-6}$alkyl;

D

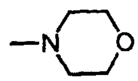

E

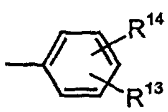

wherein $R^{13}$ and $R^{14}$ are independently hydrogen, halogen, CN, perfluro$C_{1-6}$alkyl, perfluroO$C_{1-6}$ alkyl, $C_{1-6}$alkyl, -O$C_{1-6}$alkyl, -$C_{1-6}$alkyleneO$C_{1-6}$alkyl, -S$C_{1-6}$alkyl, or aryl;

F

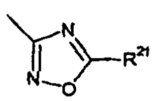

wherein $R^{21}$ is independently as defined above;

G

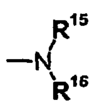

wherein $R^{15}$ and $R^{16}$ are independently hydrogen, $C_{1-6}$alkyl, $C_{3-6}$cycloalkyl optionally substituted with 1 or 2 $C_{1-3}$alkyl groups, or $R^{12}$ as defined above;

**H**

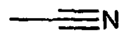

**I**

$-(CH_2)nPh$

wherein n is 1-3

**J**

$-O-R^{21}$

wherein $R^{21}$ is independently as defined above; and

**K**

$-S-R^{21}$

wherein $R^{21}$ is independently as defined above. As used herein "aryl" or in any phrase or term including "aryl" such as "-$C_{1-6}$alkylenearyl", the "aryl" means a phenyl group or a 5 or 6 membered heteroaryl group. As used hereing "heteroaryl" means a 5 or 6 membered heteroaryl group. As used herein any such "aryl" or "heteroaryl" group may optionally be substittued with one or two substituents selected from the group consisting of halogen, CN, dimethylamino, perfluro$C_{1-6}$alkyl, perfluroO$C_{1-6}$alkyl, $C_{1-6}$alkyl, -O$C_{1-6}$alkyl, -$C_{1-6}$alkyleneO$C_{1-6}$alkyl, and -S$C_{1-6}$alkyl.

In another aspect, the present invention discloses a method for prevention or treatment of a disease or condition mediated by one or more human PPAR alpha, gamma or delta ("hPPARs") comprising administration of a therapeutically effective amount of a compound of this invention. hPPAR mediated diseases or conditions include dyslipidemia including associated diabetic dyslipidemia and mixed dyslipidemia, syndrome X (as defined in this application this embraces metabolic syndrome), heart failure, hypercholesteremia, cardiovascular disease including atherosclerosis, arteriosclerosis, and hypertriglyceridemia, type I diabetes mellitus, type I diabetes, insulin resistance, hyperlipidemia, inflammation, epithelial hyperproliferative diseases including eczema and psoriasis and conditions associated with the lung and gut and regulation of appetite and food intake in subjects suffering from disorders such as obesity, anorexia bulimia, and anorexia nervosa. In particular, the compounds of this invention are useful in the treatment and prevention of diabetes and cardiovascular diseases and conditions including atherosclerosis, arteriosclerosis, hypertriglyceridemia, and mixed dyslipidaemia.

In another aspect, the present invention provides pharmaceutical compositions comprising a compound of the invention, preferably in association with a pharmaceutically acceptable diluent or carrier.

In another aspect, the present invention provides a compound of the invention for use in therapy, and in particular, in human medicine.

In another aspect, the present invention provides the use of a compound of the invention for the manufacture of a medicament for the treatment of a hPPAR mediated disease or condition.

As used herein, "a compound of the invention" means a compound of formula (I) or a pharmaceutically acceptable hydrolyzable ester or, solvate, thereof.

While hydrolyzable esters are included in the scope of this invention, the acids are preferred because the data suggests that while the esters are useful compounds, it may actually be the acids to which they hydrolyze that are the active compounds. Esters that hydrolyze readily can produce the carboxylic acid in the assay conditions or in vivo. Generally the carboxylic acid is active in both the binding and transient transfection assays, while the ester does not usually bind well but is active in the transient transfection assay presumably due to hydrolysis. Preferred hydrolysable esters are $C_{1-6}$ alkyl esters wherein the alkyl group may be straight chain or branched chain. Methyl or ethyl esters are more preferred.

Preferably $R^1$ and $R^2$ are independently H or $CH_3$. Most preferably $R^1$ and $R^2$ are either both H or both $CH_3$.

Preferably $X^2$ is O or S. More preferably $X^2$ is S;

Preferably $R^3$ is $CH_3$ or H;

Preferably $R^4$ and $R^5$ are H.

Preferably Y is S.

Preferably y is 1 or 2. When y is 2, preferably one $R^{25}$ is halogen; more preferably one is halogen and the other is $CF_3$. When y is 1, preferably the $R^{25}$ is in the para position on the ring and is more preferably $CF_3$.

Preferably $R^{26}$ is selected from the moieties shown below in Group III.

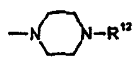
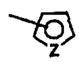
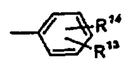

Group III

Preferably $R^{12}$ is selected from the moieties shown below in Group IV.

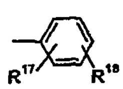
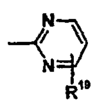
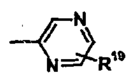

Group IV

Preferably $R^{13}$ or $R^{14}$ are independently fluorine, bromine, phenyl, thienyl, $CF_3$, $OCF_3$, $OCH_3$, $SCH_3$, or t-butyl. Most preferably $R^{14}$ is thienyl, $OCH_3$, $OCF_3$, $CF_3$, or fluorine. Most preferably $R^{14}$ is substituted para to the depicted open valence. Most preferably $R^{13}$ is hydrogen or fluorine.

Preferably $R^{17}$ and $R^{18}$ are independently hydrogen, OH, $OC_{1-3}$alkyl, CN, halogen, $CF_3$, $COCH_3$, CH(OH) $CH_3$, or $OCF_3$. Most preferably $R^{17}$ is fluorine, chlorine, $OC_{1-3}$alkyl, or $COCH_3$ and $R^{18}$ is $OCH_3$ or hydrogen. Most preferably $R^{17}$ is substituted para to the depicted open valence.

Preferably $R^{20}$ is phenyl, methyl, $OCH_3$, OH, or $CH_2OH$.

Preferably $R^{21}$ is -$C_{1-3}$alkylenephenyl, phenyl-5-methyl-1,2,4-oxadiazol-3-yl, or phenyl optionally substituted by methyl or CN.

Preferably $R^{22}$ is $C_{1-6}$alkyl, phenyl, or benzyl.

Preferably $R^{23}$ is $C_{1-6}$alkyl, furanyl, thienyl, methoxymethyl, $C_{3-6}$cyclalkyl, or phenyl optionally substituted by a halogen a methoxy or a dimethylamino group.

Preferably $R^{24}$ is H, $C_{1-6}$alkyl, cyclohexyl, m-methoxyphenyl, p-fluorophenyl, or -$CH_2CH_2$phenyl.

Preferably $R^{19}$ is hydrogen.

Particularly preferred compounds will be those is which most or all of the variables are selected from the preferred or most preferred groups for each variable.

While the preferred groups for each variable have generally been listed above separately for each variable, preferred compounds of this invention include those in which several or each variable in Formula (I) is selected from the preferred, more preferred, or most preferred groups for each variable. Therefore, this invention is intended to include all combinations of preferred, more preferred, and most preferred groups.

Suitable compounds of formula (1) include:

2-[4-({[4-{[4-(4-acetylphenyl)-1-piperazinyl]methyl}-2-(4-fluorophenyl)-1,3-thiazol-5-yl]methyl}sulfanyl)-2-methylphenoxy]-2-methylpropanoic acid,
2-methyl-2-{2-methyl-4-[({4-[4-(methylsulfanyl)benzyl]-2-[4-(trifluoromethyl)phenyl]-1,3-thiazol-5-yl}methyl)sulfanyl]phenoxy}propanoic acid,
{2-methyl-4-[({4-(3-thienylmethyl)-2-[4-(trifluoromethyl)phenyl]-1,3-thiazol-5-yl}methyl)sulfanyl]phenoxy}acetic acid,
{4-[({4-benzyl-2-[4-(trifluoromethyl)phenyl]-1,3-thiazol-5-yl}methyl)sulfanyl]-2,5-dimethylphenoxy}acetic acid,
2-{4-[({4-{[4-(4-acetylphenyl)-1-piperazinyl]methyl}-2-[4-(trifluoromethyl)phenyl]-1,3-thiazol-5-yl}methyl)sulfanyl]-2-methylphenoxy}propanoic acid,
2-{4-[({4-{[4-(4-acetylphenyl)-1-piperazinyl]methyl}-2-[4-(trifluoromethyl)phenyl]-1,3-thiazol-5-yl}methyl)sulfanyl]-2-ethylphenoxy}propanoic acid,
2-{2-methyl-4-[({4-(2-thienylmethyl)-2-[4-(trifluoromethyl)phenyl]-1,3-thiazol-5-yl}methyl)sulfanyl]phenoxy}propanoic acid,

2-{4-[({4-{[4-(4-methoxyphenyl)-1-piperazinyl]methyl}-2-[4-(trifluoromethyl)phenyl]-1,3-thiazol-5-yl}methyl)sulfanyl]-2-methylphenoxy}propanoic acid,
2-{4-[({4-{[4-(4-ethoxyphenyl)-1-piperazinyl]methyl}-2-[4-(trifluoromethyl)phenyl]-1,3-thiazol-5-yl}methyl)sulfanyl]-2-methylphenoxy}propanoic acid,
2-methyl-2-{2-methyl-4-[({4-{[4-(phenoxycarbonyl)-1-piperazinyl]methyl}-2-[4-(trifluoromethyl)phenyl]-1,3-thiazol-5-yl}methyl)sulfanyl]phenoxy}propanoic acid,
2-{4-[({4-{[4-(4-acetylphenyl)-1-piperazinyl]methyl}-2-[4-(trifluoromethyl)phenyl]-1,3-thiazol-5-yl}methyl)sulfanyl]-2-propylphenoxy}propanoic acid,
{2-methyl-4-[({4-[4-(3-thienyl)benzyl]-2-[4-(trifluoromethyl)phenyl]-1,3-thiazol-5-yl}methyl)sulfanyl]phenoxy}acetic acid,
2-(4-{[(2-(4-fluorophenyl)-4-{[4-(4-methoxyphenyl)-1-piperazinyl]methyl}-1,3-thiazol-5-yl)methyl]sulfanyl}-2-methylphenoxy)-2-methylpropanoic acid,
2-{4-[({4-{[4-(4-acetylphenyl)-1-piperazinyl]methyl}-2-[4-(trifluoromethyl)phenyl]-1,3-thiazol-5-yl}methyl)sulfanyl] phenoxy}-2-methylpropanoic acid,
2-{4-[({4-{[4-(2,4-dimethoxyphenyl)-1-piperazinyl]methyl}-2-[4-(trifluoromethyl)phenyl]-1,3-thiazol-5-yl}methyl) sulfanyl]-2-methylphenoxy}propanoic acid,
{2-isopropyl-4-[({4-{[4-(4-methoxyphenyl)-1-piperazinyl]methyl}-2-[4-(trifluoromethyl)phenyl]-1,3-thiazol-5-yl}methyl)sulfanyl]phenoxy}acetic acid,
2-{4-[({4-{[4-(4-methoxyphenyl)-1-piperazinyl]methyl}-2-[4-(trifluoromethyl)phenyl]-1,3-thiazol-5-yl}methyl)sulfanyl]-2-propylphenoxy}propanoic acid,
2-{4-[({4-benzyl-2-[4-(trifluoromethyl)phenyl]-1,3-thiazol-5-yl}methyl)sulfanyl]-2-methylphenoxy}propanoic acid,
2-{2-ethyl-4-[({4-{[4-(4-methoxyphenyl)-1-piperazinyl]methyl}-2-[4-(trifluoromethyl)phenyl]-1,3-thiazol-5-yl}methyl)sulfanyl]phenoxy}propanoic acid,
2-methyl-2-{2-methyl-4-[({4-[4-(trifluoromethyl)benzyl]-2-[4-(trifluoromethyl)phenyl]-1,3-thiazol-5-yl}methyl)sulfanyl]phenoxy}propanoic acid,
2-{4-[({4-{[4-(4-fluorophenyl)-1-piperazinyl]methyl}-2-[4-(trifluoromethyl)phenyl]-1,3-thiazol-5-yl}methyl)sulfanyl]-2-methylphenoxy}-2-methylpropanoic acid,
{4-[({4-{[4-(4-acetylphenyl)-1-piperazinyl]methyl}-2-[4-(trifluoromethyl)phenyl]-1,3-thiazol-5-yl}methyl)sulfanyl]-2-propylphenoxy}acetic acid,
{4-[({4-([1,1'-biphenyl]-4-ylmethyl)-2-[4-(trifluoromethyl)phenyl]-1,3-thiazol-5-yl}methyl)sulfanyl]-2-methylphenoxy}acetic acid,
2-{4-[({4-{[4-(4-fluorophenyl)-1-piperazinyl]methyl}-2-[4-(trifluoromethyl)phenyl]-1,3-thiazol-5-yl}methyl)sulfanyl]-2-methylphenoxy}propanoic acid,
{4-[({4-{[4-(3-methoxyphenyl)-1-piperazinyl]methyl}-2-[4-(trifluoromethyl)phenyl]-1,3-thiazol-5-yl}methyl)sulfanyl]-2-methylphenoxy}acetic acid,
2-{2-methyl-4-[({4-(3-thienylmethyl)-2-[4-(trifluoromethyl)phenyl]-1,3-thiazol-5-yl}methyl)sulfanyl]phenoxy}propanoic acid,
{4-[({4-{[4-(2-methoxyphenyl)-1-piperazinyl]methyl}-2-[4-(trifluoromethyl)phenyl]-1,3-thiazol-5-yl}methyl)sulfanyl]-2-methylphenoxy}acetic acid,
2-{2-isopropyl-4-[({4-{[4-(4-methoxyphenyl)-1-piperazinyl]methyl}-2-[4-(trifluoromethyl)phenyl]-1,3-thiazol-5-yl} methyl)sulfanyl]phenoxy}propanoic acid,
2-{4-[({4-(4-tert-butylbenzyl)-2-[4-(trifluoromethyl)phenyl]-1,3-thiazol-5-yl}methyl)sulfanyl]-2-methylphenoxy}-2-methylpropanoic acid,
2-{4-[({4-{[4-(3-methoxyphenyl)-1-piperazinyl]methyl}-2-[4-(trifluoromethyl)phenyl]-1,3-thiazol-5-yl}methyl)sulfanyl]-2-methylphenoxy}propanoic acid,
2-{4-[({4-benzyl-2-[4-(trifluoromethyl)phenyl]-1,3-thiazol-5-yl}methyl)sulfanyl]-2,3-dimethylphenoxy}propanoic acid,
2-{4-[({4-{[4-(4-chlorophenyl)-1-piperazinyl]methyl}-2-[4-(trifluoromethyl)phenyl]-1,3-thiazol-5-yl}methyl)sulfanyl]-2-methylphenoxy}propanoic acid,
2-{4-[({4-benzyl-2-[4-(trifluoromethyl)phenyl]-1,3-thiazol-5-yl}methyl)sulfanyl]-2-fluorophenoxy}propanoic acid,
2-{4-[({4-{[4-(2,4-difluorophenyl)-1-piperazinyl]methyl}-2-[4-(trifluoromethyl)phenyl]-1,3-thiazol-5-yl}methyl)sulfanyl]-2-methylphenoxy}propanoic acid,
{4-[({4-(2,4-difluorobenzyl)-2-[4-(trifluoromethyl)phenyl]-1,3-thiazol-5-yl}methyl)sulfanyl]-2-methylphenoxy}acetic acid,
2-{4-[({4-{[4-(4-acetylphenyl)-1-piperazinyl]methyl}-2-[4-(trifluoromethyl)phenyl]-1,3-thiazol-5-yl}methyl)sulfanyl]-2-methylphenoxy}-2-methylpropanoic acid,
2-methyl-2-{2-methyl-4-[({4-(3-thienylmethyl)-2-[4-(trifluoromethyl)phenyl]-1,3-thiazol-5-yl}methyl)sulfanyl]phenoxy}propanoic acid,

2-{4-[({4-{[4-(4-methoxyphenyl)-1-piperazinyl]methyl}-2-[4-(trifluoromethyl)phenyl]-1,3-thiazol-5-yl}methyl)sulfanyl]-2-methylphenoxy}propanoic acid,
{2-ethyl-4-[({4-{[4-(4-methoxyphenyl)-1-piperazinyl]methyl}-2-[4-(trifluoromethyl)phenyl]-1,3-thiazol-5-yl}methyl) sulfanyl]phenoxy}acetic acid,
2-{4-[({4-(4-methoxybenzyl)-2-[4-(trifluoromethyl)phenyl]-1,3-thiazol-5-yl}methyl)sulfanyl]-2-methylphenoxy}-2-methylpropanoic acid,
2-methyl-2-{4-[({4-{[4-(2-pyrazinyl)-1-piperazinyl]methyl}-2-[4-(trifluoromethyl)phenyl]-1,3-thiazol-5-yl}methyl)sulfanyl]phenoxy}propanoic acid,
2-{4-[({4-{[4-(4-methoxyphenyl)-1-piperazinyl]methyl}-2-[4-(trifluoromethyl)phenyl]-1,3-thiazol-5-yl}methyl)sulfanyl]-2-methylphenoxy}-2-methylpropanoic acid,
2-{4-[({4-{[4-(4-methoxyphenyl)-1-piperazinyl]methyl}-2-[4-(trifluoromethyl)phenyl]-1,3-thiazol-5-yl}methyl)sulfanyl]phenoxy}-2-methylpropanoic acid,
2-methyl-2-{2-methyl-4-[({4-[4-(trifluoromethoxy)benzyl]-2-[4-(trifluoromethyl)phenyl]-1,3-thiazol-5-yl}methyl)sulfanyl]phenoxy}propanoic acid,
2-{4-[({4-{[4-(4-isopropoxyphenyl)-1-piperazinyl]methyl}-2-[4-(trifluoromethyl)phenyl]-1,3-thiazol-5-yl}methyl)sulfanyl]-2-methylphenoxy}propanoic acid,
2-{2-methyl-4-[({4-{[4-(2-pyrimidinyl)-1-piperazinyl]methyl}-2-[4-(trifluoromethyl)phenyl]-1,3-thiazol-5-yl}methyl) sulfanyl]phenoxy}propanoic acid,
{2-methyl-4-[({4-(3-phenylpropyl)-2-[4-(trifluoromethyl)phenyl]-1,3-thiazol-5-yl}methyl)sulfanyl]phenoxy}acetic acid,
[4-[({4-benzyl-2-[4-(trifluoromethyl)phenyl]-1,3-thiazol-5-yl}methyl)sulfanyl]-2-(trifluoromethyl)phenoxy]acetic acid,
{2-methyl-4-[({4-{[4-(5-methyl-1,2,4-oxadiazol-3-yl)phenoxy]methyl}-2-[4-(trifluoromethyl)phenyl]-1,3-thiazol-5-yl} methyl)sulfanyl]phenoxy}acetic acid,
{4-[({4-benzyl-2-[4-(trifluoromethyl)phenyl]-1,3-thiazol-5-yl}methyl)sulfanyl]-5-chloro-2-methylphenoxy}acetic acid,
{4-[({4-benzyl-2-[4-(trifluoromethyl)phenyl]-1,3-thiazol-5-yl}methyl)sulfanyl]-2-methylphenoxy}acetic acid,
{4-[({4-(4-methoxybenzyl)-2-[4-(trifluoromethyl)phenyl]-1,3-thiazol-5-yl}methyl)sulfanyl]-2-methylphenoxy}acetic acid,
{2,5-dimethyl-4-[({4-(3-thienylmethyl)-2-[4-(trifluoromethyl)phenyl]-1,3-thiazol-5-yl}methyl)sulfanyl]phenoxy}acetic acid,
{2-methyl-4-[({4-{[4-(2-pyrazinyl)-1-piperazinyl]methyl}-2-[4-(trifluoromethyl)phenyl]-1,3-thiazol-5-yl}methyl)sulfanyl]phenoxy}acetic acid,
{4-[({4-benzyl-2-[4-(trifluoromethyl)phenyl]-1,3-thiazol-5-yl}methyl)sulfanyl]-2,3-dimethylphenoxy}acetic acid,
[4-({[2-(4-chlorophenyl)-4-methyl-1,3-thiazol-5-yl]methyl}sulfanyl)-2-methylphenoxy]acetic acid,
{2-methyl-4-[({4-[(4-methyl-2-thienyl)methyl]-2-[4-(trifluoromethyl)phenyl]-1,3-thiazol-5-yl}methyl)sulfanyl]phenoxy}acetic acid,
{4-[({4-benzyl-2-[4-(trifluoromethyl)phenyl]-1,3-thiazol-5-yl}methyl)sulfanyl]-2-bromophenoxy}acetic acid,
{2-methyl-4-[({4-[(2-phenylethoxy)methyl]-2-[4-(trifluoromethyl)phenyl]-1,3-thiazol-5-yl}methyl)sulfanyl]phenoxy} acetic acid,
{2-methyl-4-[({4-(2-phenylethyl)-2-[4-(trifluoromethyl)phenyl]-1,3-thiazol-5-yl}methyl)sulfanyl]phenoxy}acetic acid, and

pharmaceutically acceptable salts, solvates, and hydrolyzable esters thereof.

More preferred compounds of formula (1) include:

2-methyl-2-{2-methyl-4-[({4-(3-thienylmethyl)-2-[4-(trifluoromethyl)phenyl]-1,3-thiazol-5-yl}methyl)sulfanyl]phenoxy}propanoic acid,
2-{4-[({4-{[4-(4-methoxyphenyl)-1-piperazinyl]methyl}-2-[4-(trifluoromethyl)phenyl]-1,3-thiazol-5-yl}methyl)sulfanyl]-2-methylphenoxy}propanoic acid,
{2-ethyl-4-[({4-{[4-(4-methoxyphenyl)-1-piperazinyl]methyl}-2-[4-(trifluoromethyl)phenyl]-1,3-thiazol-5-yl}methyl) sulfanyl]phenoxy}acetic acid,
2-{4-[({4-(4-methoxybenzyl)-2-[4-(trifluoromethyl)phenyl]-1,3-thiazol-5-yl}methyl)sulfanyl]-2-methylphenoxy}-2-methylpropanoic acid,
2-methyl-2-{4-[({4-{[4-(2-pyrazinyl)-1-piperazinyl]methyl}-2-[4-(trifluoromethyl)phenyl]-1,3-thiazol-5-yl}methyl)sulfanyl]phenoxy}propanoic acid,
2-{4-[({4-{[4-(4-methoxyphenyl)-1-piperazinyl]methyl}-2-[4-(trifluoromethyl)phenyl]-1,3-thiazol-5-yl}methyl)sulfanyl]-2-methylphenoxy}-2-methylpropanoic acid,

2-{4-[({4-{[4-(4-methoxyphenyl)-1-piperazinyl]methyl}-2-[4-(trifluoromethyl)phenyl]-1,3-thiazol-5-yl}methyl)sulfanyl]phenoxy}-2-methylpropanoic acid,
2-methyl-2-{2-methyl-4-[({4-[4-(trifluoromethoxy)benzyl]-2-[4-(trifluoromethyl)phenyl]-1,3-thiazol-5-yl}methyl)sulfanyl]phenoxy}propanoic acid,
2-{4-[({4-{[4-(4-isopropoxyphenyl)-1-piperazinyl]methyl}-2-[4-(trifluoromethyl)phenyl]-1,3-thiazol-5-yl}methyl)sulfanyl]-2-methylphenoxy}propanoic acid,
2-{2-methyl-4-[({4-{[4-(2-pyrimidinyl)-1-piperazinyl]methyl}-2-[4-(trifluoromethyl)phenyl]-1,3-thiazol-5-yl}methyl)sulfanyl]phenoxy}propanoic acid, and

pharmaceutically acceptable salts, solvates, and hydrolyzable esters thereof.

Preferably, the compounds of formula (I) are hPPAR agonists. The hPPAR agonists of formula (I) may be agonists of only one type ("selective agonists"), agonists for two PPAR subtypes ("dual agonists"), or agonists for all three subtypes ("pan agonists"). As used herein, by "agonist", or "activating compound", or "activator", or the like, is meant those compounds which have a pKi of at least 5.0 preferably at least 6.0 to the relevant PPAR, for example hPPARR□ in the binding assay described below, and which achieve at least 30% activation of the relevant PPAR relative to the appropriate indicated positive control in the transfection assay described below at concentrations of $10^{-5}$ M or less. More preferably, the compounds of this invention achieve 30% activation of at least one human PPAR in the relevant transfection assay at concentrations of $10^{-6}$ M or less. More preferably the compounds of the invention achieve 30% activation of at least one human PPAR in the relevant transfection assay at concentrations of $10^{-7}$M or less.

Preferably the compounds of formula (1) are hPPARδ agonists. More preferably they are also agonists of at least one of PPARγ or PPARα. Most preferably they are pan hPPAR agonists.

It will also be appreciated by those skilled in the art that the compounds of the present invention may also be utilized in the form of a pharmaceutically acceptable salt or solvate thereof. The physiologically acceptable salts of the compounds of formula (I) include conventional salts formed from pharmaceutically acceptable inorganic or organic acids or bases as well as quaternary ammonium acid addition salts. More specific examples of suitable acid salts include hydrochloric, hydrobromic, sulfuric, phosphoric, nitric, perchloric, fumaric, acetic, propionic, succinic, glycolic, formic, lactic, maleic, tartaric, citric, patmoic, malonic, hydroxymaleic, phenylacetic, glutamic, benzoic, salicylic, fumaric, toluenesulfonic, methanesulfonic, naphthalene-2-sulfonic, benzenesulfonic hydroxynaphthoic, hydroiodic, malic, steroic, tannic and the like. Other acids such as oxalic, while not in themselves pharmaceutically acceptable, may be useful in the preparation of salts useful as intermediates in obtaining the compounds of the invention and their pharmaceutically acceptable salts. More specific examples of suitable basic salts include sodium, lithium, potassium, magnesium, aluminium, calcium, zinc, N,N'-dibenzylethylenediamine, chloroprocaine, choline, diethanolamine, ethylenediamine, N-methylglucamine and procaine salts. Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compound of formula (I) are within the scope of the invention. References hereinafter to a compound according to the invention include both compounds of formula (I) and their pharmaceutically acceptable salts and solvates.

The compounds of the invention and their pharmaceutically acceptable derivatives are conveniently administered in the form of pharmaceutical compositions. Such compositions may conveniently be presented for use in conventional manner in admixture with one or more physiologically acceptable carriers or excipients.

While it is possible that compounds of the present invention may be therapeutically administered as the raw chemical, it is preferable to present the active ingredient as a pharmaceutical formulation. The carrier(s) must be "acceptable" in the sense of being compatible with the other ingredients of the formulation and not deleterious to the recipient thereof.

Accordingly, the present invention further provides for a pharmaceutical formulation comprising a compound of formula (I) or a pharmaceutically acceptable salt or solvate thereof together with one or more pharmaceutically acceptable carriers therefore and, optionally, other therapeutic and/or prophylactic ingredients.

The formulations include those suitable for oral, parenteral (including subcutaneous e.g. by injection or by depot tablet, intradermal, intrathecal, intramuscular e.g. by depot and intravenous), rectal and topical (including dermal, buccal and sublingual) administration although the most suitable route may depend upon for example the condition and disorder of the recipient. The formulations may conveniently be presented in unit dosage form and may be prepared by any of the methods well known in the art of pharmacy. All methods include the step of bringing into association the compounds ("active ingredient") with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation.

Formulations suitable for oral administration may be presented as discrete units such as capsules, cachets or tablets (e.g. chewable tablets in particular for paediatric administration) each containing a predetermined amount

of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with other conventional excipients such as binding agents, (for example, syrup, acacia, gelatin, sorbitol, tragacanth, mucilage of starch or polyvinylpyrrolidone), fillers (for example, lactose, sugar, microcrystalline cellulose, maize-starch, calcium phosphate or sorbitol), lubricants (for example, magnesium stearate, stearic acid, talc, polyethylene glycol or silica), disintegrants (for example, potato starch or sodium starch glycollate) or wetting agents, such as sodium lauryl sulfate. Moulded tablets may be made by moulding in a suitable machine a mixture of the powdered compound moistened with an inert liquid diluent. The tablets may optionally be coated or scored and may be formulated so as to provide slow or controlled release of the active ingredient therein. The tablets may be coated according to methods well-known in the art.

Alternatively, the compounds of the present invention may be incorporated into oral liquid preparations such as aqueous or oily suspensions, solutions, emulsions, syrups or elixirs, for example. Moreover, formulations containing these compounds may be presented as a dry product for constitution with water or other suitable vehicle before use. Such liquid preparations may contain conventional additives such as suspending agents such as sorbitol syrup, methyl cellulose, glucose/sugar syrup, gelatin, hydroxyethylcellulose, carboxymethyl cellulose, aluminum stearate gel or hydrogenated edible fats; emulsifying agents such as lecithin, sorbitan mono-oleate or acacia; non-aqueous vehicles (which may include edible oils) such as almond oil, fractionated coconut oil, oily esters, propylene glycol or ethyl alcohol; and preservatives such as methyl or propyl p-hydroxybenzoates or sorbic acid. Such preparations may also be formulated as suppositories, e.g., containing conventional suppository bases such as cocoa butter or other glycerides.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The formulations may be presented in unit-dose or multi-dose containers, for example sealed ampoules and vials, and may be stored in a freeze-dried (lyophilised) condition requiring only the addition of a sterile liquid carrier, for example, water-for-injection, immediately prior to use. Extemporaneous injection solutions and suspensions may be prepared from sterile powders, granules and tablets of the kind previously described.

Formulations for rectal administration may be presented as a suppository with the usual carriers such as cocoa butter, hard fat or polyethylene glycol.

Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerin or sucrose and acacia.

The compounds may also be formulated as depot preparations. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

In addition to the ingredients particularly mentioned above, the formulations may include other agents conventional in the art having regard to the type of formulation in question, for example those suitable for oral administration may include flavouring agents.

It will be appreciated by those skilled in the art that reference herein to treatment extends to prophylaxis as well as the treatment of established diseases or symptoms. Moreover, it will be appreciated that the amount of a compound of the invention required for use in treatment will vary with the nature of the condition being treated and the age and the condition of the patient and will be ultimately at the discretion of the attendant physician or veterinarian. In general, however, doses employed for adult human treatment will typically be in the range of 0.02-5000 mg per day, preferably 1-1500 mg per day. The desired dose may conveniently be presented in a single dose or as divided doses administered at appropriate intervals, for example as two, three, four or more sub-doses per day. The formulations according to the invention may contain between 0.1-99% of the active ingredient, conveniently from 30-95% for tablets and capsules and 3-50% for liquid preparations.

The compound of formula (I) for use in the instant invention may be used in combination with other therapeutic agents for example, statins and/or other lipid lowering drugs for example MTP inhibitors and LDLR upregulators. The compounds of the invention may also be used in combination with antidiabetic agents, e.g. metformin, sulfonylureas and/or PPAR gamma, PPAR alpha or PPAR alpha/gamma agonists (for example thiazolidinediones such as e.g. Pioglitazone and Rosiglitazone). The compounds may also be used in combination with antihypertensive agents such as angistensin antagonists e.g. telmisartan, calcium channel antagonists e.g. lacidipine and ACE inhibitors e.g. enalapril. The invention thus provides in a further aspect the use of a combination comprising a compound of formula (I) with a further therapeutic agent in the treatment of a hPPAR mediated disease.

When the compounds of formula (I) are used in combination with other therapeutic agents, the compounds may be administered either sequentially or simultaneously by any convenient route.

The combinations referred to above may conveniently be presented for use in the form of a pharmaceutical formulation and thus pharmaceutical formulations comprising a combination as defined above optimally together with a pharmaceutically acceptable carrier or excipient comprise a further aspect of the invention. The individual components of such combinations may be administered either sequentially or simultaneously in separate or combined pharmaceutical formulations.

When combined in the same formulation it will be appreciated that the two compounds must be stable and compatible with each other and the other components of the formulation and may be formulated for administration. When formulated separately they may be provided in any convenient formulation, conveniently in such a manner as are known for such compounds in the art.

When a compound of formula (I) is used in combination with a second therapeutic agent active against the same hPPAR mediated disease, the dose of each compound may differ from that when the compound is used alone. Appropriate doses will be readily appreciated by those skilled in the art.

There is further provided processes for the preparation of compounds of 1. Unless otherwise indicated all definitions are as above.

In general when $X^2$ is O or S the compounds could be assembled by coupling through an alkylation step such as that shown below.

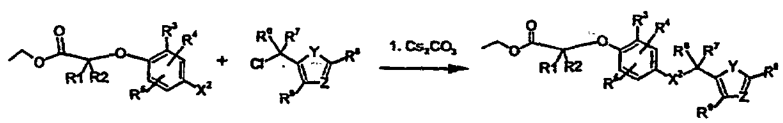


The esters are commercially available or made by the following general route when $X^2$ is S.

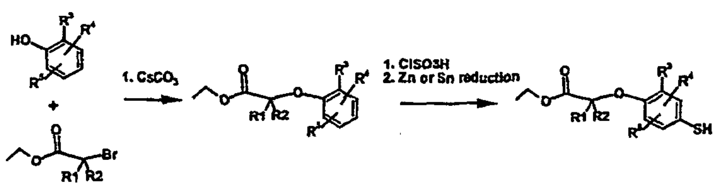


The heterocycle when Y is O or S and Z is N was generally made as shown below from an appropriate amide or thioamide:

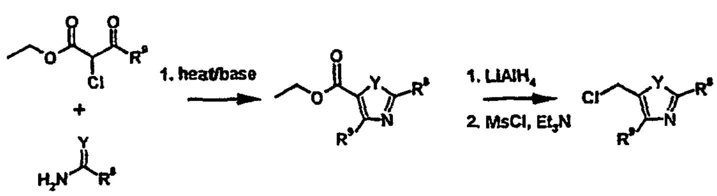


In specific cases the overall coupling step could be carried out directly after chlorosulfonation of the ester component without the need for formation of the chloride of the heterocyclic moiety, as shown below:

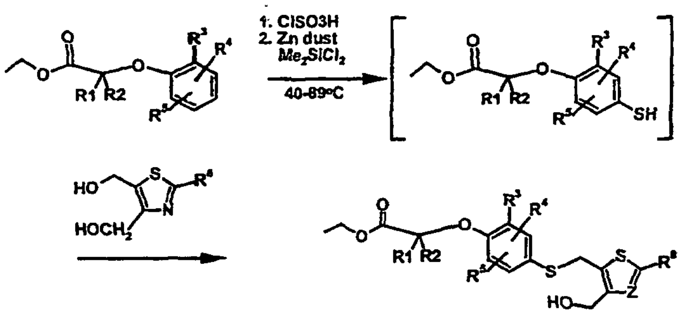


In some cases $R^9$ was further elaborated through palladium coupling at the ester stage as shown below:

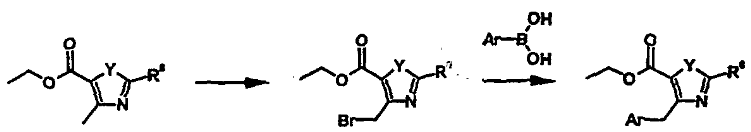

Alternatively $R^9$ was elaborated after the coupling reaction by nucleophilic displacement of a mesylate shown below:

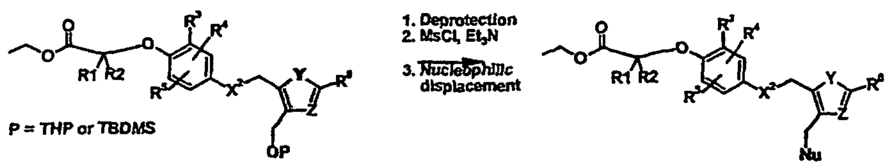


## Examples

The invention is further illustrated by the following Examples which should not be construed as constituting a limitation thereto.

**Ethyl 4-(bromomethyl)-2-[4-(trifluoromethyl)phenyl]-1,3-thiazole-5-carboxylate**

To a 2-L round-bottom flask equipped with an mechanical overhead stirrer, a reflux condenser and a $N_2$ inlet was added ethyl 4-methyl-2-[4-(trifluoromethyl)phenyl]-1,3-thiazole-5-carboxylate (85g, 0.27moles, 1.0eq) and dry carbon tetrachloride (750ml, 0.38M). Freshly recrystallized N-bromo succinimide (52.72g, 1.1 eq) was added as a solid, Benzoyl peroxide (6.5g, 10mol%) was added at room temperature all at once as a solid, and the reaction mixture was refluxed for 5 hrs. The reaction was monitored by $^1H$ NMR and was determined to be composed of a 9:1 mixture of mono-bromination product (i.e. desired product) and di-bromination product with a 90% conversion. After cooling to 0°C (to precipitate out the succinimide) the reaction was filtered through Celite and the solvent was removed under reduced pressure to yield a brown oil. The oil was crystallized using hexanes to yield 100g (94%) of an off-white product of 90% purity.

$^1H$ NMR ($CDCl_3$) 400MHz δ 8.10(d, 2H, J=8.20 Hz), 7.72(d, 2H, J=8.20 Hz), 4.99(s, 2H), 4.40(q, 2H, J=7.18 Hz), 1.41(t, 3H, J=7.18 Hz),

TLC(15% EtOAc/Hexanes) $R_f$ = 0.55

**Ethyl 4-(bromomethyl)-2-phenyl-1,3-thiazole-5-carboxylate**

The title compound was made using the same procedure as above.

$^1H$ NMR ($CDCl_3$) 400MHz δ 7.98(dd, 2H, J=7.86, 1.54 Hz), 7.47(m, 3H), 4.99(s, 2H), 4.39(q, 2H, J=7.12 Hz), 1.40(t, 3H, J=7.12 Hz),

TLC(15% EtOAc/Hexanes) $R_f$ = 0.50

**Ethyl 4-(hydroxymethyl)-2-[4-(trifluoromethyl)phenyl]-1,3-thiazole-5-carboxylate**

To a stirred solution of ethyl 4-(bromomethyl)-2-[4-(trifluoromethyl)phenyl]-1,3-thiazole-5-carboxylate (50g, 0.127moles, 1 eq) in dry DMF (300ml) under a positive $N_2$ flow was added silver trifluoroacetate (42.02g, 0.191moles, 1.5eq) all at once as a solid. This was stirred at room temperature for 3.5 hrs. The reaction was partitioned between ethyl ether (1.5L) and water (500ml). The phases were separated and the organic phase was washed twice with water (500ml). After separation of the phases, the organic fraction was dried with $Na_2SO_4$, filtered and concentrated *in vacuo*. The crude trifluoroacetate product was used without characterization. Ethanol (300ml) was added and the reaction was refluxed for 10 hrs. After cooling to room temperature the ethanol was removed *in vacuo* to yield 42g (100%) of the title compound. The product was used without purification.

$^1$H NMR ($CDCl_3$) 400MHz δ 8.09(d, 2H, J=8.20 Hz), 7.73(d, 2H, J=8.20 Hz), 5.09(s, 2H), 4.41(q, 2H, J=7.12 Hz), 1.40(t, 3H, J=7.12 Hz),

**Ethyl 4-(hydroxymethyl)-2-phenyl-1,3-thiazole-5-carboxylate**

The title compound was made using the same procedure as above.

$^1$H NMR ($CDCl_3$) 400MHz δ 7.95(m, 2H), 7.48(m, 3H), 5.09(s, 2H), 4.40(q, 2H, J=7.12 Hz), 1.41(t, 3H, J=7.12 Hz),

**Ethyl 4-[(tetrahydro-2H-pyran-2-yloxy)methyl]-2-[4-(trifluoromethyl)phenyl]-1,3-thiazole-5-carboxylate**

To a 1-L round-bottom flask equipped with a magnetic stir-bar and a $N_2$ inlet was added Ethyl 4-(hydroxymethyl)-2-[4-(trifluoromethyl)phenyl]-1,3-thiazole-5-carboxylate (42g, 0.127moles, 1eq) and dry $CH_2Cl_2$ (300ml) at room temperature. This was followed by the addition of 3,4-dihydro-2*H*-pyran (14ml, 0.152moles, 1.2eq) as a neat liquid and pyridinium *p*-toluenesulfonate (6.4g, 25.4mmoles, 20mol%). The reaction mixture was stirred at room temperature overnight (10 hrs). The volatiles were then removed *in vacuo* and the residue was purified by flash silica gel chromatography (10% EtOAc/Hexanes to 30% EtOAc/Hexanes) to yield 34g (64%) of pure title compound.

$^1$H NMR ($CDCl_3$) 400MHz δ 8.09(d, 2H, J=8.20 Hz), 7.69(d, 2H, J=8.20 Hz), 5.18(d, 1 H, J□.30 Hz), 4.99(d, 1H, J .30 Hz), 4.90(t, 1H, J=3.42 Hz), 4.36(q, 2H, J=7.12 Hz), 3.98(m, 1H), 3.56(m, 1H), 1.69(m, 6H), 1.37(t, 3H, J=7.12 Hz),

TLC(30% EtOAc/Hexanes)= 0.64

**Ethyl 2-phenyl-4-[(tetrahydro-2H-pyran-2-yloxy)methyl]-1,3-thiazole-5-carboxylate**

$^1$H NMR ($CDCl_3$) 400MHz δ 7.97(m, 2H), 7.43(m, 3H), 5.17(d, 1H, J 13 Hz), 4.98(d, 1H, J 13 Hz), 4.91 (t, 1H, J=3.33 Hz), 4.35(q, 2H, J=7.12 Hz), 3.98(m, 1H), 3.54(m, 1 H), 1.69(m, 6H), 1.36(t, 3H, J=7.12 Hz),

**Ethyl 2-(4-fluorophenyl)-4-[(tetrahydro-2H-pyran-2-yloxy)methyl]-1,3-thiazole-5-carboxylate**

$^1$H Nz δ 7.97(m, 2H), 7.11 (m, 2H), 5.16(d, 1H, J .24 Hz), 4.97(d, 1H, J .24 Hz), 4.90(t, 1H, J=3.36 Hz), 4.34 (q, 2H, J=7.13 Hz), 3.98(m, 1H), 3.55(m, 1H), 1.86(m, 2H), 1.70(m, 2H), 1.55(m, 2H), 1.36(t, 3H, J=7.13 Hz),

**Suzuki Coupling**

**Ethyl 4-[4-(trifluoromethyl)benzyl]-2-[4-(trifluoromethyl)phenyl]-1,3-thiazole-5-carboxylate**

To a solution of ethyl 4-(bromomethyl)-2-[4-(trifluoromethyl)phenyl]-1,3-thiazole-5-carboxylate (0.25g, 0.63 mmol) in 4 ml of 2-methoxyethyl ether was added tetrakis(triphenylphosphine)palladium(0), (0.02g, 0.019 mmol) and then sodium carbonate (0.13g, 1.2 mmol) in 0.5 ml water. After brief stirring, 4-(trifluoromethyl)phenyl boronic acid (0.13g, 0.7 mmol) in 1 ml ethanol was added. After heating at 110°C for 15 hours, the reaction was complete by HPLC and was treated with water (5 ml) and extracted with *tert*-butyl methyl ether (2 x 30ml). The organic layers were dried with magnesium sulfate and immediately loaded onto silica to give a crude residue which was purified on a Biotage FlashElute with a 40M silica cartridge, eluting with 10% ethyl acetate in hexanes to yield ethyl 4-[4-(trifluoromethyl) benzyl]-2-[4-(trifluoromethyl)phenyl]-1,3-thiazole-5-carboxylate as a white solid (0.09g, 35%).

$^1$H NMR ($CDCl_3$): δ 8.18 (d, 2 H), 7.78 (d, 2 H), 7.58 (m, 4 H), 4.68 (s, 2 H), 4.40 (q, 2 H), 1.40 (t, 3 H); MS *m*/*z* 460 (M+1).

The following compounds were made using the the same palladium catalyzed coupling procedure using the appropriate boronic acid.

**Ethyl 4-[4-(trifluoromethoxy)benzyl]-2-[4-(trifluoromethyl)phenyl]-1,3-thiazote-5-carboxylate**

From ethyl 4-(bromomethyl)-2-[4-(trifluoromethyl)phenyl]-1,3-thiazole-5-carboxylate (0.25g, 0.63 mmol), ethyl 4-[4-(trifluoromethoxy)benzyl]-2-[4-(trifluoromethyl)phenyl]-1,3-thiazole-5-carboxylate (0.12g, 43%) was obtained as a light yellow solid.

$^1$H NMR ($CDCl_3$): δ 8.18 (d, 2 H), 7.77 (d, 2 H), 7.46 (d, 2 H), 7.18 (d, 2 H), 4.60 (s, 2 H) 4.40 (q, 2 H), 1.40 (t, 3 H); MS m/z 476 (M+1).

**Ethyl 4-[4-methoxybenzyl]-2-[4-(trifluoromethyl)phenyl]-1,3-thiazole-5-carboxylate**

From ethyl 4-(bromomethyl)-2-[4-(trifluoromethyl)phenyl]-1,3-thiazole-5-carboxylate (0.25g, 0.63 mmol), ethyl 4-[4-methoxybenzyl]-2-[4-(trifluoromethyl)phenyl]-1,3-thiazole-5-carboxylate (0.16g, 63%) was obtained as a yellow semi-solid.

$^1$H NMR ($CDCl_3$): δ 8.18 (d, 2H), 7.70 (d, 2 H), 7.40 (d, 2H), 6.80 (d, 2 N), 4.57 (s, 2 H), 4.40 (q, 2 H), 3.80 (s, 3 H), 1.40 (t, 3 H); MS *m*/*z* 422 (M+1).

**Ethyl 4-[4-(methylsulfanyl)benzyl]-2-[4-(trifluoromethyl)phenyl]-1,3-thiazole-5-carboxylate**

From ethyl 4-(bromomethyl)-2-[4-(trifluoromethyl)phenyl]-1,3-thiazole-5-carboxylate (0.4g, 1.01 mmol), ethy) 4-[4-(methylsulfanyl)benzyl]-2-[4-(trifluoromethyl)phenyl]-1,3-thiazole-5-carboxylate (0.44g, 100%) was obtained as a light yellow solid.

$^1$H NMR ($CDCl_3$): δ 8.11 (d, 2 H), 7.71 (d, 2 H), 7.38 (d, 2 H), 7.21 (d, 2 H). 4.52 (s, 2 H). 4.38 (q, 2 H), 2.49 (s, 3 H), 1.40 (t, 3 H); MS *m*/*z* 438 (M+1).

**Ethyl 4-[4-*tert*-butylbenzyl]-2-[4-(trifluoromethyl)phenyl]-1,3-thiazole-5-carboxylate**

From ethyl 4-(bromomethyl)-2-[4-(trifluoromethyl)phenyl]-1,3-thiazole-5-carboxylate (0.4g, 1.01 mmol), ethyl 4-[4-*tert*-butylbenzyl]-2-[4-(trifluoromethyl)phenyl]-1,3-thiazole-5-carboxylate (0.24g, 54%) was obtained as a white solid.

$^1$H NMR ($CDCl_3$): δ 8.11 (d, 2 H), 7.73 (d, 2 H), 7.56 (d, 1 H), 7.49 (d, 1 H), 7.34 (m, 2 H), 4.58 (s, 2 H), 4.40 (q, 2 H), 1.40 (t, 3 H), 1.27 (s, 9 H); MS *m*/*z* 448 (M+1).

**Ethyl 4-[3-thienylmethyl]-2-[4-(trifluoromethyl)phenyl]-1,3-thiazole-5-carboxylate**

From ethyl 4-(bromomethyl)-2-[4-(trifluoromethyl)phenyl]-1,3-thiazole-5-carboxylate (0.4g, 1.01 mmol), ethyl 4-[3-thienylmethyl]-2-[4-(trifluoromethyl)phenyl]-1,3-thiazole-5-carboxylate (0.4g 100%) was obtained as a yellow solid.

$^1$H NMR ($CDCl_3$): δ 8.12 (d, 2 H), 7.77 (d, 2 H), 7.40 (d, 1 H), 7.28 (d, 1 H), 7.20 (s, 1 H), 4.61 (s, 2 H), 4.41 (q, 2 H), 1.40 (t, 3 H); MS *m*/*z* 398 (M+1).

**Ethyl 4-[2-furylmethyl]-2-[4-(trifluoromethyl)phenyl]-1,3-thiazole-5-carboxylate**

From ethyl 4-(bromomethyl)-2-[4-(trifluoromethyl)phenyl]-1,3-thiazole-5-carboxylate (0.4g, 1.01 mmol), ethyl 4-[2-furylmethyl]-2-[4-(tnfluoromethyl)phenyl]-1,3-thiazole-5-carboxylate (0.204g, 53%) was obtained as a white solid.

MS *m*/*z* 382 (M+1); HPLC RT 4.072 (C18 4.2x100mm, 0-100% ACN/$H_2O$ (0.1% TFA), 6min @ 2ml/min @254/220nm).

**Ethyl 4-[3-furylmethyl]-2-[4-(trifluoromethyl)phenyl]-1,3-thiazole-5-carboxylate**

From ethyl 4-(bromomethyl)-2-[4-(trifluoromethyl)phenyl]-1,3-thiazole-5-carboxylate (0.4g, 1.01 mmol), ethyl 4-[3-furylmethyl]-2-[4-(trifluoromethyl)phenyl]-1,3-thiazole-5-carboxylate (0.217g, 56%) was obtained as a white solid.

MS *m*/*z* 382 (M+1); HPLC RT 4.091 (C18 4.2x100mm, 0-100% ACN/$H_2O$ (0.1% TFA), 6min @ 2ml/min @254/220nm).

## Ethyl 4-[2-thienylmethyl]-2-[4-(trifluoromethyl)phenyl]-1,3-thiazole-5-carboxylate

From ethyl 4-(bromomethyl)-2-[4-(trifluoromethyl)phenyl]-1,3-thiazole-5-carboxylate (0.4g, 1.01 mmol), ethyl 4-[2-thienylmethyl]-2-[4-(trifluoromethyl)phenyl]-1,3-thiazole-5-carboxylate (0.248g, 62%) was obtained as a yellow solid.

MS *m/z* 398 (M+1); HPLC RT 4.224 (C18 4.2x100mm, 0-100% ACN/$H_2O$ (0.1% TFA), 6min @ 2ml/min @254/220nm).

## Ethyl 4-[(4-methyl-2-thienyl)methyl]-2-[4-(trifluoromethyl)phenyl]-1,3-thiazole-5-carboxylate

From ethyl 4-(bromomethyl)-2-[4-(trifluoromethyl)phenyl]-1,3-thiazole-5-carboxylate (0.6g, 1.52 mmol), ethyl 4-[(4-methyl-2-thienyl)methyl]-2-[4-(trifluoromethyl)phenyl]-1,3-thiazole-5-carboxylate (0.5g, 81 %) was obtained as a yellow solid.

MS *m/z* 412 (M+1); HPLC RT 4.682 (C18 4.2x100mm, 0-100% ACN/$H_2O$ (0.1 % TFA), 6min @ 2ml/min @254/220nm).

## Ethyl 4-[2,4-difluorobenzyl]-2-[4-(trifluoromethyl)phenyl]-1,3-thiazole-5-carboxylate

From ethyl 4-(bromomethyl)-2-[4-(trifluoromethyl)phenyl]-1,3-thiazole-5-carboxylate (0.6g, 1.52 mmol), ethyl 4-[2,4-difluorobenzyl]-2-[4-(trifluoromethyl)phenyl]-1,3-thiazole-5-carboxylate (0.222g, 35%) was obtained as a white solid.

MS *m/z* 428 (M+1); HPLC RT 4.618 (C18 4.2x100mm, 0-100% ACN/$H_2O$ (0.1% TFA), 6min @ 2ml/min @254/220nm).

## 4-[(Tetrahydro-2H-pyran-2-yloxy)methyl]-2-[4-(trifluoromethyl)phenyl]-1,3-thiazol-5-yl}methanol

To a stirred solution of lithium aluminum hydride (95%, 3.3g, 81.84mmoles, 1eq) in dry ethyl ether (300ml) at 0°C was added ethyl 4-[(tetrahydro-2*H*-pyran-2-yloxy)methyl]-2-[4-(trifluoromethyl)phenyl]-1,3-thiazole-5-carboxylate (34g, 81.84mmoles, 1eq) in dry ethyl ether (50ml) dropwise via an addition funnel maintaining the internal reaction temperature below 5°C. This was stirred at 0°C for 1hr. At 0°C 3.5ml water was added dropwise very carefully and was then allowed to warm to room temperature. This was followed by the addition 3.5ml 5N NaOH and 10ml water. The mixture was stirred at room temperature for 2hrs. At this point a fine white precipitate formed. The reaction was filtered through Celite and the resulting aluminum salts were washed with 500ml EtOAc. The ether/EtOAc solution was concentrated *in vacuo* to 30.6g (100%) of titled alcohol.

$^1H$ NMR ($CDCl_3$) 400MHz δ 8.07(d, 2H, J=8.20 Hz), 7.72(d, 2H, J=8.20 Hz), 4.93(m, 4H), 4.78(t, 1H, J=3.32 Hz), 3.90(m, 1H), 3.61 (m, 1H), 1.73(m, 6H),

TLC(30% EtOAc/Hexanes)= 0.20

The following intermediates were reduced as above for 4-[(Tetrahydro-2H-pyran-2-yloxy)methyl]-2-[4-(trifluoromethyl)phenyl]-1,3-thiazol-5-yl}methanol.

## {4-[(Tetrahydro-2H-pyran-2-yloxy)methyl]-2-[4-(trifluoromethyl)phenyl]-1,3-thiazol-5-yl}methanol

$^1H$ NMR ($CDCl_3$) 400MHz δ 8.07(d, 2H, J=8.20 Hz), 7.72(d, 2H, J=8.20 Hz), 4.93(m, 4H), 4.78(t, 1H, J=3.32 Hz), 3.90(m, 1H), 3.61 (m, 1 H), 1.73(m, 6H),

TLC(30% EtOAc/Hexanes)= 0.20

## {2-(4-Fluorophenyl)-4-[(tetrahydro-2H-pyran-2-yloxy)methyl]-1,3-thiazol-5-yl}methanol

$^1H$ NMR ($CDCl_3$) 400MHz δ 7.89(m, 2H), 7.09(m, 2H), 4.81 (m, 5H), 3.84(m, 1 H), 3.55(m, 1 H), 1.67(m, 6H),

## {2-Phenyl-4-[(tetrahydro-2H-pyran-2-yloxy)methyl]-1,3-thiazol-5-yl}methanol

1H NMR (CDCl3) 400MHz δ 7.96(m, 2H), 7.47(m, 3H), 4.92(m, 4H), 4.79(t, 1H, J=3.45 Hz); 3.91 (m, 1H), 3.60(m, 1H), 1.73(m, 6H),

## {2-(4-{trifuloromethyl}phenyl)-4-[(2-phenylethoxy)methyl]-1,3-thiazol-5-yl}methanol

$^1H$ ($CDCl_3$) 300MHz δ 7.99(d, 2H, J=8.79 Hz), 7.67(d, 2H, J=8.79 Hz), 7.26(m, 5H), 4.78(s, 2H), 4.71 (s, 2H),

3.84(t, 2H, J=6.94 Hz), 2.95(t, 2H, J=6.94 Hz), 2.63(s, 1H),

**[2-(4-{trifuloromethyl}phenyl)-4-(3-phenylpropyl)-1,3-thiazol-5-yl]methanol**

$^1$H NMR ($CDCl_3$) 300MHz δ 8.02(d, 2H, J=8.79 Hz), 7.67(d, 2H, J=8.79 Hz), 7.23(m, 4H), 4.76(s, 2H), 2.84 (t, 2H, 7.28 Hz), 2.67(t, 2H, 7.28 Hz), 2.12(m, 2H),

**[4-benzyl-2-(4-{trifluoromethyl}phenyl)-1,3-thiazol-5-yl]methanol**

$^1$H ($CDCl_3$) 300MHz δ 8.01 (d, 2H, J=8.79 Hz), 7.65(d, 2H, J=8.79 Hz), 7.26(m, 5H), 4.78(s, 2H), 4.15(s, 2H),
**[0118]** TLC(20% EtOAc/Hexanes) $R_f$= 0.18
MS($ES^+$) M+H= 350

**[2-(4-{trifluoromethyl}phenyl)-4-(2-phenylethyl)-1,3-thiazol-5-yl]methanol**

$^1$H ($CDCl_3$) 300MHz δ 8.06(d, 2H, J=9.61 Hz), 7.70(d, 1H, J=9.48 Hz), 7.23(m, 4H), 7.06(m, 2H), 4.40(d, 2H, J=5.63 Hz), 3.07(s, 4H), 1.08(s, 1 H),
**[0121]** TLC(20% EtOAc/Hexanes) $R_f$= 0.18
MS($ES^+$) M+H= 364

**[4-[(Benzyloxy)methyl]-2-(4-{trifluoromethyl}phenyl)-1,3-thiazol-5-yl]methanol**

$^1$H ($CDCl_3$) 300MHz δ 8.02(d, 2H, J=8.79 Hz), 7.68(d, 2H, J=8.79 Hz), 7.35(m, 5H), 4.82(m, 4H), 4.68(s, 2H),
**[0124]** TLC(20% EtOAc/Hexanes) $R_f$= 0.14

**[4-(4-Bromobenzyl)-2-(4-{trifluoromethyl}phenyl)-1,3-thiazol-5-yl]methanol**

$^1$H NMR ($CDCl_3$) 300MHz δ 7.99(d, 2H, J=8.10 Hz), 7.66(d, 2H, J=8.10 Hz), 7.40(d, 2H, J=8.38 Hz), 7.15(d, 2H, J=8.38 Hz), 4.81(s, 2H), 4.10(s, 2H),
**[0126]** TLC(20% EtOAc/Hexanes) $R_f$= 0.14

**{4-[4-(trifluoromethyl)benzyl]-2-[4-(trifluoromethyl)phenyl]-1,3-thiazol-5-yl}methanol**

From ethyl 4-[4-(trifluoromethyl)benzyl]-2-[4-(trifluoromethyl)phenyl]-1,3-thiazole-5-carboxylate (0.096g, 0.21 mmol), {4-[4-(trifluoromethyl)benzyl]-2-[4-(trifluoromethyl)phenyl]-1,3-thiazol-5-yl}methanol (0.09g, 100%) was obtained as a white solid.
$^1$H NMR ($CDCl_3$): δ 8.16 (d, 2 H), 7.73 (d, 2 H), 7.59 (d, 2 H), 7.44 (d, 2 H), 4.90 (d, 2 H), 4.26 (t, 2 H); MS *m*/*z* 418 (M+1).

**{4-[4-(trifluoromethoxy)benzyl]-2-[4-(trifluoromethyl)phenyl]-1,3-thiazol-5-yl}methanol**

From ethyl 4-[4-(trifluoromethoxy)benzyl]-2-[4-(trifluoromethyl)phenyl]-1,3-thiazole-5-carboxylate (0.123g 0.26 mmol), {4-[4-(trifluoromethoxy)benzyl]-2-[4-(trifluoromethyl)phenyl]-1,3-thiazol-5-yl}methanol (0.13g, 99%) was obtained as a white solid.
$^1$H NMR ($CDCl_3$): δ 8.07 (d, 2 H), 7.71 (d, 2 H), 7.38 (d, 2 H), 7.18 (d, 2 H), 4.80 (d, 2 H), 4.20 (s, 2 H); MS *m*/*z* 434 (M+1).

**{4-(4-methoxybenzyl)-2-[4-(trifluoromethyl)phenyl]-1,3-thiazol-5-yl}methanol**

From ethyl 4-[4-methoxybenzyl]-2-[4-(trifluoromethyl)phenyl]-1,3-thiazole-5-carboxylate ( 0.16g, 0.38 mmol), {4-(4-methoxybenzyl)-2-[4-(trifluoromethyl)phenyl]-1,3-thiazol-5-yl}methanol (0.06g, 40%) was obtained as a white solid.
MS *m*/*z* 380 (M+1); HPLC RT 3.552 (C18 4.2x100mm, 0-100% ACN/$H_2O$ (0.1% TFA), 6min @ 2ml/min @254/220nm).

**{4-[4-(methylsulfanyl)benzyl]-2-[4-(trifluoromethyl)phenyl]-1,3-thiazol-5-yl}methanol**

From ethyl 4-[4-(methylsulfanyl)benzyl]-2-[4-(trifluoromethyl)phenyl]-1,3-thiazole-5-carboxylate (0.44g, 1.0

mmol), {4-[4-(methylsulfanyl)benzyl]-2-[4-(trifluoromethyl)phenyl]-1,3-thiazol-5-yl}methanol (0.3g, 76%) was obtained as a white solid.

MS *m/z* 396 (M+1); HPLC RT 3.699 (C18 4.2x100mm, 0-100% ACN/$H_2O$ (0.1% TFA), 6min @ 2ml/min @254/220nm).

**{4-(4-*tert*-butylbenzyl)-2-[4-(trifluoromethyl)phenyl]-1,3-thiazol-5-yl}methanol**

From ethyl 4-[4-*tert*-butylbenzyl]-2-[4-(trifluoromethyl)phenyl]-1,3-thiazole-5-carboxylate (0.24g, 0.54 mmol), {4-(4-*tert*-butylbenzyl)-2-[4-(trifluoromethyl)phenyl]-1,3-thiazol-5-yl}methanol (0.13g, 64%) was obtained as a white solid.

MS *m/z* 406 (M+1); HPLC RT 4.002 (C18 4.2x100mm, 0-100% ACN/$H_2O$ (0.1% TFA), 6min @ 2ml/min @254/220nm).

**{4-(3-thienylmethyl)-2-[4-(trifluoromethyl)phenyl]-1,3-thiazol-5-yl}methanol**

From ethyl 4-[3-thienylmethyl]-2-[4-(trifluoromethyl)phenyl]-1,3-thiazole-5-carboxylate (0.44g, 1.11 mmol), {4-(3-thienylmethyl)-2-[4-(trifluoromethyl)phenyl]-1,3-thiazol-5-yl}methanol (0.098g, 25%) was obtained as a yellow solid.

MS *m/z* 356 (M+1); HPLC RT 3.513 (C18 4.2x100mm, 0-100% ACN/$H_2O$ (0.1% TFA), 6min @ 2ml/min @254/220nm).

**{4-(2-furylmethyl)-2-[4-(trifluoromethyl)phenyl]-1,3-thiazol-5-yl}methanol**

From ethyl 4-[2-furylmethyl]-2-[4-(trifluoromethyl)phenyl]-1,3-thiazole-5-carboxylate ( 0.204g, 0.53 mmol), {4-(2-furylmethyl)-2-[4-(trifluoromethyl)phenyl]-1,3-thiazol-5-yl}methanol (0.162g, 89%) was obtained as a white solid.

MS *m/z* 340 (M+1); HPLC RT 3.382 (C18 4.2x100mm, 0-100% ACN/$H_2O$ (0.1% TFA), 6min @ 2ml/min @254/220nm).

**{4-(3-furylmethyl)-2-[4-(trifluoromethyl)phenyl]-1,3-thiazol-5-yl}methanol**

From ethyl 4-[3-furylmethyl]-2-[4-(trifluoromethyl)phenyl]-1,3-thiazole-5-carboxylate (0.217g 0.57 mmol), {4-(3-furylmethyl)-2-[4-(trifluoromethyl)phenyl]-1,3-thiazol-5-yl}methanol (0.180g, 88%) was obtained as a white solid.

MS *m/z* 340 (M+1); HPLC RT 3.385 (C18 4.2x100mm, 0-100% ACN/$H_2O$ (0.1% TFA), 6min @ 2ml/min @254/220nm).

**{4-(2-thienylmethyl)-2-[4-(trifluoromethyl)phenyl]-1,3-thiazol-5-yl}methanol**

From ethyl 4-[2-thienylmethyl]-2-[4-(trifluoromethyl)phenyl]-1,3-thiazole-5-carboxylate ( 0.248g, 0.62 mmol), {4-(2-thienylmethyl)-2-[4-(trifluoromethyl)phenyl]-1,3-thiazol-5-yl}methanol (0.186g, 87%) was obtained as a yellow solid.

MS *m/z* 356 (M+1); HPLC RT 3.528 (C18 4.2x100mm, 0-100% ACN/$H_2O$ (0.1% TFA), 6min @ 2ml/min @254/220nm).

**{4-[(4-Methyll-2-thienyl)methyl]-2-[4-(trifluoromethyl)phenyl]-1,3-thiazol-5-yl}methanol**

From ethyl 4-[(4-methyl-2-thienyl)methyl]-2-[4-(trifluoromethyl)phenyl]-1,3-thiazole-5-carboxylate (0.5g, 1.22 mmol), {4-[(4-methyl-2-thienyl)methyl]-2-[4-(trifluoromethyl)phenyl]-1,3-thiazol-5-yl}methanol (0.084g, 19%) was obtained as a yellow solid.

MS *m/z* 370 (M+1); HPLC RT 3.913 (C18 4.2x100mm, 0-100% ACN/$H_2O$ (0.1% TFA), 6min @ 2ml/min @254/220nm).

**{4-(2,4-difluorobenzyl)-2-[4-(trifluoromethyl)phenyl]-1,3-thiazol-5-yl}methanol**

From ethyl 4-[2,4-difluorobenzyl]-2-[4-(trifluoromethyl)phenyl]-1,3-thiazole-5-carboxylate (0.46g, 1.08 mmol), {4-(2,4-difluorobenzyl)-2-[4-(trifluoromethyl)phenyl]-1,3-thiazol-5-yl}methanol (0.222g, 54%) was obtained as a white solid.

MS *m/z* 386 (M+1); HPLC RT 3.900 (C18 4.2x100mm, 0-100% ACN/$H_2O$ (0.1% TFA), 6min @ 2ml/min @254/220nm).

**5-(Chloromethyl)-4-[(tetrahydro-2H-pyran-2-yloxy)methyl]-2-[4-(trifluoromethyl)phenyl]-1,3-thiazole**

To a 500-ml round-bottom flask equipped with a magnetic stir-bar, an addition funnel and a $N_2$ inlet was added 4-[(Tetrahydro-2H-pyran-2-yloxy)methyl]-2-[4-(trifluoromethyl)phenyl]-1,3-thiazol-5-yl}methanol (15g, 40.17mmoles, 1eq) and dry $CH_2Cl_2$ (150ml, 0.27M). Methanesulfonyl chloride (3.73ml, 48.20mmoies, 1.2eq) was added neat all at once followed by the dropwise addition of triethylamine (8.44ml, 60.26mmoles, 1.5eq) over 10 minutes: This solution was stirred at room temperature for 1 hr. The reaction was transferred to a separatory funnel and washed with water and brine. After the phases were separated the $CH_2Cl_2$ fraction was dried over $Na_2SO_4$ and the solvent was removed in vacuo. This yielded 15.74g (100%) of a brown oil. The crude product was used as is and required no purification.

$^1H$ NMR ($CDCl_3$) 300MHz δ 8.08(d, 2H, J=8.20 Hz), 7.73(d, 2H, J=8.20 Hz), 5.00(m, 3H), 4.80(m, 2H), 3.97 (m, 1H), 3.64(m, 1H), 1.77(m, 6H),

TLC(25% EtOAc/Hexanes) $R_f$ = 0.64

The following intermediates were also prepared using the above mesylation/chloride displacement procedure:

**5-(Chloromethyl)-2-(4-fluorophenyl)-4-[(tetrahydro-2H-pyran-2-yloxy)methyl]-1,3-thiazole**

$^1H$ NMR ($CDCl_3$) 400MHz δ 7.90(m, 2H), 7.11(m, 2H), 4.94(s, 2H), 4.91(d, 1H, J□.45 Hz), 4.76(t, 1H, J=3.39 Hz), 4.72(d, 1H, J .45 Hz), 3.92(m, 1H), 3.58(m, 1H), 1.69(m, 6H),

**[5-(Chloromethyl)-2-phenyl-1,3-thiazol-4-yl]methyl tetrahydro-2H-pyran-2-yl ether**

$^1H$ NMR ($CDCl_3$) 300MHz δ 7.95(m, 2H), 7.47(m, 3H), 4.98(m, 3H), 4.80(m, 2H), 3.98(m, 1H), 3.63(m, 1 H), 1.73(m, 6H),

TLC(25% EtOAc/Hexanes) $R_f$ = 0.57

**5-(Chloromethyl)-2-(4-{trifluoromethyl}phenyl)-4-[4-(3-thienyl)benzyl]-1,3-thiazole**

$^1H$ NMR ($CDCl_3$) 300MHz δ 8.06(d. 2H, J=8.23 Hz), 7.71(d, 2H, J=8.23 Hz), 7.58(d, 2H, J=8.23 Hz), 7.41 (m, 5H), 4.84(s, 2H), 426(s, 2H),

TLC(20% EtOAc/Hexanes) $R_f$= 0.66

**4-[(Benzyloxy)methyl]-5-(chloromethyl)-2-[4-(trifluoromethyl)phenyl]-1,3-thiazole**

$^1H$ NMR ($CDCl_3$) 300MHz δ 8.03(d, 2H, J=8.79 Hz), 7.69(d, 2H, J=8.79 Hz), 7.37(m, 5H), 4.90(s, 2H), 4.77 (s, 2H), 4.66(s, 2H)

**4-Benzyl-5-(chloromethyl)-2-(4-(trifluoromethyl}phenyl)-1,3-thiazole**

$^1H$ ($CDCl_3$) 300MHz δ 8.02(d, 2H, J=8.79 Hz), 7.67(d, 2H, J=8.79 Hz), 7.26(m, 5H), 4.77(s, 2H), 4.21 (s, 2H),

TLC(20% EtOAc/Hexanes) $R_f$= 0.66

**5-(Chloromethyl)-2-(4-{trifluoromethyl}phenyl)-4-(2-phenylethyl)-1,3-thiazole**

$^1H$ ($CDCl_3$) 300MHz δ 8.05(d, 2H, J=8.79 Hz), 7.70(d, 2H, J=8.79 Hz), 7.22(m, 5H), 4.46(s, 2H), 3.09(s, 4H),

TLC(20% EtOAc/Hexanes) $R_f$= 0.67

**5-(Chloromethyl)-2-(4-{trifluoromethyl}phenyl)-4-[(2-phenylethoxy)methyl]-1,3-thiazole**

$^1H$ NMR ($CDCl_3$) 300MHz δ 8.01 (d, 2H, J=8.79 Hz), 7.68(d, 2H, J=8.79 Hz), 7.26(m, 5H), 4.76(s, 2H), 4.74 (s, 2H), 3.78(t, 2H, J=6.94 Hz), 2.94(t, 2H, J=6.94 Hz),

TLC(20% EtOAc/Hexanes) $R_f$= 0.56

**5-(Chloromethyl)-2-(4-{trifluoromethyl}phenyl)-4-(3-phenylpropyl)-1,3-thiazole**

TLC(20% EtOAc/Hexanes) $R_f$= 0.63

**4-(4-Bromobenzyl)-5-(chloromethyl)-2-(4-{trifluoromethyl}phenyl)-1,3-thiazole**

$^1$H NMR ($CDCl_3$) 300MHz δ 8.00(d, 2H, J=8.10 Hz), 7.67(d, 2H, J=8.10 Hz), 7.42(d, 2H, J=8.38 Hz), 7.18(d, 2H, J=8.38 Hz), 4.77(s, 2H), 4.14(s, 2H),
TLC(20% EtOAc/Hexanes) $R_f$= 0.66

**4-([1,1'-Biphenyl]-4-ylmethyl)-5-(chloromethyl)-2-(4-{trifluoromethyl}phenyl)-1,3-thiazole**

$^1$H NMR ($CDCl_3$) 300MHz δ 8.07(d, 2H, J=8.23 Hz), 7.72(d, 2H, J=8.23 Hz), 7.57(m, 4H), 7.39(m, 5H), 4.85 (s, 2H), 4.28(s, 2H),
**[0168]** TLC(20% EtOAc/Hexanes) $R_f$= 0.69

**5-(chloromethyl)-4-[4-(trifluoromethyl)benzyl]-2-[4-(trifluoromethyl)phenyl]-1,3-thiazole**

From {4-[4-(trifluoromethyl)benzyl]-2-[4-(trifluoromethyl)phenyl]-1,3-thiazol-5-yl}methanol (0.09g, 0.216 mmol), 5-(chloromethyl)-4-[4-(trifluoromethyl)benzyl]-2-[4-(trifluoromethyl)phenyl]-1,3-thiazole (0.087g, 93%) was obtained as a yellow oil and immediately taken on without purification.

**5-(chloromethyl)-4-[4-(trifluoromethoxy)benzyl]-2-[4-(trifluoromethyl)phenyl]-1,3-thiazole**

From {4-[4-(trifluoromethoxy)benzyl]-2-[4-(trifluoromethyl)phenyl]-1,3-thiazol-5-yl}methanol (0.13g, 0.3 mmol), 5-(chloromethyl)-4-[4-(trifluoromethoxy)benzyl]-2-[4-(trifluoromethyl)phenyl]-1,3-thiazole (0.135g, 100%) was obtained as a yellow oil and immediately taken on without purification.

**5-(chloromethyl)-4-(4-methoxybenzyl)-2-[4-(trifluoromethyl)phenyl]-1,3-thiazole**

From {4-(4-methoxybenzyl)-2-[4-(trifluoromethyl)phenyl]-1,3-thiazol-5-yl}methanol (0.06g, 0.158 mmol), 5-(chloromethyl)-4-(4-methoxybenzyl)-2-[4-(trifluoromethyl)phenyl]-1,3-thiazole (0.08g, 100%) was obtained as a yellow oil and immediately taken on without purification.

**5-(chloromethyl)-4-[4-(methylsulfanyl)benzyl]-2-[4-(trifluoromethyl)phenyl]-1,3-thiazole**

From {4-[4-(methylsulfanyl)benzyl]-2-[4-(trifluoromethyl)phenyl]-1,3-thiazol-5-yl}methanol (0.3g, 0.76 mmol), 5-(chloromethyl)-4-[4-(methylsulfanyl)benzyl]-2-[4-(trifluoromethyl)phenyl]-1,3-thiazole (0.33g, 100%) was obtained as a yellow oil and immediately taken on without purification.
MS *m/z* 414 (M+1).

**4-(4-*tert*-butylbenzyl)-5-(chloromethyl)-2-[4-(trifluoromethyl)phenyl]-1,3-thiazole**

From {4-(4-*tert*-butylbenzyl)-2-[4-(trifluoromethyl)phenyl]-1,3-thiazol-5-yl}methanol (0.13g, 0.32 mmol), 4-(4-*tert*-butylbenzyl)-5-(chloromethyl)-2-[4-(trifluoromethyl)phenyl]-1,3-thiazole (0.151g, 100%) was obtained as a yellow oil and immediately taken on without purification.
MS *m/z* 424 (M+1).

**5-(chloromethyl)-4-(3-thienylmethyl)-2-[4-(trifluoromethyl)phenyl]-1,3-thiazole**

From {4-(3-thienylmethyl)-2-[4-(trifluoromethyl)phenyl]-1,3-thiazol-5-yl}methanol (0.098g, 0.28 mmol), 5-(chloromethyl)-4-(3-thienylmethyl)-2-[4-(trifluoromethyl)phenyl]-1,3-thiazole (0.105g, 100%) was obtained as a yellow oil and immediately taken on without purification.
MS *m/z* 374 (M+1).

**5-(chloromethyl)-4-(2-furylmethyl)-2-[4-(trifluoromethyl)phenyl]-1,3-thiazole**

From {4-(2-furylmethyl)-2-[4-(trifluoromethyl)phenyl]-1,3-thiazol-5-yl}methanol (0.162g, 0.48 mmol), 5-(chloromethyl)-4-(2-furylmethyl)-2-[4-(trifluoromethyl)phenyl]-1,3-thiazole (0.097g, 57%) was obtained as a yellow oil and immediately taken on without purification.
MS *m/z* 358 (M+1).

**5-(chloromethyl)-4-(3-furylmethyl)-2-[4-(trifluoromethyl)phenyl]-1,3-thiazole**

From {4-(3-furylmethyl)-2-[4-(trifluoromethyl)phenyl]-1,3-thiazol-5-yl}methanol (0.18g, 0.53 mmol), 5-(chloromethyl)-4-(3-furylmethyl)-2-[4-(trifluoromethyl)phenyl]-1,3-thiazole (0.172g, 91 %) was obtained as a yellow oil and immediately taken on without purification.

MS *m*/*z* 358 (M+1).

**5-(chloromethyl)-4-(2-thienylmethyl)-2-[4-(trifluoromethyl)phenyl]-1,3-thiazole**

From {4-(2-thienylmethyl)-2-[4-(trifluoromethyl)phenyl]-1,3-thiazol-5-yl}methanol (0.186g, 0.52 mmol), 5-(chloromethyl)-4-(2-thienylmethyl)-2-[4-(trifluoromethyl)phenyl]-1,3-thiazole (0.185g, 95%) was obtained as a yellow oil and immediately taken on without purification.

MS *m*/*z* 374 (M+1).

**5-(chloromethyl)-4-[(4-methyl-2-thienyl)methyl]-2-[4-(trifluoromethyl)phenyl]-1,3-thiazole**

From {4-[(4-methyl-2-thienyl)methyl]-2-[4-(trifluoromethyl)phenyl]-1,3-thiazol-5-yl}methanol (0.084g, 0.23 mmol), 5-(chloromethyl)-4-[(4-methyl-2-thienyl)methyl]-2-[4-(trifluoromethyl)phenyl]-1,3-thiazole (0.123g, 100%) was obtained as a yellow oil and immediately taken on without purification.

**5-(chloromethyl)-4-(2,4-difluorobenzyl)-2-[4-(trifluoromethyl)phenyl]-1,3-thiazole**

From {4-(2,4-difluorobenzyl)-2-[4-(trifluoromethyl)phenyl]-1,3-thiazol-5-yl}methanol (0.222g, 0.58 mmol), 5-(chtoromethyl)-4-(2,4-difluorobenzyl)-2-[4-(trifluoromethyl)phenyl]-1,3-thiazole (0.279g, 100%) was obtained as a yellow oil and immediately taken on without purification.

**Ethyl 2-methyl-2-phenoxypropanoate**

To a solution of potassium *t*-butoxide (1M in THF, 531ml, 0.531moles, 1eq) precooled to 0°C (ice bath) was added phenol (50g, 0.531moles, 1eq) in dry THE (50ml) dropwise via an addition funnel over 20 minutes maintaining the internal temperature of the reaction below 5 degrees centigrade. Ethyl-2-bromoisobutyrate (70.14ml, 0.9eq, 0.478moles) in dry THF (20ml) was added dropwise over 10 minutes maintaining the internal reaction temperature below 5 °C. After the addition was complete, the ice bath was removed and the reaction was allowed to warm to room temperature. The reaction was brought to reflux and maintained at this reflux temperature for 8 hours. Following the cooling of the reaction to 0°C the volatiles were removed *in vacuo*. The residue was then partitioned between EtOAc and 1N NaOH. The phases were separated and the organic phase was washed with 1N NaOH, $H_2O$, brine and dried over $Na_2SO_4$. After filtration the solution was concentrated under reduced pressure to yield 83g (75%) of dean title compound.

$^1H$ NMR ($CDCl_3$) 400MHz δ 7.21 (m, 2H), 6.95(t, 1H. J=7.41 Hz), 6.82(m, 2H), 4.21 (q, 2H, J=7.13 Hz), 1.57 (s, 6H), 1.22(t, 3H, J=7.13 Hz),

**Ethyl (2-ethylphenoxy)acetate**

To a stirred solution of 2-ethylphenol (5ml, 42.4mmoles, 1eq) in dry DMF (120ml, 0.35M) was added potassium carbonate (6.45g, 46.6mmoles, 1.1eq) and ethylbromoacetate (4.7ml, 42.2mmoles, 1eq) and heated to 60°C overnight. After cooling to room temperature the reaction mixture was partitioned between ethyl ether and 1 N NaOH. The phases were separated and the organic portion was washed twice with 1N NaOH, twice with $H_2O$, brine, dried over $Na_2SO_4$, filtered and concentrated *in vacuo* to yield 7.2g (82%) of product.

$^1H$ NMR ($CDCl_3$) 400MHz δ 7.14(m, 2H), 6.92(t, 1H, J=8.24 Hz), 6.70(d. 1H, J=8.24 Hz), 4.62(s, 2H), 4.24 (q, 2H, J=7.14 Hz), 2.70(q, 2H, J=7.51 Hz), 1.27(t, 3H, J=7.14 Hz). 1.21(t, 3H, J=7.51 Hz),

The following were compounds were made using the same alkylation procedure:

**Ethyl (2-isopropylphenoxy)acetate**

$^1H$ NMR ($CDCl_3$) 400MHz δ 7.23(d, 1H, J=7.69 Hz), 7.11 (t, 1H, J=7.69 Hz), 6.96(t, 1H, J=7.69 Hz), 6.70(d, 1H, J=7.69 Hz), 4.62(s, 2H), 4.25(q, 2H, J=7.14 Hz), 3.41(m, 1H), 1.26(m, 9H),

**Ethyl (2-propylphenoxy)acetate**

$^1H$ NMR ($CDCl_3$) 400MHz δ 7.12(m, 2H), 6.90(t, 1H, J=8.24 Hz), 6.69(d, 1H, J=8.24 Hz), 4.61(s, 2H), 4.24 (q, 2H, J=7.14 Hz), 2.64(t, 2H, J=7.33 Hz), 1.64(m, 2H), 1.27(t, 3H, J=7.14 Hz), 0.94(t, 3H, J=7.33 Hz),

**Ethyl [4-(chlorosulfonyl)-2-ethylphenoxy]acetate**

To a 250ml round-bottom flask containing chlorosulfonic acid (30ml) cooled to 0°C was added ethyl (2-ethylphenoxy)acetate (7.2g, 34.6mmoles) dropwise. Once the addition was complete the icebath was removed and the reaction was allowed to warm to room temperature at which the reaction was stirred for 3 hours. The reaction was then slowly added to ice and, once the excess chlorosulfonic acid was quenched, the mixture was diluted with $CH_2Cl_2$ (200ml). The phases were separated and the aqueous fraction was washed with $CH_2Cl_2$ twice. The combined organic fractions were dried over $Na_2SO_4$ and filtered and concentrated *in vacuo* to yield 7.2g (70%) of crude product. The crude product was used with no purification.

$^1H$ NMR ($CDCl_3$) 400MHz δ 7.84(m, 2H), 6.79(d, 1 H, J=8.24 Hz), 4.75(s, 2H), 4.26(q, 2H, J=7.14 Hz), 2.77 (q, 2H, J=7.51 Hz), 1.26(m, 6H),

The following were compounds were made using the same chlorosulfonation procedure:

**Ethyl [4-(chlorosulfonyl)-2-methylphenoxy]acetate**

$^1H$ NMR (d6-DMSO) 300MHz δ 7.41(m, 2H), 6.79(d, 1H, J=8.23 Hz), 4.82(s, 2H), 4.16(q, 2H, J=7.17 Hz), 2.21(s, 3H), 1.21(t, 3H, J=7.17 Hz),

**Ethyl 2-[4-(chlorosulfonyl)-2-methylphenoxy]propanoate**

$^1H$ NMR (d6-DMSO) 300MHz δ 7.44(m, 1H), 7.39(dd, 1H, J=8.23, 2.39 Hz), 6.74(d, 1H, J=8.23 Hz), 4.96(q, 1H, J=6.81 Hz), 4.13(q, 2H, J=7.08 Hz), 2.20(s, 3H), 1.54(d, 3H, J=6.81 Hz), 1.18(t, 3H, J=7.08 Hz),

**Ethyl 2-[4-(chlorosulfonyl)-2-isopropylphenoxy]propanoate**

$^1H$ NMR ($CDCl_3$) 400MHz δ 7.81(m, 2H), 6.76(d, 1H, J=8.42 Hz), 4.87(q, 1H, J=6.78 Hz), 4.21 (q, 2H, J=7.14 Hz), 3.40(m, 1H), 1.65(d, 3H, J=6.78 Hz), 1.24(m, 9H),

**Ethyl [4-(chlorosulfonyl)-2-isopropylphenoxy]acetate**

$^1H$ NMR ($CDCl_3$) 400MHz δ 7.84(m, 2H), 6.80(d, 1H, J=8.42 Hz), 4.75(s, 2H), 4.26(q, 2H, J=7.14 Hz), 3.42 (m, 1 H), 1.27(m, 9H),

**Ethyl 2-[4-(chlorosulfonyl)-2-propylphenoxy]propanoate**

$^1H$ NMR ($CDCl_3$) 400MHz δ 7.80(m, 2H), 6.75(d, 1H, J=8.42 Hz), 4.85(q, 1H, J=6.78 Hz), 4.21 (q, 2H, J=7.14 Hz), 2.69(t, 2H, J=7.51 Hz), 1.66(m, 5H), 1.23(t, 3H, J=7.14 Hz), 0.95(t, 3H, J=7.51 Hz),

**Ethyl [4-(chlorosulfonyl)-2-propylphenoxy]acetate**

$^1H$ NMR ($CDCl_3$) 400MHz δ 7.83(m, 2H), 6.79(d, 1H, J=8.42 Hz), 4.73(s, 2H), 4.26(q, 2H, J=7.14 Hz), 2.70 (t, 2H, J=7.51 Hz), 1.67(m, 2H), 1.29(t, 3H, J=7.14 Hz), 0.95(t, 3H, J=7.51 Hz),

**Ethyl 2-[4-(chlorosulfonyl)-2-ethylphenoxy]propanoate**

$^1H$ NMR ($CDCl_3$) 400MHz δ 7.81 (m, 2H), 6.75(d, 1H, J=8.42 Hz), 4.86(q, 1H, J=6.78 Hz), 4.21(q, 2H, J=7.08 Hz), 2.75(m, 2H), 1.68(d, 3H, J=6.78 Hz), 1.23(m, 6H),

**Ethyl 2-[4-(chlorosulfonyl)phenoxy]-2-methylpropanoate**

To a 3-L three-neck round-bottom flask equipped with a magnetic stir-bar, low temperature thermometer with thermometer adapter, addition funnel and a $N_2$ inlet was added ethyl 2-methyl-2-phenoxypropanoate (83g, 0.399moles, 1eq) and dry $CH_2Cl_2$ (1L, 0.4M). After cooling the reaction to 0 °C (ice bath) chlorosulfonic acid (26.5ml, 0.399moles,

1eq) in dry $CH_2Cl_2$ (50ml) was added dropwise over 30 minutes via addition funnel maintaining the internal temperature below 5°C. Following this dropwise addition the reaction was allowed to stir at 0°C for 3 hours. The reaction was monitored by HPLC and after 3 hours complete conversion was observed [(C-18, 3μm) 0%-95% Acetonitrile/Water over 8 minutes $R_t$= 2.96minutes]. At this point dry DMF (124ml, 4eq) was added slowly maintaining the internal temperature below 5°C. This was followed by the dropwise addition of thionyl chloride (43.77ml, 0.599moles, 1.5eq) in dry $CH_2Cl_2$ (50ml) over 25minutes maintaining the internal temperature below 5°C. After stirring at 0°C for 1.5 hours and monitoring by HPLC [(C-18, 3μm) 0%-95% Acetonitrile/Water over 8 minutes $R_t$= 5.97minutes] the reaction was allowed to warm to room temperature. The reaction mixture was then washed with 0.1 N HCl and the phases were separated, with discarding the aqueous fraction. The organic fraction was washed with 0.1N HCl, $H_2O$, brine and dried over $Na_2SO_4$. The solution was filtered and concentrated *in vacuo* to yield 119.95g (98%) of pure sulfonyl chloride.

$^1H$ NMR ($CDCl_3$) 400MHz δ 7.89(d, 2H, J=9.31 Hz), 6.89(d, 2H, J=9.31 Hz), 4.21 (q, 2H, J=7.16 Hz), 1 66(s, 6H), 1.20(t, 3H, J=7.16 Hz),

HPLC (C-18, 3μm) 0%-95% Acetonitrile/Water over 8 minutes $R_t$= 5.97minutes

**Ethyl 2-methyl-2-(4-sulfanylphenoxy)propanoate**

To a 3-L three-neck round-bottom flask equipped with an overhead mechanical stirrer, addition funnel and a $N_2$ inlet was added ethyl 2-[4-(chlorosulfonyl)phenoxy]-2-methylpropanoate (53g, 0.173moles, 1eq) and absolute EtOH (500ml). Tin powder (325mesh, 123.06g, 1.04moles, 6 eq) was added as a solid. The overhead stirrer was adjusted so that the rotor is as close as possible to the bottom of the round-bottom flask and stirring speed was accelerated to a very high setting before adding the HCl to prevent the clumping of the tin metal. Hydrogen chloride (4N in dioxane, 300ml) was added dropwise over the course of 1 hour. The reaction mixture was refluxed for 4 hours at which point the hot ethanolic solution was poured into a 2-L Erlenmeyer flask containing $CH_2Cl_2$ (1L) and ice. After stirring for 10 minutes the biphasic mixture was filtered through Celite. After transferring to a separatory funnel the phases were separated and the aqueous fraction was washed with $CH_2Cl_2$ (2x 100ml). The combined organic fractions were dried over $Na_2SO_4$, filtered and concentrated *in vacuo*. A bright yellow oil with a white precipitate suspended resulted. This yellow mixture was dissolved in a minimum amount of $CH_2Cl_2$ and filtered once again through Celite to yield 30g (75%) of a bright yellow oil.

$^1H$ NMR ($CD_3OD$) 300MHz δ 7.18(m, 2H), 6.73(d, 2H, J=8.00 Hz), 4.23(q, 2H, J=7.17 Hz), 3.69(s, 1H), 1.59 (s, 6H), 1.26(t, 3H, J=7.17 Hz),

The following were compounds were made using the same reduction procedure:

**Ethyl (2-methyl-4-sulfanylphenoxy)acetate**

$^1H$ NMR ($CDCl_3$) 400MHz δ 7.15(m, 2H), 6.63(d, 1H, J=8.23 Hz), 4.64(s, 2H), 4.29(q, 2H, J=7.17 Hz), 3.36 (s, 1H), 2.29(s, 3H), 1.33(t, 3H, J=7.17 Hz),

**Ethyl 2-(2-methyl-4-sulfanylphenoxy)propanoate**

$^1H$ NMR ($CDCl_3$) 400MHz δ 7.12(d, 1H, J=2.39 Hz), 7.04(dd, 1H, J=8.37, 2.39 Hz), 6.56(d, 1H, J=8.37 Hz), 4.67(q, 1H, J=6.72 Hz), 4.19(q, 2H, J=7.12 Hz), 3.31(s, 1H), 2.22(s, 3H), 1.61(d, 3H, J=6.72 Hz), 1.23(t, 3H, J=7.12 Hz),

TLC(20% EtOAc/Hexanes) $R_f$ = 0.60

**Ethyl (2-ethyl-4-sulfanylphenoxy)acetate**

$^1H$ NMR ($CDCl_3$) 400MHz δ 7.13(d, 1H, J=2.20 Hz), 7.08(dd, 1H, J=8.42, 2.38 Hz), 6.58(d, 1H, J=8.42 Hz), 4.59(s, 2H), 4.24(q, 2H, J=7.14 Hz), 3.33(s, 1H), 2.64(q, 2H, J=7.51 Hz), 1.28(t, 3H, J=7.14 Hz), 1.18(t, 3H, J=7.51 Hz),

**Ethyl 2-(2-ethyl-4-sulfanylphenoxy)propanoate**

$^1H$ NMR ($CDCl_3$) 400MHz δ 7.15(d, 1H, J=2.20 Hz), 7.07(dd, 1H, J=8.42, 220 Hz), 6.55(d, 1H, J=8.42 Hz), 4.74(q, 1H, J=6.78 Hz), 4.17(m, 2H), 3.32(s, 1H), 2.61(q, 2H, J=7.51 Hz), 1.61(d, 3H, J=6.59 Hz), 1.19(m, 6H),

The following four compounds were made in the same way and used without further purification.

**Ethyl (2-propyl-4-sulfanylphenoxy)acetate**

**Ethyl 2-(2-propyl-4-sulfanylphenoxy)propanoate**

**Ethyl (2-isopropyl-4-sulfanylphenoxy)acetate**

**Ethyl 2-(2-isopropyl-4-sulfanylphenoxy)propanoate**

**Ethyl 2-methyl-2-{4-[({4-[(tetrahydro-2H-pyran-2-yloxy)methyl]-2-[4-(trifluoromethyl)phenyl]-1,3-thiazol-5-yl}methyl)sulfanyl]phenoxy}propanoate**

To a 250ml round-bottom flask equipped with a magnetic stir-bar and $N_2$ inlet was added 5-(chloromethyl)-4-[(tetrahydro-2H-pyran-2-yloxy)methyl]-2-[4-(trifluoromethyl)phenyl]-1,3-thiazole (7.87g, 20.09mmoles, 1eq) and dry $CH_3CN$ (100ml, 0.27M). Solid cesium carbonate (16.4g, 50.22mmoles, 2.5eq) was added all at once followed by the quick addition of ethyl 2-methyl-2-(4-sulfanylphenoxy)propanoate (5.79g, 24.11mmoles, 1.2eq) in dry $CH_3CN$ (10ml). The reaction was allowed to stir at room temperature for 2 hours at which point the solvent was removed under reduced pressure. The resulting residue was partitioned between EtOAc and 1 N NaOH. After the phases were separated the organic fraction was washed with $H_2O$, brine and dried over $Na_2SO_4$. After filtration the volatiles were removed *in vacuo* to yield the titled compound in >100% yield. Sometimes because of the difficult separation between the thiophenol and the product, the crude product was carried forward without purification.

The following compounds were made using the same alkylation procedure. Where selectivity was an issue the alkylations were carried out below room temperature.:

**Ethyl 2-{2-methyl-4-[({2-phenyl-4-[(tetrahydro-2H-pyran-2-yloxy)methyl]-1,3-thiazol-5-yl}methyl)sulfanyl]phenoxy}propanoate**

$^1H$ NMR ($CDCl_3$) 300MHz δ 7.93(m, 2H), 7.44(m, 3H), 7.28(d, 1H, J=2.39 Hz), 7.15(dd, 1H, J=8.23, 2.39 Hz), 6.61(d, 1H, J=8.23 Hz), 4.72(m, 3H), 4.50(d, 1H, J .21 Hz), 4.32(s, 2H), 4.23(q, 2H, J=7.08 Hz), 3.93(m, 1H), 3.59(m, 1H), 2.26(s, 3H), 1.71(m, 9H), 1.28(t, 3H, J=7.08 Hz),

**Ethyl 2-{2-methyl-4-[({4-[(tetrahydro-2H-pyran-2-yloxy)methyl]-2-[4-(trifluoromethyl)phenyl]-1,3-thiazol-5-yl}methyl)sulfanyl]phenoxy}propanoate**

$^1H$ NMR ($CDCl_3$) 300MHz δ 8.04(d, 2H, J=8.23 Hz), 7.70(d, 2H, J=8.23 Hz), 7.27(d, 1H, J=2.39 Hz), 7.15(dd, 1H, J=8.49, 2.39 Hz), 6.60(d, 1H, J=8.49 Hz), 4.73(m, 3H), 4,51(d, 1H, J□.21 Hz), 4.32(s, 2H), 4.20(q, 2H, J=7.17 Hz), 3.93(m, 1H), 3.60(m, 1H), 2.27(m, 3H), 1.71 (m, 9H), 1.27(t, 3H, J=7.17 Hz),

TLC(30% EtOAc/Hexanes)= 0.73

**Ethyl 2-{4-[({2-(4-fluorophenyl)-4-[(tetrahydro-2H-pyran-2-yloxy)methyl]-1,3-thiazol-5-yl}methyl)sulfanyl]-2-methylphenoxy}propanoate**

$^1H$ NMR ($CDCl_3$) 400MHz δ 7.88(m, 2H), 7.19(d, 1 H, J=2.24 Hz), 7.08(m, 3H), 6.54(d, 1 H, J=8.45 Hz), 4.65 (m, 3H), 4.44(m, 1H), 4.24(s, 2H), 4.16(q, 2H, J=7.13 Hz), 3.86(m, 1 H), 3.53(m, 1H), 2.21 (s, 3H), 1.66(m, 9H), 1.20 (t, 3H, J=7.13 Hz),

**Ethyl {2-ethyl-4-[({4-[(tetrahydro-2H-pyran-2-yloxy)methyl]-2-[4-(trifluoromethyl)phenyl]-1,3-thiazol-5-yl}methyl)sulfanyl]phenoxy}acetate**

$^1H$ NMR ($CDCl_3$) 400MHz δ 7.98(d, 2H, J=8.24 Hz), 7.64(d, 2H, J=8.24 Hz), 7.20(d, 1H, J=2.20 Hz), 7.15(dd, 1H, J=8.42, 2.20 Hz), 6.60(d, 1H, J=8.42 Hz), 4.63(m, 4H), 4.42(d, 1H, J .27 Hz), 4.24(m, 4H), 3.87(m, 1H), 3.54(m, 1H), 2.64(q, 2H, J=7.51 Hz), 1.66(m, 6H), 1.26(t, 3H, J=7.14 Hz), 1.15(t, 3H, J=7.51 Hz),

**Ethyl 2-{2-ethyl-4-[({4-[(tetrahydro-2H-pyran-2-yloxy)methyl]-2-[4-(trifluoromethyl)phenyl]-1,3-thiazol-5-yl}methyl)sulfanyl]phenoxy}propanoate**

$^1H$ NMR ($CDCl_3$) 400MHz δ 7.98(d, 2H, J=8.24 Hz), 7.64(d, 2H, J=8.24 Hz), 7.17(d, 1H, J=2.38 Hz), 7.11(dd, 1H, J=8.42, 2.38 Hz), 6.56(d, 1H, J=8.42 Hz), 4.71 (q, 1H, J=6.78 Hz), 4.66(t, 1H, J=3.39 Hz), 4.60(d, 1H, J .27 Hz), 4.41 (d, 1H, J .27 Hz), 4.26(s, 2H), 4.16(q, 2H, J=7.14 Hz), 3.87(m, 1H), 3.54(m, 1H), 2.62(q, 2H, J=7.51 Hz), 1.60(m,

9H), 120(t, 3H, J=7.14 Hz), 1.15(t, 3H, J=7.51 Hz),

**Ethyl {2-propyl-4-[({4-[(tetrahydro-2H-pyran-2-yloxy)methyl]-2-[4-(trifluoromethyl)phenyl]-1,3-thiazol-5-yl} methyl)sulfanyl]phenoxy}acetate**

$^1$H NMR ($CDCl_3$) 400MHz δ 7.97(d, 2H, J=8.20 Hz), 7.64(d, 2H, J=8.20 Hz), 7.16(m, 2H), 6.59(d, 1H, J=8.24 Hz), 4.66(m, 1 H), 4.61 (m, 3H), 4.43(d, 1 H, J .27 Hz), 4.23(m, 4H), 3.88(m, 1H), 3.54(m, 1 H), 2.57(t, 2H, J=7.33 Hz), 1.68(m, 8H), 1.26(t, 3H, J=7.14 Hz), 0.88(t, 3H, J=7.33 Hz),

**Ethyl 2-{2-propyl-4-[({4-[(tetrahydro-2H-pyran-2-yloxy)methyl]-2-[4-(trifluoromethyl)phenyl]-1,3-thiazol-5-yl} methyl)sulfanyl]phenoxy}propanoate**

$^1$H NMR ($CDCl_3$) 400MHz δ 7.97(d, 2H, J=8.24 Hz), 7.64(d, 2H, J=8.24 Hz), 7.17(d, 1H, J=2.38 Hz), 7.11(dd, 1H, J=8.42, 2.38 Hz), 6.55(d, 1H, J=8.42 Hz), 4.70(q, 1H, J=6.78 Hz), 4.66(t, 1H, J=3.39 Hz), 4.62(d, 1H, J .27 Hz), 4.43(d, 1H, J .27 Hz), 4.25(s, 2H), 4.15(q, 2H, J=7.14 Hz), 3.88(m, 1 H), 3.54(m, 1H), 2.56(t, 2H, J=7.33 Hz), 1.60(m, 11H), 1.21(t, 3H, J=7.14 Hz), 0.88(t, 3H, J=7.33 Hz),

**Ethyl {2-isopropyl-4-[({4-[(tetrahydro-2H-pyran-2-yloxy)methyl]-2-[4-(trifluoromethyl)phenyl]-1,3-thiazol-5-yl} methyl)sulfanyl]phenoxy}acetate**

$^1$H NMR ($CDCl_3$) 400MHz δ 7.98(d, 2H, J=8.24 Hz), 7.64(d, 2H, J=8.24 Hz), 7.20(d, 1H, J=2.38 Hz), 7.15(dd, 1H, J=8.42, 2.38 Hz), 6.60(d, 1H, J=8.42 Hz), 4.65(t, 1H, J=3.48 Hz), 4.60(s, 2H), 4.56(d, 1 H, J .09 Hz), 4.38(d, 1 H, J .09 Hz), 4.23(m, 4H), 3.87(m, 1 H), 3.53(m, 1 H), 3.32(m, 1 H), 1.66(m, 6H), 1.26(t, 3H, J=7.14 Hz), 1.15(d, 6H, J=6.96 Hz),

**Ethyl 2-{4-[({4-(2-furylmethyl)-2-[4-(trifluoromethyl)phenyl]-1,3-thiazol-5-yl}methyl)sulfanyl]-2-methylphenoxy}propanoate**

From 5-(chloromethyl)-4-(2-furylmethyl)-2-[4-(trifluoromethyl)phenyl]-1,3-thiazole (0.097g, 0.27 mmol), ethyl 2-{4-[({4-(2-furylmethyl)-2-[4-(trifluoromethyl)phenyl]-1,3-thiazol-5-yl}methyl)sulfanyl]-2-methylphenoxy}propanoate (0.091 g, 60%) was obtained as a white solid.

$^1$H NMR ($CDCl_3$): δ 8.00 (d, 2 H), 7.68 (d, 2 H), 7.23 (m, 2 H), 6.62 (m 2 H), 6.30 (s, 1 H), 6.02 (s, 1 H), 4.76 (q, 1 H), 4.21 (q, 2 H), 4.17 (s, 2 H), 3.98 (s, 2 H), 2.29 (s, 3 H), 1.63 (s, 3 H), 1.24 (t, 3 H); MS *m/z* 562 (M+1).

**Ethyl 2-{4-[({4-(3-furylmethyl)-2-[4-(trifluoromethyl)phenyl]-1,3-thiazol-5-yl}methyl)sulfanyl]-2-methylphenoxy}propanoate**

From 5-(chloromethyl)-4-(3-furylmethyl)-2-[4-(trifluoromethyl)phenyl]-1,3-thiazole (0.172g, 0.48 mmol), ethyl 2-{4-[({4-(3-furylmethyl)-2-[4-(trifluoromethyl)phenyl]-1,3-thiazol-5-yl}methyl)sulfanyl]-2-methylphenoxy}propanoate (0.177g, 65%) was obtained as a white solid.

$^1$H NMR ($CDCl_3$): δ 8.00 (d, 2 H), 7.70 (d, 2 H), 7.28 (m, 2 H), 7.16, (d, 1 H), 6.61 (m, 2 H), 6.31 (s, 1 H), 4.78 (q, 1 H), 4.27 (q, 2 H), 4.18 (s, 2 H), 3.68 (s, 2 H), 2.22 (s, 3 H), 1.68 (s, 3 H), 1.30 (t, 3 H); MS *m/z* 578 (M+1).

**Ethyl 2-{4-[({4-(2-thienylmethyl)-2-[4-(trifluoromethyl)phenyl]-1,3-thiazol-5-yl}methyl)sulfanyl]-2-methylphenoxy}propanoate**

From 5-(chloromethyl)-4-(3-furylmethyl)-2-[4-(trifluoromethyl)phenyl]-1,3-thiazole (0.185g, 0.50 mmol), ethyl 2-{4-[({4-(2-thienylmethyl)-2-[4-(trifluoromethyl)phenyl]-1,3-thiazol-5-yl}methyl)sulfanyl]-2-methylphenoxy}propanoate (0.21g, 73%) was obtained as a yellow solid.

$^1$H NMR ($CDCl_3$): δ 8.01 (d, 2 H), 7.70 (d, 2 H), 7.20 (s, 1H), 7.17 (m, 1 H), 6.93 (m, 1 H), 6.80 (s, 1H), 6.60 (m, 2 H), 4.74 (q, 1 H), 4.20 (q, 2 H), 4.19 (s, 2 H), 4.17 (s, 2 H), 2.29 (s, 3 H), 1.67 (s, 3 H), 1.30 (t, 3 H); MS *m/z* 578 (M+1).

**Ethyl 2-methyl-2-{4-[({4-[4-(trifluoromethoxy)benzyl]-2-[4-(trifluoromethyl)phenyl]-1,3-thiazol-5-yl}methyl) sulfanyl]phenoxy}propanoate**

From 5-(chloromethyl)-4-[4-(trifluoromethoxy)benzyl]-2-[4-(trifluoromethyl)phenyl]-1,3-thiazole (0.166g, 0.37 mmol) (prepared as in U16097-118-2), ethyl 2-methyl-2-{4-[({4-[4-(trifluoromethoxy)benzyl]-2-[4-(trifluoromethyl)phenyl]-1,3-thiazol-5-yl}methyl)sulfanyl]phenoxy}propanoate (0.210g, 87%) was obtained as a white solid.

MS *m/z* 656 (M+1); HPLC RT 4.862 (C18 4.2x100mm, 0-100% ACN/$H_2O$ (0.1% TFA), 6min @ 2ml/min @254/220nm).

**Ethyl 2-methyl-2-{4-[({4-[(4-methyl-2-thienyl)methyl]-2-[4-(trifluoromethyl)phenyl]-1,3-thiazol-5-yl}methyl) sulfanyl]phenoxy}propanoate**

From 5-(chloromethyl)-4-(2-thienylmethyl)-2-[4-(trifluoromethyl)phenyl]-1,3-thiazole (0.062g, 0.16 mmol), ethyl 2-methyl-2-{4-[({4-[(4-methyl-2-thienyl)methyl]-2-[4-(trifluoromethyl)phenyl]-1,3-thiazol-5-yl}methyl)sulfanyl]phenoxy}propanoate (0.17g, 100%) was obtained as a yellow oil.

MS *m/z* 592 (M+1); HPLC RT 4.534 (C18 4.2x100mm, 0-100% ACN/$H_2O$ (0.1% TFA), 6min @ 2ml/min @254/220nm).

**Ethyl {2-methyl-4-[({4-[(4-methyl-2-thienyl)methyl]-2-[4-(trifluoromethyl)phenyl]-1,3-thiazol-5-yl}methyl) sulfanyl]phenoxy}acetate**

From 5-(chloromethyl)-4-(2-thienylmethyl)-2-[4-(trifluoromethyl)phenyl]-1,3-thiazole (0.062g, 0.16 mmol), ethyl {2-methyl-4-[({4-[(4-methyl-2-thienyl)methyl]-2-[4-(trifluoromethyl)phenyl]-1,3-thiazol-5-yl}methyl)sulfanyl]phenoxy}acetate (0.13g, 100%) was obtained as a yellow oil.

MS *m/z* 578 (M+1); HPLC RT 4.338 (C18 4.2x100mm, 0-100% ACN/$H_2O$ (0.1% TFA), 6min @ 2ml/min @254/220nm).

**Ethyl {4-[({4-(2,4-difluorobenzyl)-2-[4-(trifluoromethyl)phenyl]-1,3-thiazol-5-yl}methyl)sulfanyl]-2-methylphenoxy}acetate**

From 5-(chloromethyl)-4-(2,4-difluorobenzyl)-2-[4-(trifluoromethyl)phenyl]-1,3-thiazole (0.139g, 0.34 mmol), ethyl {4-[({4-(2,4-difluorobenzyl)-2-[4-(trifluoromethyl)phenyl]-1,3-thiazol-5-yl}methyl)sulfanyl]-2-methylphenoxy}acetate, (0.1 g, 49%) was obtained as a white solid.

MS *m/z* 594 (M+1); HPLC RT 4.337 (C18 4.2x100mm, 0-100% ACN/$H_2O$ (0.1% TFA), 6min @ 2ml/min @254/220nm).

**Ethyl {4-[({4-(4-methoxybenzyl)-2-[4-(trifluoromethyl)phenyl]-1,3-thiazol-5-yl}methyl)sulfanyl]-2-methylphenoxy}acetate**

From 5-(chloromethyl)-4-(4-methoxybenzyl)-2-[4-(trifluoromethyl)phenyl]-1,3-thiazole (0.09g, 0.4 mmol) (prepared as in U17097-118-3), ethyl {4-[({4-(4-methoxybenzyl)-2-[4-(trifluoromethyl)phenyl]-1,3-thiazol-5-yl}methyl)sulfanyl]-2-methylphenoxy}acetate (0.160g, 68%) was obtained as a white solid.

MS *m/z* 588 (M+1); HPLC RT 4.631 (C18 4.2x100mm, 0-100% ACN/$H_2O$ (0.1% TFA), 6min @ 2ml/min @254/220nm).

**2-Methyl-4-[({4-[(tetrahydro-2*H*-pyran-2-yloxy)methyl]-2-[4-(trifluoromethyl)phenyl]-1,3-thiazol-5-yl}methyl) sulfanyl]phenol**

$^1H$ NMR ($CDCl_3$) 400MHz δ 7.97(d, 2H, J=8.10 Hz), 7.63(d, 2H, J=8.10 Hz), 7.16(d, 1H, J=2.24 Hz), 7.06(dd, 1 H, J=8.28, 2.24 Hz), 6.63(d, 1H, J=8.28 Hz), 4.64(t, 1H, J=3.53 Hz), 4.59(d, 1H, J .24 Hz), 4.40(d, 1H, J .24 Hz), 4.23(s, 2H), 3.86(m, 1H), 3.53(m, 1H), 2.16(s, 3H), 1.66(m, 6H),

**2-Methyl-4-[({4-(4-trifluoromethyl)benzyl]-2-[4-(trifluoromethyl)phenyl]-1,3-thiazol-5-yl}methyl)sulfanyl] phenol**

From 5-(chloromethyl)-4-[4-(trifluoromethyl)benzyl]-2-[4-(trifluoromethyl)phenyl]-1,3-thiazole (0.82g, 0.19 mmol), 2-methyl-4-[({4-(4-trifluoromethyl)benzyl]-2-[4-(trifluoromethyl)phenyl]-1,3-thiazol-5-yl}methyl)sulfanyl]phenol (0.021g, 21 %) was obtained as a white solid.

$^1H$ NMR ($CDCl_3$): δ 8.00 (d, 2 H), 7.69 (d, 2 H), 7.52 (d, 2 H), 729 (d, 2 H), 7.18 (s, 1 H), 7.16 (d 1 H), 6.70 (d, 1 H), 4.15 (s, 2 H), 4.00 (s, 2 H), 2.20 (s, 3 H); MS *m/z* 540 (M+1).

**2-Methyl-4-[({4-(4-trifluoromethoxy)benzyl]-2-[4-(trifluoromethyl)phenyl]-1,3-thiazol-5-yl}methyl)sulfanyl] phenol**

From 5-(chloromethyl)-4-[4-(trifluoromethoxy)benzyl]-2-[4-(trifluoromethyl)phenyl]-1,3-thiazole (0.147g, 0.33 mmol), 2-methyl-4-[({4-(4-trifluoromethoxy)benzyl]-2-[4-(trifluoromethyl)phenyl]-1,3-thiazol-5-yl}methyl)sulfanyl]phenol (0.048g, 27%) was obtained as a white solid.
$^1$H NMR ($CDCl_3$): δ 8.01 (d, 2 H), 7.71 (d, 2H), 7.13 (m, 6 H), 6.69 (d, 1 H), 4.18 (s, 2 H), 3.96 (s, 2 H), 2.22 (s, 3 H); MS *m*/*z* 556 (M+1).

**4-[({4-(4-methoxybenzyl)-2-[4-(trifluoromethyl)phenyl]-1,3-thiazol-5-yl}methyl)sulfanyl]-2-methylphenol**

From 5-(chloromethyl)-4-(4-methoxybenzyl)-2-[4-(trifluoromethyl)phenyl]-1,3-thiazole (0.063g, 0.16 mmol), 4-[({4-(4-methoxybenzyl)-2-[4-(trifluoromethyl)phenyl]-1,3-thiazol-5-yl}methyl)sulfanyl}-2-methylphenol (0.022g, 28%) was obtained as a white solid.
$^1$H NMR ($CDCl_3$): δ 8.00 (d, 2 H), 7.68 (d, 2 H), 7.19 (s, 1 H), 7.09 (m, 3 H), 6.82 (d, 2 H), 6.70 (d, 1H), 4.14 (s, 2 H), 3.90 (s, 2 H), 2.20 (s, 3 H); MS *m*/*z* 502 (M+1).

**2-Methyl-4-[({4-(4-methylsulfanyl)benzyl]-2-[4-(trifluoromethyl)phenyl]-1,3-thiazol-5-yl}methyl)sulfanyl] phenol**

From 5-(chloromethyl)-4-[4-(methylsulfanyl)benzyl]-2-[4-(trifluoromethyl)phenyl]-1,3-thiazole (0.33g, 0.78 mmol), 2-methyl-4-[({4-(4-methylsulfanyl)benzyl]-2-[4-(trifluoromethyl)phenyl]-1,3-thiazol-5-yl}methyl)sulfanyl]phenol (0.296g, 72%) was obtained as a white solid.
MS *m*/*z* 518 (M+1).

**4-[({4-(4-*tert*-butylbenzyl)-2-[4-(trifluoromethyl)phenyl]-1,3-thiazol-5-yl}methyl)sulfanyl]-2-methylphenol**

From 4-(4-*tert*-butylbenzyl)-5-(chloromethyl)-2-[4-(trifluoromethyl)phenyl]-1,3-thiazole (0.151g, 0.36 mmol), 4-[({4-(4-*tert*-butylbenzyl)-2-[4-(trifluoromethyl)phenyl]-1,3-thiazol-5-yl}methyl)sulfanyl]-2-methylphenol (0.113g, 60%) was obtained as a white solid. MS *m*/*z* 528 (M+1).

**2-Methyll-4-[({4-(3-thienylmethyl)-2-[4-(trifluoromethyl)phenyl]-1,3-thiazol-5-yl}methyl)sulfanyl]phenol**

From 5-(chloromethyl)-4-(3-thienylmethyl)-2-[4-(trifluoromethyl)phenyl]-1,3-thiazole (0.105g, 0.28 mmol), 2-methyl-4-[({4-(3-thienylmethyl)-2-[4-(trifluoromethyl)phenyl]-1,3-thiazol-5-yl}methyl)sulfanyl]phenol (0.072g, 54%) was obtained as a yellow oil. MS *m*/*z* 478 (M+1).
The following three compounds were also prepared by the same route but were carried on without purification:

**Ethyl 2-{2-isopropyl-4-[({4-[tetrahydro-2H-pyran-2-yloxy)methyl]-2-[4-(trifluoromethyl)phenyl]-1,3-thiazol-5-yl} methyl)sulfanyl]phenoxy}propanoate**

**4-[({4-[(Tetrahydro-2*H*-pyran-2-yloxy)methyl]-2-[4-(trifluoromethyl)phenyl]-1,3-thiazol-5-yl}methyl)sulfanyl] phenol**

**4-[({2-(4-Fluorophenyl)-4-[(tetrahydro-2*H*-pyran-2-yloxy)methyl]-1,3-thiazol-5-yl}methyl)sulfanyl]-2-methylphenol**

**Ethyl 2-{2-methyl-4-[({4-[4-(trifluoromethoxy)benzyl]-2-[4-(trifluoromethyl)phenyl]-1,3-thiazol-5-yl}methyl) sulfanyl]phenoxy}propanoate**

From 2-methyl-4-[({4-(4-trifluoromethoxy)benzyl]-2-[4-(trifluoromethyl)phenyl]-1,3-thiazol-5-yl}methyl)sulfanyl]phenol (0.17g, 0.31 mmol), ethyl 2-{2-methyl-4-[({4-[4-(trifluoromethoxy)benzyl]-2-[4-(trifluoromethyl)phenyl]-1,3-thiazol-5-yl}methyl)sulfanyl]phenoxy}propanoate (0.17g, 83%) was obtained as a white solid.
MS *m*/*z* 656 (M+1); HPLC RT 4.553 (C18 4.2x100mm, 0-100% ACN/$H_2O$ (0.1% TFA), 6min @ 2ml/min @254/220nm).

**Methyl {2-methyl-4-[({4-[4-(trifluoromethoxy)benzyl]-2-[4-(trifluoromethyl)phenyl]-1,3-thiazol-5-yl}methyl) sulfanyl]phenoxy}acetate**

From 2-methyl-4-[({4-(4-trifluoromethoxy)benzyl]-2-[4-(trifluoromethyl)phenyl]-1,3-thiazol-5-yl}methyl)sulfanyl]phenol (0.17g, 0.31 mmol), methyl {2-methyl-4-[({4-[4-(trifluoromethoxy)benzyl]-2-[4-(trifluoromethyl)phenyl]-1,3-thiazol-5-yl}methyl)sulfanyl]phenoxy}acetate (0.15g, 80%) was obtained as a white solid. MS *m/z* 628 (M+1); HPLC RT 4.398 (C18 4.2x100mm, 0-100% ACN/$H_2O$ (0.1% TFA), 6min @ 2ml/min @254/220nm).

**Ethyl 2-{2-methyl-4-[({4-(3-thienylmethyl)-2-[4-(trifluoromethyl)phenyl]-1,3-thiazol-5-yl}methyl)sulfanyl] phenoxy}propanoate**

From 2-methyl-4-[({4-(3-thienylmethyl)-2-[4-(trifluoromethyl)phenyl]-1,3-thiazol-5-yl}methyl)sulfanyl]phenol, ethyl 2-{2-methyl-4-[({4-(3-thienylmethyl)-2-[4-(trifluoromethyl)phenyl]-1,3-thiazol-5-yl}methyl)sulfanyl]phenoxy}propanoate (0.225g, 0.47 mmol), (0.255g, 91 %) was obtained as a yellow oil.
MS *m/z* 578 (M+1); HPLC RT 4.412 (C18 4.2x100mm, 0-100% ACN/$H_2O$ (0.1% TFA), 6min @ 2ml/min @254/220nm).

**Methyl {2-methyl-4-[({4-(3-thienylmethyl)-2-[4-(trifluoromethyl)phenyl]-1,3-thiazol-5-yl}methyl)sulfanyl] phenoxy}acetate**

From 2-methyl-4-[({4-(3-thienylmethyl)-2-[4-(frifluoromethyl)phenyl]-1,3-thiazol-5-yl}methyl)sulfanyl]phenol, methyl {2-methyl-4-[({4-(3-thienylmethyl)-2-[4-(trifluoromethyl)phenyl]-1,3-thiazol-5-yl}methyl)sulfanyl]phenoxy}acetate (0.225g, 0.47 mmol), (0.259g, 94%) was obtained as a yellow oil.
MS *m/z* 550 (M+1); HPLC RT 4.243 (C18 4.2x100mm, 0-100% ACN/$H_2O$ (0.1 % TFA), 6min @ 2ml/min @254/220nm).

**Ethyl 2-{4-[({4-(hydroxymethyl)-2-[4-(trifluoromethyl)phenyl]-1,3-thiazol-5-yl}ethyl)sulfanyl]phenoxy}-2-methylpropanoate**

To a stirred solution of crude ethyl {2-methyl-4-[({4-[(tetrahydro-2H-pyran-2-yloxy)methyl]-2-[4-(trifluoromethyl)phenyl]-1,3-thiazol-5-yl}methyl)sulfanyl]phenoxy}acetate (11.98g, 20.09mmoles, 1 eq) in MeOH (100ml, 0.20M) was added as a solid *p*-toluenesulfonic acid (800mg, 25mol%) at room temperature. The reaction mixture was stirred at room temperature for 3 hours. The MeOH was removed *in vacuo* and the residue was purified by silica gel chromatography (15% EtOAc/Hexanes to 30% EtOAc/Hexanes) to yield 8g (78%) of pure titled alcohol.
$^1H$ NMR ($CDCl_3$) 400MHz δ 7.96(d, 2H, J=8.06 Hz), 7.65(d, 2H, J=8.06 Hz), 7.23(d, 2H, J=8.79 Hz), 6.73(d, 2H. J=8.79 Hz), 4.44(s, 2H), 4.17(m, 4H), 2.33(br s, 1 H), 1.56(s, 6H), 1.21 (t, 3H, J=7.14 Hz).
TLC(30% EtOAc/Hexanes) $R_f$ = 0.32

**4-[({4-(Hydroxymethyl)-2-[4-(trifluoromethyl)phenyl]-1,3-thiazol-5-yl}methyl)sulfanyl]-2-methylphenol**

$^1H$ NMR ($CDCl_3$) 400MHz δ 7.95(d, 2H, J=7.93 Hz), 7.64(d, 2H, J=7.93 Hz), 7.15(d, 1H, J=2.07 Hz), 6.98(dd, 1H, J=8.10, 2.07 Hz), 6.62(d, 1 H, J=8.10 Hz), 4.39(s, 2H), 4.11 (s, 2H), 2.14(s, 3H),

**Ethyl 2-{4-[({4-(hydroxymethyl)-2-[4-(trifluoromethyl)phenyl]-1,3-thiazol-5-yl}methyl)sulfanyl]-2-propylphenoxy}propanoate**

$^1H$ NMR ($CDCl_3$) 400MHz δ 7.97(d, 2H, J=8.06 Hz), 7.66(d, 2H, J=8.06 Hz), 7.13(d, 1H, J=2.38 Hz), 7.10(dd, 1H, J=8.24, 2.38 Hz), 6.55(d, 1H, J=8.24 Hz), 4.70(q, 1H, J=6.78 Hz), 4.43(s, 2H), 4.14(m, 4H), 2.55(t, 2H, J=7.33 Hz), 2.19(br s, 1H), 1.55(m, 5H), 1.21(t, 3H, J=7.14 Hz), 0.85(t, 3H, J=7.33 Hz),

**Methyl {4-[({4-(hydroxymethyl)-2-[4-(trifluoromethyl)phenyl]-1,3-thiazol-5-yl}methyl)sulfanyl]-2-isopropylphenoxy}acetate**

$^1H$ NMR ($CDCl_3$) 400MHz δ 7.97(d, 2H, J=8.42 Hz), 7.66(d, 2H, J=8.42 Hz), 7.15(m, 2H), 6.60(d, 1H, J=8.79 Hz), 4.64(s, 2H), 4.38(s, 2H), 4.15(s, 2H), 3.77(s, 3H), 3.31 (m, 1H), 2.03(br s, 1H), 1.12(d, 6H, J=6.96 Hz),

**Ethyl 2-{4-[({4-(hydroxymethyl)-2-[4-(trifluoromethyl)phenyl]-1,3-thiazol-5-yl}methyl)sulfanyl]-2-isopropylphenoxy}propanoate**

$^1$H NMR ($CDCl_3$) 400MHz δ 7.97(d, 2H, J=8.24 Hz), 7.66(d, 2H, J=8.24 Hz), 7.15(d, 1H, J=2.38 Hz), 7.11 (dd, 1 H, J=8.42, 2.38 Hz), 6.56(d, 1H, J=8.42 Hz), 4.73(q, 1 H, J=6.78 Hz), 4.38(s, 2H), 4.14(m, 4H), 3.30(m, 1H), 1.60(d, 3H, J=6.78 Hz), 1.17(m, 9H),

**Ethyl 2-{4-[({4-(hydroxymethyl)-2-[4-(trifluoromethyl)phenyl]-1,3-thiazol-5-yl}methyl)sulfanyl]-2-methylphenoxy}propanoate**

$^1$H NMR ($CDCl_3$) 300MHz δ 8.00(d, 2H, J=8.23 Hz), 7.69(d, 2H, J=8.23 Hz), 7.22(d, 1H, J=2.39 Hz), 7.12(dd, 1H, J=8.23, 2.39 Hz), 6.59(d, 1H, J=8.23 Hz), 4.74(q, 1H, J=6.77 Hz), 4.51(s, 2H), 4.19(m, 4H), 3.68(br s, 1H), 2.26 (s, 3H), 1.65(d, 3H, J=6.77 Hz), 1.26(t, 3H, J=7.17 Hz),

TLC(50% EtOAc/Hexanes) $R_f$ = 0.40

The following four compounds were deprotected as above but used without further purification:

**Ethyl 2-[4-({[2-(4-fluorophenyl)-4-(hydroxymethyl)-1,3-thiazol-5-yl]methyl}sulfanyl)-2-methylphenoxy] propanoate**

**Ethyl {2-ethyl-4-[({4-(hydroxymethyl)-2-[4-(trifluoromethyl)phenyl]-1,3-thiazol-5-yl}methyl)sulfanyl]phenoxy} acetate**

**Ethyl 2-{2-ethyl-4-[({4-(hydroxymethyl)-2-[4-(trifluoromethyl)phenyl]-1,3-thiazol-5-yl}methyl)sulfanyl] phenoxy}propanoate**

**Ethyl {4-[({4-(hydroxymethyl)-2-[4-(trifluoromethyl)phenyl]-1,3-thiazol-5-yl}methyl)sulfanyl]-2-propylphenoxy} acetate**

**Ethyl {[tert-butyl(diphenyl)silyl]oxy}acetate**

To a 500ml round-bottom flask equipped with a magnetic stir-bar, $N_2$ inlet was added ethyl glycolate (10g, 96.0mmoles, 1 eq) and dry $CH_2Cl_2$ (200ml, 0.5M). This was followed by the addition of triethylamine (40ml, 0.288moles, 3eq) and DMAP (1.17g, 9.6mmoles, 10mol%) followed by the dropwise addition of TBDPSCl (27.5ml, 0.106moles, 1.1eq) in dry $CH_2Cl_2$ (20ml). The reaction mixture was allowed to stir at room temperature overnight at which time the reaction mixture was diluted with $CH_2Cl_2$ and washed with 1N HCl, saturated sodium bicarbonate, $H_2O$ and dried over $Na_2SO_4$. After filtration the volatiles were removed *in vacuo* to yield 30g (91%) of titled compound.

$^1$H NMR ($CDCl_3$) 300MHz δ 7.69(m, 4H), 7.39(m, 6H), 4.23(s, 2H), 4.14(q, 2H, J=7.14 Hz), 1.22(t, 3H, J=7.14 Hz), 1.08(m, 9H),

TLC(20% EtOAc/Hexanes) $R_f$= 0.67

**{[tert-Butyl(diphenyl)silyl]oxy}acetic acid**

To a stirred solution of ethyl {[tert-butyl(diphenyl)silyl]oxy}acetate (20g, 58.4mmoles, 1eq) in THF (100ml, 0.58M) was added 1N NaOH (6ml, 0.117moles, 2eq) and was allowed to stir at room temperature overnight. The THF was removed *in vacuo* and the residue was partitioned between $CH_2Cl_2$ and 1 N HCl until a pH of 2 was reached. The phases were separated and the aqueous phase was washed twice with $CH_2Cl_2$. The combined organic fractions were dried over $Na_2SO_4$, filtered and concentrated *in vacuo* to yield 17g (90%) of product.

$^1$H NMR ($CDCl_3$) 300MHz δ 7.68(m, 4H), 7.41 (m, 6H), 4.22(s, 2H), 1.11(s, 9H),

TLC(5% MeOH/$CH_2Cl_2$) $R_f$= 0.37

**{[tert-Butyl(diphenyl)silyl]oxy}acetyl chloride**

In a 500ml round-bottom flask was mixed {[tert-butyl(diphenyl)silyl]oxy}acetic acid (17g, 54.0mmoles, 1eq), thionyl chloride (11.7g, 0.162moles, 3eq) and dry $CH_2Cl_2$ (120ml, 0.45M). This mixture was refluxed for 5 hours. After cooling to room temperature the volatiles were removed *in vacuo*. The resulting residue was washed twice with toluene and the toluene was subsequently removed *in vacuo* to remove excess thionyl chloride. This resulted in 18g (100%) of titled compound.

$^1$H NMR ($CDCl_3$) 300MHz δ 7.72(m, 4H), 7.44(m, 6H), 4.54(s, 2H), 1.11(m, 9H),

**Ethyl 4-{[tert-butyl(diphenyl)silyl]oxy}-3-oxobutanoate**

To a 1-L round-bottom flask equipped with a magnetic stir-bar, addition funnel, low temperature thermometer with thermometer adapter and a $N_2$ inlet was added monoethyl malonate (14.53g, 0.11 moles, 2eq) in dry THF (150ml, 0.73M) and 20mg of 2,2'-dipyridyl. After cooling the reaction mixture to -78°C (dry ice/acetone), *n*-BuLi (2.5M in Hexanes, 88ml, 0.22moles, 4eq) was added at a rate to maintain the internal temperature below -10°C. Once the addition was complete the reaction was allowed to warm to -10°C by removal of the cold bath. The reaction remained a light pink color; this designates that there was ample amount of *n*-BuLi to deprotonate the monoethyl malonate. (If the color had turned yellow the reaction would have had to have been re-cooled to - 78°C and additional *n*-BuLi would have had to have been added followed be re-warming to -10°C.) At this point the reaction mixture was cooled to -78°C followed by the dropwise addition of neat {[tert-Butyl(diphenyl)silyl]oxy}acetyl chloride (18g, 54mmoles, 1eq) over a period of 15 minutes maintaining the internal reaction temperature below -60°C. This was allowed to stir at -78°C for 10 minutes at which point the reaction was transferred to a separatory funnel containing diethyl ether (900ml) and 1N HCl (450ml). This was agitated and vented until further gas evolution ceased after which the phases were separated and the organic phase was washed with saturated sodium bicarbonate, brine and dried over $Na_2SO_4$. This was then filtered, concentrated *in vacuo* and purified by silica gel chromatography (5% EtOAc/Hexanes to 20% EtOAc/Hexanes) to yield 12.2g (60%) of product.

$^1H$ NMR ($CDCl_3$) 300MHz δ 7.63(m, 4H), 7.41(m, 6H), 4.19(m, 4H), 3.63(s, 2H), 1.27(t, 3H, J=7.14 Hz), 1.08 (s, 9H),

TLC(20% EtOAc/Hexanes) $R_f$= 0.53

The following compounds were made according to W. Wierenga (J.Org.Chem. 1979 vol 44 p 310):

**Ethyl 4-(4-bromophenyl)-3-oxobutanoate**

$^1H$ NMR ($CDCl_3$) 300MHz δ 7.45(d, 2H, J=8.38 Hz), 7.10(d, 2H, J=8.38 Hz), 4.17(q, 2H, J=7.14 Hz), 3.79(s, 2H), 3.45(s, 2H), 1.26(t, 3H, J=7.14 Hz),

**Ethyl 3-oxo-4-(2-phenylethoxy)butanoate**

$^1H$ NMR ($CDCl_3$) 300MHz δ 7.26(m, 5H), 4.15(q, 4H, J=7.14 Hz), 3.71(t, 2H, J=6.94 Hz), 3.46(s, 2H), 2.92(t, 2H, J=6.94 Hz), 1.27(t, 3H, J=7.14 Hz),

**Ethyl 3-oxo-6-phenylhexanoate**

$^1H$ NMR ($CDCl_3$) 300MHz δ 7.22(m, 5H), 4.18(q, 2H, J=7.14 Hz), 3.39(s, 2H), 2.62(t, 2H, J=7.28 Hz), 2.53(t, 2H, J=7.28 Hz), 1.92(m, 2H), 1.25(t, 3H, J=7.14 Hz),

**Ethyl 3-oxo-4-phenylbutanoate**

$^1H$ ($CDCl_3$) 300MHz 7.29(m, 5H), 4.18(q, 2H, J=7.14 Hz), 3.83(s, 2H), 3.44(s, 2H), 1.26(t, 3H, J=7.14 Hz),

TLC(20% EtOAc/Hexanes) $R_f$= 0.36

**Ethyl 4-(benzyloxy)-3-oxobutanoate**

$^1H$ ($CDCl_3$) 300MHz 7.35(m, 5H), 4.59(s, 2H), 4.16(q, 4H, J=7.14 Hz), 3.53(s, 2H), 1.26(t, 3H, J=7.14 Hz),

**Ethyl 3-oxo-5-phenylpentanoate**

$^1H$ NMR ($CDCl_3$) 300MHz 7.24(m, 5H), 4.18(q, 2H, J=7.14 Hz), 3.42(s, 2H), 2.90(m, 4H), 1.27(t, 3H, J=7.14 Hz)

**Ethyl 4-{[tert-butyl(diphenyl)silyl]oxy}-2-chloro-3-oxobutanoate**

To a 100ml round-bottom flask equipped with a magnetic stir-bar and a $N_2$ inlet was added ethyl 4-{[tert-butyl (diphenyl)silyl]oxy}-3-oxobutanoate (4g, 10.4mmoles, 1eq) and dry $CH_2Cl_2$ (25ml, 0.42M) at room temperature. This was followed by the addition of neat sulfuryl chloride (0.833ml, 10.4mmoles, 1eq) and the reaction was allowed to stir overnight at room temperature. After dilution with $CH_2Cl_2$ (50ml) the reaction mixture was treated with saturated sodium bicarbonate until bubbling ceased. The phases were separated and the organic fraction was washed with sat. $NaHCO_3$,

brine and dried over $Na_2SO_4$. After filtration and concentration *in vacuo* was yielded 4.2g (96%) of crude chloride. This crude product was used without purification.

$^1H$ NMR ($CDCl_3$) 400MHz δ 7.62(m, 4H), 7.41 (m, 6H). 5.26(s, 1H), 4.40(m, 2H), 4.25(m, 2H), 1.28(t, 3H, J=7.14 Hz), 1.09(s, 9H),

The following intermediates were made by the same procedure as that used for Ethyl 4-{[tert-butyl(diphenyl) silyl]oxy}-2-chloro-3-oxobutanoate

**<u>Ethyl 4-(benzyloxy)-2-chloro-3-oxobutanoate</u>**

$^1H$ ($CDCl_3$) 300MHz δ 7.36(m, 5H), 5.10(s, 1H), 4.59(s, 2H), 4.32(s, 2H), 4.23(q, 2H, J=7.23 Hz), 1.28(t, 3H, J=7.14 Hz),

**<u>Ethyl 2-chloro-3-oxo-6-phenylhexanoate</u>**

$^1H$ ($CDCl_3$) 300MHz δ 7.23(m, 5H), 4.75(s, 1 H), 4.27(q, 2H, J=7.14 Hz), 2.72(t, 2H, J=7.28 Hz), 2.63(t, 2H, J=7.28 Hz), 1.97(m, 2H, J=7.28 Hz), 1.28(t, 3H, J=7.14 Hz),

**<u>Ethyl 2-chloro-3-oxo-4-(2-phenylethoxy)butanoate</u>**

$^1H$ NMR ($CDCl_3$) 300MHz δ 7.25(m, 5H), 5.03(s, 1H), 4.29(m, 2H), 4.24(q, 2H, J=7.14 Hz), 3.73(t, 2H, J=7.00 Hz), 2.91 (t, 2H, J=7.00 Hz), 1.29(t, 3H, J=7.14 Hz),

**<u>Ethyl 2-chloro-3-oxo-4-phenylbutanoate</u>**

$^1H$ ($CDCl_3$) 300MHz 7.29(m, 5H), 4.87(s, 1H), 4.23(m, 2H, J=7.14, 7.00, 7.14, 1.10, 1.24, 1.24, 0.82 Hz), 4.02 (d, 2H, J=4.53 Hz), 1.31(t, 3H, J=7.14 Hz),

TLC(20% EtOAc/Hexanes) $R_f$= 0.51

**<u>Ethyl 2-chloro-3-oxo-5-phenylpentanoate</u>**

$^1H$ ($CDCl_3$) 300MHz 7.25(m, 5H), 4.76(s, 1H), 4.25(q, 2H, J=7.14 Hz), 2.99(m, 4H), 1.31(t. 3H, J=7.14 Hz),

TLC(20% EtOAc/Hexanes) $R_f$= 0.46

**<u>Ethyl 4-(4-bromophenyl)-2-chloro-3-oxobutanoate</u>**

$^1H$ NMR ($CDCl_3$) 300MHz δ 7.48(d, 2H, J=8.51 Hz), 7.10(d, 2H, J=8.51 Hz), 4.84(s, 1H), 4.25(q, 2H, J=7.14 Hz), 3.97(s, 2H), 1.29(t, 3H, J=7.14 Hz),

TLC(20% EtOAc/Hexanes) $R_f$= 0.58

**<u>Ethyl 4-({[tert-butyl(diphenyl)silyl]oxy}methyl)-2-(4-{trifluoromethyl}phenyl)-1,3-thiazole-5-carboxylate</u>**

To a 500ml round-bottom flask equipped with a magnetic stir-bar was mixed ethyl 4-{[tert-butyl(diphenyl)silyl] oxy}-2-chloro-3-oxobutanoate (20.4g, 52.88mmoles, 1eq), 4-trifluoromethylthiobenzamide (12.2g, 59.5mmoles, 1.1eq), 1,2-dichloroethane (150ml, 0.44M) and $H_2O$ (3ml). This mixture was refluxed for 12 hrs. After cooling to room temperature, the reaction mixture was diluted with $CH_2Cl_2$ (100ml) and washed with sat. $NaHCO_3$. Once the phases were separated, the organic phase was washed with water, brine and dried over $Na_2SO_4$. This was then filtered, concentrated *in vacuo* and purified via silica gel chromatography (5% EtOAc/Hexanes to 20% EtOAc/Hexanes) to yield 20.3g (76%) of the titled compound.

$^1H$ NMR ($CDCl_3$) 400MHz δ 8.07(d, 2H, J=8.37 Hz), 7.76(m, 4H), 7.71(d, 2H, J=8.37 Hz), 7.37(m, 6H), 5.24 (s, 2H), 4.26(q, 2H, J=7.18 Hz), 1.29(t, 3H, J=7.18 Hz), 1.11(s, 9H),

TLC(20% EtOAc/Hexanes) $R_f$ = 0.72

**<u>Ethyl 4-({[tert-butyl(diphenyl)silyl]oxy}methyl)-2-phenyl-1,3-thiazole-5-carboxylate</u>**

Analogous procedure to that used for ethyl 4-({[*tert*-butyl(diphenyl)silyl]oxy}methyl)-2-(4-{trifluoromethyl}phenyl)-1,3-thiazole-5-carboxylate except thiobenzamide is the starting material.

$^1H$ NMR ($CDCl_3$) 400MHz δ 7.98(m, 2H), 7.76(m, 4H), 7.40(m, 9H), 5.21 (s, 2H), 4.23(q, 2H, J=7.12 Hz), 1.28 (t, 3H, J=7.12 Hz), 1.08(s, 9H),

TLC(20% EtOAc/Hexanes) $R_f$ = 0.67

The following intermediates were made using the same procedure as Ethyl 4-({[tert-butyl(diphenyl)silyl]oxy}methyl)-2-(4-{trifluoromethyl}phenyl)-1,3-thiazole-5-carboxylate:

**Ethyl 2-(4-{trifluoromethyl}phenyl)-4-[(2-phenylethoxy)methyl]-1,3-thiazole-5-carboxylate**

$^1H$ ($CDCl_3$) 300MHz δ 8.10(d, 2H, J=8.79 Hz), 7.71(d, 2H, J=8.79 Hz), 7.23(m, 5H), 5.02(s, 2H), 4.37(q, 2H, J=7.14 Hz), 3.86(t, 2H, J=7.42 Hz), 2.99(t, 2H, J=7.42 Hz), 1.41 (t, 3H, J=7.14 Hz),

**Ethyl 2-(4-{trifluoromethyl}phenyl)-4-(3-phenylpropyl)-1,3-thiazole-5-carboxylate**

$^1H$ ($CDCl_3$) 300MHz δ 8.08(d, 2H, J=8.24 Hz), 7.71(d, 2H, J=8.24 Hz), 7.23(m, 5H), 4.34(q, 2H, J=7.14 Hz), 3.25(t, 2H, J=7.69 Hz), 2.71 (t, 2H, J=7.69 Hz), 2.13(m, 2H), 1.35(t, 3H, J=7.14 Hz),

**Ethyl 4-[(benzyloxy)methyl]-2-(4-{trifluoromethyl}phenyl)-1,3-thiazole-5-carboxylate**

$^1H$ ($CDCl_3$) 300MHz δ 8.12(d, 2H, J=8.79 Hz), 7.72(d, 2H, J=8.79 Hz), 7.35(m, 5H), 5.04(s, 2H), 4.74(s, 2H), 4.36(q, 2H, J=7.10 Hz), 1.38(t, 3H, J=7.14 Hz),

TLC(20% EtOAc/Hexanes) $R_f$= 0.49

**Ethyl 4-(4-bromobenzyl)-2-(4-{trifluoromethyl}phenyl)-1,3-thiazole-5-carboxylate**

$^1H$ NMR ($CDCl_3$) 300MHz δ 8.07(d, 2H, J=8.79 Hz), 7.69(d, 2H, J=8.79 Hz), 7.43(d, 2H, J=8.51 Hz), 7.28(d, 2H, J=8.51 Hz), 4.51 (s, 2H), 4.38(q, 2H, J=7.14 Hz), 1.39(t, 3H, J=7.14 Hz),

TLC(20% EtOAc/Hexanes) $R_f$= 0.66

**Ethyl 4-(2-phenylethyl)-2-[4-(trifluoromethyl)phenyl]-1,3-thiazole-5-carboxylate**

$^1H$ ($CDCl_3$) 300MHz 8.10(d, 2H, J=8.79 Hz), 7.72(d, 2H, J=8.79 Hz), 7.24(m, 5H), 4.37(q, 2H, J=7.14 Hz), 3.51 (m, 2H), 3.10(m, 2H), 1.40(t, 3H, J=7.14 Hz),

MS($ES^+$) M+H= 405.99

**Ethyl 4-benzyl-2-[4-(trifluoromethyl)phenyl]-1,3-thiazole-5-carboxylate**

$^1H$ ($CDCl_3$) 300MHz 8.08(d, 2H, J=8.79 Hz), 7.70(d, 2H, J=8.79 Hz), 7.42(d, 2H, J=9.61 Hz), 7.23(m, 3H), 4.58(s, 2H), 4.38(q, 2H, J=7.14 Hz), 1.39(t, 3H, J=7.14 Hz),

TLC(20% EtOAc/Hexanes) $R_f$= 0.57

MS($ES^+$) M+H= 391.9

**{4-({[tert-Butyl(diphenyl)silyl]oxy}methyl)-2-[4-(trifluoromethyl)phenyl]-1,3-thiazol-5-yl}methanol**

Analogous reduction as in the synthesis of 4-[(tetrahydro-2*H*-pyran-2-yloxy)methyl]-2-[4-(trifluoromethyl)phenyl]-1,3-thiazol-5-yl}methanol.

$^1H$ NMR ($CDCl_3$) 400MHz δ 7.97(d, 2H, J=8.03 Hz), 7.68(m, 6H), 7.41(m, 6H), 4.97(s, 2H), 4.84(s, 2H), 1.08 (s, 9H),

**[4-({[tert-Butyl(diphenyl)silyl]oxy}methyl)-2-phenyl-1,3-thiazol-5-yl]methanol**

Analogous reduction as in the synthesis of 4-[(tetrahydro-2*H*-pyran-2-yloxy)methyl]-2-[4-(trifluoromethyl)phenyl]-1,3-thiazol-5-yl}methanol.

$^1H$ NMR ($CDCl_3$) 300MHz δ 7.90(m, 2H), 7.75(m, 4H), 7.45(m, 9H), 5.00(s, 2H), 4.86(s, 2H), 1.13(s, 9H),

The following compounds were all made by the general alkylation procedure with the appropriate thiols made above and the alkyl halides made from either {4-({[*tert*-Butyl(diphenyl)silyl]oxy}methyl)-2-[4-(trifluoromethyl)phenyl]-1,3-thiazol-5-yl}methanol or {4-({[*tert*-Butyl(diphenyl)silyl]oxy}methyl)-2-[4-(trifluoromethyl)phenyl]-1,3-thiazol-5-yl}methanol via the chlorides as described above.

**Ethyl [4-({[4-({[tert-butyl(diphenyl)silyl]oxy}methyl)-2-phenyl-1,3-thiazol-5-yl]methyl}sulfanyl)-2-methylphenoxy]acetate**

$^1H$ NMR ($CDCl_3$) 400MHz δ 7.85(m, 2H), 7.68(m, 4H), 7.39(m, 9H), 7.12(d, 1H, J=2.39 Hz), 7.03(dd, 1H, J=8.37, 2.39 Hz), 6.50(d, 1H, J=8.37 Hz), 4.61(s, 2H), 4.55(s, 2H), 4.24(q, 2H, J=7.12 Hz), 4.10(s, 2H), 2.18(s, 3H), 1.26(t, 3H, J=7.12 Hz), 1.05(s, 9H),

TLC(20% EtOAc/Hexanes) $R_f$ = 0.43

**Ethyl 2-{4-[({4-({[tert-butyl(diphenyl)silyl]oxy}methyl)-2-[4-(trifluoromethyl)phenyl]-1,3-thiazol-5-yl}methyl) sulfanyl]-2-methylphenoxy}propanoate**

$^1H$ NMR ($CDCl_3$) 400MHz δ 7.94(d, 2H, J=8.20 Hz), 7.67(m, 6H), 7.39(m, 6H), 7.11(d, 1H, J=2.39 Hz), 7.00 (dd, 1H, J=8.37, 2.39 Hz), 6.49(d, 1H, J=8.37 Hz), 4.65(m, 2H), 4.17(q, 2H, J=7.18 Hz), 4.09(s, 2H), 2.17(s, 3H), 1.60 (d, 3H, J=6.84 Hz), 1.21(t, 3H, J=7.18 Hz), 1.05(s, 9H),

TLC(20% EtOAc/Hexanes) $R_r$ = 0.57

**Ethyl 2-[4-({[4-({[tert-butyl(diphenyl)silyl]oxy}methyl)-2-phenyl-1,3-thiazol-5-yl]methyl}sulfanyl)-2-methylphenoxy]propanoate**

$^1H$ NMR ($CDCl_3$) 400MHz δ 7.85(m, 2H), 7.68(m, 4H), 7.38(m, 9H), 7.11(d, 1H, J=2.39 Hz), 6.99(dd, 1H, J=8.55, 2.39 Hz), 6.49(d, 1H, J=8.55 Hz), 4.64(m, 3H), 4.16(q, 2H, J=7.12 Hz), 4.07(s, 2H), 2.17(s, 3H), 1.59(d, 3H, J=6.84 Hz), 1.20(t, 3H, J=7.12 Hz), 1.05(m, 9H),

TLC(20% EtOAc/Hexanes) $R_f$ = 0.48

**Ethyl {4-[({3-({[tert-butyl(diphenyl)silyl]oxy}methyl)-5-[4-(trifluoromethyl)phenyl]-2-thienyl}methyl)sulfanyl]-2-methylphenoxy}acetate**

$^1H$ NMR ($CDCl_3$) 400MHz δ 7.94(d, 2H, J=8.20 Hz), 7.66(m, 6H), 7.38(m, 6H), 7.11(d, 1H, J=2.22 Hz), 7.03 (dd, 1H, J=8.37, 2.22 Hz), 6.50(d, 1H, J=8.37 Hz), 4.63(s, 2H), 4.56(s, 2H), 4.23(q, 2H, J=7.12 Hz), 4.10(s, 2H), 2.18 (s, 3H), 1.27(t, 3H, J=7.12 Hz), 1.04(s, 9H),

TLC(20% EtOAc/Hexanes) $R_f$ = 0.50

**Ethyl [4-({[4-(hydroxymethyl)-2-(4-{trifluoromethyl}phenyl)-1,3-thiazol-5-yl]methyl}sulfanyl)-2-methylphenoxy] acetate**

$^1H$ NMR ($CDCl_3$) 300MHz δ 7.97(d, 2H, J=8.23 Hz), 7.67(d, 2H, J=8.23 Hz), 7.22(d, 1H, J=2.39 Hz), 7.14(dd, 1H, J=8.23, 2.39 Hz), 6.61(d, 1H, J=8.23 Hz), 4.63(s, 2H), 4.50(s, 2H), 4.26(q, 2H, J=7.17 Hz), 4.18(s, 2H), 2.83(s, 1H), 2.25(s, 3H), 1.29(t, 3H, J=7.17 Hz),

TLC(50% EtOAc/Hexanes) $R_f$= 0.51

**(4-Bromophenyl)acetyl chloride**

To a stirred solution of 4-bromophenylacetic acid (10g, 46.5mmoles, 1 eq) in dry $CH_2Cl_2$ (100ml, 0.47M) was added thionyl chloride (20.2ml, 0.280moles, 6eq) and refluxed for 36 hours. After cooling to room temperature the reaction was concentrated *in vacuo* to yield 10.86g (100%) of acid chloride.

$^1H$ ($CDCl_3$) 300MHz δ 7.50(d, 2H, J=8.38 Hz), 7.14(d, 2H, J=8.38 Hz), 4.09(s, 2H),

**4-Phenylbutanoyl chloride**

$^1H$ NMR ($CDCl_3$) 300MHz δ 7.25(m, 5H), 2.90(t, 2H, J=7.28 Hz), 2.69(t, 2H, J=7.28 Hz), 2.05(m, 2H),

**(2-Phenylethoxy)acetyl chloride**

$^1H$ NMR ($CDCl_3$) 300MHz δ 7.26(m, 5H), 4.39(s, 2H), 3.80(t, 2H, J=6.94 Hz), 2.93(t, 2H, J=6.94 Hz)

**[4-([1,1'-Biphenyl]-4-ylmethyl)-2-(4-{trifluoromethyl}phenyl)-1,3-thiazol-5-yl]methanol**

To a stirred solution of [4-(4-Bromobenzyl)-2-(4-{trifluoromethyl}phenyl)-1,3-thiazol-5-yl]methanol (0.33g,

0.78mmoles, 1eq) in dry 1,2-dimethoxyethane (5ml, 0.16M) was added tetrakis(triphenylphosphino) palladium I (0.45g, 0.39mmoles, 0.5eq) and stirred for 5 minutes at room temperature. Phenylboronic acid (0.143g, 1.2mmoles, 1.5eq) was then added followed by the addition of sodium carbonate (2M aqueous solution, 2.3ml, 4.68mmoles, 6eq). The reaction mixture was heated at 100 degrees centigrade for 13 hours at which point, after cooling to room temperature, the reaction was partitioned between EtOAc and water. After separation of the phases the organic phase was washed with brine, dried over anhydrous sodium sulfate, filtered, concentrated *in vacuo* to yield after purification by silica gel chromatography ($CH_2Cl_2$ to 2% MeOH/$CH_2Cl_2$) 268mg (80%) of product.

$^1$H NMR ($CDCl_3$) 400MHz δ 8.03(d, 2H, J=8.20 Hz), 7.67(d, 2H, J=8.20 Hz), 7.54(m, 4H), 7.36(m, 5H), 4.85 (s, 2H), 4.22(s, 2H),

The following intermediate was prepared in using the same procedure:

**{2-(4-{trifluoromethyl}phenyl)-4-[4-(3-thienyl)benzyl]-1,3-thiazol-5-yl}methanol**

$^1$H NMR ($CDCl_3$) 400MHz δ 8.03(d, 2H, J=8.20 Hz), 7.67(d, 2H, J=8.20 Hz), 7.52(d, 2H, J=8.37 Hz), 7.35(m, 5H), 4.84(s, 2H), 4.20(s, 2H),

The following componds were made by the same prcedure for phenol alkylation.

**Ethyl {2-methyl-4-[({4-[(tetrahydro-2H-pyran-2-yloxy)methyl]-2-[4-(trifluoromethyl)phenyl]-1,3-thiazol-5-yl}methyl)sulfanyl]phenoxy}acetate**

To a 250ml round-bottom flask equipped with a magnetic stir-bar and $N_2$ inlet was added 5-(chloromethyl)-4-[(tetrahydro-2H-pyran-2-yloxy)methyl]-2-[4-(trifluoromethyl)phenyl]-1,3-thiazole (7.87g, 20.09mmoles, 1eq) and dry $CH_3CN$ (100ml, 0.27M). Solid cesium carbonate (16.4g, 50.22mmoles, 2.5eq) was added all at once followed by the quick addition of ethyl 2-methyl-2-(4-sulfanylphenoxy)propanoate (5.79g, 24.11mmoles, 1.2eq) in dry $CH_3CN$ (10ml). The reaction was allowed to stir at room temperature for 2 hours at which point the solvent was removed under reduced pressure. The resulting residue was partitioned between EtOAc and 1 N NaOH. After the phases were separated the organic fraction was washed with $H_2O$, brine and dried over $Na_2SO_4$. After filtration the volatiles were removed *in vacuo* to yield the titled compound in >100% yield. Because of the difficult separation between the thiophenol and the product, the crude product was carried forward without purification.

**4-[({4-(Bromomethyl)-2-[4-(trifluoromethyl)phenyl]-1,3-thiazol-5-yl}methyl)sulfanyl]-2-methylphenol**

$^1$H NMR ($CDCl_3$) 400MHz δ 8.01(d, 2H, J=8.10 Hz), 7.68(d, 2H, J=8.10 Hz), 7.17(d, 1H, J=2.41 Hz), 7.08(dd, 1H, J=8.10, 2.41 Hz), 6.67(d, 1H, J=8.10 Hz), 4.63(s, 2H), 4.14(s, 2H),

**Ethyl 2-{4-[({4-{[4-(4-methoxyphenyl)-1-piperazinyl]methyl}-2-[4-(trifluoromethyl)phenyl]-1,3-thiazol-5-yl}methyl)sulfanyl]phenoxy}-2-methylpropanoate**

To a 500ml 3-neck round-bottom flask equipped with a magnetic stir-bar, low temperature thermometer with thermometer adapter, addition funnel and $N_2$ inlet was added ethyl 2-{4-[({4-(hydroxymethyl)-2-[4-(trifluoromethyl)phenyl]-1,3-thiazol-5-yl}methyl)sulfanyl]phenoxy}-2-methylpropanoate (16g, 31.28mmoles, 1eq) and dry $CH_2Cl_2$ (120ml, 0.26M) and cooled to 0°C. Methanesulfonyl chloride (2.91ml, 37.54mmoles, 1.2eq) was added neat all at once. Triethylamine (6.6ml, 46.92mmoles, 1.5eq) was added dropwise over 20 minutes maintaining the internal temperature below 5°C and was stirred at 0 °C for 30 minutes. The reaction mixture was transferred to a separatory funnel and washed with $H_2O$, brine and the organic fraction was dried over $Na_2SO_4$. After filtration the solvent was removed under reduced pressure to yield the corresponding mesylate in quantitative yield. Because of the unstable nature of the mesylate, the product was not characterized and was progressed onto the next stage without purification.

To the crude mesylate dissolved in dry THF (200ml, 0.16M) was added 4-methoxyphenyl piperazine (13g, 62.56mmoles, 2eq) and the reaction mixture was refluxed for 5 hours. After cooling to room temperature the solvent was removed *in vacuo* to yield a yellow solid residue. The residue was washed with a minimal amount of EtOAc and filtered through Celite to remove the 4-methoxyphenyl piperazine hydrochloride salt. The EtOAc was removed *in vacuo* and the resulting solid was filtered through a "plug" of silica gel using 30% EtOAc/Hexanes to yield 20.37g (95%)of a light-yellow solid.

$^1$H NMR ($CDCl_3$) 400MHz δ 7.96(d, 2H, J=8.24 Hz), 7.63(d, 2H, J=8.24 Hz), 7.27(d, 2H, J=8.79 Hz), 6.87(d, 2H, J=9.16 Hz), 6.80(d, 2H, J=9.16 Hz), 6.74(d, 2H, J=8.79 Hz), 4.32(s. 2H), 4.17(q, 2H, J=7.14 Hz), 3.73(s, 3H), 3.56 (s, 2H), 3.06(br s, 4H), 2.59(br s, 4H), 1.55(s, 6H), 1.21(t, 3H, J=7.14 Hz),

HPLC (C-18, 3μm) 0%-95% Acetonitrile/Water over 8 minutes $R_t$= 6.06minutes

The follow intermediates were made using the same alkylation conditions:

**4-[({4-{[4-(4-Methoxyphenyl)-1-piperazinyl]methyl}-2-[4-(trifluoromethyl)phenyl]-1,3-thiazol-5-yl}methyl)sulfanyl]-2-methylphenol**

$^1H$ NMR ($CDCl_3$) 400MHz δ 7.94(d, 2H, J=8.10 Hz), 7.64(d, 2H, J=8.10 Hz), 7.16(d, 1H, J=2.07 Hz), 7.07(dd, 1H, J=8.10, 2.07 Hz), 6.86(m, 2H), 6.80(d, 2H, J=8.97 Hz), 6.66(d, 1H, J=8.10 Hz), 4.27(s, 2H), 3.73(s, 3H), 3.59(s, 2H), 3.15(br s, 4H), 2.67(br s, 4H), 2.16(s, 3H),

**Ethyl [2-methyl-4-({[2-{4-{trifluoromethyl}phenyl)-4-(4-morpholinylmethyl)-1,3-thiazol-5-yl]methyl}sulfanyl)phenoxy]acetate**

$^1H$ NMR ($CDCl_3$) 300MHz δ 8.02(d, 2H, J=8.23 Hz), 7.69(d, 2H, J=8.23 Hz), 727(m, 1H), 7.17(dd, 1H, J=8.23, 2.39 Hz), 6.62(d, 1H, J=8.23 Hz), 4.64(s, 2H), 4.36(s, 2H), 4.25(q, 2H, J=7.17 Hz), 3.72(t, 4H. J=4.51 Hz), 3.53(s, 2H), 2.48(t, 4H, J=4.51 Hz), 2.27(s, 3H), 1.32(t, 3H, J=7.17 Hz),

TLC(50% EtOAc/Hexanes) $R_f$= 0.26

**Ethyl [4-({[4-[(4-benzyl-1-piperazinyl)methyl]-2-(4-{trifluoromethyl}phenyl)-1,3-thiazol-5-yl]methyl}sulfanyl)-2-methylphenoxy]acetate**

$^1H$ NMR ($CDCl_3$) 300MHz δ 8.02(d, 2H, J=8.76 Hz), 7.68(d, 2H, J=8.76 Hz), 7.31(m. 6H), 7.16(dd, 1H, J=8.49, 2.39 Hz), 6.62(d, 1H, J=8.49 Hz), 4.63(s, 2H), 4.35(s, 2H), 4.27(q, 2H, J=7.17 Hz), 3.54(m, 4H), 2.51 (br s, 8H), 2.27 (s, 3H), 1.32(t, 3H, J=7.17 Hz),

TLC(50% EtOAc/Hexanes)= 0.19

**Ethyl 2-{4-[({4-{[4-(4-methoxyphenyl)-1-piperazinyl]methyl]-2-[4-(trifluoromethyl)phenyl]-1,3-thiazol-5-yl}methyl)sulfanyl]-2-methylphenoxy}propanoate**

$^1H$ NMR ($CDCl_3$) 400MHz δ7.99(d, 2H, J=8.20 Hz), 7.66(d, 2H, J=8.20 Hz), 7.23(d, 1H, J=2.39 Hz), 7.13(dd, 1H, J=8.37, 2.39 Hz), 6.89(d, 2H, J=9.23 Hz), 6.83(d, 2H, J=9.23 Hz), 6.57(d, 1H, J=8:37 Hz), 4.70(q, 1H, J=6.84 Hz), 4.34(s, 2H), 4.17(q, 2H, J=7.18 Hz), 3.76(s, 3H), 3.58(s, 2H), 3.09(m, 4H), 2.63(m, 4H), 2.24(s, 3H), 1.62(d, 3H, J=6.84 Hz), 1.21(t, 3H. J=7.18 Hz),

TLC(30% EtOAc/Hexanes)= 0.29

**Ethyl {2-methyl-4-[({2-(4-{trifluoromethyl}phenyl)-4-[(4-phenyl-1-piperazinyl)methyl]-1,3-thiazol-5-yl}methyl)sulfanyl]phenoxy}acetate**

$^1H$ NMR ($CDCl_3$) 300MHz δ 8.04(d, 2H, J=8.23 Hz), 7.70(d, 2H, J=8.23 Hz), 7.29(m, 3H), 7.21 (dd, 1H, J=8.23, 2.39 Hz), 6.92(m, 3H), 6.63(d. 1H, J=8.23 Hz), 4.64(s, 2H), 4.38(s, 2H), 4.27(q, 2H, J=7.17 Hz), 3.63(s, 2H), 3.21(m, 4H), 2.66(m, 4H), 228(s, 3H), 1.32(t, 3H, J=7.17 Hz),

TLC(50% EtOAc/Hexanes) $R_f$ = 0.52

**Ethyl 4-{[5-({[4-(2-ethoxy-2-oxoethoxy)-3-methylphenyl]sulfanyl}methyl)-2-(4-{trifluoromethyl}phenyl)-1,3-thiazol-4-yl]methyl}-1-piperazinecarboxylate**

$^1H$ NMR ($CDCl_3$) 300MHz δ 7.99(d, 2H, J=8.23 Hz), 7.68(d, 2H, J=8.23 Hz), 7.25(m, 1H), 7.17(dd, 1H, J=8.49, 2.12 Hz), 6.61(d, 1H, J=8.49 Hz), 4.64(s, 2H), 4.28(m, 4H), 4.14(t, 2H, J=7.17 Hz); 3.50(m, 6H), 2.44(br s, 4H), 2.26 (s, 3H), 1.29(t, 3H, J=7.17 Hz),

TLC(50% EtOAc/Hexanes) $R_f$ = 0.17

**Ethyl {2-methyl-4-[({2-(4-{trifluoromethyl}phenyl)-4-[(4-phenyl-1-piperidinyl)methyl]-1,3-thiazol-5-yl}methyl)sulfanyl]phenoxy}acetate**

$^1H$ NMR ($CDCl_3$) 300MHz δ 8.04(d, 2H, J=823 Hz), 7.70(d, 2H, J=8.23 Hz), 7.27(m, 7H). 6.64(d, 1H, J=8.49 Hz), 4.64(s, 2H), 4.41 (s, 2H), 4.28(q, 2H, J=7.17 Hz), 3.60(s, 2H), 3.02(m, 2H), 2.53(m, 1H), 2.30(s, 3H), 2.18(m, 2H), 1.84(m, 4H), 1.32(t, 3H, J=7.17 Hz),

TLC(50% EtOAc/Hexanes) $R_f$ = 0.48

**Ethyl {2-methyl-4-[({2-(4-{trifluoromethyl}phenyl)-4-[(4-methyl-1-piperidinyl)methyl]-1,3-thiazol-5-yl}methyl) sulfanyl]phenoxy}acetate**

$^1H$ NMR ($CDCl_3$) 300MHz δ 8.02(d, 2H, J=8.23 Hz), 7.68(d, 2H, J=8.23 Hz), 7.28(d. 1 H, J=2.39 Hz), 7.19 (dd, 1H, J=8.49, 2.39 Hz), 6.62(d, 1H. J=8.49 Hz), 4.64(s, 2H), 4.38(s, 2H), 4.28(q, 2H. J=7.17 Hz), 3.51 (s, 2H), 2.84 (m, 4H), 2.28(s, 3H), 2.02(m, 4H), 1.61(m, 4H), 1.30(m, 8H), 0.94(d, 3H, J=6.11 Hz),

TLC(50% EtOAc/Hexanes) $R_f$ = 0.36

**Ethyl (2-methyl-4-{[(2-(4-{trifluoromethyl}phenyl)-4-{[4-(2-methylphenyl)-1-piperazinyl]methyl}-1,3-thiazol-5-yl)methyl]sulfanyl}phenoxy)acetate**

$^1H$ ($CDCl_3$) 400MHz δ 7.99(d, 2H, J=8.20 Hz), 7.66(d, 2H, J=8.20 Hz), 7.25(m, 1H), 7.16(m, 3H), 6.98(m, 2H), 6.60(d, 1H, J=8.55 Hz), 4.60(s, 2H), 4.37(s, 2H), 4.23(q, 2H, J=7.12 Hz), 3.59(s, 2H), 2.93(s, 4H), 2.63(s, 4H), 2.29(s, 3H), 2.24(s, 3H), 1.27(t, 5H, J=7.12 Hz),

TLC(50% EtOAc/Hexanes) $R_f$ = 0.73

**Ethyl [4-({[4-{[4-(4-methoxyphenyl)-1-piperazinyl]methyl}-2-(4-{trifluoromethyl}phenyl)-1,3-thiazol-5-yl] methyl}sulfanyl)-2-methylphenoxy]acetate**

$^1H$ ($CDCl_3$) 400MHz δ 7.99(d, 2H, J=8.20 Hz), 7.65(d, 2H, J=8.20 Hz), 7.24(dd, 1H, J=2.39 Hz), 7.16(dd, 1H, J=8.37, 2.39 Hz), 6.84(m, 4H), 6.58(d, 1H, J=8.37 Hz), 4.59(s, 2H), 4.33(s, 2H), 4.23(q, 2H, J=7.18 Hz), 3.75(s, 3H), 3.57(s, 2H), 3.07(m, 4H), 2.62(s, 4H), 2.24(s, 3H), 1.27(t, 3H, J=7.18 Hz),

TLC(50% EtOAc/Hexanes) $R_f$ = 0.44

**Ethyl (2-methyl-4-{[(2-(4-{trifluoromethyl}phenyl)-4-{[4-(3-methylphenyl)-1-piperazinyl]methyl}-1,3-thiazol-5-yl)methyl]sulfanyl}phenoxy)acetate**

$^1H$ ($CDCl_3$) 400MHz δ 7.99(d, 2H, J=8.20 Hz), 7.66(d, 2H, J=8.20 Hz), 7.24(m, 1H), 7.14(m, 2H), 6.70(s, 3H), 6.59(d, 1H, J=8.55 Hz), 4.60(s, 2H), 4.33(s, 2H), 4.23(q, 2H, J=7.12 Hz), 3.57(s, 2H), 3.16(br s, 4H), 2.62(br s, 4H), 2.30(s, 3H), 2.24(s, 3H), 1.26(t, 3H, J=7.12 Hz),

TLC(50% EtOAc/Hexanes) $R_f$ = 0.64

**Ethyl (2-methyl-4-{[(2-(4-{trifluoromethyl}phenyl)-4-{[4-(4-methylphenyl)-1-piperazinyl]methyl}-1,3-thiazol-5-yl)methyl]sulfanyl}phenoxy)acetate**

$^1H$ ($CDCl_3$) 400MHz δ 7.99(d, 2H, J=8.20 Hz), 7.65(d, 2H, J=8.20 Hz), 7.24(d, 1H, J=2.39 Hz), 7.15(dd, 1H, J=8.37, 2.39 Hz), 7.04(d, 2H, J=8.55 Hz), 6.82(d, 2H, J=8.55 Hz), 6.58(d, 1H, J=8.37 Hz), 4.60(s, 2H), 4.32(s, 2H), 4.23(q, 2H, J=7.12 Hz), 3.57(s, 2H), 3.10(s, 4H), 2.60(s, 4H), 2.26(s, 3H), 2.23(s, 3H), 1.26(t, 3H, J=7.12 Hz),

TLC(50% EtOAc/Hexanes) $R_f$ = 0.64

**Ethyl [4-({[4-{[4-(2-furoyl)-1-piperazinyl]methyl}-2-(4-{trifluoromethyl}phenyl)-1,3-thiazol-5-yl]methyl} sulfanyl)-2-methylphenoxy]acetate**

$^1H$ ($CDCl_3$) 400MHz δ 7.98(d, 2H, J=8.20 Hz), 7.65(d, 2H, J=8.20 Hz), 7.46(m, 1H), 7.22(d, 1H, J=2.39 Hz), 7.13(dd, 1H, J=8.37, 2.39 Hz), 6.96(d, 1H, J=3.42 Hz), 6.59(d, 1H, J=8.37 Hz), 6.46(m, 1H), 4.62(s, 2H), 4.29(s, 2H), 4.21(q, 2H, J=7.12 Hz), 3.80(s, 4H), 3.50(s, 2H), 2.53(s, 4H), 2.23(s, 3H), 1.26(t, 3H, J=7.18 Hz),

TLC(50% EtOAc/Hexanes) $R_f$ = 0.06

**Ethyl (2-methyl-4-{[(2-(4-{trifluoromethyl}phenyl)-4-{[4-(2-pyridinyl)-1-piperazinyl]methyl}-1,3-thiazol-5-yl) methyl]sulfanyl}phenoxy)acetate**

$^1H$ ($CDCl_3$) 400MHz δ 8.16(m, 1 H), 7.98(d, 2H, J=8.20 Hz), 7.63(d, 2H, J=8.20 Hz), 7.45(s, 1H), 7.25(d, 1H, J=2.22 Hz), 7.15(dd, 1H, J=8.37, 2.22 Hz), 6.56(m, 3H), 4.60(s, 2H), 4.33(s, 2H), 4.21(q, 2H, J=7.12 Hz), 3.53(m, 6H), 2.57(s, 4H), 2.23(s, 3H), 1.27(t, 3H, J=7.12 Hz),

TLC(50% EtOAc/Hexanes) $R_f$ = 0.25

**Ethyl [4-({[4-{[4-(4-chlorobenzyl)-1-piperazinyl]methyl}-2-(4-{trifluoromethyl}phenyl)-1,3-thiazol-5-yl]methyl}sulfanyl)-2-methylphenoxy]acetate**

$^1H$ ($CDCl_3$) 400MHz δ 7.96(d, 2H, J=8.20 Hz), 7.64(d, 2H, J=8.20 Hz), 7.25(m, 5H), 7.13(dd, 1H, J=8.37, 2.39 Hz), 6.58(d, 1H, J=8.37 Hz), 4.58(s, 2H), 4.31 (s, 2H), 4.22(q, 2H, J=7.18 Hz), 3.52(s, 2H), 3.42(s, 2H), 2.48(br s, 8H), 2.20(s, 3H), 1.26(t, 3H, J=7.18 Hz),

TLC(50% EtOAc/Hexanes) $R_f$ = 0.23

**Ethyl [4-({[4-{[4-(4-acetylphenyl)-1-piperazinyl]methyl}-2-(4-{trifluoromethyl}phenyl)-1,3-thiazol-5-yl]methyl}sulfanyl)-2-methylphenoxy]acetate**

$^1H$ ($CDCl_3$) 400MHz δ 7.98(d, 2H, J=8.20 Hz), 7.85(d, 2H, J=9.06 Hz), 7.66(d, 2H, J=8.20 Hz), 7.24(d, 1H, J=2.39 Hz), 7.16(dd, 1H, J=8.20, 2.39 Hz), 6.84(d. 2H, J=9.06 Hz), 6.58(d, 1H, J=8.20 Hz), 4.61 (s, 2H), 4.31 (s, 2H), 4.22(q, 2H, J=7.18 Hz), 3.58(s, 2H), 3.33(br s, 4H), 2.60(br s, 4H), 2.50(m, 3H), 2.24(s, 3H), 1.27(t, 3H, J=7.18 Hz),

TLC(50% EtOAc/Hexanes) $R_f$ = 0.23

**Ethyl (4-({[4-{[4-(2-hydroxyethyl)-1-piperazinyl]methyl}-2-(4-{trifluoromethyl}phenyl)-1,3-thiazol-5-yl]methyl}sulfanyl)-2-methylphenoxy]acetate**

$^1H$ ($CDCl_3$) 400MHz δ 7.97(d, 2H, J=8.20 Hz), 7.64(d, 2H, J=8.20 Hz), 7.23(d, 1H, J=2.22 Hz), 7.14(dd, 1H, J=8.37, 2.22 Hz), 6:58(d, 1H, J=8.37 Hz), 4.60(s, 2H), 4.30(s, 2H), 4.22(q, 2H, J=7.12 Hz), 3.60(m, 2H), 3.50(s, 2H), 2.94(s, 1H), 2.53(m, 10H), 2.23(s, 3H), 1.26(t, 3H, J=7.12 Hz),

**Ethyl (2-methyl-4-{[(2-(4-{trifluoromethyl}phenyl)-4-{[(3-pyridinylmethyl)amino]methlyl}-1,3-thiazol-5-yl)methyl]sulfanyl}phenoxy)acetate**

$^1H$ ($CDCl_3$) 400MHz δ 8.55(m, 1H), 8.50(m, 1 H), 7.98(d, 2H, J=8.20 Hz), 7.71 (m, 1H), 7.65(m, 2H), 7.24(m, 1H), 7.17(m, 1H), 7.10(m, 1H), 6.55(d, 1H, J=8.37 Hz), 4.58(s, 2H), 4.22(q, 2H, J=7.12 Hz), 4.12(s, 2H), 3.77(s, 2H), 3,63(s, 2H), 2.64(br s, 1H), 2.21 (s, 3H), 1.27(t, 3H, J=7.12 Hz),

**Ethyl (4-{[(4-{[4-(4-methoxyphenyl)-1-piperazinyl]methyl}-2-phenyl-1,3-thiazol-5-yl)methyl]sulfanyl}-2-methylphenoxy)acetate**

$^1H$ NMR ($CDCl_3$) 400MHz δ7.88(m, 2H), 7.40(m, 3H), 7.25(d, 1H, J=2.39 Hz), 7.17(dd, 1H, J=8.37, 2.39 Hz), 6.89(d, 2H, J=9.06 Hz), 6.81 (d, 2H, J=9.06 Hz), 6.58(d, 1H, J=8.37 Hz), 4.59(s, 2H), 4.32(s, 2H), 4.23(q, 2H, J=7.12 Hz), 3.74(s. 3H), 3.56(s, 2H), 3.06(m, 4H), 2.62(m, 4H), 2.24(s, 3H), 1.27(t, 3H, J=7.12 Hz),

**Ethyl 2-(4-{[(4-{[4-(4-methoxyphenyl)-1-piperazinyl]methyl}-2-phenyl-1,3-thiazol-5-yl)methyl]sulfanyl}-2-methylphenoxy)propanoate**

$^1H$ NMR ($CDCl_3$) 400MHz δ7.88(m, 2H), 7.40(m, 3H), 7.25(d, 1H, J=2.39 Hz), 7.14(dd, 1H, J=8.37, 2.39 Hz), 6.89(d, 2H, J=9.40 Hz), 6.82(d, 2H, J=9.40 Hz), 6.57(d, 1H, J=8.37 Hz), 4.70(q, 1H, J=6.84 Hz), 4.32(s, 2H), 4.17(q, 2H, J=7.18 Hz), 3.76(s, 3H), 3.56(s, 2H), 3.08(m, 4H), 2.63(m, 4H), 2.23(m, 3H), 1.61(d, 3H, J=6.84 Hz), 1.25(t, 3H, J=7.18 Hz),

**Ethyl {2-methyl-4-[({2-(4-{trifluoromethyl}phenyl)-4-[(pentylamino)methyl]-1,3-thiazol-5-yl}methyl)sulfanyl]phenoxy}acetate**

$^1H$ ($CDCl_3$) 400MHz δ 7.97(d, 2H, J=8.20 Hz), 7.65(d, 2H, J=8.20 Hz), 7.20(d, 1H, J=2.39 Hz), 7.12(dd, 1H, J=8.37, 2.39 Hz), 6.58(d, 1H, J=8.37 Hz), 4.60(s, 2H), 4.23(q, 2H, J=7.18 Hz), 4.18(s, 2H), 3.64(s, 2H), 2.58(t, 2H, J=6.92 Hz), 2.22(s, 3H), 1.50(m, 2H), 1.28(m, 7H), 0.87(t, 3H, J=6.92 Hz),

**Ethyl 2-{4-[({4-{[4-(4-hydroxyphenyl)-1-piperazinyl]methyl}-2-[4-(trifluoromethyl)phenyl]-1,3-thiazol-5-yl}methyl)sulfanyl]-2-methylphenoxy}propanoate**

$^1H$ NMR ($CDCl_3$) 300MHz δ 8.08(d, 2H, J=8.28 Hz), 7.75(d, 2H, J=8.28 Hz), 7.27(d, 1H, J=2.21 Hz), 7 17(dd, 1H, J=8.28, 2.21 Hz), 6.87(d, 2H, J=8.83 Hz), 6.73(d, 2H, J=8.83 Hz), 6.66(d, 1H, J=8.28 Hz), 4.83(q, 1H, J=6.81 Hz), 4.34(s, 2H). 4.15(q, 2H, J=7.08 Hz), 3.47(s, 2H), 3.00(t, 4H, J=4.83 Hz), 2.57(t, 4H, J=4.83 Hz), 2.20(s, 3H), 1.57(d,

3H, J=6.81 Hz), 1.20(t, 3H, J=7.08 Hz),

**Ethyl 2-{4-[({4-{[4-(3,4-dimethoxyphenyl)-1-piperazinyl]methyl}-2-[4-(trifluoromethyl)phenyl]-1,3-thiazol-5-yl} methyl)sulfanyl]-2-methylphenoxy}propanoate**

$^1H$ NMR ($CDCl_3$) 300MHz δ 8.06(d, 2H, J=8.28 Hz), 7.72(d, 2H, J=8.28 Hz), 7.26(d, 1H, J=2.21 Hz), 7.16(dd, 1H, J=8.55, 2.21 Hz), 6.82(d, 1H, J=8.55 Hz), 6.64(m, 2H), 6.47(dd, 1H, J=8.55, 2.21 Hz), 4.81(q, 1H, J=6.99 Hz), 4.34(s, 2H), 4.14(q, 2H, J=7.17 Hz), 3.82(s, 3H), 3.77(s, 3H), 3.52(s, 2H), 3.07(t, 4H, J=4.55 Hz), 2.63(t, 4H, J=4.55 Hz), 2.20(s, 3H), 1.57(d, 3H, J=6.99 Hz), 1.18(t, 3H, J=7.17 Hz),

**Ethyl 2-(4-{[(4-{(4-methoxyphenyl)-1-piperazinyl]methyl}-2-phenyl-1,3-thiazol-5-yl)methyl]sulfanyl}phenoxy)-2-methylpropanoate**

$^1H$ NMR ($CDCl_3$) 400MHz δ 7.87(m, 2H), 7.40(m, 3H), 7.28(d, 2H, J=8.89 Hz), 6.89(d, 2H, J=9.23 Hz), 6.82 (d, 2H, J=9.23 Hz), 6.75(d, 2H, J=8.89 Hz), 4.33(s, 2H), 4.19(q, 2H, J=7.18 Hz), 3.76(s, 3H), 3.56(s, 2H), 3.09(br s, 4H), 2.65(br s, 4H), 1.58(s, 6H), 1.20(t, 3H, J=7.18 Hz),

**Ethyl {4-[({4-{[4-(4-methoxyphenyl)-1-piperazinyl]methyl}-2-[4-(trifluoromethyl)phenyl]-1,3-thiazol-5-yl} methyl)sulfanyl]phenoxy}acetate**

$^1H$ NMR ($CDCl_3$) 400MHz s7.99(d, 2H, J=8.20 Hz), 7.66(d, 2H, J=8.20 Hz), 7.35(d, 2H, J=8.89 Hz), 6.88(d, 2H, J=9.40 Hz), 6.83(m, 4H), 4.58(s, 2H), 4.34(s, 2H), 4.24(q, 2H, J=7.18 Hz), 3.76(s, 3H), 3.57(s, 2H), 3.08(m, 4H), 2.63(m, 4H), 1.27(t, 3H, J=7.18 Hz),

**Ethyl 2-{4-[({4-{[4-(4-methoxyphenyl)-1-piperazinyl]methyl}-2-[4-(trifluoromethyl)phenyl]-1,3-thiazol-5-yl} methyl)sulfanyl]phenoxy}propanoate**

$^1H$ NMR ($CDCl_3$) 400MHz δ 7.99(d, 2H, J=8.20 Hz), 7.66(d, 2H, J=8.20 Hz), 7.32(d, 2H, J=8.89 Hz), 6.89(d, 2H, J=9.23 Hz), 6.83(d, 2H, J=9.23 Hz), 6.79(d, 2H, J=8.89 Hz), 4.70(q, 1H, J=6.78 Hz), 4.33(s, 2H), 4.16(q, 2H, J=7.09 Hz), 3.75(s, 3H), 3.57(s, 2H), 3.08(m, 4H), 2.63(m, 4H), 1.60(d, 3H, J=6.78 Hz), 1.24(t, 3H, J=7.09 Hz),

**Ethyl 2-(4-{[(4-{[4-(4-methoxyphenyl)-1-piperazinyl]methyl}-2-phenyl-1,3-thiazol-5-yl)methyl]sulfanyl} phenoxy)propanoate**

$^1H$ NMR ($CDCl_3$) 400MHz δ7.87(m, 2H), 7.39(m, 3H), 7.32(d, 2H, J=8.85 Hz), 6.87(d, 2H, J=9.06 Hz), 6.82 (d; 2H, J=9.06 Hz), 6.77(d, 2H, J=8.85 Hz), 4.69(q, 1 H, J=6.78 Hz). 4.31(s, 2H), 4.18(q, 2H, J=7.12 Hz), 3.75(s, 3H), 3.54(s, 2H), 3.08(m, 4H), 2.62(m, 4H), 1.59(d, 3H, J=6.78 Hz), 1.20(t, 3H, J=7.12 Hz),

**Ethyl 2-{4-[({4-{[4-(3-methoxyphenyl)-1-piperazinyl]methyl}-2-[4-(trifluoromethyl)phenyl]-1,3-thiazol-5-yl} methyl)sulfanyl]-2-methylphenoxy}-2-methylpropanoate**

$^1H$ NMR ($CDCl_3$) 400MHz δ 7.96(d, 2H, J=8.28 Hz), 7.63(d, 2H, J=8.28 Hz), 7.21 (d, 1H, J=2.41 Hz), 7.13(t, 1H, J=8.10 Hz), 7.07(dd, 1H. J=8.45, 2.41 Hz), 6.53(m, 2H), 6.43(t, 1H, J=2.24 Hz), 6.38(dd, 1H, J=8.10, 2.24 Hz), 4.31(s, 2H), 4.18(q, 2H, J=7.16 Hz), 3.75(s, 3H), 3.55(s, 2H), 3.16(t, 4H, J=4.83 Hz), 2.58(t, 4H, J=4.83 Hz), 2.17(s, 3H), 1.57(s, 6H), 1.22(t, 3H, J=7.16 Hz),

**Ethyl 2-{4-[({4-{[4-(4-fluorophenyl)-1-piperazinyl]methyl}-2-[4-(trifluoromethyl)phenyl]-1,3-thiazol-5-yl}methyl) sulfanyl]-2-methylphenoxy}-2-methypropanoate**

$^1H$ NMR ($CDCl_3$) 400MHz δ 7.96(d, 2H, J=8.28 Hz), 7.62(d, 2H, J=8.28 Hz), 7.21(d, 1H, J=2.41 Hz), 7.06(dd, 1H, J=8.45, 2.41 Hz), 6.91 (m, 2H), 6.83(m, 2H), 6.53(d, 1H, J=8.45 Hz), 4.30(s, 2H), 4.13(q, 2H, J=7.16 Hz), 3.55(s, 2H), 3.06(t, 4H, J=4.66 Hz), 2.57(t, 4H. J=4.66 Hz), 2.15(s, 3H), 1.55(s, 6H), 1.21(t, 3H, J=7.16 Hz),

**Ethyl 2-{4-[({4-{[4-(3-methoxyphenyl)-1-piperazinyl]methyl}-2-[4-(trifluoromethyl)phenyl]-1,3-thiazol-5-yl} methyl)sulfanyl]phenoxy}-2-methylpropanoate**

$^1H$ NMR ($CDCl_3$) 400MHz δ 7.96(d, 2H, J=8.10 Hz), 7.63(d, 2H. J=8.10 Hz), 7.26(d, 2H, J=8.79 Hz), 7.14(t, 1H, J=8.28 Hz), 6.74(d, 2H, J=8.79 Hz), 6.51 (dd, 1H, J=8.28, 2.24 Hz), 6.43(t, 1H, J=2.24 Hz), 6.39(dd, 1H, J=8.28,

2.24 Hz), 4.31(s, 2H), 4.16(q, 2H, J=7.07 Hz), 3.74(s. 3H), 3.54(s, 2H), 3.17(t, 4H, J=4.66 Hz), 2.58(t, 4H, J=4.66 Hz), 1.56(s, 6H), 1.20(t, 3H, J=7.07 Hz),

**Ethyl 2-{4-[({4-{[4-(4-chlorophenyl)-1-piperazinyl]methyl}-2-[4-(trifluoromethyl)phenyl]-1,3-thiazol-5-yl}methyl)sulfanyl]phenoxy}-2-methylpropanoate**

$^1$H NMR ($CDCl_3$) 400MHz δ 7.96(d, 2H, J=8.10 Hz), 7.63(d, 2H, J=8.10 Hz), 7.27(d, 2H, J=8.79 Hz), 7.15(d, 2H, J=9.14 Hz), 6.80(d, 2H, J=9.14 Hz), 6.73(d, 2H, J=8.79 Hz), 4.30(s, 2H), 4.17(q, 2H, J=7.16 Hz), 3.54(s, 2H), 3.12 (t, 4H, J=4.74 Hz), 2.57(m, 4H), 1.55(s. 6H), 1.17(t, 3H, J=7.16 Hz),

**Ethyl 2-{4-[({4-{[4-(4-acetylphenyl)-1-piperazinyl]methyl}-2-[4-(trifluoromethyl)phenyl]-1,3-thiazol-5-yl}methyl)sulfanyl]phenoxy}-2-methylpropanoate**

$^1$H NMR ($CDCl_3$) 400MHz δ 7.95(d, 2H, J=8.28 Hz), 7.83(d, 2H, J=9.14 Hz), 7.62(d, 2H, J=8.28 Hz), 7.26(d, 2H, J=8.62 Hz), 6.82(d, 2H, J=9.14 Hz), 6.73(d, 2H, J=8.62 Hz), 4.29(s, 2H), 4.17(q, 2H, J=7.07 Hz), 3.53(s, 2H), 3.32 (t, 4H, J=4.66 Hz), 2.57(br s, 4H), 2.48(s, 3H), 1.55(s, 6H), 1.17(t, 3H, J=7.07 Hz),

**Ethyl 2-{4-[({4-{[4-(4-methoxyphenyl)-1-piperazinyl]methyl}-2-[4-(trifluoromethyl)phenyl]-1,3-thiazol-5-yl}methyl)sulfanyl]phenoxy}-2-methylpropanoate**

$^1$H NMR ($CDCl_3$) 400MHz δ 7.96(d, 2H, J=8.28 Hz), 7.63(d, 2H, J=8.28 Hz), 7.26(d, 2H, J=8.79 Hz), 6.87(d, 2H, J=9.14 Hz), 6.81 (d, 2H, J=9.14 Hz), 6.73(d, 1H, J=8.79 Hz), 4.32(s, 2H), 4.17(q, 2H, J=7.16 Hz), 3.73(s, 3H), 3.54 (s, 2H), 3.06(t, 4H, J=4.83 Hz), 2.60(br s, 4H), 1.55(s, 6H), 1.20(t, 3H, J=7.16 Hz),

**Ethyl 2-(4-{[(2-(4-fluorophenyl)-4-{[4-(4-methoxyphenyl)-1-piperazinyl]methyl}-1,3-thiazol-5-yl)methyl]sulfanyl}-2-methylphenoxy)-2-methylpropanoate**

$^1$H NMR ($CDCl_3$) 400MHz δ 7.84(m, 2H), 7.20(d, 1H, J=2.20 Hz), 7.07(m, 3H), 6.87(d, 2H, J=9.16 Hz), 6.81 (d, 2H, J=9.16 Hz), 6.54(d, 1H, J=8.42 Hz), 4.29(s, 2H), 4.19(q, 2H, J=7.14 Hz), 3.75(s, 3H), 3.54(s, 2H), 3.07(t, 4H, J=4.76 Hz), 2.61(br s, 4H), 2.15(s, 3H), 1.54(s, 6H), 1.21(t, 3H, J=7.14 Hz),

**Ethyl 2-[4-({[4-{[4-(4-acetylphenyl)-1-piperazinyl]methyl}-2-(4-fluorophenyl)-1,3-thiazol-5-yl]methyl}sulfanyl)-2-methylphenoxy]-2-methylpropanoate**

$^1$H NMR ($CDCl_3$) 400MHz δ 7.84(m, 4H), 7.20(d, 1H, J=2.38 Hz), 7.07(m, 3H), 6.83(d, 2H, J=9.16 Hz), 6.53 (d, 1H, J=8.42 Hz), 4.28(s, 2H), 4.18(q, 2H, J=7.14 Hz), 3.53(s, 2H), 3.33(t, 4H, J=4.58 Hz), 2.58(br s, 4H), 2.48(s, 3H), 2.16(s, 3H), 1.58(s, 6H), 1.23(t, 3H, J=7.14 Hz),

**Ethyl 2-(4-{[(2-(4-fluorophenyl)-4-{[4-(3-methoxyphenyl)-1-piperazinyl]methyl}-1,3-thiazol-5-yl)methyl]sulfanyl}-2-methylphenoxy)-2-methylpropanoate**

$^1$H NMR ($CDCl_3$) 400MHz δ 7.85(m, 2H), 7.20(d, 1H, J=2.38 ), 7.14(t, 1H, J=8.24 Hz), 7.07(m, 3H), 6.53(m, 2H), 6.44(t, 1H, J=2.29 Hz), 6.39(dd, 1H, J=8.06, 2.38 Hz), 4.29(s, 2H), 4.19(q, 2H, J=7.14 Hz), 3.76(s, 3H), 3.53(s, 2H), 3.17(t, 4H, J=4.67 Hz), 2.59(br s, 4H), 2.16(s, 3H), 1.55(s, 6H), 1.21(t, 3H, J=7.14 Hz),

**Ethyl 4-{[5-({[4-(2-ethoxy-1,1-dimethyl-2-oxoethoxy)-3-methylphenyl]sulfanyl}methyl)-2-(4-fluorophenyl)-1,3-thiazol-4-yl]methyl}-1-piperazinecarboxylate**

$^1$H NMR ($CDCl_3$) 400MHz δ 7.82(m, 2H), 7.18(d, 1H, J=2.38 Hz), 7.06(m, 3H), 6.53(d, 1H, J=8.61 Hz), 4.25 (s, 2H), 4.19(q, 2H, J=7.14 Hz), 4.10(q, 2H, J=7.08 Hz), 3.45(m, 6H), 2.40(br s, 4H), 2.16(s, 3H), 1.55(s, 6H), 1.21(m, 6H),

**Ethyl 2-{4-[({4-{[4-(4-methoxyphenyl)-1-piperazinyl]methyl}-2-[4-(trifluoromethyl)phenyl]-1,3-thiazol-5-yl}methyl)sulfanyl]-2-methylphenoxy}-2-methylpropanoate**

$^1$H NMR ($CDCl_3$) 400MHz δ 7.96(d, 2H, J=8.28 Hz), 7.63(d, 2H, J=8.28 Hz), 7.21 (d, 1H, J=2.24 Hz), 7.07 (dd, 1H, J=8.45, 2.24 Hz), 6.86(d, 2H, J=9.14 Hz), 6.80(d, 2H, J=9.14 Hz), 6.53(d, 1 H, J=8.45 Hz), 4.31 (s, 2H), 4.17 (q, 2H, J=7.16 Hz), 3.72(s, 3H), 3.55(s, 2H), 3.05(t, 4H, J=4.66 Hz), 2.59(t, 4H, J=4.66 Hz), 2.16(s, 3H), 1.55(s, 6H),

1.20(t, 3H, J=7.16 Hz),

**Ethyl 2-{4-[({4-{[4-(4-acetylphenyl)-1-piperazinyl]methyl}-2-[4-(trifluoromethyl)phenyl]-1,3-thiazol-5-yl}methyl) sulfanyl]-2-methylphenoxy}-2-methylpropanoate**

$^1$H NMR ($CDCl_3$) 400MHz δ 7.95(d, 2H, J=8.10 Hz), 7.82(d, 2H, J=8.97 Hz), 7.62(d, 2H, J=8.10 Hz), 7.19(d, 1H, J=2.41 Hz), 7.06(dd, 1H, J=8.45, 2.41 Hz), 6.82(d, 2H, J=8.97 Hz), 6.52(d, 1H, J=8.45 Hz), 4.27(s, 2H), 4.16(q, 2H, J=7.07 Hz), 3.53(s, 2H), 3.29(t, 4H, J=4.66 Hz), 2.54(t, 4H, J=4.66 Hz), 2.47(s, 3H), 2.14(s, 3H), 1.55(s, 6H), 1.18 (t, 3H, J=7.07 Hz),

**Ethyl 2-{4-[({4-[(4-acetyl-1-piperazinyl)methyl]-2-[4-(trifluoromethyl)phenyl]-1,3-thiazol-5-yl}methyl)sulfanyl]-2-methylphenoxy}propanoate**

$^1$H NMR ($CDCl_3$) 300MHz δ 8.00(d, 2H, J=8.23 Hz), 7.68(d, 2H, J=8.23 Hz), 7.27(d, 1H, J=2.39 Hz), 7.14(dd, 1H, J=8.23, 2.39 Hz), 6.59(d, 1H, J=8.23 Hz), 4.73(q, 1H, J=6.72 Hz), 4.30(s, 2H), 4.20(q, 2H, J=7.17 Hz), 3.65(t, 2H, J=4.65 Hz), 3.54(s, 2H), 3.45(t, 2H, J=4.65Hz), 2.48(t, 4H, J=4.65 Hz), 2.26(s, 3H), 2.09(s, 3H), 1.65(d, 3H, J=6.72 Hz), 1.25(dd, 3H, J=7.17 Hz),

**2-Methyl-2-{4-[({4-{[4-(phenoxycarbonyl)-1-piperazinyl]methyl}-2-[4-(trifluoromethyl)phenyl]-1,3-thiazol-5-yl} methyl)sulfanyl]phenoxy}propanoic acid**

$^1$H NMR ($CDCl_3$) 400MHz δ 7.95(d, 2H, J=8.28 Hz), 7.65(d, 2H, J=8.28 Hz), 7.33(m, 2H), 7.26(d, 2H, J=8.79 Hz), 7.17(t, 1H, J=7.59 Hz), 7.06(d, 2H, J=7.59 Hz), 6.74(d, 2H, J=8.79 Hz), 4.32(s, 2H), 4.18(q, 2H, J=7.07 Hz), 3.61 (m, 6H), 2.51 (br s, 4H), 1.57(s, 6H), 1.20(t, 3H, J=7.07 Hz),

**tert-Butyl 4-({5-({[4-(2-ethoxy-1,1-dimethyl-2-oxoethoxy)phenyl]sulfanyl}methyl)-2-[4-(trifluoromethyl)phenyl]-1,3-thiazol-4-yl}methyl)-1-piperazinecarboxylate**

$^1$H NMR ($CDCl_3$) 400MHz δ 7.94(d, 2H, J=8.28 Hz), 7.63(d, 2H, J=8.28 Hz), 7.24(d, 2H, J=8.79 Hz), 6.72(d, 2H, J=8.79 Hz), 4.29(s, 2H), 4.18(q, 2H, J=7.07 Hz), 3.44(m, 6H), 2.43(br s, 4H), 1.56(s, 6H), 1.42(s, 9H), 1.19(t, 3H, J=7.07 Hz),

**Ethyl 2-methyl-2-{4-[({4-{[4-(2-pyrazinyl)-1-piperazinyl]methyl}-2-[4-(trifluoromethyl)phenyl]-1,3-thiazol-5-yl} methyl)sulfanyl]phenoxy}propanoate**

$^1$H NMR ($CDCl_3$) 400MHz δ 8.12(s, 1H), 8.04(s, 1H), 7.94(d, 2H, J=8.28 Hz), 7.83(s, 1H), 7.65(d, 2H, J=8.28 Hz), 7.26(d, 2H, J=8.79 Hz), 6.73(d, 2H, J=8.79 Hz), 4.32(s, 2H), 4.17(q, 2H, J=7.07 Hz), 3.62(m, 6H), 2.64(br s, 4H), 1.56(s, 6H), 1.18(t, 3H, J=7.07 Hz),

**Ethyl 2-{4-[({4-{[4-(2-methoxyphenyl)-1-piperazinyl]methyl}-2-[4-(trifluoromethyl)phenyl]-1,3-thiazol-5-yl} methyl)sulfanyl]phenoxy}-2-methylpropanoate**

$^1$H NMR ($CDCl_3$) 400MHz δ 7.96(d, 2H, J=8.28 Hz), 7.64(d, 2H, J=8.28 Hz), 7.27(d, 2H, J=8.97 Hz), 6.98(m, 1H), 6.90(m, 2H), 6.83(m, 1H), 6.73(d, 2H, J=8.97 Hz), 4.35(s, 2H), 4.17(q, 2H, J=7.07 Hz), 3.83(s, 3H), 3.60(s, 2H), 3.11 (br s, 4H), 2.72(br s, 4H), 1.58(s, 6H), 1.18(t, 3H, J=7.07 Hz),

**tert-Butyl 4-({5-({[4-(2-methoxy-2-oxoethoxy)-3-methylphenyl]sulfanyl}methyl)-2-[4-(trifluoromethyl)phenyl]-1,3-thiazol-4-yl}methyl)-1-piperazinecarboxylate**

$^1$H NMR ($CDCl_3$) 400MHz δ 7.90(d, 2H, J=8.28 Hz), 7.58(d, 2H, J=8.28 Hz), 7.16(d, 1H, J=2.24 Hz), 7.08(dd, 1H, J=8.45, 2.24 Hz), 6.52(d, 1H, J=8.45 Hz), 4.56(s, 2H), 4.20(s, 2H), 3.70(s, 3H), 3.44(s, 2H), 3.36(t, 4H, J=4.48 Hz), 2.32(br s, 4H), 2.17(s, 3H), 1.38(s, 9H),

**Ethyl 2-{2-methyl-4-[({4-{[4-(4-pyridinyl)-1-piperazinyl]methyl}-2-[4-(trifluoromethyl)phenyl]-1,3-thiazol-5-yl} methyl)sulfanyl]phenoxy}propanoate**

$^1$H NMR ($CDCl_3$) 300MHz δ 8.28(d, 2H. J=6.37 Hz), 8.02(d, 2H, J=8.23 Hz), 7.69(d, 2H, J=8.23 Hz), 7.28(d, 1H, J=2.39 Hz), 7.16(dd, 1H, J=8.49, 2.39 Hz), 6.68(d, 2H, J=6.37 Hz), 6.60(d, 1H, J=8.49 Hz), 4.73(q, 1H, J=6.72

Hz), 4.32(s, 2H), 4.20(q, 2H, J=7.08 Hz), 3.59(s, 2H), 3.34(t, 4H, J=5.04 Hz), 2.58(t, 4H, J=5.04 Hz), 2.26(s, 3H), 1.65 (d, 3H, J=6.72 Hz), 1.25(t, 3H, J=7.08 Hz),

**Ethyl 2-{4-[({4-{[4-(4-methoxyphenyl)-1-piperazinyl]methyl}-2-[4-(trifluoromethyl)phenyl]-1,3-thiazol-5-yl} methyl)sulfanyl]-2-methylphenoxy}propanoate**

$^1H$ NMR ($CDCl_3$) 400MHz δ7.99(d, 2H, J=8.20 Hz), 7.66(d, 2H, J=8.20 Hz), 7.23(d, 1H, J=2.39 Hz), 7.13(dd, 1H, J=8.37, 2.39 Hz), 6.89(d, 2H, J=9.23 Hz), 6.83(d, 2H, J=9.23 Hz), 6.57(d, 1H, J=8.37 Hz), 4.70(q, 1H, J=6.84 Hz), 4.34(s, 2H), 4.17(q, 2H, J=7.18 Hz), 3.76(s, 3H), 3.58(s, 2H), 3.09(m, 4H), 2.63(m, 4H), 2.24(s, 3H), 1.62(d, 3H, J=6.84 Hz), 1.21(t, 3H, J=7.18 Hz),

TLC(30% EtOAc/Hexanes)= 0.29

**Ethyl 2-{4-[({4-{[4-(2,4-difluorophenyl)-1-piperazinyl]methyl)-2-[4-(trifluoromethyl)phenyl]-1,3-thiazol-5-yl} methyl)sulfanyl]-2-methylphenoxy}propanoate**

$^1H$ NMR ($CDCl_3$) 300MHz δ 8.03(d, 2H, J=8.28 Hz), 7.69(d, 2H, J=8.28 Hz), 7.27(d, 1H, J=2.21 Hz), 7.17(dd, 1H, J=8.28, 2.21 Hz), 6.86(m, 3H), 6.61(d, 1H, J=8.28 Hz), 4.73(q, 1H, J=6.71 Hz), 4.36(s, 2H), 4.21 (q, 2H, J=7.17 Hz), 3.62(s, 2H), 3.06(t, 4H, J=4.55 Hz), 2.67(t, 4H, J=4.55 Hz), 2.27(s, 3H), 1.65(d, 3H, J=6.71 Hz), 1.26(t, 3H, J=7.17 Hz),

**Ethyl 2-{2-methyl-4-[({4-({4-[4-(trifluoromethoxy)phenyl]-1-piperazinyl}methyl)-2-[4-(trifluoromethyl)phenyl]-1,3-thiazol-5-yl}methyl)sulfanyl]phenoxy}propanoate**

$^1H$ NMR ($CD_3OD$) 400MHz δ 7.97(d, 2H, J=8.24 Hz), 7.63(d. 2H, J=8.24 Hz), 7.19(s, 1H), 7.10(dd, 1H, J=8.42, 2,20 Hz), 7.03(d, 2H, J=9.16 Hz), 6.85(d, 2H, J=9.16 Hz), 6.57(d, 1H, J=8.42 Hz), 4.73(q, 1H, J=6.78 Hz), 4.27(s, 2H), 4.07(m, 2H), 3.41 (s, 2H), 3.03(br s, 4H), 2.48(br s, 4H), 2.13(s, 3H), 1.51 (d, 3H, J=6.78 Hz), 1.11 (t, 3H, J=7.14 Hz),

**Ethyl 2-{4-[({4-{[4-(4-ethoxyphenyl)-1-piperazinyl]methyl}-2-[4-(trifluoromethyl)phenyl]-1,3-thiazol-5-yl} methyl)sulfanyl]-2-methylphenoxy}propanoate**

$^1H$ NMR ($CD_3OD$) 400MHz δ 8.01 (d, 2H, J=8.28 Hz), 7.70(d, 2H, J=8.28 Hz), 7.21 (d, 1H, J=2.24 Hz), 7.11 (dd, 1H, J=8.45, 2.24 Hz), 6.86(d, 2H, J=9.14 Hz), 6.76(d, 2H, J=9.14 Hz), 6.61(d, 1H, J=8.45 Hz), 4.77(q, 1H, J=6.72 Hz), 4.29(s, 2H), 4.10(q, 2H, J=7.16 Hz), 3.91(q, 2H, J=6.98 Hz), 3.40(s, 2H), 2.96(t, 4H, J=4.83 Hz), 2.50(t, 4H, J=4.83 Hz), 2.14(s, 3H), 1.52(d, 3H, J=6.72 Hz), 1.30(t, 3H, J=6.98 Hz), 1.14(t, 3H, J=7.16 Hz),

**Ethyl 2-{2-methyl-4-[({4-{[4-(4-propoxyphenyl)-1-piperazinyl]methyl}-2-[4-(trifluoromethyl)phenyl]-1,3-thiazol-5-yl}methyl)sulfanyl]phenoxy}propanoate**

$^1H$ NMR ($CD_3OD$) 400MHz δ 7.96(d, 2H, J=8.10 Hz), 7.63(d, 2H, J=8.10 Hz), 7.18(s, 1H), 7.09(d, 1H, J=8.45 Hz), 6.81 (d, 2H, J=8.97 Hz), 6.73(d, 2H, J=8.97 Hz), 6.56(d, 1 H, J=8.45 Hz), 4.71 (q, 1H, J=6.47 Hz), 4.25(s, 2H), 4.06(q, 2H, J=7.07 Hz), 3.76(t, 2H, J=7.41 Hz), 3.39(s, 2H), 2.92(br s, 4H), 2.48(br s, 4H), 2.12(s, 3H), 1.67(m, 2H), 1.49(d, 3H, J=6.47 Hz). 1.11(t, 3H, J=7.07 Hz), 0.94(t, 3H, J=7.41 Hz),

**Ethyl 2-{4-[({4-{[4-(4-isopropoxyphenyl)-1-piperazinyl]methyl}-2-[4-(trifluoromethyl)phenyl]-1,3-thiazol-5-yl} methyl)sulfanyl]-2-methylphenoxy}propanoate**

$^1H$ NMR ($CD_3OD$) 400MHz δ 7.96(d, 2H, J=8.28 Hz), 7.64(d, 2H, J=8.28 Hz), 7.18(d, 1H, J=2.24 Hz), 7.09 (dd, 1H, J=8.45, 2.24 Hz), 6.81(d, 2H, J=9.14 Hz), 6.73(d, 2H, J=9.14 Hz), 6.57(d, 1H, J=8.45 Hz), 4.71 (q, 1H, J=6.78 Hz), 4.36(m, 1H), 4.24(s, 2H), 4.06(q, 2H, J=7.16 Hz), 3.39(s, 2H), 2.92(t, 4H, J=4.57 Hz), 2.47(t, 4H, J=4.57 Hz), 2.11 (s, 3H), 1.48(d, 3H, J=6.78 Hz), 1.19(d, 6H, J=6.21 Hz), 1.11(t, 3H, J=7.16 Hz),

**Ethyl 4-({5-({[4-(2-ethoxy-1,1-dimethyl-2-oxoethoxy)phenyl]sulfanyl}methyl)-2-[4-(trifluoromethyl)phenyl]-1,3-thiazol-4-yl}methyl)-1-piperazinecarboxylate**

$^1H$ NMR ($CDCl_3$) 400MHz δ 7.94(d, 2H, J=8.28 Hz), 7.63(d, 2H, J=8.28 Hz), 7.24(m, 2H), 6.72(d; 2H, J=8.79 Hz), 4.30(s, 2H), 4.18(q, 2H, J=7.07 Hz), 4.10(q, 2H, J=7.13 Hz), 3.49(m, 6H), 2.46(br s, 4H), 1.58(s, 6H), 1.21(m, 6H),

**Ethyl 4-({5-({[4-(2-methoxy-2-oxoethoxy)-3-methylphenyl]sulfanyl}methyl)-2-[4-(trifluoromethyl)phenyl]-1,3-thiazol-4-yl}methyl)-1-piperazinecarboxylate**

$^1$H NMR ($CDCl_3$) 400MHz δ 7.95(d, 2H, J=8.10 Hz), 7.64(d, 2H, J=8.10 Hz), 7.20(d, 1H, J=2.21 Hz), 7.13(dd, 1H, J=8.45, 2.21 Hz), 6.57(d, 1H, J=8.45 Hz), 4.62(s, 2H), 4.30(s, 2H), 4.10(q, 2H, J=7.16 Hz), 3.77(s, 3H), 3.49(m, 6H), 2.45(br s, 4H), 2.21 (s, 3H), 1.23(t, 3H, J=7.16 Hz),

**Methyl {4-[({4-{[4-(3-methoxyphenyl)-1-piperazinyl]methyl}-2-[4-(trifluoromethyl)phenyl]-1,3-thiazol-5-yl}methyl)sulfanyl]-2-methylphenoxy}acetate**

$^1$H NMR ($CDCl_3$) 400MHz δ 7.97(d, 2H, J=8.28 Hz), 7.64(d, 2H, J=8.28 Hz), 7.21(d, 1H, J=2.24 Hz), 7.14(m, 2H), 6.57(d, 1H, J=8.45 Hz), 6.49(dd, 1H, J=8.10, 2.20 Hz), 6.40(s, 2H), 4.60(s, 2H), 4.33(s, 2H), 3.76(s, 6H), 3.59(s, 2H), 3.21 (br s, 4H), 2.68(br s, 4H), 2.21 (s, 3H),

**Methyl {4-[({4-{[4-(4-acetylphenyl)-1-piperazinyl]methyl}-2-[4-(trifluoromethyl)phenyl]-1,3-thiazol-5-yl}methyl)sulfanyl]-2-methylphenoxy}acetate**

$^1$H NMR ($CDCl_3$) 400MHz δ 7.93(d, 2H, J=8.28 Hz), 7.82(d, 2H, J=8.97 Hz), 7.61(d, 2H, J=8.28 Hz), 7.20(d, 1H, J=2.24 Hz), 7.13(dd, 1H, J=8.45, 2.24 Hz), 6.80(d, 2H, J=8.97 Hz), 6.55(d, 1H, J=8.45 Hz), 4.57(s, 2H), 4.27(s, 2H), 3.73(s, 3H), 3.52(s, 2H), 3.27(t, 4H, J=4.83 Hz), 2.54(t, 4H, J=4.83 Hz), 2.45(s, 3H), 2.20(s, 3H),

**Methyl {4-[({4-{[4-(2-methoxyphenyl)-1-piperazinyl]methyl}-2-[4-(trifluoromethyl)phenyl]-1,3-thiazol-5-yl}methyl)sulfanyl]-2-methylphenoxy}acetate**

$^1$H NMR ($CDCl_3$) 400MHz δ 7.97(d, 2H, J=8.10 Hz), 7.65(d, 2H, J=8.10 Hz), 7.21(m, 1H), 7.15(dd, 1H, J=8.45, 2.07 Hz), 6.98(br s, 1H), 6.89(m, 2H), 6.83(d, 1 H, J=7.41 Hz), 6.57(d, 1H, J=8.45 Hz), 4.61 (s, 2H), 4.35(s, 2H), 3.83 (s, 3H), 3.75(s, 3H), 3.61 (s, 2H), 3.11 (br s, 4H), 2.70(br s, 4H), 2.22(s, 3H),

**Methyl {2-methyl-4-[({4-{[4-(2-pyrazinyl)-1-piperazinyl]methyl}-2,4-(trifluoromethyl)phenyl]-1,3-thiazol-5-yl}methyl)sulfanyl]phenoxy}acetate**

$^1$H NMR ($CDCl_3$) 400MHz δ 8.07(s, 1H), 7.99(m, 1H), 7.94(d, 2H, J=8.10 Hz), 7.77(d, 1H, J=2.59 Hz), 7.60 (d, 2H, J=8.10 Hz), 7.20(d, 1H, J=2.24Hz), 7.12(dd, 1H, J=8.45, 2.24 Hz), 6.54(d, 1H, J=8.45 Hz), 4.58(s, 2H), 4.26 (s, 2H), 3.73(s, 3H), 3.52(m, 6H), 2.52(t, 4H, J=4.83 Hz), 2.19(s, 3H),

**Ethyl (4-{[(4-{[4-(4-methoxyphenyl)-1-piperazinyl]methyl}-2-phenyl-1,3-thiazol-5-yl)methyl]sulfanyl}-2-methylphenoxy)acetate**

$^1$H NMR ($CDCl_3$) 400MHz δ7.88(m, 2H), 7.40(m, 3H), 7.25(d, 1H, J=2.39 Hz), 7.17(dd, 1H, J=8.37, 2.39 Hz), 6.89(d, 2H, J=9.06 Hz), 6.81(d, 2H, J=9.06 Hz), 6.58(d, 1H, J=8.37 Hz), 4.59(s, 2H), 4.32(s, 2H), 4.23(q, 2H, J=7.12 Hz), 3.74(s, 3H), 3.56(s, 2H), 3.06(m, 4H), 2.62(m, 4H), 2.24(s, 3H), 1.27(t, 3H, J=7.12 Hz),

**Ethyl 2-(4-{[(4-{[4-(4-methoxyphenyl)-1-piperazinyl]methyl}-2-phenyl-1,3-thiazol-5-yl)methyl]sulfanyl}-2-methylphenoxy)propanoate**

$^1$H NMR ($CDCl_3$) 400MHz δ7.88(m, 2H), 7.40(m, 3H), 7.25(d, 1H, J=2.39 Hz), 7.14(dd, 1H, J=8.37, 2.39 Hz), 6.89(d, 2H, J=9.40 Hz), 6.82(d, 2H, J=9.40 Hz), 6.57(d, 1H, J=8.37 Hz), 4.70(q, 1H, J=6.84 Hz), 4.32(s, 2H), 4.17(q, 2H, J=7.18 Hz), 3.76(s, 3H), 3.56(s, 2H), 3.08(m, 4H), 2.63(m, 4H), 2.23(m, 3H), 1.61 (d, 3H, J=6.84 Hz), 1.25(t, 3H, J=7.18 Hz),

**Ethyl 2-(4-{[(4-{[4-(4-methoxyphenyl)-1-piperazinyl]methyl}-2-phenyl-1,3-thiazol-5-yl)methyl]sulfanyl}phenoxy)-2-methylpropanoate**

$^1$H NMR ($CDCl_3$) 400MHz δ 7.87(m, 2H), 7.40(m, 3H), 7.28(d, 2H, J=8.89 Hz), 6.89(d, 2H, J=9.23 Hz), 6.82 (d, 2H, J=9.23 Hz), 6.75(d, 2H, J=8.89 Hz), 4.33(s, 2H), 4.19(q, 2H, J=7.18 Hz), 3.76(s, 3H), 3.56(s, 2H), 3.09(br s, 4H), 2.65(br s, 4H), 1.58(s, 6H), 1.20(t, 3H, J=7.18 Hz),

**Ethyl 2-{4-[({4-{[4-(2-methoxyphenyl)-1-piperazinyl]methyl}-2-[4-(trifluoromethyl)phenyl]-1,3-thiazol-5-yl}methyl)sulfanyl]-2-methylphenoxy}propanoate**

$^1$H NMR ($CDCl_3$) 300MHz δ 8.03(d, 2H, J=8.28 Hz), 7.69(d, 2H, J=8.28 Hz), 7.28(d, 1H, J=2.21 Hz), 7.17(dd, 1H, J=8.28, 2.21 Hz), 7.00(m, 3H), 6.88(d, 1H, J=7.73 Hz), 6.61(d, 1H, J=8.28 Hz), 4.74(q, 1H, J=6.81 Hz), 4.39(s, 2H), 4.21(q, 2H, J=7.17 Hz), 3.89(s, 3H), 3.63(s, 2H), 3.12(br s, 4H), 2.72(br s, 4H), 2.27(s, 3H), 1.65(d, 3H, J=6.81 Hz), 1.26(t, 3H, J=7.17 Hz),

**Ethyl 2-[2-methyl-4-({[2-[4-(trifluoromethyl)phenyl]-4-({4-[3-(trifluoromethyl)phenyl]-1-piperazinyl}methyl)-1,3-thiazol-5-yl]methyl}sulfanyl)phenoxy]propanoate**

$^1$H NMR ($CDCl_3$) 300MHz δ 8.03(d, 2H, J=8.28 Hz), 7.70(d, 2H, J=8.28 Hz), 7.36(t, 1H, J=8.00 Hz), 7.29(d, 1H, J=2.21 Hz), 7.13(m, 4H), 6.61 (d, 1H, J=8.28 Hz), 4.74(q, 1H, J=6.90 Hz), 4.36(s, 2H), 4.18(q, 2H, J=7.08 Hz), 3.62(s, 2H), 3.26(t, 4H, J=4.83 Hz), 2.65(t, 4H, J=4.83 Hz), 2.26(s, 3H), 1.65(d, 3H, J=6.90 Hz), 1.27(t, 3H, J=7.08 Hz),

**Ethyl 2-{2-methyl-4-[({4-({4-[2-oxo-2-(1-pyrrolidinyl)ethyl]-1-piperazinyl}methyl)-2-[4-(trifluoromethyl)phenyl]-1,3-thiazol-5-yl}methyl)sulfanyl]phenoxy}propanoate**

$^1$H NMR ($CDCl_3$) 300MHz δ 7.96(d, 2H, J=8.28 Hz), 7.63(d, 2H, J=8.28 Hz), 7.20(d, 1 H, J=2.21 Hz), 7.10 (dd, 1H, J=8.28, 2.21 Hz), 6.56(d, 1H, J=8.28 Hz), 4.69(q, 1H, J=6.71 Hz), 4.30(s, 2H), 4.16(q, 2H, J=7.08 Hz), 3.47 (m, 8H), 3.10(s, 2H), 2.54(m, 6H), 2.20(s, 3H), 1.85(m, 4H), 1.60(d, 3H, J=6.71 Hz), 1.20(t, 3H, J=7.08 Hz),

**Ethyl 2-{2-methyl-4-[({4-{[4-(2-pyrimidinyl)-1-piperazinyl]methyl}-2-[4-(trifluoromethyl)phenyl]-1,3-thiazol-5-yl}methyl)sulfanyl]phenoxy}propanoate**

$^1$H NMR ($CDCl_3$) 300MHz δ 8.31(d, 2H, J=4.69 Hz), 8.01(d, 2H, J=8.28 Hz), 7.68(d, 2H, J=8.28 Hz), 7.27(d, 1H, J=2.21 Hz), 7.16(dd, 1H, J=8.28, 2.21 Hz), 6.60(d, 1H, J=8.28 Hz), 6.48(t, 1H, J=4.69 Hz), 4.74(q, 1H, J=6.71 Hz), 4.35(s, 2H), 4.20(q, 2H, J=7.08 Hz), 3.85(t, 4H, J=4.97 Hz), 3.57(s, 2H), 2.54(t, 4H, J=4.97 Hz), 2.24(s, 3H), 1.64(d, 3H, J=6.71 Hz), 1.24(t, 3H, J=7.08 Hz),

**Ethyl 2-{2-methyl-4-[({4-{(4-(2-pyrazinyl)-1-piperazinyl]methyl}-2-[4-(trifluoromethyl)phenyl]-1,3-thiazol-5-yl}methyl)sulfanyl]phenoxy}propanoate**

$^1$H NMR ($CDCl_3$) 300MHz δ 8.14(m, 1H), 8.06(m, 1H), 8.01(d, 2H, J=8.28 Hz), 7.85(d, 1H, J=2.48 Hz), 7.67 (d, 2H, J=8.28 Hz), 7.27(d, 1H, J=2.21 Hz), 7.15(dd, 1H, J=8.28, 2.21 Hz), 6.59(d, 1H, J=8.28 Hz), 4.73(q, 1H, J=6.71 Hz), 4.33(s, 2H), 4.16(q, 2H, J=7.17 Hz), 3.60(m, 6H), 2.58(t, 4H, J=4.83 Hz), 2.25(s, 3H), 1.64(d, 3H, J=6.71 Hz), 1.25(t, 3H, J=7.17 Hz),

**Ethyl 2-[2-methyl-4-({[2-[4-(trifluoromethyl)phenyl]-4-({4-[4-(trifluoromethyl)phenyl]-1-piperazinyl}methyl)-1,3-thiazol-5-yl]methyl}sulfanyl)phenoxy]propanoate**

$^1$H NMR ($CDCl_3$) 300MHz δ 8.03(d, 2H, J=8.28 Hz), 7.70(d, 2H, J=8.28 Hz), 7.51(d, 2H, J=8.55 Hz), 7.28(d, 1H, J=2.21 Hz), 7.18(dd, 1H, J=8.28, 2.21 Hz), 6.94(d, 2H, J=8.55 Hz), 6.61(d, 1H, J=8.28 Hz), 4.74(q, 1H, J=6.71 Hz), 4.35(s, 2H), 4.21 (q, 2H, J=7.17 Hz), 3.62(s, 2H), 3.33(t, 4H, J=4.55 Hz), 2.66(t, 4H, J=4.55 Hz), 2.27(s, 3H), 1.66 (d, 3H, J=6.71 Hz), 1.26(t, 3H, J=7.17 Hz),

**Ethyl 2-{4-[({4-[(4-acetyl-1-piperazinyl)methyl]-2-[4-(trifluoromethyl)phenyl]-1,3-thiazol-5-yl}methyl)sulfanyl]-2-isopropylphenoxy}propanoate**

$^1$H NMR ($CDCl_3$) 400MHz δ 7.94(d, 2H, J=8.10 Hz), 7.64(d, 2H, J=8.10 Hz), 7.17(d, 1H, J=2.24 Hz), 7.11 (dd, 1 H, J=8.45, 2.24 Hz), 6.54(d, 1H, J=8.45 Hz), 4.72(q, 1H, J=6.78 Hz), 4.23(s, 2H), 4.14(q, 2H, J=7.13 Hz), 3.59(s, 2H), 3.42(br s, 4H), 3.30(m, 1H), 2.42(br s, 4H), 2.04(s, 3H), 1.59(d, 3H, J=6.78 Hz), 1.17(m, 9H),

**Ethyl 2-{4-[({4-{[4-(4-fluorophenyl)-1-piperazinyl]methyl}-2-[4-(trifluoromethyl)phenyl]-1,3-thiazol-5-yl}methyl)sulfanyl]-2-isopropylphenoxy}propanoate**

$^1$H NMR ($CDCl_3$) 400MHz δ 7.97(d, 2H, J=8.28 Hz), 7.64(d, 2H, J=8.28 Hz), 7.20(d, 1H, J=2.24 Hz), 7.13(dd, 1H, J=8.45, 2.24 Hz), 6.92(m, 2H), 6.83(m, 2H), 6.55(d, 1H, J=8.45 Hz), 4.71(q, 1H, J=6.78 Hz), 4.28(s, 2H), 4.14(q,

2H, J=7.18 Hz), 3.48(s, 2H), 3.31 (m, 1H), 3.07(t, 4H, J=4.83 Hz), 2.59(br s, 4H), 1.59(d, 3H, J=6.78 Hz), 1.15(m, 9H),

**Ethyl 2-{2-isopropyl-4-[({4-(4-morpholinylmethyl)-2-[4-(trifluoromethyl)phenyl]-1,3-thiazol-5-yl}methyl) sulfanyl]phenoxy}propanoate**

$^1$H NMR ($CDCl_3$) 400MHz δ 7.95(d, 2H, J=8.28 Hz), 7.63(d, 2H, J=8.28 Hz), 7.19(d, 1H, J=2.24 Hz), 7.12(dd, 1H, J=8.45, 2.24 Hz), 6.55(d, 1H, J=8.45 Hz), 4.71 (q, 1H, J=6.78 Hz), 4.26(s, 2H), 4.14(q, 2H, J=7.13 Hz), 3.67(m, 4H), 3.41(s, 2H), 3.30(m, 1H), 2.42(br s, 4H), 1.59(d, 3H, J=6.78 Hz), 1.16(m, 9H),

**Ethyl 2-{2-methyl-4-[({4-(1-piperazinylmethyl)-2-[4-(trifluoromethyl)phenyl]-1,3-thiazol-5-yl}methyl)sulfanyl] phenoxy}propanoate**

$^1$H NMR ($CDCl_3$) 300MHz δ 8.01(d, 2H, J=8.23 Hz), 7.67(d, 2H, J=8.23 Hz), 7.27(d, 1H, J=2.39 Hz), 7.15(dd, 1H, J=8.23, 2.39 Hz), 6.59(d, 1H, J=8.23 Hz), 4.73(q, 1H, J=6.64 Hz), 4.34(s, 2H), 4.20(q, 2H, J=7.08 Hz), 3.52(s, 2H), 2.91 (t, 4H, J=4.91 Hz), 2.46(m, 4H), 2.33(br s, 1H), 2.26(s, 3H), 1.64(d, 3H, J=6.64 Hz), 1.25(t, 3H, J=7.08 Hz),

***tert*-Butyl 4-({5-({[4-(2-ethoxy-1-methyl-2-oxoethoxy)-3-methylphenyl]sulfanyl}methyl)-2-[4-(trifluoromethyl) phenyl]-1,3-thiazol-4-yl}methyl)-1-piperazinecarboxylate**

$^1$H NMR ($CDCl_3$) 300MHz δ 8.01(d, 2H, J=8.23 Hz), 7.68(d, 2H, J=8.23 Hz), 7.27(d, 1H, J=2.39 Hz), 7.15(dd, 1H, J=8.49, 2.39 Hz), 6.60(d, 1H, J=8.49 Hz), 4.74(q, 1H, J=6.72 Hz), 4.33(s, 2H), 4.22(q, 2H, J=7.08 Hz), 3.54(s, 2H), 3.46(m, 4H), 2.44(m, 4H), 2.27(s, 3H), 1.65(d, 3H, J=6.72 Hz), 1.48(s, 9H), 1.26(t, 3H, J=7.08 Hz),

**Ethyl 2-{4-[({4-{[4-(4-chlorophenyl)-1-piperazinyl]methyl}-2-[4-(trifluoromethyl)phenyl]-1,3-thiazol-5-yl}methyl) sulfanyl]-2-methylphenoxy}propanoate**

$^1$H NMR ($CDCl_3$) 300MHz δ 8.03(d, 2H, J=8.23 Hz), 7.70(d, 2H, J=8.23 Hz), 7.22(m, 4H), 6.86(d, 2H, J=9.03 Hz), 6.61 (d, 1H, J=8.49 Hz), 4.73(q, 1H, J=6.81 Hz), 4.36(s, 2H), 4.18(q, 2H, J=7.08 Hz), 3.61(s, 2H), 3.17(m, 4H), 2.64(m, 4H), 2.27(s, 3H), 1.65(d, 3H, J=6 84 Hz), 1.27(t, 3H, J=7.08 Hz),

**Ethyl 2-(4-[({4-[(3,5-dimethyl-1-piperazinyl)methyl]-2-[4-(trifluoromethyl)phenyl]-1,3-thiazol-5-yl}methyl) sulfanyl]-2-methylphenoxy}propanoate**

$^1$H NMR ($CDCl_3$) 300MHz δ 8.01(d, 2H, J=8.23 Hz), 7.68(d, 2H, J=8.23 Hz), 7.27(d, 1H, J=2.39 Hz), 7.15(dd, 1H, J=8.49, 2.39 Hz), 6.60(d, 1H, J=8.49 Hz), 4.74(q, 1H, J=6.72 Hz), 4.35(s, 2H), 4.21 (q, 2H. J=7.08 Hz), 3.53(s, 2H), 2.96(m, 2H), 2.78(m, 2H), 2.26(s, 3H), 1.73(m, 2H), 1.65(d, 3H, J=6.72 Hz), 1.26(t, 3H, J=7.08 Hz), 1.09(d, 6H, J=6.37 Hz),

**Ethyl 2-{4-[({4-{[4-(4-fluorophenyl)-1-piperazinyl]methyl}-2-[4-(trifluoromethyl)phenyl]-1,3-thiazol-5-yl}methyl) sulfanyl]-2-methylphenoxy}propanoate**

$^1$H NMR ($CDCl_3$) 300MHz δ 8.03(d, 2H, J=8.49 Hz), 7.70(d, 2H, J=8.49 Hz), 7.28(d, 1H, J=2.39 Hz), 7.18(dd. 1H, J=8.23, 2.39 Hz), 6.94(m, 4H), 6.62(d, 1H, J=823 Hz), 4.74(q, 1H, J=6.72 Hz), 4.37(s, 2H), 4.21 (q, 2H, J=7.08 Hz), 3.63(s, 2H), 3.14(t, 4H, J=4.51 Hz), 2.67(t, 4H, J=4.51 Hz), 2.2.8(s, 3H), 1.65(d, 3H, J=6.72 Hz), 1.26(t, 3H, J=7.08 Hz),

**Ethyl 2-{4-[({4-{[4-(4-acetylphenyl)-1-piperazinyl]methyl}-2-[4-(trifluoromethyl)phenyl]-1,3-thiazol-5-yl}methyl) sulfanyl]-2-methylphenoxy}propanoate**

$^1$H NMR ($CDCl_3$) 300MHz δ 8.02(d, 2H, J=8.23 Hz), 7.89(d, 2H, J=8.76 Hz), 7.69(d, 2H, J=8.23 Hz), 7.28(br s, 1H), 7.17(dd, 1H, J=8.23, 2.39 Hz), 6.88(d, 2H, J=8.76 Hz), 6.60(d, 1H, J=8.23 Hz), 4.73(q, 1H, J=6.81 Hz), 4.34(s, 2H), 4.18(q. 2H, J=7.17 Hz), 3.60(s, 2H), 3.37(m, 4H), 2.63(m, 4H), 2.54(s, 3H), 2.26(s, 3H), 1.65(d, 3H, J=6.81 Hz), 1.27(t, 3H, J=7.17 Hz),

**Ethyl 4-({5-({[4-(2-ethoxy-1-methyl-2-oxoethoxy)-3-methylphenyl]sulfanyl}methyl)-2-[4-(trifluoromethyl) phenyl]-1,3-thiazol-4-yl}methyl)-1-piperazinecarboxylate**

$^1$H NMR ($CDCl_3$) 300MHz δ 8.00(d, 2H, J=8.23 Hz), 7.68(d, 2H, J=8.23 Hz), 7.27(d, 1H, J=2.39 Hz), 7.14(dd,

1H, J=8.23, 2.39 Hz), 6.60(d, 1H, J=8.23 Hz), 4.73(q, 1H, J=6.81 Hz), 4.31 (s, 2H), 4.18(m, 4H), 3.50(m, 6H), 2.44(m, 4H), 2.26(s, 3H), 1.65(d, 3H, J=6.81 Hz), 1.26(m, 6H),

**Ethyl 2-{2-methyl-4-[({4-(4-morpholinylmethyl)-2-[4-(trifluoromethyl)phenyl]-1,3-thiazol-5-yl}methyl)sulfanyl] phenoxy}propanoate**

$^1$H NMR ($CDCl_3$) 300MHz δ 8.01(d, 2H, J=8.23 Hz), 7.68(d, 2H, J=8.23 Hz). 7.27(d. 1H, J=2.39 Hz), 7.16(dd, 1H, J=8.49, 2.39 Hz), 6.60(d, 1H, J=8.49 Hz), 4.73(q, 1H, J=6.72 Hz), 4.34(s, 2H), 4.21(q, 2H, J=7.08 Hz), 3.73(t, 4H, J=4.51 Hz), 3.54(s, 2H), 2.49(t, 4H, J=4.51 Hz), 2.26(s, 3H), 1.65(d, 3H, J=6.72 Hz), 1.26(t, 3H, J=7.08 Hz),

**Ethyl 2-[4-[({4-{[4-(3-methoxyphenyl)-1-piperazinyl]methyl}-2-[4-(trifluoromethyl)phenyl]-1,3-thiazol-5-yl} methyl)sulfanyl]-2-methylphenoxy}propanoate**

$^1$H NMR ($CDCl_3$) 300MHz δ 8.04(d, 2H, J=8.23 Hz), 7.70(d, 2H, J=823 Hz), 728(m, 1H), 7.18(m, 2H), 6.62 (d, 1H, J=8.23 Hz), 6.56(dd, 1H, J=8.23, 2.39 Hz), 6.50(t, 1H, J=2.26 Hz), 6.45(dd, 1H, J=8.23, 2.39 Hz), 4.74(q, 1H, J=6.81 Hz), 4.37(s, 2H), 4.21(q, 2H, J=7.08 Hz), 3.82(s, 3H), 3.61(s, 2H), 3.22(t, 4H, J=4.65 Hz), 2.65(t, 4H, J=4.65 Hz), 2.28(s, 3H), 1.66(d, 3H, J=6.81 Hz), 1.26(t, 3H, J=7.08 Hz),

**Ethyl 2-{4-[({4-[(4-acetyl-1-piperazinyl)methyl]-2-[4-(trifluoromethyl)phenyl]-1,3-thiazol-5-yl}methyl)sulfanyl]-2-propylphenoxy}propanoate**

$^1$H NMR ($CDCl_3$) 400MHz δ 7.95(d, 2H, J=8.06 Hz), 7.63(d, 2H, J=8.06 Hz), 7.16(d, 1H, J=2.38 Hz), 7.11(dd, 1H, J=8.24, 2.38 Hz), 6.55(d, 1H, J=8.24 Hz), 4.70(q, 1H, J=6.84 Hz), 4.23(s, 2H), 4.13(q, 2H, J=7.14 Hz), 3.59(br s, 2H), 3.47(s, 2H), 3.40(t, 2H, J=4.58 Hz), 2.55(t, 2H, J=7.33 Hz), 2.40(m, 4H), 2.05(s, 3H), 1.56(m, 5H), 1.20(t, 3H, J=7.14 Hz), 0.86(t, 3H, J=7.33. Hz),

**Ethyl 2-{4-[({4-{[4-(4-fluorophenyl)-1-piperazinyl]methyl}-2-[4-(trifluoromethyl)phenyl]-1,3-thiazol-5-yl}methyl) sulfanyl]-2-propylphenoxy}propanoate**

$^1$H NMR ($CDCl_3$) 400MHz δ 7.97(d, 2H, J=8.24 Hz), 7.64(d, 2H, J=8.24 Hz), 7.19(d, 1H, J=2.38 Hz), 7.14(dd, 1H, J=8.42, 2.38 Hz), 6.93(m, 2H), 6.84(m, 2H), 6.56(d, 1H, J=8.42 Hz), 4.69(q, 1H, J=6.78 Hz), 4.30(s, 2H), 4.14(q, 2H, J=7.14 Hz), 3.54(s, 2H), 3.07(t, 4H, J=4.58 Hz), 2.58(m, 6H), 1.57(m, 5H), 1.22(t, 3H, J=7.14 Hz), 0.86(t, 3H, J=7.33 Hz),

**Ethyl 2-{4-[({4-(4-morpholinylmethyl)-2-[4-(trifluoromethyl)phenyl]-1,3-thiazol-5-yl}methyl)sulfanyl]-2-propylphenoxy}propanoate**

$^1$H NMR ($CDCl_3$) 400MHz δ 7.95(d, 2H, J=8.24 Hz), 7.63(d, 2H, J=8.24 Hz), 7.17(d, 1H, J=2.38 Hz), 7.12(dd, 1H, J=8.42, 2.38 Hz), 6.55(d, 1H, J=8.42 Hz), 4.69(q, 1H, J=6.78 Hz), 4.27(s, 2H), 4.14(q, 2H, J=7.14 Hz), 3.66(t, 4H, J=4.67 Hz), 3.45(s, 2H), 2.56(t, 2H, J=7.33 Hz), 2.42(m, 4H), 1.56(m, 5H), 1.21(t, 3H, J=7.14 Hz), 0.86(t, 3H, J=7.33 Hz),

**Methyl {4-[({4-[(4-acetyl-1-piperazinyl)methyl]-2-[4-(trifluoromethyl)phenyl]-1,3-thiazol-5-yl}methyl)sulfanyl]-2-isopropylphenoxy}acetate**

$^1$H NMR ($CDCl_3$) 400MHz δ 7.95(d, 2H, J=8.61 Hz), 7.64(d, 2H, J=8.61 Hz), 7.20(d, 1H, J=2.20 Hz), 7.15(dd, 1H, J=8.42, 2.20 Hz), 6.59(d, 1H, J=8.42 Hz), 4.63(s, 2H), 4.25(s, 2H), 3.76(s, 3H), 3.56(s, 2H), 3.41 (m, 4H), 3.31 (m, 1H), 2.38(m, 4H), 2.05(s, 3H), 1.11(d, 6H, J=6.78 Hz),

**Methyl {4-[({4-{[4-(4-fluorophenyl)-1-piperazinyl]methyl}-2-[4-(trifluoromethyl)phenyl]-1,3-thiazol-5-yl}methyl) sulfanyl]-2-isopropylphenoxy}acetate**

$^1$H NMR ($CDCl_3$) 400MHz δ 7.98(d, 2H, J=8.24 Hz), 7.65(d, 2H, J=8.24 Hz), 7.23(d, 1H, J=2.20 Hz), 7.18(dd, 1H, J=8.42, 2.20 Hz), 6.94(m, 2H), 6.83(m, 2H), 6.60(d, 1H, J=8.42 Hz), 4.61(s, 2H), 4.30(s, 2H), 3.76(s, 3H), 3.49(s, 2H), 3.34(m, 1H), 3.07(t, 4H, J=4.58 Hz), 2.59(m, 4H), 1.13(d, 6H, J=6.96 Hz),

**Methyl {2-isopropyl-4-[({4-(4-morpholinylmethyl)-2-[4-(trifluoromethyl)phenyl]-1,3-thiazol-5-yl}methyl) sulfanyl]phenoxy}acetate**

$^1H$ NMR ($CDCl_3$) 400MHz δ 7.96(d, 2H, J=8.24 Hz), 7.64(d, 2H, J=8.24 Hz), 7.21 (d, 1H, J=2.38 Hz), 7.16 (dd, 1H, J=8.42, 2.38 Hz), 6.59(d, 1H, J=8.42 Hz), 4.62(s, 2H), 4.28(s, 2H), 3.76(s, 3H), 3.66(t, 4H, J=4.58 Hz), 3.41 (s, 2H), 3.32(m, 1H), 2.42(m, 4H), 1.15(d, 6H, J=6.96 Hz),

**Methyl {2-isopropyl-4-[({4-{[4-(4-methoxyphenyl)-1-piperazinyl]methyl}-2-[4-(trifluoromethyl)phenyl]-1,3-thiazol-5-yl}methyl)sulfanyl]phenoxy}acetate**

$^1H$ NMR ($CDCl_3$) 400MHz δ 7.98(d, 2H, J=8.24 Hz), 7.65(d, 2H, J=8.24 Hz), 7.23(d, 1H, J=2.20 Hz), 7.18(dd, 1H, J=8.42, 220 Hz), 6.87(d, 2H, J=9.16 Hz), 6.81(d, 2H, J=9.16 Hz), 6.60(d, 1H, J=8.42 Hz), 4.61 (m, 2H), 4.31 (s, 2H), 3.77(s, 3H), 3.74(s, 3H), 3.50(s, 2H), 3.33(m, 1H), 3.05(m, 4H), 2.60(br s, 4H), 1.15(d, 6H, J=6.96 Hz),

**Methyl {4-[({4-{[4-(4-acetylphenyl)-1-piperazinyl]methyl}-2-[4-(trifluoromethyl)phenyl]-1,3-thiazol-5-yl}methyl) sulfanyl]-2-isopropylphenoxy}acetate**

$^1H$ NMR ($CDCl_3$) 400MHz δ 7.95(d, 2H, J=8.28 Hz), 7.84(d, 2H, J=9.14 Hz), 7.62(d, 2H, J=8.28 Hz), 7.21 (d, 1H, J=2.24 Hz), 7.16(dd, 1H, J=8.45, 2.24 Hz), 6.80(d, 2H, J=9.14 Hz), 6.58(d, 1H, J=8.45 Hz), 4.59(s, 2H), 4.27(s, 2H), 3.73(s, 3H), 3.46(s, 2H), 3.30(m, 5H), 2.54(t, 4H, J=4.57 Hz), 2.47(s, 3H), 1.12(d, 6H, J=6.90 Hz),

**Methyl {2-isopropyl-4-[({4-{[4-(3-methoxyphenyl)-1-piperazinyl]methyl}-2-[4-(trifluoromethyl)phenyl]-1,3-thiazol-5-yl}methyl)sulfanyl]phenoxy}acetate**

$^1H$ NMR ($CDCl_3$) 400MHz δ 7.98(d, 2H, J=8.28 Hz), 7.65(d, 2H, J=8.28 Hz), 7.24(d, 1H, J=2.38 Hz), 7.19(dd, 1H, J=8.42, 2.38 Hz), 7.14(t, 1 H, J=8.24 Hz), 6.60(d, 1H, J=8.42 Hz), 6.51 (dd, 1H, J=8.24, 2.38 Hz), 6.44(t, 1H, J=2.29 Hz), 6.39(dd, 1H, J=8.24, 2.38 Hz), 4.62(s, 2H), 4.30(s, 2H), 3.75(m, 6H), 3.48(s, 2H), 3.34(m, 1H), 3.16(t, 4H, J=4.67 Hz), 2.57(t, 4H, J=4.67 Hz), 1.14(d, 6H, J=6.78 Hz),

**Ethyl 2-{2-isopropyl-4-[({4-{[4-(4-methoxyphenyl)-1-piperazinyl]methyl}-2-[4-(trifluoromethyl)phenyl]-1,3-thiazol-5-yl}methyl)sulfanyl]phenoxy}propanoate**

$^1H$ NMR ($CDCl_3$) 400MHz δ 8.04(d, 2H, J=8.24 Hz), 7.71(d, 2H, J=8.24 Hz), 7.16(m, 2H), 6.87(d, 2H, J=9.16 Hz), 6.78(d, 2H, J=9.16 Hz), 6.64(d, 1H, J=8.42 Hz), 4.81(q, 1H, J=6.71 Hz), 4.27(s, 2H), 4.11 (q, 2H, J=7.08 Hz), 3.69 (s, 3H), 3.28(m, 3H), 2.96(t, 4H, J=4.94 Hz), 2.51 (t, 4H, J=4.94 Hz), 1.54(d, 3H, J=6.71 Hz), 1.12(m, 9H),

**Ethyl 2-{4-[({4-{[4-(4-acetylphenyl)-1-piperazinyl]methyl}-2-[4-(trifluoromethyl)phenyl]-1,3-thiazol-5-yl}methyl) sulfanyl]-2-isopropylphenoxy}propanoate**

$^1H$ NMR ($CDCl_3$) 400MHz δ 7.96(d, 2H, J=8.28 Hz), 7.83(d, 2H, J=9.14 Hz), 7.64(d, 2H, J=8.28 Hz), 7.19(d, 1H, J=2.24 Hz), 7.12(dd, 1H, J=8.45, 2.24 Hz), 6.81 (d, 2H, J=9.14 Hz), 6.55(d, 1H, J=8.45 Hz), 4.71(q, 1H, J=6.78 Hz), 4.26(s, 2H), 4.12(q, 2H, J=7.16 Hz), 3.47(s, 2H), 3.29(m, 5H), 2.56(br s, 4H), 2.48(s, 3H), 1.58(d, 3H, J=6.78 Hz), 1.15(m, 9H),

**Ethyl 2-{2-isopropyl-4-[({4-{[4-(3-methoxyphenyl)-1-piperazinyl]methyl}-2-[4-(trifluoromethyl)phenyl]-1,3-thiazol-5-yl}methyl)sulfanyl]phenoxy}propanoate**

$^1H$ NMR ($CDCl_3$) 400MHz δ 7.98(d, 2H, J=8.24 Hz), 7.65(d, 2H, J=8.24 Hz), 7.21(d, 1H, J=2.38 Hz), 7.14(m, 2H), 6.58(d, 1H, J=8.61 Hz), 6.51(dd, 1H, J=8.24, 2.20 Hz), 6.43(t, 1H, J=2.29 Hz), 6.39(dd, 1H, J=8.24, 2.20 Hz), 4.72(q, 1H, J=6.78 Hz), 4.29(s, 2H), 4.15(q, 2H, J=7.14 Hz), 3.76(s, 3H), 3.48(s, 2H), 3.33(m, 1 H), 3.16(br s, 4H), 2.59(br s, 4H), 1.60(d, 3H, J=6.78 Hz), 1.16(m, 9H),

**Ethyl {4-[({4-(4-morpholinylmethyl)-2-[4-(trifluoromethyl)phenyl]-1,3-thiazol-5-yl}methyl)sulfanyl]-2-propylphenoxy}acetate**

$^1H$ NMR ($CDCl_3$) 400MHz δ 7.95(d, 2H, J=8.24 Hz), 7.63(d, 2H, J=8.24 Hz), 7.19(m, 2H), 6.58(d, 1H, J=8.24 Hz), 4.59(s, 2H), 4.28(s, 2H), 4.21 (q, 2H, J=7.14 Hz), 3.66(t, 4H, J=4.49 Hz), 3.45(s, 2H), 2.56(t, 2H, J=7.33 Hz), 2.42 (m, 4H), 1.56(m, 2H), 1.24(t, 3H, J=7.14 Hz), 0.87(t, 3H, J=7.33 Hz),

**Ethyl {4-[({4-{[4-(4-acetylphenyl)-1-piperazinyl]methyl}-2-[4-(trifluoromethyl)phenyl]-1,3-thiazol-5-yl}methyl) sulfanyl]-2-ethylphenoxy}acetate**

$^1$H NMR ($CDCl_3$) 400MHz δ 7.97(d, 2H, J=8.24 Hz), 7.83(d, 2H, J=9.16 Hz), 7.63(d, 2H, J=8.24 Hz), 7.21(d, 1H, J=2.20 Hz), 7.16(dd, 1H, J=8.42, 2.20 Hz), 6.82(d, 2H, J=9.16 Hz), 6.59(d, 1H, J=8.42 Hz), 4.59(s, 2H), 4.29(s, 2H), 4.21 (q, 2H, J=7.14 Hz), 3.52(s, 2H), 3.31 (t, 4H, J=4.80 Hz), 2.64(q, 2H, J=7.51 Hz), 2.55(t, 4H, J=4.80 Hz), 2.47 (s, 3H), 1.24(t, 3H, J=7.14 Hz), 1.14(t, 3H, J=7.51 Hz),

**Ethyl {2-ethyl-4-[({4-{[4-(3-methoxyphenyl)-1-piperazinyl]methyl}-2-[4-(trifluoromethyl)phenyl]-1,3-thiazol-5-yl}methyl)sulfanyl]phenoxy}acetate**

$^1$H NMR ($CDCl_3$) 400MHz δ 7.98(d, 2H, J=8.24 Hz), 7.65(d, 2H, J=8.24 Hz), 7.22(s, 1H), 7.16(m, 2H), 6.60 (d, 1H, J=8.42 Hz), 6.51(d, 1H, J=8.42 Hz), 6.44(s, 1H), 6.39(dd, 1H, J=8.24, 1.28 Hz), 4.60(s, 2H), 4.32(s, 2H), 4.22 (q, 2H, J=7.14 Hz), 3.76(s, 3H), 3.52(s, 2H), 3.16(t, 4H, J=4.67 Hz), 2.65(q, 2H, J=7.51 Hz), 2.57(t, 4H, J=4.67 Hz), 1.26(t, 3H, J=7.14 Hz), 1.16(t, 3H, J=7.51 Hz),

**Ethyl {4-[({4-[(4-acetyl-1-piperazinyl)methyl]-2-[4-(trifluoromethyl)phenyl]-1,3-thiazol-5-yl}methyl)sulfanyl]-2-ethylphenoxy}acetate**

$^1$H NMR ($CDCl_3$) 400MHz δ 7.93(d, 2H, J=8.28 Hz), 7.61(d, 2H, J=8.28 Hz), 7.16(d, 1H, J=2.24 Hz), 7.12(dd, 1H, J=8.28, 2.24 Hz), 6.56(d,1 H, J=8.28 Hz), 4.58(s, 2H), 4.24(m, 4H), 3.55(t, 4H, J=4.91 Hz), 3.43(s, 2H), 3.37(t, 4H, J=4.91 Hz), 2.60(q, 2H, J=7.50 Hz), 2.02(s, 3H), 1.22(t, 3H, J=7.14 Hz), 1.11(t, 3H, J=7.50 Hz),

**Ethyl {2-ethyl-4-[({4-{[4-(4-fluorophenyl)-1-piperazinyl]methyl}-2-[4-(trifluoromethyl)phenyl]-1,3-thiazol-5-yl} methyl)sulfanyl]phenoxy}acetate**

$^1$H NMR ($CDCl_3$) 400MHz δ 8.03(d, 2H, J=8.28 Hz), 7.69(d, 2H, J=8.28 Hz), 7.25(m, 2H), 6.93(m, 4H), 6.64 (d, 1H, J=8.28 Hz), 4.64(s, 2H), 4.36(s, 2H), 4.26(q, 2H, J=7.08 Hz), 3.58(s, 2H), 3.11(t, 4H, J=4.97 Hz), 2.66(m, 6H), 1.29(t, 3H, J=7.08 Hz), 1.19(t, 3H, J=7.54 Hz),

**Ethyl {2-ethyl-4-[({4-(4-morpholinylmethyl)-2-[4-(trifluoromethyl)phenyl]-1,3-thiazol-5-yl}methyl)sulfanyl] phenoxy}acetate**

$^1$H NMR ($CDCl_3$) 400MHz δ 8.01 (d, 2H, J=8.28 Hz), 7.68(d, 2H, J=8.28 Hz), 7.24(m, 2H), 6.63(d, 1H, J=8.28 Hz), 4.64(s, 2H), 4.34(s, 2H), 4.26(q, 2H, J=7.17 Hz), 3.70(t, 4H, J=4.42 Hz), 3.49(s, 2H), 2.67(q, 2H, J=7.54 Hz), 2.46 (t, 4H, J=4.42 Hz), 1.30(t, 3H, J=7.17 Hz), 1.19(t, 3H, J=7.54 Hz),

**Ethyl 2-{2-ethyl-4-[({4-{[4-(4-methoxyphenyl)-1-piperazinyl]methyl}-2-[4-(trifluoromethyl)phenyl]-1,3-thiazol-5-yl}methyl)sulfanyl]phenoxy}propanoate**

$^1$H NMR ($CDCl_3$) 400MHz δ 8.03(d, 2H, J=8.28 Hz), 7.70(d, 2H, J=8.28 Hz), 7.26(d, 1 H, J=2.21 Hz), 7.19 (dd, 1H, J=8.28, 2.21 Hz), 6.93(d, 2H, J=9.11 Hz), 6.86(d, 2H, J=9.11 Hz), 6.62(d, 1H, J=8.28 Hz), 4.76(q, 1H, J=6.90 Hz), 4.36(s, 2H), 4.19(q, 2H, J=7.17 Hz), 3.80(s, 3H), 3.58(s, 2H), 3.11(t, 4H, J=4.69 Hz), 2.67(m, 6H), 1.65(d, 3H, J=6.90 Hz), 1.24(m, 6H),

**Ethyl 2-{4-[({4-{[4-(4-acetylphenyl)-1-piperazinyl]methyl}-2-[4-(trifluoromethyl)phenyl]-1,3-thiazol-5-yl}methyl) sulfanyl]-2-ethylphenoxy}propanoate**

$^1$H NMR ($CDCl_3$) 400MHz δ 8.02(d, 2H, J=8.28 Hz), 7.89(d, 2H, J=8.83 Hz), 7.69(d, 2H, J=8.28 Hz), 7.25(d, 1H, J=2.21 Hz), 7.18(dd, 1H, J=8.28, 2.21 Hz), 6.88(d, 2H, J=8.83 Hz), 6.61(d, 1H, J=8.28 Hz), 4.76(q, 1H, J=6.90 Hz), 4.33(s, 2H), 4.18(q, 2H, J=7.17 Hz), 3.57(s, 2H), 3.36(m, 4H), 2.66(m, 6H), 2.53(s, 3H), 1.65(d, 3H, J=6.90 Hz), 1.23(m, 6H),

**Ethyl 2-{2-ethyl-4-[({4-{[4-(3-methoxyphenyl)-1-piperazinyl]methyl}-2-[4-(trifluoromethyl)phenyl]-1,3-thiazol-5-yl}methyl)sulfanyl]phenoxy}propanoate**

$^1$H NMR ($CDCl_3$) 400MHz δ 8.03(d, 2H, J=8.28 Hz), 7.70(d, 2H, J=8.28 Hz), 7.26(d, 1H, J=2.21 Hz), 7.18(m, 2H), 6.62(d, 1H, J=8.28 Hz), 6.56(dd, 1H, J=8.00, 1.66 Hz), 6.49(m, 1H), 6.44(dd, 1H, J=8.00, 1.66 Hz), 4.76(q, 1H,

J=6.62 Hz), 4.35(s, 2H), 4.19(q, 2H, J=7.17 Hz), 3.81(s, 3H), 3.57(s, 2H), 3.21 (t, 4H, J=4.83 Hz), 2.66(m, 6H), 1.65 (d, 3H, J=6.62 Hz), 1.24(m, 6H),

**Ethyl 2-{4-[({4-{[4-(4-methoxyphenyl)-1-piperazinyl]methyl}-2-[4-(trifluoromethyl)phenyl]-1,3-thiazol-5-yl}methyl)sulfanyl]-2-propylphenoxy}propanoate**

$^1H$ NMR ($CDCl_3$) 400MHz δ 7.97(d, 2H, J=8.24 Hz), 7.64(d, 2H, J=8.24 Hz), 7.19(d, 1 H, J=2.38 Hz), 7.14 (dd, 1H, J=8.42, 2.38 Hz), 6.88(d, 2H, J=9.16 Hz), 6.81 (d, 2H, J=9.16 Hz), 6.56(d, 1H, J=8.42 Hz), 4.70(q, 1H, J=6.78 Hz), 4.31 (s, 2H), 4.15(q, 2H, J=7.14 Hz), 3.74(s, 3H), 3.54(s, 2H), 3.05(t, 4H, J=4.85 Hz), 2.57(m, 6H), 1.56(m, 5H), 1.20(t, 3H, J=7.14 Hz), 0.86(t, 3H, J=7.33 Hz),

**Ethyl 2-{4-[({4-{[4-(4-acetylphenyl)-1-piperazinyl]methyl}-2-[4-(trifluoromethyl)phenyl]-1,3-thiazol-5-yl}methyl)sulfanyl]-2-propylphenoxy}propanoate**

$^1H$ NMR ($CDCl_3$) 400MHz δ 7.95(d, 2H, J=8.24 Hz), 7.84(d, 2H, J=9.14 Hz), 7.63(d, 2H, J=8.24 Hz), 7.17(d, 1H, J=2.24 Hz), 7.12(dd, 1H, J=8.45, 2.24 Hz), 6.82(d, 2H, J=9.14 Hz), 6.54(d, 1 H, J=8.45 Hz), 4.68(q, 1H, J=6.78 Hz), 4.27(s, 2H), 4.13(q, 2H, J=7.07 Hz), 3.51 (m, 2H), 3.31 (t, 4H, J=4.91 Hz), 2.55(m, 6H), 2.47(s, 3H), 1.55(m, 5H), 1.17(t, 3H, J=7.07 Hz), 0.85(t, 3H, J=7.41 Hz),

**Ethyl 2-{4-[({4-{[4-(3-methoxyphenyl)-1-piperazinyl]methyl}-2-[4-(trifluoromethyl)phenyl]-1,3-thiazol-5-yl}methyl)sulfanyl]-2-propylphenoxy}propanoate**

$^1H$ NMR ($CDCl_3$) 400MHz δ 7.97(d, 2H, J=8.28 Hz), 7.64(d, 2H, J=8.28 Hz), 7.15(m, 3H), 6.56(d, 1H, J=8.45 Hz), 6.50(dd, 1 H, J=8.10, 2.07 Hz), 6.43(t, 1H, J=2.07 Hz), 6.39(dd, 1H, J=8.10, 2.07 Hz), 4.70(q, 1H, J=6.72 Hz), 4.29(s, 2H), 4.14(q, 2H, J=7.07 Hz), 3.76(s, 3H), 3.52(s, 2H), 3.16(t, 4H, J=4.83 Hz), 2.58(m, 6H), 1.57(m, 5H), 1.19 (t, 3H, J=7.07 Hz), 0.87(t, 3H, J=7.33 Hz),

**Ethyl 2-(4-{[(2-(4-fluorophenyl)-4-{[4-(4-methoxyphenyl)-1-piperazinyl]methyl}-1,3-thiazol-5-yl)methyl]sulfanyl}-2-methylphenoxy)propanoate**

$^1H$ NMR ($CDCl_3$) 400MHz δ 7.85(m, 2H), 7.22(d, 1H, J=2.38 Hz), 7.09(m, 3H), 6.87(d, 2H, J=9.16 Hz), 6.81 (d, 2H, J=9.16 Hz), 6.56(d, 1H, J=8.42 Hz), 4.68(q, 1H, J=6.78 Hz), 4.30(s, 2H), 4.16(q, 2H, J=7.20 Hz), 3.74(s, 3H), 3.53(s, 2H), 3.07(t, 4H, J=4.58 Hz), 2.62(br s, 4H), 2.21 (s, 3H), 1.60(d, 3H, J=6.78 Hz), 1.20(t, 3H, J=7.20 Hz),

**Ethyl 2-[4-({[4-{[4-(4-acetylphenyl)-1-piperazinyl]methyl}-2-(4-fluorophenyl)-1,3-thiazol-5-yl]methyl}sulfanyl}-2-methylphenoxy]propanoate**

$^1H$ NMR ($CDCl_3$) 400MHz δ 7.85(m, 4H), 7.23(d, 1H, J=2.38 Hz), 7.09(m, 3H), 6.83(d, 2H, J=9.16 Hz), 6.55 (d, 1H, J=8.42 Hz), 4.68(q, 1H, J=6.78 Hz), 4.27(s, 2H), 4.16(q, 2H, J=7.14 Hz), 3.52(s, 2H), 3.32(t, 4H, J=4.94 Hz), 2.59(br s, 4H), 2.49(s, 3H), 2.21 (s, 3H), 1.60(d, 3H, J=6.78 Hz), 1.21(t, 3H, J=7.14 Hz),

**Ethyl 2-(4-{[(2-(4-fluorophenyl)-4-{[4-(3-methoxyphenyl)-1-piperazinyl]methyl}-1,3-thiazol-5-yl)methyl]sulfanyl}-2-methylphenoxy)propanoate**

$^1H$ NMR ($CDCl_3$) 400MHz δ 7.85(m, 2H), 7.23(d, 1H, J=2.20 Hz), 7.11(m, 4H), 6.56(d, 1H, J=8.24 Hz), 6.51 (dd, 1H, J=8.24, 2.20 Hz), 6.44(t, 1H, J=2.20 Hz), 6.39(dd, 1H, J=8.24, 2.20 Hz), 4.69(q, 1H, J=6.78 Hz), 4.29(s, 2H), 4.16(q, 2H, J=7.14 Hz), 3.76(s, 3H), 3.52(s, 2H), 3.16(t, 4H, J=4.76 Hz), 2.60(br s, 4H), 2.21(s, 3H), 1.59(d, 3H, J=6.78 Hz), 1.22(t, 3H, J=7.14 Hz),

**Ethyl 4-{[5-({[4-(2-ethoxy-1-methyl-2-oxoethoxy)-3-methylphenyl]sulfanyl}methyl)-2-(4-fluorophenyl)-1,3-thiazol-4-yl]methyl}-1-piperazinecarboxylate**

$^1H$ NMR ($CDCl_3$) 400MHz δ 7.83(m, 2H), 7.20(d, 1H, J=2.20 Hz), 7.08(m, 3H), 6.55(d, 1H, J=8.42 Hz), 4.68 (q, 1H, J=6.78 Hz), 4.23(s, 2H), 4.16(q, 2H, J=7.14 Hz), 4.09(q, 2H, J=7.14 Hz), 3.42(m, 6H), 2.38(br s, 4H), 2.18(s, 3H), 1.57(d, 3H, J=6.78 Hz), 1.13(m, 6H),

**Ethyl {2-ethyl-4-[({4-{[4-(4-methoxyphenyl)-1-piperazinyl]methyl}-2-[4-(trifluoromethyl)phenyl]-1,3-thiazol-5-yl}methyl)sulfanyl]phenoxy}acetate**

$^1$H NMR ($CDCl_3$) 400MHz δ 7.98(d, 2H, J=8.24 Hz), 7.65(d, 2H, J=8.24 Hz), 7.22(s, 1H), 7.17(d, 1H, J=8.42 Hz), 6.87(d, 2H, J=9.16 Hz), 6.81 (d, 2H, J=9.16 Hz), 6.59(d, 1H, J=8.42 Hz), 4.60(s, 2H), 4.32(s, 2H), 4.22(q, 2H, J=7.14 Hz), 3.74(s, 3H), 3.53(s, 2H), 3.05(t, 4H, J=4.76 Hz), 2.62(m, 6H), 1.26(t, 3H, J=7.14 Hz), 1.16(t, 3H, J=7.33 Hz),

**Ethyl 2-{4-[({4-[(4-acetyl-1-piperazinyl)methyl]-2-[4-(trifluoromethyl)phenyl]-1,3-thiazol-5-yl}methyl)sulfanyl]-2-ethylphenoxy}propanoate**

$^1$H NMR ($CDCl_3$) 400MHz δ 7.99(d, 2H, J=8.28 Hz), 7.68(d, 2H, J=8.28 Hz), 7.22(d, 1H, J=2.21 Hz), 7.15(dd, 1H, J=8.28, 2.21 Hz), 6.60(d, 1H, J=8.28 Hz), 4.75(q, 1H, J=6.81 Hz), 4.29(s, 2H), 4.19(q, 2H, J=7.17 Hz), 3.62(t, 2H, J=4.69 Hz), 3.50(s, 2H), 3.43(t, 2H, J=4.69 Hz), 2.66(q, 2H, J=7.45 Hz), 2.43(br s, 4H), 2.09(s, 3H), 1.64(d, 3H, J=6.81 Hz), 1.22(m, 6H),

**Ethyl 2-{2-ethyl-4-[({4-{[4-(4-fluorophenyl)-1-piperazinyl]methyl}-2-[4-(trifluoromethyl)phenyl]-1,3-thiazol-5-yl}methyl)sulfanyl]phenoxy}propanoate**

$^1$H NMR ($CDCl_3$) 400MHz δ 8.03(d, 2H, J=8.28 Hz), 7.69(d, 2H, J=8.28 Hz), 7.26(d, 1H, J=2.21 Hz), 7.19(dd, 1H, J=8.55, 2.21 Hz), 6.94(m, 4H), 6.62(d, 1H, J=8.55 Hz), 4.75(q, 1H, J=6.90 Hz), 4.35(s, 2H), 4.19(q, 2H, J=7.17 Hz), 3.58(s, 2H), 3.12(t, 4H, J=4.97 Hz), 2.66(m, 6H), 1.64(d, 3H, J=6.90 Hz), 1 :24(m, 6H),

**Ethyl 2-{2-ethyl-4-[({4-(4-morpholinylmethyl)-2-[4-(trifluoromethyl)phenyl]-1,3-thiazol-5-yl}methyl)sulfanyl]phenoxy}propanoate**

$^1$H NMR ($CDCl_3$) 400MHz δ 8.01(d, 2H, J=8.28 Hz), 7.68(d, 2H, J=8.28 Hz), 7.24(d, 1H, J=2.21 Hz), 7.16(dd, 1H, J=8.28, 2.21 Hz), 6.60(d, 1H, J=8.28 Hz), 4.75(q, 1H, J=6.62 Hz), 4.32(s, 2H). 4.17(s, 2H), 3.70(t, 4H, J=4.42 Hz), 3.49(s, 2H), 2.66(q, 2H, J=7.54 Hz), 2.45(t, 4H, J=4.42 Hz), 1.63(d, 3H, J=6.62 Hz), 1.22(m, 6H),

**Ethyl {4-[({4-{[4-(4-methoxyphenyl)-1-piperazinyl]methyl}-2-[4-(trifluoromethyl)phenyl]-1,3-thiazol-5-yl}methyl)sulfanyl]-2-propylphenoxy}acetate**

$^1$H NMR ($CDCl_3$) 400MHz δ 8.03(d, 2H, J=8.28 Hz), 7.69(d, 2H, J=8.28 Hz), 7.23(m, 2H), 6.89(m, 4H), 6.64 (d, 1H, J=828 Hz), 4.62(s, 2H), 4.36(s, 2H), 4.26(q, 2H, J=7.08 Hz), 3.79(s, 3H), 3.60(s, 2H), 3.11(m, 4H), 2.64(m, 6H), 1.62(m, 2H), 1.30(t, 3H, J=7.08 Hz), 0.93(t, 3H, J=7.45 Hz),

**Ethyl {4-[({4-{[4-(4-acetylphenyl)-1-piperazinyl]methyl}-2-(4-(trifluoromethyl)phenyl]-1,3-thiazol-5-yl}methyl)sulfanyl]-2-propylphenoxy}acetate**

$^1$H NMR ($CDCl_3$) 400MHz δ 8.02(d, 2H, J=8.28 Hz), 7.89(d, 2H, J=9.11 Hz), 7.69(d, 2H, J=8.28 Hz), 7.24(m, 2H), 6.87(d, 2H, J=9.11 Hz), 6.64(d, 1H, J=8.28 Hz), 4.62(s. 2H), 4.34(s, 2H), 4.26(q, 2H, J=7.17 Hz), 3.58(s, 2H), 3.35(t, 4H, J=4.97 Hz), 2.62(m, 6H), 2.54(s, 3H), 1.61(m, 2H), 1.29(t, 3H, J=7.17 Hz), 0.91 (t, 3H, J=7.45 Hz),

**Ethyl {4-[({4-{[4-(3-methoxyphenyl)-1-piperazinyl]methyl}-2-[4-(trifluoromethyl)phenyl]-1,3-thiazol-5-yl}methyl)sulfanyl]-2-propylphenoxy}acetate**

$^1$H NMR ($CDCl_3$) 400MHz δ 7.97(d, 2H, J=8.28 Hz), 7.64(d, 2H, J=8.28 Hz), 7.17(m, 3H), 6.58(d, 1H, J=8.10 Hz), 6.51 (dd, 1 H, J=8.10, 2.07 Hz), 6.43(t, 1H, J=2.07 Hz), 6.38(dd, 1 H, J=8.10, 2.07 Hz), 4.58(s, 2H), 4.30(s, 2H), 4.21 (q, 2H, J=7.13 Hz), 3.75(s, 3H), 3.53(s, 2H), 3.15(t, 4H, J=4.66 Hz), 2.57(m, 6H), 1.57(m, 2H), 1.24(t, 3H, J=7.13 Hz), 0.87(t, 3H, J=7.41 Hz),

**Ethyl {4-[({4-[(4-acetyl-1-piperazinyl)methyl]-2-[4-(trifluoromethyl)phenyl]-1,3-thiazol-5-yl}methyl)sulfanyl]-2-propylphenoxy}acetate**

$^1$H NMR ($CDCl_3$) 400MHz δ 7.93(d, 2H, J=8.28 Hz), 7.62(d, 2H, J=8.28 Hz), 7.14(m, 2H), 6.57(d, 1H, J=8.28 Hz), 4.58(s, 2H), 4.20(m, 4H), 3.56(t, 2H, J=4.91 Hz), 3.45(s, 2H), 3.38(t, 2H, J=4.91 Hz), 2.55(t, 2H, J=7.33 Hz), 2.37 (m, 4H), 2.03(s, 3H), 1.53(m, 2H), 1.22(t, 3H, J=7.16 Hz), 0.85(t, 3H, J=7.33 Hz),

**Ethyl {4-[({4-{[4-(4-fluorophenyl)-1-piperazinyl]methyl}-2-[4-(trifluoromethyl)phenyl]-1,3-thiazol-5-yl}methyl) sulfanyl]-2-propylphenoxy}acetate**

$^1$H NMR ($CDCl_3$) 400MHz δ 7.97(d, 2H, J=8.28 Hz), 7.64(d, 2H, J=8.28 Hz), 7.19(m, 2H), 6.92(m, 2H), 6.83 (m, 2H), 6.58(d, 1H, J=8.28 Hz), 4.56(s, 2H), 4.29(s, 2H). 4.20(q, 2H, J=7.13 Hz), 3.53(s, 2H), 3.06(t, 4H, J=4.91.Hz), 2.57(m, 6H), 1.55(m, 2H), 1.24(t, 3H, J=7.13 Hz), 0.86(t, 3H, J=7.41 Hz),

**Ethyl 2-{4-[({4-{[4-(2,4-dimethoxyphenyl)-1-piperazinyl]methyl}-2-[4-(trifluoromethyl)phenyl]-1,3-thiazol-5-yl} methyl)sulfanyl]-2-methylphenoxy}propanoate**

$^1$H NMR ($CDCl_3$) 300MHz δ 8.02(d, 2H, J=8.28 Hz), 7.67(d, 2H, J=8.28 Hz), 7.26(d, 1 H, J=2.21 Hz), 7.16 (dd, 1H, J=8.55, 2.21 Hz), 6.87(d, 1H, J=8.55 Hz), 6.60(d, 1H, J=8.55 Hz), 6.50(d, 1H, J=2.48 Hz), 6.42(dd, 1H, J=8.55, 2.48 Hz), 4.72(q, 1H, J=6.90 Hz), 4.38(s, 2H), 4.21(q, 2H, J=7.08 Hz), 3.85(s, 3H), 3.79(s, 3H), 3.61(s, 2H), 3.04(br s, 4H), 2.70(br s, 4H), 2.26(s, 3H), 1.63(d, 3H, J=6.90 Hz), 1.24(t, 3H, J=7.04 Hz),

**phenyl 4-({5-({[4-(2-ethoxy-1,1-dimethyl-2-oxoethoxy)phenyl]thio}methyl)-2-[4-(trifluoromethyl)phenyl]-1,3-thiazol-4-yl}methyl)piperazine-1-carboxylate**

To a 500ml 3-neck round-bottom flask equipped with a magnetic stir-bar, low temperature thermometer with thermometer adapter, addition funnel and $N_2$ inlet was added ethyl 2-{4-[({4-(hydroxymethyl)-2-[4-(trifluoromethyl)phenyl]-1,3-thiazol-5-yl}methyl)sulfanyl]phenoxy}-2-methylpropanoate (300mg, 0.59mmoles, 1eq) and dry $CH_2Cl_2$ (4m), 0.15M) and cooled to 0°C. Methanesulfonyl chloride (0.055ml, 0.71 mmoles, 1.2eq) was added neat all at once. Triethylamine (0.12ml, 0.89mmoles, 1.5eq) was added dropwise maintaining the internal temperature below 5°C and was stirred at 0 °C for 30 minutes. The reaction mixture was transferred to a separatory funnel and washed with $H_2O$, brine and the organic fraction was dried over $Na_2SO_4$. After filtration the solvent was removed under reduced pressure to yield the corresponding mesylate in quantitative yield. Because of the unstable nature of the mesylate, the product was not characterized and was progressed onto the next stage without purification.

To the crude mesylate dissolved in dry THF (3ml, 0.20M) was added piperazine (559mg, 5.9mmoles, 10eq) and the reaction mixture was refluxed for 5 hours. After cooling to room temperature the solvent was removed *in vacuo*. The residue was partitioned between EtOAc and $H_2O$ and after the phases were separated the organic fraction was dried over $Na_2SO_4$, filtered and concentrated *in vacuo* to yield a quantitative amount of product. The product was used without characterization and purification.

The crude piperazine was dissolved in dry $CH_2Cl_2$ (5ml, 0.12M) and to it was added phenylchloroformate (0.08ml, 0.65mmoles, 1.1eq) and triethylamine (0.248ml, 1.8mmoles, 3eq) and was stirred at room temperature overnight. The reaction mixture was diluted with EtOAc and washed with 0.1N HCl twice, $H_2O$, brine, dried over $Na_2SO_4$, filtered and concentrated *in vacuo* to yield after silica gel chromatography 125mg (32% over three steps) of product.

$^1$H NMR ($CDCl_3$) 400MHz δ 7.95(d, 2H, J=8.28 Hz), 7.65(d, 2H, J=8.28 Hz), 7.33(m, 2H), 7.26(d, 2H, J=8.79 Hz), 7.17(t, 1H, J=7.59 Hz), 7.06(d, 2H, J=7.59 Hz), 6.74(d, 2H, J=8.79 Hz), 4.32(s, 2H), 4.18(q, 2H, J=7.07 Hz), 3.61 (m, 6H), 2.51(br s, 4H), 1.57(s, 6H), 1.20(t, 3H, J=7.07 Hz),

The following compounds were made the same procedure used for phenyl 4-({5-({[4-(2-ethoxy-1,1-dimethyl-2-oxoethoxy)phenyl]thio}methyl)-2-[4-(trifluoromethyl)phenyl]-1,3-thiazol-4-yl}methyl)piperazine-1-carboxylate except no extra base was used when the other reactant was an isocyanate.

**Phenyl 4-({5-({[4-(2-ethoxy-1-methyl-2-oxoethoxy)-3-methylphenyl]sulfanyl}methyl)-2-[4-(trifluoromethyl) phenyl]-1,3-thiazol-4-yl}methyl)-1-piperazinecarboxylate**

$^1$H NMR ($CDCl_3$) 300MHz δ 7.97(d, 2H, J=8.28 Hz), 7.77(d, 2H, J=8.28 Hz), 7.60(m, 5H), 7.20(d, 1H, J=2.21 Hz), 7.10(dd, 1H, J=8.55, 221 Hz), 6.57(d, 1H, J=8.55 Hz), 4.74(q, 1H, J=6.71 Hz), 4.20(m, 4H), 3.48(s, 2H), 3.06(br s, 4H), 2.56(br s, 4H), 2.24(s, 3H), 1.65(d, 3H, J=6.71 Hz), 1.25(t, 3H, J=7.04 Hz),

**benzyl 4-({5-({[4-(2-ethoxy-1-methyl-2-oxoethoxy)-3-methylphenyl]thio}methyl)-2-[4-(trifluoromethyl)phenyl]-1,3-thiazol-4-yl}methyl)piperazine-1-carboxylate**

$^1$H NMR ($CDCl_3$) 300MHz δ 8.01 (d, 2H, J=8.00 Hz), 7.69(d, 2H, J=8.00 Hz), 7.36(m, 5H), 7.26(d, 1H, J=2.21 Hz), 7.15(dd, 1H, J=8.55, 2.21 Hz), 6.60(d, 1H, J=8.55 Hz), 5.16(s, 2H), 4.74(q, 1H, J=6.62 Hz), 4.31 (s, 2H), 4.21 (q, 2H, J=7.08 Hz), 3.55(m, 6H), 2.47(br s, 4H), 2.26(s, 3H), 1.65(d, 3H, J=6.62 Hz), 1.25(t, 3H, J=7.08 Hz),

**Isopropyl 4-{[5-({[4-(2-ethoxy-1-methyl-2-oxoethoxy)-3-methylphenyl]sulfanyl}methyl)-2-(4-fluorophenyl)-1,3-thiazol-4-yl]methyl}-1-piperazinecarboxylate**

$^1$H NMR ($CDCl_3$) 400MHz δ 7.84(m, 2H), 7.22(d, 1H, J=2.20 Hz), 7.09(m, 3H), 6.55(d, 1H, J=8.42 Hz), 4.89 (m, 1H), 4.68(q, 1H, J=6.78 Hz), 4.26(s, 2H), 4.16(q, 2H, J=7.20 Hz), 3.47(m, 6H), 2.40(br s, 4H), 2.22(s, 3H), 1.61(d, 3H, J=6.78 Hz), 1.27(m, 9H),

**Ethyl 2-{4-[({4-{[4-(cyclopentylcarbonyl)-1-piperazinyl]methyl}-2-[4-(trifluoromethyl)phenyl]-1,3-thiazol-5-yl} methyl)sulfanyl]-2-methylphenoxy}propanoate**

$^1$H NMR ($CDCl_3$) 300MHz δ 8.00(d, 2H, J=8.28 Hz), 7.67(d, 2H, J=8.28 Hz), 7.24(d, 1 H, J=2.21 Hz), 7.14 (dd, 1H, J=8.28, 2.21 Hz), 6.59(d, 1H, J=8.28 Hz), 4.73(q, 1H, J=6.71 Hz), 4.31(s, 2H), 4.19(q, 2H, J=7.17 Hz), 3.65 (br s, 2H), 3.50(br s, 4H), 2.87(m, 1H), 2.45(t, 4H, J=4.69 Hz), 2.23(s, 3H), 1.73(m, 11H), 1.24(t, 3H, J=7.17 Hz),

**Ethyl 2-{4-[({4-{[4-(cyclopropylcarbonyl)-1-piperazinyl]methyl}-2-[4-(trifluoromethyl)phenyl]-1,3-thiazol-5-yl} methyl)sulfanyl]-2-methylphenoxy}propanoate**

$^1$H NMR ($CDCl_3$) 300MHz δ 8.00(d, 2H, J=8.28 Hz), 7.68(d, 2H, J=8.28 Hz), 7.27(d, 1H, J=2.21 Hz), 7.15(dd, 1H, J=8.28, 2.21 Hz), 6.59(d, 1H, J=8.28 Hz), 4.73(q, 1H, J=6.71 Hz), 4.31(s, 2H), 4.20(q, 2H, J=7.08 Hz), 3.67(br s, 4H), 3.55(s, 2H), 2.49(br s, 4H), 2.26(s, 3H), 1.74(m, 1H), 1.64(d, 3H, J=6.71 Hz), 1.25(t, 3H, J=7.08 Hz), 1.00(m, 2H), 0.76(m, 2H),

**Ethyl 2-{4-[({4-{[4-(cyclobutylcarbonyl)-1-piperazinyl]methyl}-2-[4-(trifluoromethyl)phenyl]-1,3-thiazol-5-yl} methyl)sulfanyl]-2-methylphenoxy}propanoate**

$^1$H NMR ($CDCl_3$) 300MHz δ 7.99(d, 2H, J=8.28 Hz), 7.67(d, 2H, J=8.28 Hz), 7.24(d, 1 H, J=2.21 Hz), 7.13 (dd, 1H, J=8.28, 2.21 Hz), 6.58(d, 1H, J=8.28 Hz), 4.73(q, 1H, J=6.71 Hz), 4.28(s, 2H), 4.19(q, 2H, J=7.17 Hz), 3.64 (t, 2H, J=4.83 Hz), 3.52(s, 2H), 3.36(t, 2H, J=4.83 Hz), 3.24(m, 1H), 2.47(m, 4H), 2.08(m, 9H), 1.63(d, 3H, J=6.71 Hz), 124(t, 3H, J=7.17 Hz),

**Methyl 4-({5-({[4-(2-ethoxy-1-methyl-2-oxoethoxy)-3-methylphenyl]sulfanyl}methyl)-2-[4-(trifluoromethyl) phenyl]-1,3-thiazol-4-yl}methyl)-1-piperazinecarboxylate**

$^1$H NMR ($CDCl_3$) 300MHz δ 8.00(d, 2H, J=8.28 Hz), 7.68(d, 2H, J=8.28 Hz), 7.26(d, 1H, J=2.21 Hz), 7.14(dd, 1H, J=8.28, 2.21 Hz), 6.59(d, 1H, J=8.28 Hz), 4.73(q, 1H, J=6.71 Hz), 4.31(s, 2H), 4.20(q, 2H, J=7.17 Hz), 3.71 (s, 3H), 3.50(m, 6H), 2.44(br s, 4H), 2.26(s, 3H), 1.65(d, 3H, J=6.71 Hz), 1.25(t, 3H, J=7.17 Hz),

**Ethyl 2-{2-methyl-4-[({4-{[4-(3-methylbutanoyl)-1-piperazinyl]methyl}-2-[4-(trifluoromethyl)phenyl]-1,3-thiazol-5-yl}methyl)sulfanyl]phenoxy}propanoate**

$^1$H NMR ($CDCl_3$) 300MHz δ 8.00(d, 2H, J=8.28 Hz), 7.67(d, 2H, J=8.28 Hz), 7.24(d, 1H, J=2.21 Hz), 7.14(dd, 1H, J=8.55, 2.48 Hz), 6.59(d, 1H, J=8.55 Hz), 4.73(q, 1H, J=6.71 Hz), 4.30(s, 2H), 4.20(q, 2H, J=7.08 Hz), 3.65(br s, 2H), 3.54(s, 2H), 3.47(t, 2H, J=4.69 Hz), 2.45(t, 4H, J=4.83 Hz), 2.26(s, 3H), 2.12(m, 3H), 1.64(d, 3H, J=6.71 Hz), 1.24 (t, 3H, J=7.08 Hz), 0.96(d, 6H, J=6.35 Hz),

**Ethyl 2-{4-[({4-{[4-(4-fluorobenzoyl)-1-piperazinyl]methyl}-2-[4-(trifluoromethyl)phenyl]-1,3-thiazol-5-yl} methyl)sulfanyl]-2-methylphenoxy}propanoate**

$^1$H NMR ($CDCl_3$) 300MHz δ 8.00(d, 2H, J=8.28 Hz), 7.69(d, 2H, J=8.28 Hz), 7.43(m, 2H), 7.24(d, 1H, J=2.39 Hz), 7.11(m, 3H), 6.59(d, 1H, J=8.55 Hz), 4.73(q, 1 H, J=6.71 Hz), 4.30(s, 2H), 4.19(q, 2H, J=7.17 Hz), 3.65(m, 6H), 2.53(m, 4H), 2.25(s, 3H), 1.64(d, 3H, J=6.71 Hz), 1.25(t, 3H, J=7.17 Hz),

**Ethyl 2-{2-methyl-4-[({4-{[4-(propylsulfonyl)-1-piperazinyl]methyl}-2-[4-(trifluoromethyl)phenyl]-1,3-thiazol-5-yl}methyl)sulfanyl]phenoxy}propanoate**

$^1$H NMR ($CDCl_3$) 300MHz δ, 8.00(d, 2H, J=8.28 Hz), 7.68(d, 2H, J=8.28 Hz), 7.26(d, 1H, J=2.21 Hz), 7.15 (dd, 1H, J=8.28, 2.21 Hz), 6.59(d, 1H, J=8.28 Hz), 4.74(q, 1H, J=6.71 Hz), 4.28(s, 2H), 4.20(q, 2H, J=7.08 Hz), 3.55 (s, 2H), 3.30(t, 4H, J=4.55 Hz), 2.89(m, 2H), 2.56(t, 4H, J=4.28 Hz), 2.26(s, 3H), 1.87(m, 2H), 1.65(d, 3H, J=6.62 Hz),

1.25(t, 3H, J=7.04 Hz), 1.07(t, 3H, J=7.17 Hz).

**Ethyl 2-{4-[({4-[(4-butyryl-1-piperazinyl)methyl]-2-[4-(trifluoromethyl)phenyl]-1,3-thiazol-5-yl}methyl)sulfanyl]-2-methylphenoxy}propanoate**

$^1$H NMR ($CDCl_3$) 300MHz δ 8.00(d, 2H, J=8.28 Hz), 7.67(d, 2H, J=8.28 Hz), 7.26(d, 1H, J=2.21 Hz), 7.14(dd, 1H, J=8.55, 2.21 Hz), 6.59(d, 1H, J=8.55 Hz), 4.73(q, 1H, J=6.71 Hz), 4.30(s, 2H), 4.20(q, 2H, J=7.08 Hz), 3.64(m, 2H), 3.54(s, 2H), 3.45(t, 2H, J=4.83 Hz), 2.45(t, 4H, J=4.83 Hz), 2.31(t, 2H, J=7.31 Hz), 2.25(s, 3H), 1.66(m, 5H), 1.24 (t, 3H, J=7.08 Hz), 0.98(t, 3H, J=7.31 Hz),

**Ethyl 2-{2-methyl-4-[({4-[(4-pentanoyl-1-piperazinyl)methyl]-2-[4-(trifluoromethyl)phenyl]-1,3-thiazol-5-yl} methyl)sulfanyl]phenoxy}propanoate**

$^1$H NMR ($CDCl_3$) 300MHz δ 8.00(d, 2H, J=8.28 Hz), 7.67(d, 2H, J=8.28 Hz), 726(d, 1H, J=2.21 Hz), 7.14(dd, 1H, J=8.28, 2.21 Hz), 6.58(d, 1H, J=8.28 Hz), 4.73(q, 1H, J=6.71 Hz), 4.30(s, 2H), 4.19(q, 2H, J=7.27 Hz), 3.64(m, 2H), 3.54(s, 2H), 3.46(t, 2H, J=4.83 Hz), 2.45(t, 4H, J=4.83 Hz), 2.32(t, 2H, J=7.45 Hz), 2.24(s, 3H), 1.61(m, 5H), 1.37 (m, 2H), 1.24(t, 3H, J=7.27 Hz), 0.93(t, 3H, J=7.45 Hz),

**Ethyl 2-{4-[({4-{[4-(4-methoxybenzoyl)-1-piperazinyl]methyl}-2-[4-(trifluoromethyl)phenyl]-1,3-thiazol-5-yl} methyl)sulfanyl]-2-methylphenoxy}propanoate**

$^1$H NMR ($CDCl_3$) 300MHz δ 8.00(d, 2H, J=8.28 Hz), 7.67(d, 2H, J=8.28 Hz), 7.40(d, 2H, J=8.83 Hz), 7.24(d, 1 H, J=2.21 Hz), 7.14(dd, 1H, J=8.28, 2.21 Hz), 6.92(d, 2H, J=8.83 Hz), 6.59(d, 1H, J=8.28 Hz), 4.73(q, 1 H, J=6.71 Hz), 4.30(s, 2H), 4.19(q, 2H, J=7.08 Hz), 3.84(s, 3H), 3.63(m, 6H), 2.49(br s, 4H), 2.25(s, 3H), 1.64(d, 3H, J=6.71 Hz), 1.24(t, 3H, J=7.08 Hz),

**Ethyl 2-{4-[({4-[(4-benzoyl-1-piperazinyl)methyl]-2-[4-(trifluoromethyl)phenyl]-1,3-thiazol-5-yl}methyl) sulfanyl]-2-methylphenoxy}propanoate**

$^1$H NMR ($CDCl_3$) 300MHz δ 7.99(d, 2H, J=8.55 Hz), 7.67(d, 2H, J=8.55 Hz), 7.41(m, 5H), 7.24(d, 1H, J=2.21 Hz), 7.15(dd, 1H, J=8.55, 2.21 Hz), 6.59(d, 1H, J=8.55 Hz), 4.73(q, 1H, J=6.71 Hz), 4.30(s, 2H), 4.19(q, 2H, J=7.04 Hz), 3.83(br s, 2H), 3.56(s, 2H), 3.39(br s, 2H), 2.50(br s, 4H), 2.25(s, 3H), 1.64(d, 3H, J=6.71 Hz), 1.24(t, 3H, J=7.04 Hz),

**isobutyl 4-({5-({[4-(2-ethoxy-1-methyl-2-oxoethoxy)-3-methylphenyl]sulfanyl}methyl)-2-[4-(trifluoromethyl) phenyl]-1,3-thiazol-4-yl}methyl)-1-piperazinecarboxylate**

$^1$H NMR ($CDCl_3$) 300MHz δ, 8.00(d, 2H, J=8.28 Hz), 7.68(d, 2H, J=8.28 Hz), 7.26(d, 1H, J=2.21 Hz), 7.14 (dd, 1H, J=8.28, 2.21 Hz), 6.59(d, 1H, J=8.55 Hz), 4.73(q, 1H, J=6.71 Hz), 4.32(s, 2H), 4.20(q, 2H, J=7.08 Hz), 3.88 (d, 2H, J=6.62 Hz), 3.53(m, 6H), 2.46(br s, 4H), 2.25(s, 3H), 1.94(m, 1H), 1.65(d, 3H, J=6.62 Hz), 1.25(t, 3H, J=7.17 Hz), 0.95(d, 6H, J=6.62 Hz).

**Ethyl 2-{2-methyl-4-[({4-{[4-(2-thienylcarbonyl)-1-piperazinyl]methyl}-2-[4-(trifluoromethyl)phenyl]-1,3-thiazol-5-yl}methyl)sulfanyl]phenoxy}propanoate**

$^1$H NMR ($CDCl_3$) 300MHz δ 8.01 (d, 2H, J=8.28 Hz), 7.68(d, 2H, J=8.28 Hz), 7.45(d, 1H, J=4.97 Hz), 7.30(d, 1H, J=3.59 Hz), 7.25(d, 1H, J=2.21 Hz), 7.15(dd, 1 H, J=8.28, 2.21 Hz), 7.05(m, 1 H), 6.60(d, 1H, J=8.28 Hz), 4.74(q, 1H, J=6.71 Hz), 4.31(s, 2H), 4.19(q, 2H, J=7.08 Hz), 3.78(t, 4H, J=4.69 Hz), 3.56(s, 2H), 2.55(t, 4H, J=4.69 Hz), 2.25 (s, 3H), 1.65(d, 3H, J=6.71 Hz), 1.25(t, 3H, J=7.08 Hz).

**Phenyl 4-{[5-({[4-(2-ethoxy-1-methyl-2-oxoethoxy)-3-methylphenyl]sulfanyl}methyl)-2-(4-fluorophenyl)-1,3-thiazol-4-yl]methyl}-1-piperazinecarboxylate**

$^1$H NMR ($CDCl_3$) 400MHz δ 7.85(m, 2H), 7.33(m, 2H), 7.15(m, 7H), 6.57(d, 1H, J=8.61 Hz), 4.69(q, 1H, J=6.78 Hz), 4.27(s, 2H), 4.14(q, 2H, J=7.14 Hz), 3.63(br s, 4H), 3.50(s, 2H), 2.49(br s, 4H), 2.23(s, 3H), 1.60(d, 3H, J=6.78 Hz), 1.22(t, 3H, J=7.14 Hz),

**Ethyl 2-{4-[({4-({4-[4-(dimethylamino)benzoyl]-1-piperazinyl}methyl)-2-[4-(trifluoromethyl)phenyl]-1,3-thiazol-5-yl}methyl)sulfanyl]-2-methylphenoxy}propanoate**

$^1$H NMR ($CDCl_3$) 300MHz δ 8.01 (d, 2H, J=8.28 Hz), 7.68(d, 2H, J=8.28 Hz), 7.37(d, 2H, J=8.83 Hz), 7.25(d, 1H, J=2.21 Hz), 7.15(dd, 1H, J=8.28, 2.21 Hz), 6.68(d, 2H, J=8.83 Hz), 6.60(d, 1H, J=8.28 Hz), 4.73(q, 1H, J=6.71 Hz), 4.32(s, 2H), 4.20(q, 2H, J=7.17 Hz), 3.67(br s, 4H), 3.55(s, 2H), 3.02(s, 6H), 2.51 (br s, 4H), 2.26(s, 3H), 1.65(d, 3H, J=6.71 Hz), 1.25(t, 3H, J=7.17 Hz),

**Ethyl 2-{4-[({4-{[4-(cyclohexylcarbonyl)-1-piperazinyl]methyl}-2-[4-(trifluoromethyl)phenyl]-1,3-thiazol-5-yl}methyl)sulfanyl]-2-methylphenoxy}propanoate**

$^1$H NMR ($CDCl_3$) 300MHz δ 8.00(d, 2H, J=8.28 Hz), 7.68(d, 2H, J=8.28 Hz), 7.26(d, 1H, J=2.21 Hz), 7.14(dd, 1H, J=8.28, 2.21 Hz), 6.59(d, 1H, J=8.28 Hz), 4.73(q, 1H, J=6.71 Hz), 4.30(s, 2H), 4.20(q, 2H, J=7.08 Hz), 3.58(m, 6H), 2.47(m, 5H), 2.26(s, 3H), 1.63(m, 11H), 1.27(m, 5H),

**Ethyl 2-{2-methyl-4-[({4-({4-[(methylamino)carbonyl]-1-piperazinyl}methyl)-2-[4-(trifluoromethyl)phenyl]-1,3-thiazol-5-yl}methyl)sulfanyl]phenoxy}propanoate**

$^1$H NMR ($CDCl_3$) 300MHz δ 8.01(d, 2H, J=8.28 Hz), 7.67(d, 2H, J=8.28 Hz), 7.27(d, 1H, J=2.21 Hz), 7.14(dd, 1H, J=8.55, 2.21 Hz), 6.53(d, 1H, J=8.55 Hz), 4.84(m, 1H), 4.70(q, 1H, J=6.90 Hz), 4.25(m, 4H), 3.52(m, 2H), 3.29(m, 4H), 2.80(d, 3H, J=4.42 Hz), 2.35(t, 4H, J=4.83 Hz), 2.22(s, 3H), 1.64(d, 3H, J=6.90 Hz), 1.25(t, 3H, J=7.17 Hz),

**Ethyl 2-{4-[({4-({4-[(*tert*-butylamino)carbonyl]-1-piperazinyl}methyl)-2-[4-(trifluoromethyl)phenyl]-1,3-thiazol-5-yl}methyl)sulfanyl]-2-methylphenoxy}propanoate**

$^1$H NMR ($CDCl_3$) 300MHz δ 8.00(d, 2H, J=8.28 Hz), 7.67(d, 2H, J=8.28 Hz), 7.27(d, 1H, J=2.21 Hz), 7.14(dd, 1H, J=8.28, 2.21 Hz), 6.56(d, 1H, J=8.28 Hz), 4.72(q, 1 H, J=6.81 Hz), 4.42(s, 1H), 4.33(d, 1 H, J .63 Hz), 4.26(d, 1H, J=63 Hz), 4.20(q, 2H, J=7.08 Hz), 3.53(s, 2H), 3.29(m, 4H), 2.40(t, 4H, J=4.69 Hz), 2.25(s, 3H), 1.63(d, 3H, J=6.81 Hz), 1.35(s, 9H), 1.25(t, 3H, J=7.09 Hz),

**Ethyl 2-{4-[({4-({4-[(4-methoxyanilino)carbonyl]-1-piperazinyl}methyl)-2-[4-(trifluoromethyl)phenyl]-1,3-thiazol-5-yl}methyl)sulfanyl]-2-methylphenoxy}propanoate**

$^1$H NMR ($CDCl_3$) 300MHz δ 8.05(d, 2H, J=8.28 Hz), 7.73(d, 2H, J=8.28 Hz), 7.23(m, 4H), 6.84(d, 2H, J=6.90 Hz), 6.66(d, 1H, J=8.55 Hz), 4.83(q, 1H, J=6.76 Hz), 4.36(d, 1H, J=63 Hz), 4.30(d, 1H, J=.63 Hz), 4.16(q, 2H, J=7.08 Hz), 3.75(s, 3H), 3.46(m, 6H), 2.43(t, 4H, J=4.83 Hz), 2.21 (s, 3H), 1.58(d, 3H, J=6.76 Hz), 1.20(t, 3H, J=7.08 Hz),

**Ethyl 2-{2-methyl-4-[({4-[(4-{[(2-phenylethyl)amino]carbonyl}-1-piperazinyl)methyl]-2-[4-(trifluoromethyl)phenyl]-1,3-thiazol-5-yl}methyl)sulfanyl]phenoxy}propanoate**

$^1$H NMR ($CDCl_3$) 300MHz δ 8.01 (d, 2H, J=8.28 Hz), 7.68(d, 2H, J=8.28 Hz), 7.24(m, 7H), 6.57(d, 1H, J=8.55 Hz), 4.74(m, 2H), 4.33(d, 1H, J .35 Hz), 4.26(d, 1H, J .35 Hz), 4.20(q, 2H, J=7.04 Hz), 3.50(m, 4H), 3.28(m, 4H), 2.84 (t, 2H, J=7.04 Hz), 2.38(t, 4H, J=4.83 Hz), 2.25(s, 3H), 1.65(d, 3H, J=6.62 Hz), 1.26(t, 3H, J=7.04 Hz),

**Ethyl 2-{2-methyl-4-[({4-{[4-(phenylsulfonyl)-1-piperazinyl]methyl}-2-[4-(trifluoromethyl)phenyl]-1,3-thiazol-5-yl}methyl)sulfanyl]phenoxy}propanoate**

$^1$H NMR ($CDCl_3$) 300MHz δ 7.97(d, 2H, J=8.28 Hz), 7.77(d, 2H, J=8.28 Hz), 7.59(m, 5H), 7.20(d, 1H, J=2.21 Hz), 7.10(dd, 1H, J=8.55, 2.21 Hz), 6.58(d, 1H, J=8.55 Hz), 4.73(q, 1H, J=6.71 Hz), 4.19(m, 4H), 3.48(s, 2H), 3.07(br s, 4H), 2.56(br s, 4H), 2.25(s, 3H), 1.65(d, 3H, J=6.71 Hz), 1.25(t, 3H, J=7.04 Hz),

**Ethyl 2-{2-methyl-4-[({2-[4-(trifluoromethyl)phenyl]-4-[(4-{[4-(trifluoromethyl)phenyl]sulfonyl}-1-piperazinyl)methyl]-1,3-thiazol-5-yl}methyl)sulfanyl]phenoxy}propanoate**

$^1$H NMR ($CDCl_3$) 300MHz δ 7.98(d, 2H, J=8.28 Hz), 7.90(d, 2H, J=8.55 Hz), 7.81(d, 2H, J=8.55 Hz), 7.67(d, 2H, J=8.28 Hz), 7.21(d, 1H, J=2.21 Hz), 7.10(dd, 1H, J=828, 2.21 Hz), 6.58(d, 1H, J=8.28 Hz), 4.74(q, 1H, J=6.71 Hz), 4.21 (m, 4H), 3.49(s, 2H), 3.09(br s, 4H), 2.58(br s, 4H), 224(s, 3H), 1.66(d, 3H, J=6.71 Hz), 1.26(t, 3H, J=7.17 Hz),

**Ethyl 2-{4-[({4-({4-[(4-methoxyphenyl)sulfonyl]-1-piperazinyl}methyl)-2-[4-(trifluoromethyl)phenyl]-1,3-thiazol-5-yl}methyl)sulfanyl]-2-methylphenoxy}propanoate**

$^1$H NMR ($CDCl_3$) 300MHz δ 7.97(d, 2H, J=8.28 Hz), 7.67(m, 4H), 7.20(d, 1H, J=2.21 Hz), 7.09(dd, 1H, J=8.55, 2.21 Hz), 6.99(d, 2H, J=8.83 Hz), 6.58(d, 1H, J=8.55 Hz), 4.74(q, 1H, J=6.71 Hz), 4.20(m, 4H), 3.87(s, 3H), 3.49(s, 2H), 3.05(br s, 4H), 2.54(br s, 4H), 2.24(s, 3H), 1.66(d, 3H, J=6.71 Hz), 1.25(t, 3H, J=7.04 Hz),

**Ethyl 2-{4-[({4-{[4-(ethylsulfonyl)-1-piperazinyl]methyl}-2-[4-(trifluoromethyl)phenyl]-1,3-thiazol-5-yl}methyl)sulfanyl]-2-methylphenoxy}propanoate**

$^1$H NMR ($CDCl_3$) 300MHz δ 8.00(d, 2H, J=8.28 Hz), 7.68(d, 2H, J=8.28 Hz), 7.26(d, 1H, J=2.48 Hz), 7.15(d, 1H, J=2.48 Hz), 6.59(d, 1H, J=8.28 Hz), 4.74(q, 1H, J=6.81 Hz), 4.28(s, 2H), 4.20(q, 2H, J=7.17 Hz), 3.55(s, 2H), 3.32 (t, 4H, J=4.69Hz), 2.96(q, 2H, J=7.45 Hz), 2.55(br s, 4H), 2.25(s, 3H), 1.65(d, 3H, J=6.81 Hz), 1.38(t, 3H, J=7.45 Hz), 1.25(t, 3H, J=7.17 Hz),

**Ethyl 2-{2-methyl-4-[({4-{[4-(methylsulfonyl)-1-piperazinyl]methyl}-2-[4-(trifluoromethyl)phenyl]-1,3-thiazol-5-yl}methyl)sulfanyl]phenoxy}propanoate**

$^1$H NMR ($CDCl_3$) 300MHz δ 8.00(d, 2H, J=8.55 Hz), 7.68(d, 2H, J=8.55 Hz), 7.26(d, 1H, J=2.21 Hz), 7.15(dd, 1H, J=8.55, 2.21 Hz), 6.59(d, 1H, J=8.55 Hz), 4.73(q, 1H, J=6.71 Hz), 4.27(s, 2H), 4.20(q, 2H, J=7.17 Hz), 3.56(s, 2H), 3.24(t, 4H, J=4.55 Hz), 2.78(s, 3H), 2.58(t, 4H, J=4.55 Hz), 2.24(s, 3H), 1.64(d, 3H, J=6.71 Hz), 1.25(t, 3H, J=7.17 Hz),

**Ethyl 2-{4-[({4-[(4-{[4-(acetylamino)phenyl]sulfonyl}-1-piperazinyl)methyl]-2-[4-(trifluoromethyl)phenyl]-1,3-thiazol-5-yl}methyl)sulfanyl]-2-methylphenoxy}propanoate**

$^1$H NMR ($CDCl_3$) 300MHz δ 8.40(s, 1H), 7.96(d, 2H, J=8.28 Hz), 7.66(m, 6H), 7.16(d, 1H, J=2.21 Hz), 7.07 (dd, 1H, J=8.28, 2.21 Hz), 6.56(d, 1H, J=8.28 Hz), 4.73(q, 1H, J=6.71 Hz), 4.22(m, 4H), 3.51(s, 2H), 3.03(br s, 4H), 2.55(br s, 4H), 2.19(m, 6H), 1.65(d, 3H, J=6.71 Hz), 1.27(t, 3H, J=7.04 Hz),

**Ethyl 2-{4-[({4-({4-[(4-fluorophenyl)sulfonyl]-1-piperazinyl}methyl)-2-[4-(trifluoromethyl)phenyl]-1,3-thiazol-5-yl}methyl)sulfanyl]-2-methylphenoxy}propanoate**

$^1$H NMR ($CDCl_3$) 300MHz δ 7.97(d, 2H, J=8.28 Hz), 7.77(m, 2H), 7.66(d, 2H, J=8.28 Hz), 7.22(m, 3H), 7.10 (dd, 1H, J=8.55, 2.21 Hz), 6.58(d, 1H, J=8.55 Hz), 4.74(q, 1H, J=6.81 Hz), 4.20(m, 4H), 3.49(s, 2H), 3.07(br s, 4H), 2.57(t, 4H, J=4.42 Hz), 2.24(s, 3H), 1.65(d, 3H, J=6.81 Hz), 1.27(t, 3H, J=7.17 Hz),

**Ethyl 2-{4-[({4-{[4-(2-furoyl)-1-piperazinyl]methyl}-2-[4-(trifluoromethyl)phenyl]-1,3-thiazol-5-yl}methyl)sulfanyl]-2-methylphenoxy}propanoate**

$^1$H NMR ($CDCl_3$) 300MHz δ 8.00(d, 2H, J=8.28 Hz), 7.67(d, 2H, J=8.28 Hz), 7.48(s, 1H), 7.24(d, 1H, J=2.21 Hz), 7.15(dd, 1H, J=8.28, 2.21 Hz), 6.99(d, 1H, J=3.59 Hz), 6.60(d, 1H, J=8.28 Hz), 6.48(m, 1H), 4.73(q, 1H, J=6.71 Hz), 4.31 (s, 2H), 4.20(q, 2H, J=7.08 Hz), 3.83(br s, 4H), 3.55(s, 2H), 2.54(t, 4H, J=4.83 Hz), 2.25(s, 3H), 1.64(d, 3H, J=6.71 Hz), 1.24(t, 3H, J=7.08 Hz),

**Ethyl 2-{4-[({4-({4-[(isopropylamino)carbonyl]-1-piperazinyl}methyl)-2-[4-(trifluoromethyl)phenyl]-1,3-thiazol-5-yl}methyl)sulfanyl]-2-methylphonoxy}propanoate**

$^1$H NMR ($CDCl_3$) 300MHz δ, 8.01 (d, 2H, J=8.28 Hz), 7.67(d, 2H, J=8.28 Hz), 7.27(d, 1H, J=1.93 Hz), 7.14 (ddd, 1H, J=8.55, 2.21, 0.55 Hz), 6.55(d, 1H, J=8.28 Hz), 4.72(q, 1H, J=6.81 Hz), 4.47(d, 1H, J=7.17 Hz), 4.26(m, 4H), 3.99(m, 1 H), 3.52(m, 2H), 3.29(m, 4H), 2.37(t, 4H, J=4.69 Hz), 2.24(s, 3H), 1.64(d, 3H, J=6.62 Hz), 1.25(t, 3H, J=7.17 Hz), 1.15(m, 6H),

**Ethyl 2-{4-[({4-{[4-(methoxyacetyl)-1-piperazinyl]methyl}-2-[4-(trifluoromethyl)phenyl]-1,3-thiazol-5-yl}methyl)sulfanyl]-2-methylphenoxy}propanoate**

$^1$H NMR ($CDCl_3$) 300MHz δ 8.00(d, 2H, J=8.28 Hz), 7.67(d, 2H, J=8.28 Hz), 7.24(d, 1H, J=2.21 Hz), 7.14(dd, 1H, J=8.28, 2.21 Hz), 6.58(d, 1H, J=8.28 Hz), 4.73(q, 1H, J=6.71 Hz), 4.29(s, 2H), 4.20(q, 2H, J=7.17 Hz), 4,10(s, 2H), 3.64(m, 2H), 3.54(s, 2H), 3.48(m, 2H), 3.42(s, 3H), 2.47(m, 4H), 2.25(s, 3H), 1.64(d, 3H, J=6.71 Hz), 1.24(t, 3H, J=7.17

Hz),

Ethyl 2-{4-[({4-[(4-isobutyryl-1-piperazinyl)methyl]-2-[4-(trifluoromethyl)phenyl]-1,3-thiazol-5-yl}methyl) sulfanyl]-2-methylphenoxy}propanoate

$^1$H NMR ($CDCl_3$) 300MHz δ 8.00(d, 2H, J=8.28 Hz), 7.68(d, 2H, J=8.28 Hz), 7.24(d, 1H, J=2.48 Hz), 7.14(dd, 1H, J=8.55, 2.48 Hz), 6.59(d, 1H, J=8.55 Hz), 4.74(q, 1H, J=6.71 Hz), 4.30(s, 2H), 420(q, 2H, J=7.17 Hz), 3.58(m, 6H), 2.79(m, 1H), 2.46(t, 4H, J=4.55 Hz), 2.24(s, 3H), 1.64(d, 3H, J=6.71 Hz), 1.24(t, 3H, J=7.17 Hz), 1.13(d, 6H, J=6.71 Hz),

Ethyl 2-{4-[({4-{[4-(2,2-dimethylpropanoyl)-1-piperazinyl]methyl}-2-[4-(trifluoromethyl)phenyl]-1,3-thiazol-5-yl} methyl)sulfanyl]-2-methylphenoxy}propanoate

$^1$H NMR ($CDCl_3$) 300MHz δ 8.00(d, 2H, J=8.28 Hz), 7.68(d, 2H, J=8.28 Hz), 7.24(d, 1H, J=2.21 Hz), 7.15(dd, 1H, J=8.28, 2.21 Hz), 6.60(d, 1H, J=8.28 Hz), 4.73(q, 1H, J=6.71 Hz), 4.31 (s, 2H), 4.20(q, 2H, J=7.08 Hz), 3.66(t, 4H, J=4.69 Hz), 3.52(s, 2H), 2.48(t, 4H, J=4.69 Hz), 2.26(s, 3H), 1.65(d, 3H, J=6.71 Hz), 1.27(m, 12H),

Ethyl 2-{4-[({4-[{4-[(4-fluoroanilino)carbonyl]-1-piperazinyl}methyl)-2-[4-(trifluoromethyl)phenyl]-1,3-thiazol-5-yl}methyl)sulfanyl]-2-methylphenoxy}propanoate

$^1$H NMR ($CDCl_3$) 300MHz δ 8.04(d, 2H, J=8.28 Hz), 7.69(d, 2H, J=8.28 Hz), 7.33(m, 2H), 7.17(dd, 1H, J=8.55, 2.21 Hz), 6.96(m, 2H), 6.52(d, 1H, J=8.55 Hz), 4.72(q, 1H, J=6.90 Hz), 4.27(m, 4H), 3.59(d, 1H, J .52 Hz), 3.51 (d, 1H, J .52 Hz), 3.34(m, 4H), 2.33(t, 4H, J=4.97Hz), 2.22(s, 3H), 1.62(d, 3H, J=6.90 Hz), 1.26(t, 3H, J=7.17 Hz),

Ethyl 2-{4-[({4-({4-[(3-methoxyanilino)carbonyl]-1-piperazinyl}methyl)-2-[4-(trifluoromethyl)phenyl]-1,3-thiazol-5-yl}methyl)sulfanyl]-2-methylphenoxy}propanoate

$^1$H NMR ($CDCl_3$) 300MHz δ 8.04(d, 2H, J=8.28 Hz), 7.69(d, 2H, J=8.28 Hz), 7.31(d, 1H, J=2.21 Hz), 7.16(m, 2H), 6.89(m, 2H), 6.59(dd, 1H, J=8.28, 2.21 Hz), 6.53(m, 1 H), 4.73(q, 1H, J=6.90 Hz), 4.27(m, 4H), 3.79(s, 3H), 3.56 (m, 2H), 3.37(m, 4H), 2.36(t, 4H, J=4.69 Hz), 2.23(s, 3H), 1.63(d, 3H, J=6.90 Hz), 1.26(t, 3H, J=7.17 Hz),

Ethyl 2-{4-[({4-{[4-(aminocarbonyl)-1-piperazinyl]methyl}-2-[4-(trifluoromethyl)phenyl]-1,3-thiazol-5-yl}methyl) sulfanyl]-2-methylphenoxy}propanoate

$^1$H NMR ($CDCl_3$) 300MHz δ 8.01(d, 2H, J=8.28 Hz), 7.68(d, 2H, J=8.28 Hz), 7.26(d, 1H, J=2.21 Hz), 7.15(dd, 1H, J=8.55, 2.21 Hz), 6.56(d, 1H, J=8.55 Hz), 4.83(s, 2H), 4.71 (q, 1H, J=6.81 Hz), 4.26(m, 4H), 3.55(m, 2H), 3.34(m, 4H), 2.41(t, 4H, J=4.55 Hz), 2.24(s, 3H), 1.63(d, 3H, J=6.81 Hz), 1.25(t, 3H, J=7.04 Hz),

Ethyl 2-{4-[({4-({4-[(cyclohexylamino)carbonyl]-1-piperazinyl}methyl)-2-[4-(trifluoromethyl)phenyl]-1,3-thiazol-5-yl}methyl)sulfanyl]-2-methylphenoxy}propanoate

$^1$H NMR ($CDCl_3$) 300MHz δ 8.00(d, 2H, J=8.28 Hz), 7.67(d, 2H, J=8.28 Hz), 7.26(d, 1H, J=2.21 Hz), 7.14(dd, 1H, J=8.55, 2.21 Hz), 6.54(d, 1H, J=8.55 Hz), 4.72(q, 1H, J=6.81 Hz), 4.49(d, 1H, J=7.45 Hz), 4.25(m, 4H), 3.64(m, 1H), 3.52(m, 2H), 3.28(m, 4H), 2.38(t, 4H, J=4.83 Hz), 2.24(s, 3H), 1.95(m, 2H), 1.65(m, 7H), 1.38(m, 2H), 1.24(t, 3H, J=7.04 Hz), 1.10(m, 2H),

Ethyl 2-{2-methyl-4-[({4-({4-[(propylamino)carbonyl]-1-piperazinyl}methyl)-2-[4-(trifluoromethyl)phenyl]-1,3-thiazol-5-yl}methyl)sulfanyl]phenoxy}propanoate

$^1$H NMR ($CDCl_3$) 300MHz δ 8.01(d, 2H, J=8.00 Hz), 7.68(d, 2H, J=8.00 Hz), 7.27(d, 1H, J=2.21 Hz), 7.14(dd, 1H, J=8.28, 2.21 Hz), 6.54(d, 1H, J=8.28 Hz), 4.75(m, 2H), 4.26(m, 4H), 3.53(m, 2H), 3.33(m, 4H), 3.19(m, 2H), 2.36 (t, 4H, J=4.69 Hz), 2.23(s, 3H), 1.64(d, 3H, J=6.90 Hz), 1.52(m, 2H), 1.25(t, 3H, J=7.17 Hz), 0.92(t, 3H, J=7.45 Hz),

Ethyl 2-{4-[({4-({4-[(ethylamino)carbonyl]-1-piperazinyl}methyl)-2-[4-(trifluoromethyl)phenyl]-1,3-thiazol-5-yl} methyl)sulfanyl]-2-methylphenoxy}propanoate

$^1$H NMR ($CDCl_3$) 300MHz δ 8.02(d, 2H, J=8.28 Hz), 7.69(d, 2H, J=8.28 Hz), 7.27(d, 1H, J=2.21 Hz), 7.15(dd, 1H, J=8.55, 2.21 Hz), 6.54(d, 1H, J=8.55 Hz), 4.72(m, 2H), 4.26(m, 4H), 3.54(m, 2H), 3.29(m, 6H), 2.38(t, 4H, J=4.28

Hz), 2.25(s, 3H), 1.65(d, 3H, J=6.90 Hz), 1.26(t, 3H, J=7.04 Hz), 1.15(t, 3H, J=7.31 Hz),

**Ethyl [2-methyl-4-({[4-{[3-(5-methyl-1,2,4-oxadiazol-3-yl)phenoxy]methyl}-2-(4-{trifluoromethyl}phenyl)-1,3-thiazol-5-yl]methyl}sulfanyl)phenoxy]acetate**

To a stirred solution of ethyl [4-({[4-(hydroxymethyl)-2-(4-{trifluoromethyl}phenyl)-1,3-thiazol-5-yl]methyl}sulfanyl)-2-methylphenoxy]acetate (40mg, 0.08mmoles, 1eq) in dry toluene (2ml) was added 3-(5-methyl-1,2,4-oxadiazol-3-yl)phenol (15mg, 0.088mmoles, 1.1eq) followed by triphenylphosphine (25mg, 0.096mmoles, 1.2eq) as a solid. Diisopropylazodicarboxylate (0.017ml, 0.088mmoles, 1.1eq) was then added dropwise and the reaction was stirred for 2 hours at room temperature. The reaction was then partitioned between EtOAc and $H_2O$. After the separation of the phases the organic phase was washed with 0.1 N NaOH, brine, dried over $Na_2SO_4$, filtered, concentrated *in vacuo* and purified via flash chromatography (10% EtOAc/Hexanes to 35% EtOAc/Hexanes) to yield 40mg (76%) of product.

$^1H$ ($CDCl_3$) 400MHz $\delta$ 8.02(d, 2H, J=8.20 Hz), 7.68(m, 4H), 7.38(t, 1H, J=7.95 Hz), 7.19(d, 1H, J=1.54), 7.12 (dd, 1H, J=8.37, 2.39 Hz), 7.06(dd, 1H, J=8.20, 2.39 Hz), 6.57(d, 1H, J=8.20 Hz), 4.95(s, 2H), 4.59(s, 2H), 4.27(s, 2H), 4.22(q, 2H, J=7.12 Hz), 2.65(s, 3H), 2.18(s, 3H), 1.25(t, 3H, J=7.12 Hz). TLC(50% EtOAc/Hexanes) $R_f$ = 0.76

The following compounds were made using the general Mitsunobu reaction conditions detailed above:

**Ethyl 2-{2-methyl-4-[({4-{[3-(5-methyl-1,2,4-oxadiazol-3-yl)phenoxy]methyl}-2-[4-(trifluoromethyl)phenyl]-1,3-thiazol-5-yl}methyl)sulfanyl]phenoxy}propanoate**

$^1H$ NMR ($CDCl_3$) 400MHz $\delta$ 8.03(d, 2H, J=8.20 Hz), 7.69(m, 4H), 7.39(m, 1H), 7.20(m, 1 H), 7.09(m, 2H), 6.55(d, 1H, J=8.37 Hz), 4.99(d, 1H, J .62 Hz), 4.95(d, 1H, J .62 Hz), 4.70(q, 1H, J=6.78 Hz), 4.16(q, 2H, J=7.18 Hz), 2.65(m, 3H), 2.18(s, 3H), 1.61(d, 3H, J=6.78 Hz), 1.20(t, 3H, J=7.18 Hz),

**Ethyl (2-methyl-4-{[(4-{[3-(5-methyl-1,2,4-oxadiazol-3-yl)phenoxy]methyl}-2-phenyl-1,3-thiazol-5-yl)methyl]sulfanyl}phenoxy)acetate**

$^1H$ NMR ($CDCl_3$) 400MHz $\delta$ 7.91(m, 2H), 7.69(m, 2H), 7.40(m, 4H), 7.20(d, 1H, J=2.39 Hz), 7.13(dd, 1H, J=8.37, 2.39 Hz), 7.07(dd, 1H, J=8.37, 2.39 Hz), 6.57(d, 1H, J=8.37 Hz), 5.29(s, 2H), 4.59(s, 2H), 4.27(s, 2H), 4.23(q, 2H, J=7.18 Hz), 2.65(s, 3H), 2.19(s, 3H), 1.27(t, 3H, J=7.18 Hz).

**Ethyl [2-methyl-4-({[2-(4-{trifluoromethyl}phenyl)-4-(phenoxymethyl)-1,3-thiazol-5-yl]methyl}sulfanyl)phenoxy]acetate**

$^1H$ NMR ($CDCl_3$) 300MHz $\delta$ 8.04(d, 2H, J=8.23 Hz), 7.71 (d, 2H, J=8.23 Hz), 7.34(m, 2H), 7.23(d, 1H, J=2.39 Hz), 7.15(dd, 1H, J=8.49, 2.39 Hz), 7.00(m, 3H), 6.59(d, 1H, J=8.49 Hz), 4.94(s, 2H), 4.64(s, 2H), 4.27(m, 4H), 2.26 (s, 3H), 1.32(t, 3H, J=7.17 Hz). TLC(30% EtOAc/Hexanes) $R_f$= 0.71

**Ethyl [2-methyl-4-({[4-[(2-methylphenoxy)methyl]-2-(4-{trifluoromethyl}phenyl)-1,3-thiazol-5-yl]methyl}sulfanyl)phenoxy]acetate**

$^1H$ ($CDCl_3$) 300MHz $\delta$ 8.05(d, 2H, J=8.23 Hz), 7.72(d, 2H, J=8.23 Hz), 7.21(m, 4H), 6.93(m, 2H), 6.59(d, 1H, J=8.49 Hz), 5.00(s, 2H), 4.64(s, 2H), 4.29(m, 4H), 2.26(m, 6H), 1.32(t, 3H, J=7.17 Hz). TLC(20% EtOAc/Hexanes) $R_f$ = 0.70

**Ethyl [2-methyl-4-({[4-[(3-methylphenoxy)methyl]-2-(4-{trifluoromethyl}phenyl)-1,3-thiazol-5-yl]methyl}sulfanyl)phenoxy]acetate**

$^1H$ ($CDCl_3$) 300MHz $\delta$ 8.05(d, 2H, J=8.49 Hz), 7.71 (d, 2H, J=8.49 Hz), 7.35(m, 1H), 7.26(dd, 1H, J=2.39, 0.53 Hz), 7.21(t, 1H, J=7.43 Hz), 7.15(ddd, 1H, J=8.49, 2.39, 0.53 Hz), 6.81 (m, 2H), 6.60(d, 1H, J=8.49 Hz), 4.92(s, 2H), 4.65(s, 2H), 4.29(m, 4H), 2.38(s, 3H), 2.25(s, 3H), 1.32(t, 3H, J=7.17 Hz). TLC(20% EtOAc/Hexanes) $R_f$ = 0.70

**Ethyl [2-methyl-4-({[4-[(4-methylphenoxy)methyl]-2-(4-{trifluoromethyl}phenyl)-1,3-thiazol-5-yl]methyl}sulfanyl)phenoxy]acetate**

$^1H$ ($CDCl_3$) 300MHz $\delta$ 8.04(d, 2H, J=8.23 Hz), 7.71 (d, 2H, J=8.23 Hz), 7.27(dd, 1H, J=2.39, 0.80 Hz), 7.14 (m, 3H), 6.88(d, 2H, J=8.49 Hz), 6.60(d, 1H, J=8.23 Hz), 4.92(s, 2H), 4.64(s, 2H), 4.29(m, 4H), 2.33(s, 3H), 2.26(s, 3H), 1.32(t, 3H, J=7.17 Hz). TLC(20% EtOAc/Hexanes) $R_f$ = 0.70

**Ethyl [4-({[4-[(3-cyanophenoxy)methyl]-2-(4-{trifluoromethyl}phenyl)-1,3-thiazol-5-yl]methyl}sulfanyl)-2-methylphenoxy]acetate**

$^1$H ($CDCl_3$) 300MHz δ 8.03(d, 2H, J=8.23 Hz), 7.71(d, 2H, J=8.23 Hz), 7.24(m, 6H), 6.61(d, 1H, J=8.23 Hz), 4.88(s, 2H), 4.67(s, 2H), 4.28(m, 4H), 2.24(s, 3H), 1.31(t, 3H, J=7.17 Hz). TLC(20% EtOAc/Hexanes) $R_f$ = 0.52

**Ethyl [4-({[4-[(4-cyanophenoxy)methyl]-2-(4-{trifluoromethyl}phenyl)-1,3-thiazol-5-yl]methyl}sulfanyl)-2-methylphenoxy]acetate**

$^1$H ($CDCl_3$) 300MHz δ 8.03(d, 2H, J=8.23 Hz), 7.73(d, 2H, J=8.23 Hz), 7.61(d, 2H, J=9.03 Hz), 7.23(dd, 1H, J=2.39, 0.53 Hz), 7.14(ddd, 1H, J=8.49, 2.39, 0.53 Hz), 7.01(d, 2H, J=9.03 Hz), 6.59(d, 1H, J=8.49 Hz), 4.91 (s, 2H), 4.66(s, 2H), 4.28(m, 4H), 2.25(s, 3H), 1.32(t, 3H, J=7.17 Hz). TLC(20% EtOAc/Hexanes) $R_f$ = 0.52

**Ethyl [2-methyl-4-({[4-{[4-(5-methyl-1,2,4-oxadiazol-3-yl)phenoxy]methyl}-2-(4-{trifluoro}methylphenyl)-1,3-thiazol-5-yl]methyl}sulfanyl)phenoxy]acetate**

$^1$H ($CDCl_3$) 400MHz δ 7.99(m, 4H), 7.67(d, 2H, J=8.20 Hz), 7.21(dd, 1H, J=2.39, 0.68 Hz), 7.10(m, 1H), 7.02 (m, 2H), 6.54(d, 1H, J=8.37 Hz), 4.90(s, 2H), 4.59(s, 2H), 4.23(m, 4H), 2.62(s, 3H), 2.20(s, 3H), 1.26(t, 3H, J=7.18 Hz). TLC(50% EtOAc/Hexanes) $R_f$ = 0.68

**Ethyl (2-methyl-4-{[(4-{[4-(5-methyl-1,2,4-oxadiazol-3-yl)phenoxy]methyl}-2-phenyl-1,3-thiazol-5-yl)methyl] sulfanyl}phenoxy)acetate**

$^1$H NMR ($CDCl_3$) 400MHz δ 7.98(d, 2H, J=8.89 Hz), 7.89(m, 2H), 7.42(m, 3H), 7.21(d, 1H, J=2.39 Hz), 7.10 (dd, 1H, J=8.37, 2.39 Hz), 7.02(d, 2H, J=8.89 Hz), 6.54(d, 1H, J=8.37 Hz), 4.89(s, 2H), 4.59(s, 2H), 4.23(q, 2H, J=7.18 Hz), 3.47(s, 2H), 2.62(s, 3H), 2.20(s, 3H), 1.27(t, 3H, J=7.18 Hz),

**Ethyl 2-{2-methyl-4-[({4-{[4-(5-methyl-1,2,4-oxadiazol-3-yl)phenoxy]methyl}-2-[4-(trifluoromethyl)phenyl]-1,3-thiazol-5-yl}methyl)sulfanyl]phenoxy}propanoate**

$^1$H NMR ($CDCl_3$) 300MHz δ 8.04(m, 4H), 7.71(d, 2H, J=8.23 Hz), 7.25(d, 1H, J=2.39 Hz), 7.11(dd, 1H, J=8.49, 2.39 Hz), 7.06(d, 2H, J=9.03 Hz), 6.57(d, 1H, J=8.49 Hz), 4.97(d, 1H, J .68 Hz), 4.91 (d, 1H, J .68 Hz), 4.73(q, 1H, J=6.81 Hz), 4.29(s, 2H), 4.20(q, 2H, J=7.17 Hz), 2.67(s, 3H), 2.23(s, 3H), 1.65(d, 3H, J=6.81 Hz), 1.25(t, 3H, J=7.17 Hz),

**Ethyl 2-(2-methyl-4-{[(4-{[4-(5-methyl-1,2,4-oxadiazol-3-yl)phenoxy]methyl)-2-phenyl-1,3-thiazol-5-yl)methyl] sulfanyl}phenoxy)propanoate**

$^1$H NMR ($CDCl_3$) 400MHz δ 7.99(d, 2H, J=9.06 Hz), 7.89(m, 2H), 7.42(m, 3H), 7.20(d, 1H, J=2.22 Hz), 7.06 (dd, 1H, J=8.37, 2.22 Hz), 7.02(d, 2H, J=9.06 Hz), 6.52(d, 1H, J=8.37 Hz), 4.89(d, 1H, J .62 Hz), 4.85(d, 1H, J .62 Hz), 4.68(q, 1H, J=6.78 Hz), 4.23(s, 2H), 4.17(q, 2H, J=7.12 Hz), 2.62(s, 2H), 2.19(s, 3H), 1.61(d, 3H, J=6.78 Hz), 1.21(t, 3H, J=7.12 Hz),

**4-(Chloromethyl)-2-methylphenyl methyl ether**

To a stirred solution of (4-methoxy-3-methylphenyl)methanol (2.31g, 15.18mmoles, 1eq) in anhydrous $CH_2Cl_2$ (50ml, 0.3M) was added hexachloroethane (3.59g, 15.18mmoles, 1eq) and triphenylphosphine (3.98g, 15.18mmoles, 1eq). This mixture was stirred at room temperature overnight at which point the reaction was transferred to a separatory funnel and washed with $H_2O$, brine, dried over $Na_2SO_4$, filtered, concentrated *in vacuo* and filtered through a plug of silica gel (30% EtOAc/Hexanes) to yield 2.59g (100%) of product.

$^1$H NMR ($CDCl_3$) 400MHz δ 7.16(m, 2H), 6.76(d, 1H, J=8.10 Hz), 4.52(s, 2H), 3.81 (s, 3H), 2.19(s, 3H),

**(4-Methoxy-3-methylbenzyl)(triphenyl)phosphonium chloride**

To a 250ml round-bottom flask equipped with a magnetic stir-bar and $N_2$ inlet was added 4-(Chloromethyl)-2-methylphenyl methyl ether (2.59g, 15.18mmoles, 1eq), dry toluene (50ml, 0.3M) and triphenylphosphine (3.98g, 15.18mmoles, 1 eq). The reaction mixture was refluxed overnight. After cooling to room temperature the solvent was removed *in vacuo*, the residue washed with hexanes and the solid/liquid mixture was filtered to yield 4.48g (71 %) of solid product.

$^1H$ NMR ($CDCl_3$) 400MHz δ 7.66(m, 15H), 6.93(m, 1H), 6.54(m, 2H), 5.24(d, 2H, J .79 Hz), 3.68(s, 3H), 1.90 (s, 3H),

**4-[(Tetrahydro-2*H*-pyran-2-yloxy)methyl]-2-[4-(trifluoromethyl)phenyl]-1,3-thiazole-5-carbaldehyde**

To a stirred mixtureof pyridinium chlorochromate (6.9g, 32.12mmoles, 4eq) in dry $CH_2Cl_2$ (40ml, 0.2M) was added {4-[(tetrahydro-2*H*-pyran-2-yloxy)methyl]-2-[4-(trifluoromethyl)phenyl]-1,3-thiazol-5-yl}methanol (3.0g, 8.03mmoles, 1eq) in $CH_2Cl_2$ (10ml). The mixture was stirred at room temperature for 4 hours at which time the reaction mixture was quenched by allowing it to stir with sat. $NaHCO_3$. Once the quenching had ceased the reaction was filtered through Celite and the filtrate was transferred to a separatory funnel where the phases were separated. The organic phase was dried over $Na_2SO_4$ and concentrated *in vacuo* to yield 2.18g (73%) of clean aldehyde. The crude product was used without purification.

$^1H$ NMR ($CDCl_3$) 400MHz δ 10.39(s, 1H), 8.09(d, 2H, J=8.28 Hz), 7.70(d, 2H, J=8.28 Hz), 5.22(d, 1H, J .97 Hz), 4.96(d, 1H, J .97 Hz), 4.83(m, 1H), 3.87(m, 1H), 3.58(m, 1H), 1.81 (m, 2H), 1.61 (m, 4H),

**5-[(*E*)-2-(4-Methoxy-3-methylphenyl)ethenyl]-4-[(tetrahydro-2*H*-pyran-2-yloxy)methyl]-2-[4-(trifluoromethyl) phenyl]-1,3-thiazole**

To a suspension of NaH (60% dispersion in mineral oil, 242mg, 6.32mmoles, 1.4eq) in dry $CH_2Cl_2$ (15ml) was added (4-Methoxy-3-methylbenzyl)(triphenyl)phosphonium chloride (2.62g, 6.32mmoles, 1.4eq). This was allowed to stir at room temperature for 1.5 hours followed by the dropwise addition of 4-[(tetrahydro-2*H*-pyran-2-yloxy)methyl]-2-[4-(trifluoromethyl)phenyl]-1,3-thiazole-5-carbaldehyde (1.68g, 4.51 mmoles, 1 eq) in anhydrous carbon tetrachloride (25ml). The resulting reaction mixture was refluxed overnight at which point (after cooling to room temperature) the reaction was washed with 1 N NaOH, $H_2O$, brine, dried over $Na_2SO_4$ and concentrated *in vacuo* to yield a >100% yield of a light green oil. The crude material was used without purification.

$^1H$ NMR ($CDCl_3$) 400MHz δ 8.05(d, 2H, J=8.24 Hz), 7.68(d, 2H, J=8.24 Hz), 7.29(m, 3H), 6.85(m, 2H), 4.98 (d, 1H, J=12.09 Hz), 4.81 (m, 2H), 4.01(m, 1H), 3.86(s, 3H), 3.62(m, 1H), 2.26(s, 3H), 1.72(m, 6H),

**{5-[2-(4-Methoxy-3-methylphenyl)ethyl]-2-[4-(trifluoromethyl)phenyl]-1,3-thiazol-4-yl}methanol**

To a stirred solution of 5-[(*E*)-2-(4-Methoxy-3-methylphenyl)ethenyl]-4-[(tetrahydro-2*H*-pyran-2-yloxy)methyl]-2-[4-(trifluoromethyl)phenyl]-1,3-thiazole (2.20g, 4.51mmoles, 1eq) in EtOH (50ml, 0.1M) was added 10%Pd/C (500mg). The system was degassed using an aspirator and $H_2$ was introduced via a balloon. The reaction was heated to 60°C overnight which, after cooling to room temperature, was filtered through Celite, washed with EtOAc and concentrated *in vacuo*. This reaction yielded after chromatography 760mg (41 %) of clean alcohol.

$^1H$ NMR ($CDCl_3$) 400MHz δ 7.98(d, 2H, J=8.24 Hz), 7.66(d, 2H, J=8.24-Hz), 6.91(m, 2H), 6.72(d, 1H, J=8.10 Hz), 4.54(s, 2H), 3.80(s, 3H), 3.11 (t, 2H, J=7.42 Hz), 2.87(t, 2H, J=7.42 Hz), 2.18(s, 3H), 2.05(br s, 1H),

**4-(Bromomethyl)-5-[2-(4-methoxy-3-methylphenyl)ethyl]-2-[4-(trifluoromethyl)phenyl]-1,3-thiazole**

To a 100ml round-bottom flask equipped with a magnetic stir-bar and $N_2$ inlet was added {5-[2-(4-Methoxy-3-methylphenyl)ethyl]-2-[4-(trifluoromethyl)phenyl]-1,3-thiazol-4-yl}methanol (0.708g, 1.74mmoles, 1eq), $CH_2Cl_2$ (20ml), carbon tetrabromide (0.634g, 1.91mmoles, 1.1eq) and triphenylphosphine (0.501g, 1.91 mmoles, 1.1eq) in that order. The reaction was stirred overnight at which time it was diluted with $CH_2Cl_2$ and washed with $H_2O$, brine, dried over $Na_2SO_4$, concentrated *in* vacuo and purified via silica gel chromatography to yield 573mg (70%) of product.

$^1H$ NMR ($CDCl_3$) 400MHz δ 7.97(d, 2H, J=8.10 Hz), 7.64(d, 2H, J=8.10 Hz), 6.94(m, 2H), 6.73(d, 1H, J=8.10 Hz), 4.46(m, 2H), 3.79(m, 3H), 3.12(t, 2H, J= 7.24 Hz), 2.91(t, 2H, J=7.24 Hz), 2.19(s, 3H),

**4-(2-{4-(Bromomethyl)-2-[4-(trifluoromethyl)phenyl]-1,3-thiazol-5-yl}ethyl)-2-methylphenol**

To a 50ml round-bottom flask equipped with a magnetic stir-bar, an addition funnel and $N_2$ inlet was added 4-(Bromomethyl)-5-[2-(4-methoxy-3-methylphenyl)ethyl]-2-[4-(trifluoromethyl)phenyl]-1,3-thiazole (468mg, 1.0mmoles, 1eq) and dry $CH_2Cl_2$ (15ml, 0.1M). The mixture was cooled to -78°C (dry ice/acetone) after which boron tribromide (1M in $CH_2Cl_2$, 3ml, 3.0mmoles, 3eq) was added dropwise over the course of 15minutes. After the addition was complete, the cold bath was removed and the reaction was allowed to warm to room temperature and stirred for 1 hour. After this time, the reaction was cooled to 0°C and quenched very carefully with water. Once the reaction was quenched, it was transferred to a separatory funnel where the phases were separated. The aqueous fraction was washed three times with $CH_2Cl_2$ and the combined organic fractions were dried over $Na_2SO_4$, filtered, concentrated

*in vacuo* to yield a quantitative yield of the titled phenol. The product was used without purification.

$^1H$ NMR ($CDCl_3$) 400MHz δ 7.96(d, 2H, J=8.28 Hz), 7.65(d, 2H, J=8.28 Hz), 6.93(m, 1H), 6.85(d, 1H, J=8.10 Hz), 6.68(d, 1H, J=8.10 Hz), 5.42(br s, 1H), 4.45(s, 2H), 3.10(t, 2H, J=7.41 Hz), 2.89(t, 2H, J=7.41 Hz), 2.20(s, 3H),

The following compounds were made by amine displacement as described above for General Alkylation with an Amine:

**4-(2-{4-{[4-(4-Methoxyphenyl)-1-piperazinyl]methyl}-2-[4-(trifluoromethyl)phenyl]-1,3-thiazol-5-yl}ethyl)-2-methylphenol**

$^1H$ NMR ($CDCl_3$) 400MHz δ 7.94(d, 2H, J=8.28 Hz), 7.59(d, 2H, J=8.28 Hz), 6.91 (d, 1H, J=2.24 Hz), 6.86(d, 2H, J=9.31 Hz), 6.80(d, 2H, J=9.31 Hz), 6.74(dd, 1H, J=8.10, 2.24 Hz), 6.58(s, 1H), 6.51 (d, 1H, J=8.10 Hz), 3.73(s, 3H), 3.58(s, 2H), 3.12(t, 2H, J=7.50 Hz), 3.05(t, 4H, J=4.48 Hz), 2.84(t, 2H, J=7.50 Hz), 2.64(t, 4H, J=4.48 Hz), 2.20 (s, 3H),

**1-{4-[4-({5-[2-(4-Hydroxy-3-methylphenyl)ethyl]-2-[4-(trifluoromethyl)phenyl]-1,3-thiazol-4-yl}methyl)-1-piperazinyl]phenyl}ethanone**

$^1H$ NMR ($CD_3OD$) 400MHz δ 8.07(d, 2H, J=8.28 Hz), 7.85(d, 2H, J=9.14 Hz), 7.73(d, 2H, J=8.28 Hz), 6.92 (d, 2H, J=9.14 Hz), 6.88(d, 1H, J=2.24 Hz), 6.77(dd, 1H, J=8.28, 2.24 Hz), 6.60(d, 1H, J=8.28 Hz), 3.49(s, 2H), 3.32 (t, 4H, J=4.83 Hz), 3.18(t, 2H, J=7.07 Hz), 2.88(t, 2H, J=7.07 Hz), 2.51(t, 4H, J=4.83 Hz), 2.47(s, 3H), 2.10(s, 3H),

**4-(2-{4-{[4-(3-Methoxyphenyl)-1-piperazinyl]methyl}-2-[4-(trifluoromethyl)phenyl]-1,3-thiazol-5-yl}ethyl)-2-methylphenol**

$^1H$ NMR ($CD_3OD$) 400MHz δ 8.07(d, 2H, J=8.10 Hz), 7.72(d, 2H, J=8.10 Hz), 7.09(t, 1H, J=8.28 Hz), 6.88(s, 1H), 6.77(dd, 1H, J=8.45, 2.24 Hz), 6.59(d, 1H, J=8.45 Hz), 6.51(dd, 1H, J=8.28, 2.24 Hz), 6.46(t, 1H, J=2.24 Hz), 6.38(dd, 1H, J=8.28, 2.24 Hz), 3.72(s, 3H), 3.49(s, 2H), 3.18(t, 2H, J=6.47 Hz), 3.09(br s, 4H), 2.87(t, 2H, J=6.47 Hz), 2.52(br s, 4H), 2.10(s, 3H),

**4-(2-{4-{[4-(4-Chlorophenyl)-1-piperazinyl]methyl}-2-[4-(trifluoromethyl)phenyl]-1,3-thiazol-5-yl}ethyl)-2-methylphenol**

$^1H$ NMR ($CD_3OD$) 400MHz δ 8.07(d, 2H, J=8.10 Hz), 7.73(d, 2H, J=8.10 Hz), 7.15(d, 2H, J=9.14 Hz), 6.89 (m, 3H), 6.77(dd, 1H, J=8.45, 2.41 Hz), 6.59(d, 1H, J=8.45 Hz), 3.49(s, ZH), 3.18(t, 2H, J=7.16 Hz), 3.09(t, 4H, J=5.09 Hz), 2.87(t, 2H, J=7.16 Hz), 2.53(t, 4H, J=5.09 Hz), 2.10(s, 3H),

**2-[4-(2-{4-{[4-(4-Methoxyphenyl)-1-piperazinyl]methyl}-2-[4-(trifluoromethyl)phenyl]-1,3-thiazol-5-yl}ethyl)-2-methylphenoxy]-2-methylpropanoic acid**

To a 25ml round-bottom flask equipped with a magnetic stir-bar and $N_2$ inlet was added 4-(2-{4-{[4-(4-Methoxyphenyl)-1-piperazinyl]methyl}-2-[4-(trifluoromethyl)phenyl]-1,3-thiazol-5-yl}ethyl)-2-methylphenol (53mg, 0.094mmoles, 1eq) in acetone (2ml, 0.05M) followed by the addition of 2-trichloromethyl-2-propanol (33mg, 0.188mmoles, 2eq) and NaOH (pellets, 30mg, 0.752mmoles, 8eq). This was stirred at room temperature overnight after which the acetone was removed *in vacuo* and the resulting residue was partitioned between EtOAc and 1N HCl. The phases were then separated and the organic fraction was washed with brine, dried over $Na_2SO_4$ and concentrated *in vacuo* to yield after chromatography 23mg (40%) of product.

$^1H$ NMR ($CDCl_3$) 400MHz δ 7.95(d, 2H, J=8.28 Hz), 7.62(d, 2H, J=8.28 Hz), 6.88(m, 5H), 6.67(br s, 1H), 6.54 (br s, 1 H), 3.72(s, 3H), 3.61(s, 2H), 3.23(m, 8H), 2.80(m, 4H), 2.15(s, 3H), 1.54(s, 6H),

MS($ES^-$) M-H= 652.2

The following compounds were also made by alkylation of a phenol with trichloromethyl-2-propanol as above:

**2-[4-(2-{4-{[4-(4-Chlorophenyl)-1-piperazinyl]methyl)-2-[4-(trifluoromethyl)phenyl]-1,3-thiazol-5-yl}ethyl)-2-methylphenoxy]-2-methylpropanoic acid**

$^1H$ NMR ($CD_3OD$) 400MHz δ 7.99(d, 2H, J=8.28 Hz), 7.66(d, 2H, J=8.28 Hz), 7.55(s, 1H), 7.14(d, 2H, J=8.10 Hz), 6.91(s, 1H), 6.82(d, 2H, J=8.10 Hz), 6.66(br s, 1H), 3.55(s, 2H), 3.28(m, 2H) buried under MeOH signal, 3.12(br s, 4H), 2.85(s, 2H), 2.65(br s, 4H), 2.13(s, 3H), 1.52(s, 6H),

MS($ES^+$) M+H= 659.0

**2-[4-(2-{4-{[4-(3-Methoxyphenyl)-1-piperazinyl]methyl}-2-[4-(trifluoromethyl)phenyl]-1,3-thiazol-5-yl}ethyl)-2-methylphenoxy]-2-methylpropanoic acid**

$^1H$ NMR ($CD_3OD$) 400MHz δ 8.02(d, 2H, J=8.10 Hz), 7.68(d, 2H, J=8.10 Hz), 7.09(t, 1 H, J=8.10 Hz), 6.92 (s, 1H), 6.76(m, 2H), 6.50(dd, 1H, J=8.10, 2.07 Hz), 6.42(t, 1H, J=2.07 Hz), 6.37(dd, 1H, J=8.10, 2.07 Hz), 3.72(s, 3H), 3.51 (s, 2H), 3.28(m, 2H) buried under MeOH signal, 3.12(m, 4H), 2.83(t, 2H, J=7.16 Hz), 2.61 (m, 4H), 2.15(s, 3H), 1.48(s, 6H),

MS($ES^-$) M-H= 652.1

**2-[4-(2-{4-{[4-(4-Acetylphenyl)-1-piperazinyl]methyl}-2-[4-(trifluoromethyl)phenyl]-1,3-thiazol-5-yl}ethyl)-2-methylphenoxy]-2-methylpropanoic acid**

$^1H$ NMR ($CD_3OD$) 400MHz δ 8.01(d, 2H, J=8.10 Hz), 7.82(d, 2H, J=9.14 Hz), 7.67(d, 2H, J=8.10 Hz), 6.90 (m, 3H), 6.66(m, 2H), 3.61 (s, 2H), 3.37(br s, 4H), 3.13(t, 2H, J=6.81 Hz), 2.82(t, 2H, J=6.81 Hz), 2.68(br s, 4H), 2.44 (s, 3H), 2.11 (s, 3H), 1.50(s, 6H),

**2-Methyll-2-{2-methyl-4-[({4-[4-(trifluoromethyl)benzyl]-2-[4-(trifluoromethyl)phenyl]-1,3-thiazol-5-yl}methyl)sulfanyl]phenoxy}propanoic acid**

From 2-methyl-4-[({4-(4-trifluoromethyl)benzyl]-2-[4-(trifluoromethyl)phenyl]-1,3-thiazol-5-yl}methyl)sulfanyl] phenol (0.021 g, 0.04 mmol), 2-methyl-2-{2-methyl-4-[({4-[4-(trifluoromethyl)benzyl]-2-[4-(trifluoromethyl)phenyl]-1,3-thiazol-5-yl}methyl)sulfanyl]phenoxy}propanoic acid (0.006g, 25%) was obtained as a white solid.

$^1H$ NMR ($CD_3OD$): δ 8.02 (d, 2 H), 7.78 (d, 2 H), 7.60 (d, 2 H), 7.30 (d, 2 H), 7.23 (s, 1 H), 7.16 (d, 1H), 6.73 (d, 1 H), 4.29 (s, 2 H), 4.00 (s, 2 H), 2.17 (s, 3H), 1.61 (s, 6 H); $^{19}F$ NMR ($CD_3OD$): δ-64.18 (s), -64.73 (s); MS *m/z* 626 (M+1); HPLC RT 4.273 (C18 4.2x100mm, 0-100% ACN/$H_2O$ (0.1% TFA), 6min @ 2ml/min @254/220nm).

**2-Methyll-2-{2-methyl-4-[({4-[4-(trifluoromethoxy)benzyl]-2-[4-(trifluoromethyl)phenyl]-1,3-thiazol-5-yl}methyl)sulfanyl]phenoxy}propanoic acid**

From 2-methyl-4-[({4-(4-trifluoromethoxy)benzyl]-2-[4-(trifluoromethyl)phenyl]-1,3-thiazol-5-yl}methyl)sulfanyl]phenol (0.048g, 0.086 mmol), 2-methyl-2-{2-methyl-4-[({4-[4-(trifluoromethoxy)benzyl]-2-[4-(trifluoromethyl)phenyl]-1,3-thiazol-5-yl}methyl)sulfanyl]phenoxy}propanoic acid (0.013g, 23%) was obtained as a white solid.

$^1H$ NMR ($CD_3OD$): δ 8.04 (d, 2 H), 7.74 (d, 2 H), 7.20 (m, 6 H), 6.72 (d, 1 H), 4.26 (s, 2 H), 3.95 (s, 2 H), 2.15 (s, 3 H), 1.61 (s, 6H); $^{19}F$ NMR ($CD_3OD$): δ -59.86 (s), -64.72 (s); MS *mlz* 642 (M+1); HPLC RT 4.307 (C18 4.2x100mm, 0-100% ACN/$H_2O$ (0.1% TFA), 6min @ 2ml/min @254/220nm).

**2-{4-[({4-(4-methoxybenzyl)-2-[4-(trifluoromethyl)phenyl]-1,3-thiazol-5-yl}methyl)sulfanyl]-2-methylphenoxy}-2-methylpropanoic acid**

From 4-[({4-(4-methoxybenzyl)-2-[4-(trifluoromethyl)phenyl]-1,3-thiazol-5-yl}methyl)sulfanyl]-2-methylphenol (0.022g, 0.04 mmol), 2-{4-[({4-(4-methoxybenzyl)-2-[4-(trifluoromethyl)phenyl]-1,3-thiazol-5-yl}methyl)sulfanyl]-2-methylphenoxy}-2-methylpropanoic acid (0.003g, 12%) was obtained as a white solid.

$^1H$ NMR ($CD_3OD$): δ 8.04 (d, 2 H), 7.76 (d, 2 H), 7.19 (s, 1 H), 7.14 (d, 1 H), 7.02 (d, 2 H), 6.81 (d, 2H), 6.69 (d, 1 H), 4.21 (s, 2 H), 3.83 (s, 2 H), 3.78 (s, 3 H), 2.17 (s, 3 H), 1.60 (s, 6 H); MS m/z 588 (M+1); HPLC RT 4.136 (C18 4.2x100mm, 0-100% ACN/$H_2O$ (0.1% TFA), 6min @ 2ml/min @254/220nm).

**2-Methyll-2-{2-methyl-4-[({4-[4-(methylsulfanyl)benzyl]-2-[4-(trifluoromethyl)phenyl]-1,3-thiazol-5-yl}methyl)sulfanyl]phenoxy}propanoic acid**

From 2-methyl-4-[({4-(4-methylsulfanyl)benzyl]-2-[4-(trifluoromethyl)phenyl]-1,3-thiazol-5-yl}methyl)sulfanyl] phenol (0.296g, 0.57 mmol), 2-methyl-2-{2-methyl-4-[({4-[4-(methylsulfanyl)benzyl]-2-[4-(trifluoromethyl)phenyl]-1,3-thiazol-5-yl}methyl)sulfanyl]phenoxy}propanoic acid (0.087g, 25%) was obtained as a white solid.

$^1H$ NMR ($CD_3OD$): δ 8.04 (d, 2 H), 7.78 (d, 2 H), 7.13 (m, 6 H), 6.70 (d, 1 H), 4.22 (s, 2 H), 3.87 (s, 2 H), 2.47 (s, 3 H), 2.15 (s, 3 H), 1.60 (s, 6 H); MS *m/z* 604 (M+1); HPLC RT 4.220 (C18 4.2x100mm, 0-100% ACN/$H_2O$ (0.1% TFA), 6min @ 2ml/min @254/220nm).

**2-{4-[({4-(4-*tert*-butylbenzyl)-2-[4-(trifluoromethyl)phenyl]-1,3-thiazol-5-yl}methyl)sulfanyl]-2-methylphenoxy}-2-methylpropanoic acid**

From 4-[({4-(4-*tert*-butylbenzyl)-2-[4-(trifluoromethyl)phenyl]-1,3-thiazol-5-yl}methyl)sulfanyl]-2-methylphenol (0.113g, 0.21 mmol), 2-{4-[({4-(4-*tert*-butylbenzyl)-2-[4-(trifluoromethyl)phenyl]-1,3-thiazol-5-yl}methyl)sulfanyl]-2-methylphenoxy}-2-methylpropanoic acid (0.012g, 9%) was obtained as a white solid.
$^1$H NMR ($CD_3OD$): δ 8.04 (d, 2 H), 7.76 (d, 2 H), 7.29 (d, 2 H), 7.22 (s, 1 H), 7.16 (d, 1 H), 7.03 (d, 2 H), 6.74 (d, 1 H); MS m/z 614 (M+1); HPLC RT 4.464 (C18 4.2x100mm, 0-100% ACN/$H_2O$ (0.1% TFA), 6min @ 2ml/min @254/220nm).

**2-Methyll-2-{2-methyl-4-[({4-(3-thienylmethyl)-2-[4-(trifluoromethyl)phenyl]-1,3-thiazol-5-yl}methyl)sulfanyl] phenoxy}propanoic acid**

From 2-methyl-4-[({4-(3-thienylmethyl)-2-[4-(trifluoromethyl)phenyl]-1,3-thiazol-5-yl}methyl)sulfanyl]phenol (0.072g, 0.15 mmol), 2-methyl-2-{2-methyl-4-[({4-(3-thienylmethyl)-2-[4-(trifluoromethyl)phenyl]-1,3-thiazol-5-yl}methyl)sulfanyl]phenoxy}propanoic acid (0.039g, 46%) was obtained as a cream solid.
$^1$H NMR ($CD_3OD$): δ 8.05 (d, 2 H), 7.76 (d, 2 H), 7.37 (t, 1 H), 7.20 (s, 1 H), 7.15 (d, 1H), 7.02 (s, 1 H), 6.96 (d, 1 H), 6.70 (d, 1 H), 4.23 (s, 2 H), 3.96 (s, 2 H), 2.20 (s, 3 H), 1.60 (s, 6 H); MS *m/z* 564 (M+1); HPLC RT 4.112 (C18 4.2x100mm, 0-100% ACN/$H_2O$ (0.1% TFA), 6min @ 2ml/min @254/220nm).

**Ethyl 2-{2-methyl-4-[({4-[4-(trifluoromethoxy)benzyl]-2-[4-(trifluoromethyl)phenyl]-1,3-thiazol-5-yl}methyl) sulfanyl]phenoxy}propanoate**

From 2-methyl-4-[({4-(4-trifluoromethoxy)benzyl]-2-[4-(trifluoromethyl)phenyl]-1,3-thiazol-5-yl}methyl)sulfanyl]phenol (0.17g, 0.31 mmol), ethyl 2-{2-methyl-4-[({4-[4-(trifluoromethoxy)benzyl]-2-[4-(trifluoromethyl)phenyl]-1,3-thiazol-5-yl}methyl)sulfanyl]phenoxy}propanoate (0.17g, 83%) was obtained as a white solid. MS *m/z* 656 (M+1); HPLC RT 4.553 (C18 4.2x100mm, 0-100% ACN/$H_2O$ (0.1% TFA), 6min @ 2ml/min @254/220nm).

**Methyl {2-methyl-4-[({4-[4-(trifluoromethoxylbenzyl]-2-[4-(trifluoromethyl)phenyl]-1,3-thiazol-5-yl}methyl) sulfanyl]phenoxy}acetate**

From 2-methyl-4-[({4-(4-trifluoromethoxy)benzyl]-2-[4-(trifluoromethyl)phenyl]-1,3-thiazol-5-yl}methyl)sulfanyl]phenol (0.17g, 0.31 mmol), methyl {2-methyl-4-[({4-[4-trifluoromethoxy)benzyl]-2-[4-(trifluoromethyl)phenyl]-1,3-thiazol-5-yl}methyl)sulfanyl]phenoxy}acetate (0.15g, 80%) was obtained as a white solid. MS *m/z* 628 (M+1); HPLC RT 4.398 (C18 4.2x100mm, 0-100% ACN/$H_2O$ (0.1% TFA), 6min @ 2ml/min @254/220nm).

**Ethyl 2-{2-methyl-4-[({4-(3-thienylmethyl)-2-[4-(trifluoromethyl)phenyl]-1,3-thiazol-5-yl}methyl)sulfanyl] phenoxy}propanoate**

From 2-methyl-4-[({4-(3-thienylmethyl)-2-[4-(trifluoromethyl)phenyl]-1,3-thiazol-5-yl}methyl)sulfanyl]phenol, ethyl 2-{2-methyl-4-[({4-(3-thienylmethyl)-2-[4-(trifluoromethyl)phenyl]-1,3-thiazol-5-yl}methyl)sulfanyl]phenoxy}propanoate (0.225g, 0.47 mmol), (0.255g, 91%) was obtained as a yellow oil.
MS *m/z* 578 (M+1); HPLC RT 4.412 (C18 4.2x100mm, 0-100% ACN/$H_2O$ (0.1% TFA), 6min @ 2ml/min @254/220nm).

**Methyl {2-methyl-4-[({4-(3-thienylmethyl)-2-[4-(trifluoromethyl)phenyl]-1,3-thiazol-5-yl}methyl)sulfanyl] phenoxy}acetate**

From 2-methyl-4-[({4-{3-thienylmethyl)-2-[4-(trifluoromethyl)phenyl]-1,3-thiazol-5-yl}methyl)sulfanyl]phenol, methyl {2-methyl-4-[({4-(3-thienylmethyl)-2-[4-(trifluoromethyl)phenyl]-1,3-thiazol-5-yl}methyl)sulfanyl]phenoxy}acetate (0.225g, 0.47 mmol), (0.259g, 94%) was obtained as a yellow oil.
MS *m/z* 550 (M+1); HPLC RT 4.243 (G18 4.2x100mm, 0-100% ACN/$H_2O$ (0.1% TFA), 6min @ 2ml/min @254/220nm).
The following 2compounds were made by the Mitsunobu reaction of 4-[({4-{[4-(4-Methoxyphenyl)-1-piperazinyl]methyl}-2-[4-(trifluoromethyl)phenyl]-1,3-thiazol-5-yl}methyl)sulfanyl]-2-methylphenol with R and S Methyl lactate:

**Methyl (2*S*)-2-{4-[({4-{[4-(4-methoxyphenyl)-1-piperazinyl]methyl}-2-[4-(trifluoromethyl)phenyl]1,3-thiazol-5-yl}methyl)sulfanyl]-2-methylphenoxy}propanoate**

$^1$H NMR ($CDCl_3$) 400MHz δ 7.97(d, 2H, J=8.24 Hz), 7.64(d, 2H, J=8.24 Hz), 7.21 (d, 1H, J=2.20 Hz), 7.11 (dd, 1H, J=8.42, 2.20 Hz), 6.86(d, 2H, J=9.16 Hz), 6.80(d, 2H, J=9.16 Hz), 6.54(d, 1H, J=8.42 Hz), 4.70(q, 1H, J=6.78 Hz), 4.30(s, 2H), 3.74(s, 3H), 3.69(s, 3H), 3.55(s, 2H), 3.06(br s, 4H), 2.62(br s, 4H), 2.21 (s, 3H), 1.60(d, 3H, J=6.78 Hz),

**Methyl (2*R*)-2-{4-[({4-{[4-(4-methoxyphenyl)-1-piperazinyl]methyl}-2-[4-trifluoromethyl)phenyl]1,3-thiazol-5-yl}methyl)sulfanyl]-2-methylphenoxy}propanoate**

$^1$H NMR ($CDCl_3$) 400MHz δ 7.97(d, 2H, J=8.24 Hz), 7.64(d, 2H, J=8.24 Hz), 722(d, 1H. J=2.01 Hz), 7.12(dd, 1H, J=8.42, 2.01 Hz), 6.88(d, 2H, J=9.16 Hz), 6.80(d, 2H, J=9.16 Hz), 6.55(d, 1H, J=8.42 Hz), 4.70(q, 1H, J=6.78 Hz), 4.32(s, 2H), 3.73(s, 3H), 3.69(s, 3H), 3.55(s, 2H), 3.06(t, 4H, J=4.76 Hz), 2.61(br s, 4H), 2.22(s, 3H), 1.60(d, 3H, J=6.78 Hz),

**2-{4-[({4-{[4-(4-Methoxyphenyl)-1-piperazinyl]methyl}-2-[4-(trifluoromethyl)phenyl]-1,3-thiazol-5-yl}methyl)sulfanyl]phenoxy}-2-methylpropanoic acid**

To a stirred solution of ethyl 2-{4-[({4-{[4-(4-methoxyphenyl}-1-piperazinyl]methyl}-2-[4-(trifluoromethyl)phenyl]-1,3-thiazol-5-yl}methyl)sulfanyl]phenoxy}-2-methylpropanoate (77.0g, 0.112moles, 1eq) in THE (600ml, 0.19M) was added MeOH (50ml) and a 1N LiOH solution (6.18g in 250ml $H_2O$, 2.3eq). The mixture was refluxed for 5 hrs after which the THF was removed *in vacuo*. The residue was diluted with EtOAc and to it was added 1 N HCl until a pH of about 5 was reached. The phases were separated and the organic fraction was concentrated *in vacuo*, then titrated with isopropyl acetate twice which was subsequent removed *in vacuo* each time. The crude product was then recrystallized from EtOH to yield 52g (71%) of a white solid.

$^1$H NMR ($CD_3OD$) 400MHz δ 8.08(d, 2H, J=8.24 Hz), 7.75(d, 2H, J=8.24 Hz), 7.25(d, 2H, J=8.61 Hz), 6.94 (d, 2H, J=9.16 Hz), 6.82(m, 4H), 4.28(s, 2H), 3.72(s, 3H), 3.59(s, 2H), 3.16(t, 4H, J=4.94 Hz), 2.96(t, 4H, J=4.94 Hz), 1.54(s, 6H),

CHN Analysis: Theory (C, 60.26%; H, 5.21%; N, 6.39%) Found (C, 60.11%; H, 5.31%; N, 6.23%)

HPLC (C-18, 3µm) 0%-95% Acetonitrile/Water over 8 minutes $R_t$= 5.48minutes

**{4-[({4-benzyl-2-[4-(trifluoromethyl)phenyl]-1,3-thiazol-5-yl}methyl)sulfanyl]-2,5-dimethylphenoxy}acetic acid**

Mass spec: calculated for $C_{28}H_{24}F_3NO_3S_2$: 543. Found: 544 ($MH^+$). HPLC trace: retention time = 13.5 min (Alltima $C_{18}$, 5 micron, 250mm column, Gradient elution with 70-100% $CH_3CN/H_2O$).

**2-{4-[({4-benzyl-2-[4-(trifluoromethyl)phenyl]-1,3-thiazol-5-yl}methyl)sulfanyl]-2-methylphenoxy}propanoic acid**

Elemental analysis calculated for $C_{28}H_{24}F_3NO_3S_2$: C: 61.8%, H: 4.5%, N: 2.6%. Found: C: 61.77%, H: 4.64%, N: 2.51%. HPLC trace: retention Time .7 min (Alltima $C_{18}$, 5 micron, 250 mm column, gradient elution with 70-100% $CH_3CN/H_2O$).

**2-{4-[({4-benzyl-2-[4-(trifluoromethyl)phenyl]-1,3-thiazol-5-yl}methyl)sulfanyl]-2,3-dimethylphenoxy}propanoic acid**

Elememtal analysis calculated for $C_{29}H_{26}F_3NO_3S_2$: C: 62.4%, H: 4.7%, N: 2.5%. Found: C: 62.58%, H: 4.93%, N: 2.44%. HPLC trace: retention time= 14.7 min (Alltima $C_{18}$, 5 micron, 250 mm column using gradient elution with 70-100%$CH_3CN/H_2O$).

**2-{4-[({4-benzyl-2-[4-(trifluoromethyl)phenyl]-1,3-thiazol-5-yl}methyl)sulfanyl]-2-fluorophenoxy}propanoic acid**

Mass spec calculated for $C_{27}H_{21}F_4NO_3S_2$: 547. Found: 548 ($MH^+$). HPLC, Trace: retention time = 12.1 min (Alltima $C_{18}$, 5 micron, 250 mm column using gradient elution with 70-100% $CH_3CN/H_2O$).

**(2S)-2-{4-[({4-{[4-(4-Methoxyphenyl)-1-piperazinyl]methyl}-2-[4-(trifluoromethyl)phenyl]-1,3-thiazol-5-yl}methyl)sulfanyl]-2-methylphenoxy}propanoic acid**

$^1H$ NMR ($CD_3OD$) 400MHz δ 8.07(d, 2H, J=8.24 Hz), 7.74(d, 2H, J=8.24 Hz), 7.19(d, 1H, J=2.20 Hz), 7.09 (dd, 1H, J=8.42, 2.20 Hz), 6.91 (d, 2H, J=9.16 Hz), 6.80(d, 2H, J=9.16 Hz), 6.62(d, 1H, J=8.42 Hz), 4.68(q, 1H, J=6.78 Hz), 4.28(s, 2H), 3.71 (s, 3H), 3.48(s, 2H), 3.05(t, 4H, J=4.76 Hz), 2.69(t, 4H, J=4.76 Hz), 2.18(s, 3H), 1.57(d, 3H, J=6.78 Hz),

Chiral HPLC (Chiralpak, 2cm) 75% Carbon Dioxide/25% Methanol over 65minutes $R_f$@.88 minutes

**(2*R*)-2-{4-[({4-{[4-(4-Methoxyphenyl)-1-piperazinyl]methyl}-2-[4-(trifluoromethyl)phenyl]-1,3-thiazol-5-yl}methyl)sulfanyl]-2-methylphenoxy}propanoic acid**

$^1H$ NMR ($CD_3OD$) 400MHz δ 8.11(d, 2H, J=8.24 Hz), 7.76(d, 2H, J=8.24 Hz), 7.15(d, 1H, J=2.20 Hz), 7.08 (dd, 1H, J=8.42, 2.20 Hz), 6.93(d, 2H, J=9.16 Hz), 6.82(d, 2H, J=9.16 Hz), 6.67(d, 1H, J=8.42 Hz), 4.57(q, 1H, J=6.78 Hz), 4.24(s, 2H), 3.71 (s, 3H), 3.54(s, 2H), 3.17(t, 4H, J=4.76 Hz), 3.02(t, 4H, J=4.76 Hz), 2.18(s, 3H), 1.55(d, 3H, J=6.78 Hz),

Chiral HPLC (Chiralpak, 2cm) 75% Carbon Dioxide/25% Methanol over 65minutes $R_t$T.58 minutes

**2-(4-{[(2-(4-Fluorophenyl)-4-{[4-(4-methoxyphenyl)-1-piperazinyl]methyl}-1,3-thiazol-5-yl)methyl]sulfanyl}-2-methylphenoxy)-2-methylpropanoic acid**

$^1H$ NMR ($CD_3OD$) 400MHz δ 7.95(m, 2H), 7.18(m, 3H), 7.05(br s, 1H), 6.93(d, 2H, J=8.61 Hz), 6.81 (d, 2H, J=8.61 Hz), 6.69(br s, 1H), 4.22(s, 2H), 3.72(s, 3H), 3.55(s, 2H), 3.17(br s, 4H), 2.93(br s; 4H), 2.14(s, 3H), 1.59(s, 6H),

**[4-({[4-[(4-Benzyl-1-piperazinyl)methyl]-2-(4-{trifluoromethyl}phenyl)-1,3-thiazol-5-yl]methyl}sulfanyl)-2-methylphenoxy]acetic acid**

$^1H$ ($CD_3OD$) 300MHz δ 8.15(d, 2H, J=8.23 Hz), 7.81(d, 2H, J=8.23 Hz), 7.48(m, 5H), 7.24(s, 2H), 6.74(s, 1H), 4.55(s, 2H), 4.28(s, 2H), 4.15(s, 2H), 3.46(s, 2H), 3.06(s, 4H), 2.49(s, 4H), 2.09(s, 3H). MS($ES^-$) M-H= 625.98. TLC (10% MeOH/$CH_2Cl_2$) $R_f$ = 0.35

**{2-Methyl-4-[({2-(4-{trifluoromethyl}phenyl)-4-[(4-methyl-1-piperidinyl)methyl]-1,3-thiazol-5-yl}methyl)sulfanyl]phenoxy}acetic acid**

$^1H$ ($CD_3OD$) 300MHz δ 8.20(d, 2H, J=7.97 Hz), 7.85(d, 2H, J=7.97 Hz), 7.27(s, 1H), 7.08(s, 1H), 6.68(s, 1 H), 4.62(s, 2H), 4.29(s, 2H), 3.70(s, 2H), 2.86(s, 2H), 2.26(s, 3H), 1.90(s, 2H), 1.48(m, 5H), 1.06(s, 3H). MS(ES-) M-H= 548.91. TLC(10% MeOH/$CH_2Cl_2$) $R_f$ = 0.24

**[2-Methyl-4-({[4-{[4-(5-methyl-1,2,4-oxadiazol-3-yl)phenoxy]methyl}-2-(4-{trifluoromethyl}phenyl)-1,3-thiazol-5-yl]methyl}sulfanyl)phenoxy]acetic acid**

$^1H$ ($CDCl_3$) 400MHz δ 8.03(d, 2H, J=8.03 Hz), 7.93(d, 2H, J=8.89 Hz), 7.70(d, 2H, J=8.03 Hz), 7.19(d, 1H, J=2.22 Hz), 7.07(dd, 1H, J=8.37, 2.22 Hz), 6.96(d, 2H, J=8.89 Hz), 6.53(d, 1H, J=8.37 Hz), 4.88(s, 2H), 4.64(s, 2H), 4.27(s, 2H), 2.65(s, 3H), 2.17(s, 3H). TLC(5% MeOH/$CH_2Cl_2$) $R_f$ = 0.13. MS($ES^-$) M-H= 625.92

**[2-Methyl-4-({[4-{[3-(5-methyl-1,2,4-oxadiazol-3-yl)phenoxy]methyl}-2-(4-{trifluoromethyl}phenyl)-1,3-thiazol-5-yl]methyl}sulfanyl)phenoxy]acetic acid**

$^1H$ ($CDCl_3$) 400MHz δ 8.04(d, 2H, J=8.20 Hz), 7.69(m, 3H), 7.37(s, 2H), 7.16(dd, 1H, J=8.20, 2.22 Hz), 7.05 (dd, 1H, J=8.20, 2.22 Hz), 6.91(d, 1H, J=2.22 Hz), 6.62(d, 1H, J=8.20 Hz), 4.72(s, 2H), 4.43(s, 2H), 4.19(s, 2H), 2.73 (s, 3H), 2.09(s, 3H). TLC(5% MeOH/$CH_2Cl_2$) $R_f$ = 0.13. MS($ES^-$) M-H= 625.86

**(2-Methyl-4-{[(2-(4-{trifluoromethyl}phenyl)-4-{[4-(2-methylphenyl)-1-piperazinyl]methyl}-1,3-thiazol-5-yl)methyl]sulfanyl}phenoxy)acetic acid**

$^1H$ ($CDCl_3$) 400MHz δ 8.10(d, 2H, J=8.03 Hz), 7.73(d, 2H, J=8.03 Hz), 7.16(m, 4H), 7.01 (br s, 2H), 6.73(d, 1H, J=8.37 Hz), 4.79(s, 2H), 4.08(s, 2H), 3.80(m, 4H), 3.53(m, 2H), 3.24(m, 4H), 2.40(s, 3H), 2.18(s, 3H). TLC(5% MeOH/$CH_2Cl_2$) $R_f$ = 0.10. MS($ES^-$) M-H= 625.94

**[4-({[4-{[4-(4-Methoxyphenyl)-1-piperazinyl]methyl}-2-(4-{trifluoromethyl}phenyl)-1,3-thiazol-5-yl]methyl} sulfanyl)-2-methylphenoxy]acetic acid**

$^1H$ ($CDCl_3$) 400MHz δ 8.04(d, 2H, J=8.20 Hz), 7.72(d, 2H, J=8.20 Hz), 7.12(s, 1H), 6.96(m, 3H), 6.81(d, 2H, J=8.89 Hz), 6.74(d, 1H, J=8.37 Hz), 4.76(s, 2H), 4.05(s, 2H), 3.74(s, 3H), 3.38(m, 10H), 2.16(s, 3H). TLC(5% MeOH/$CH_2Cl_2$) $R_f$ = 0.13. MS($ES^-$) M-H= 641.90

**(2-Methyl-4-{[(2-(4-{trifluoromethyl}phenyl)-4-{[4-(3-methylphenyl)-1-piperazinyl]methly}-1,3-thiazol-5-yl) methyl]sulfanyl}phenoxy)acetic acid**

$^1H$ ($CDCl_3$) 400MHz δ 8.05(d, 2H, J=8.20 Hz), 7.72(d, 2H, J=8.20 Hz), 7.20(s, 1H), 7.06(d, 2H, J=9.06 Hz), 6.91 (m, 3H), 6.72(d, 1H, J=8.37 Hz), 4.77(s, 2H), 4.06(s, 2H), 3.54(br s, 8H), 3.27(s, 2H), 2.30(s, 3H), 2.16(s, 3H). TLC(5% MeOH/$CH_2Cl_2$) $R_f$ = 0.10. MS($ES^-$) M-H= 625.99

**(2-Methyl-4-{[(2-(4-{trifluoromethyl}phenyl)-4-{[4-(4-methylphenyl)-1-piperazinyl]methyl}-1,3-thiazol-5-yl) methyl]sulfanyl}phenoxy)acetic acid**

$^1H$ ($CDCl_3$) 400MHz δ 8.03(d, 2H, J=8.20 Hz), 7.71 (d, 2H, J=8.20 Hz), 7.02(m, 6H), 6.71(d, 1H, J=8.55 Hz), 4.76(s, 2H), 4.08(s, 2H), 3.52(br s, 8H), 3.31(s, 2H), 2.27(s, 3H), 2.16(s, 3H). TLC(5% MeOH/$CH_2Cl_2$) $R_f$ = 0.10. MS ($ES^-$) M-H= 625.94

**[4-({[4-{[4-(2-Furoyl)-1-piperazinyl]methyl}-2-(4-{trifluoromethyl}phenyl)-1,3-thiazol-5-yl]methyl}sulfanyl)-2-methylphenoxy]acetic acid**

$^1H$ ($CDCl_3$) 400MHz δ 8.02(d, 2H, J=8.20 Hz), 7.71 (d, 2H, J=8.20 Hz), 7.48(d, 1 H, J=2.05 Hz), 7.16(dd, 1H, J=8.20, 2.05 Hz), 7.07(m, 1H), 6.90(d, 1H, J=2.39 Hz), 6.74(d, 1H, J=8.20 Hz), 6.49(m, 1H), 4.77(s, 2H) 4.62(s, 2H), 4.05(s, 2H), 3.46(s, 2H), 3.27(s, 2H), 3.05(br s, 4H), 2.15(s. 3H) TLC(5% MeOH/$CH_2Cl_2$) $R_f$ = 0.10. MS($ES^-$) M-H= 629.83

**(2-Methyl-4-{[(2-(4-{trifluoromethylphenyl)-4-{[4-(2-pyridinyl)-1-piperazinyl]methyl}-1,3-thiazol-5-yl)methyl] sulfanyl}phenoxy)acetic acid**

$^1H$ ($CDCl_3$) 400MHz δ 8.22(m, 1H), 7.99(d, 2H, J=8.20 Hz), 7.68(d, 2H, J=8.20 Hz), 7.60(s, 1H), 7.20(dd, 1H, J=8.37, 2.39 Hz), 7.14(s, 1 H), 6.76(m, 1H), 6.68(m, 1H), 4.68(s, 2H), 4.14(s, 2H), 3.72(br s, 4H), 3.59(s, 2H), 2.87(br s, 4H), 2.17(s, 3H). TLC(5% MeOH/$CH_2Cl_2$) $R_f$ = 0.10. MS(ES-) M-H= 612.99

**[4-({[4-{[4-(4-Chlorobenzyl)-1-piperazinyl]methyl}-2-(4-{trifluoromethyl}phenyl)-1,3-thiazol-5-yl]methyl} sulfanyl)-2-methylphenoxy]acetic acid**

$^1H$ ($CDCl_3$) 400MHz δ 8.04(d, 2H, J=8.20 Hz), 7.70(d, 2H, J=8.20 Hz), 7.41 (m, 4H), 7.14(m, 1H), 7.03(m, 1H), 6.69(d, 1H, J=8.37 Hz), 4.72(s, 2H), 4.02(s, 2H), 3.18(m, 12H), 2.10(s, 3H). TLC(5% MeOH/$CH_2Cl_2$) $R_f$ = 0.10. MS($ES^-$) M-H= 659.78

**[4-({[4-{[4-(4-acetylphenyl)-1-piperazinyl]methyl}-2-(4-{trifluoromethyl}phenyl)-1,3-thiazol-5-yl]methyl} sulfanyl)-2-methylphenoxy]acetic acid**

$^1H$ ($CDCl_3$) 400MHz δ 7.97(d, 2H, J=8.03 Hz), 7.85(d, 2H, J=8.89 Hz), 7.70(d, 2H, J=8.03 Hz), 7.16(dd, 1H, J=8.37, 2.22 Hz), 6.86(m, 3H), 6.75(d, 1H, J=8.37 Hz), 4.77(s, 2H), 4.04(s, 2H), 3,80(m, 4H), 3.45(m, 4H), 3.29(s, 2H), 2.51(s, 3H), 2.17(s, 3H). TLC(5% MeOH/$CH_2Cl_2$) $R_f$ = 0.10. MS($ES^-$) M-H= 653.99

**(4-{[(4-{[4-(4-Methoxyphenyl)-1-piperazinyl]methyl}-2-phenyl-1,3-thiazol-5-yl)methyl]sulfanyl}-2-methylphenoxy)acetic acid**

$^1H$ NMR ($CDCl_3$) 400MHz 9.94(s, 1H), 7.84(m, 2H), 7.41(m, 3H), 7.11(d, 1H, J=2.22 Hz), 7.06(dd, 1H, J=8.37, 2.22 Hz), 6.79(m, 4H), 6.60(d, 1H, J=8.37 Hz), 4.54(s, 2H), 4.18(s, 2H), 3.76(s, 2H), 3.22(m, 8H), 2.18(s, 3H). HPLC (C-18, 3μm) 1%MeOH/0-90% $CH_3CN$/Water (0.1% TFA)/(50mM $Et_3N$/TFA) 4min run $R_t$=2.67 min

**2-{4-[({4-{[4-(4-Methoxyphenyl)-1-piperazinyl]methyl}-2-[4-(trifluoromethyl)phenyl]-1,3-thiazol-5-yl}methyl) sulfanyl]-2-methylphenoxy}propanoic acid**

$^1H$ NMR ($CDCl_3$) 400MHz δ9.69(s, 1 H), 7.96(d, 2H, J=8.20 Hz), 7.65(d, 2H, J=8.20 Hz), 7.07(d, 1H, J=2.05 Hz), 7.02(dd, 1H, J=8.55, 2.05 Hz), 6.87(d, 2H, J=9.23 Hz), 6.80(d, 2H, J=9.23 Hz), 6.66(d, 1H, J=8.55 Hz), 4.66(q, 1H, J=6.95 Hz), 4.10(d, 1H, J .70 Hz), 4.05(d, 1H, J .70 Hz), 3.74(s, 3H), 3.57(d, 1H, J .18 Hz), 3.51(d, 1H, J .18 Hz), 3.15(br s, 4H), 2.96(br s, 4H), 2.17(s, 3H), 1.59(d, 3H, J=6.95 Hz). HPLC(C-18, 3μm) 1%MeOH/0-90% $CH_3CN$/Water (0.1% TFA)/(50mM $Et_3N$/TFA) 4min run $R_t$=2.91 min

**2-(4-{[(4-{[4-(4-Methoxyphenyl)-1-piperazinyl]methyl}-2-phenyl-1,3-thiazol-5-yl)methyl]sulfanyl} 2-methylphenoxy)propanoic acid**

$^1H$ NMR ($CDCl_3$) 400MHz δ7.81(m, 2H), 7.34(m, 3H), 7.09(m, 1H), 6.90(m, 1H), 6.79(m, 4H), 6.48(d, 1H, J=8.37 Hz), 4.35(m, 1H), 4.16(s, 2H), 3.70(s, 3H), 3.32(s, 2H), 3.00(m, 4H), 2.60(m, 4H), 2.09(s, 3H), 1.34(m, 3H). HPLC(C-18, 3μm) 1%MeOH/0-90% $CH_3CN$/Water (0.1% TFA)/(50mM $Et_3N$/TFA) 4min run $R_t$=2.78 min

**{2-Methyl-4-[({2-(4-{trifluoromethyl}phenyl)-4-[(4-phenyl-1-piperazinyl)methyl]-1,3-thiazol-5-yl}methyl) sulfanyl]phenoxy}acetic acid**

$^1H$ ($CD_3OD$) 300MHz δ 8.16(d, 2H, J=8.49 Hz), 7.81 (d, 2H, J=8.49 Hz), 7.26(br s, 3H), 7.09(br s, 1H), 6.98 (d, 2H, J=7.96 Hz), 6.88(m, 1H), 6.66(br s, 1H), 4.57(s, 2H), 4.29(s, 2H), 3.55(s, 2H), 3.26(br s, 4H), 2.91 (br s, 4H), 2.23(s, 3H). MS($ES^-$) M-H= 611.85. TLC(10% MeOH/$CH_2Cl_2$) $R_f$ = 0.30

**[4-({[4-{[4-(Ethoxycarbonyl)-1-piperazinyl]methyl}-2-(4-{trifluoromethyl}phenyl)-1,3-thiazol-5-yl]methyl} sulfanyl)-2-methylphenoxy]acetic acid**

$^1H$ ($CD_3OD$) 300MHz δ 8.14(d, 2H, J=8.23 Hz), 7.81(d, 2H, J=8.23 Hz), 7.26(s, 1H), 7.12(s, 1H), 6.71 (s, 1H), 4.63(s, 2H), 4.32(s, 2H), 4.16(q, 2H, J=7.08 Hz), 3.55(br s, 4H), 3.44(s, 2H), 2.60(br s, 4H), 2.25(s, 3H), 1.30(t, 3H, J=7.08 Hz). MS($ES^-$) M-H= 607.86. TLC(10% MeOH/$CH_2Cl_2$) $R_f$ = 0.28

**{2-Methyl-4-[({2-(4-{trifluoromethyl}phenyl)-4-[(4-phenyl-1-piperidinyl)methyl]-1,3-thiazol-5-yl}methyl) sulfanyl]phenoxy}acetic acid**

$^1H$ ($CD_3OD$) 300MHz δ 8.14(d, 2H, J=8.23 Hz), 7.77(d, 2H, J=8.23 Hz), 7.28(s, 7H), 6.75(d, 1H, J=8.23 Hz), 4.45(s, 2H), 4.34(s, 2H), 3.53(s, 2H), 3.08(m, 2H), 2.57(m, 1H), 2.35(m, 2H), 2.22(s, 3H), 1.80(m, 4H). MS($ES^-$) M-H= 610.91. TLC(10% MeOH/$CH_2Cl_2$) $R_f$ = 0.30

**[4-({[4-{[(Cyclopropylmethyl)amino]methyl}-2-(4-{trifluoromethyl}phenyl)-1,3-thiazol-5-yl]methyl}sulfanyl)- 2-methylphenoxy]acetic acid**

$^1H$ NMR 300MHz δ 8.08(d, 2H, J=8.20 Hz), 7.74(d, 2H, J=8.20 Hz), 7.14(dd, 1H, J=8.49, 2.39 Hz), 7.01(s, 1H), 6.72(d, 1H, J=8.49 Hz), 4.77(s, 2H), 4.03(s, 2H), 3.29(s, 2H), 2.77(d, 2H, J=7.43 Hz), 2.17(s, 3H), 1.17(m, 1H), 0.62(m, 2H), 0.28(m, 2H). MS($ES^-$) M-H= 520.90. HPLC(C-18, 3μm) 1 %MeOH/0-90% $CH_3CN$/Water (0.1 % TFA)/ (50mM $Et_3N$/TFA) 4min run $R_t$=2.67 min

**{2-Methyl-4-[({2-(4-{trifluoromethyl}phenyl)-4-[(pentylamino)methyl]-1,3-thiazol-5-yl}methyl)sulfanyl] phenoxy}acetic acid**

$^1H$ NMR 300MHz δ 8.06(d, 2H, J=8.23 Hz), 7.69(d, 2H, J=8.23 Hz), 7.05(m, 2H), 6.66(d, 1H, J=8.23 Hz), 4.67 (s, 2H), 4.06(s, 2H), 3.35(s, 2H), 2.78(t, 2H, J=6.64 Hz), 2.17(s, 3H), 1.71 (m, 2H), 1.22(m, 4H), 0.83(t, 3H, J=6.64 Hz). MS($ES^-$) M-H= 536.90. HPLC(C-18, 3μm) 1%MeOH/0-90% $CH_3CN$/Water (0.1% TFA)/(50mM $Et_3N$/TFA) 4min run $R_t$=2.80 min

**4-({[4-{[4-(2-Hydroxyethyl)-1-piperazinyl]methyl}-2-(4-{trifluoromethyl}phenyl)-1,3-thiazol-5-yl]methyl} sulfanyl)-2-methylphenoxy]acetic acid**

$^1H$ NMR ($CD_3OD$) 300MHz δ 8.16(d, 2H, J=8.23 Hz), 7.80(d, 2H, J=8.23 Hz), 7.26(m, 2H), 6.80(d, 1H, J=8.49 Hz), 4.76(s, 2H), 4.40(s, 2H), 3.95(m, 2H), 3.84(s, 2H), 3.54(br s, 4H), 3.33(m, 2H), 3.20(br s, 4H), 2.22(s, 3H). HPLC

(C-18, 3μm) 1%MeOH/0-90% $CH_3CN$/Water (0.1% TFA)/(50mM $Et_3N$/TFA) 4min run $R_t$=2.48 min

**(2-Methyl-4-{[(2-(4-{trifluoromethyl}phenyl)-4-{[(3-pyridinylmethyl)amino]methyl}-1,3-thiazol-5-yl)methyl] sulfanyl}phenoxy)acetic acid**

$^1H$ NMR ($CDCl_3$) 300MHz δ 8.58(d, 1H, J=1.59 Hz), 8.48(dd, 1H, J=4.78, 1.59 Hz), 8.03(m, 3H), 7.66(d, 2H, J=8.23 Hz), 7.24(m, 1H), 7.06(d, 1H, J=2.39 Hz), 6.99(d, 1H, J=2.39 Hz), 6.59(d, 1H, J=8.49 Hz), 4.61 (s, 2H), 4.04 (s, 2H), 3.93(s, 2H), 3.28(s, 2H), 2.13(s, 3H). MS($ES^-$) M-H= 557.80. HPLC(C-18, 3μm) 1%MeOH/0-90% $CH_3CN$/Water (0.1% TFA)/(50mM $Et_3N$/TFA) 4min run $R_t$=2.44 min

**[4-({[4-[(3-Hydroxy-1-piperidinyl)methyl]-2-(4-{trifluoromethyl}phenyl)-1,3-thiazol-5-yl]methyl}sulfanyl)-2-methylphenoxy]acetic acid**

$^1H$ NMR ($CDCl_3$) 300MHz δ 8.00(d, 2H, J=8.37 Hz), 7.69(d, 2H, J=8.37 Hz), 7.23(dd, 1H, J=8.55, 2.20 Hz), 6.94(d, 1H, J=2.20 Hz), 6.69(d, 1H, J=8.55 Hz), 4.68(s, 2H), 4.21(s, 2H), 3.16(m, 7H), 2.12(s, 3H), 1.63(m, 4H). MS ($ES^-$) M-H= 550.8. HPLC(C-18, 3μm) 1%MeOH/0-90% $CH_3CN$/Water (0.1 % TFA)/(50mM $Et_3N$/TFA) 4min run $R_t$=2.58 min

**[4-({[4-[(4-Hydroxy-1-piperidinyl)methyl]-2-(4-{trifluoromethyl}phenyl)-1,3-thiazol-5-yl]methyl}sulfanyl)-2-methylphenoxy]acetic acid**

$^1H$ NMR ($CDCl_3$) 300MHz δ 7.97(d, 2H, J=8.23 Hz), 7.65(d, 2H, J=8.23 Hz), 7.11(m, 2H), 6.58(d, 1H, J=8.23 Hz), 4.53(s, 2H), 4.18(s, 2H), 3.86(br s, 1H), 3.62(m, 2H), 3.12(m, 2H), 2.95(m, 2H), 2.15(s, 3H), 2.04(m, 2H), 1.77(m, 2H). HPLC(C-18, 3μm) 1%MeOH/0-90% $CH_3CN$/Water (0.1% TFA)/(50mM $Et_3N$/TFA) 4min run $R_t$=2.54 min

**[4-({[4-{[2-(hydroxymethyl)-1-piperidinyl]methyl}-2-(4-{trifluoromethyl}phenyl)-1,3-thiazol-5-yl]methyl} sulfanyl)-2-methylphenoxy]acetic acid**

MS($ES^-$) M-H= 564.94. HPLC(C-18, 3μm) 1%MeOH/0-90% $CH_3CN$/Water (0.1% TFA)/(50mM $Et_3N$/TFA) 4min run $R_t$=2.66 min

**[4-({[4-{[4-(Hydroxymethyl)-1-piperidinyl]methyl}-2-(4-{trifluoromethyl}phenyl)-1,3-thiazol-5-yl]methyl} sulfanyl)-2-methylphenoxy]acetic acid**

$^1H$ NMR ($CDCl_3$) 400MHz δ 7.94(d, 2H, J=8.20 Hz), 7.64(d, 2H, J=8.20 Hz), 7.13(dd, 1H, J=8.55, 2.39 Hz), 7.06(d, 1H, J=2.39 Hz), 6.58(d, 1H, J=8.55 Hz), 4.60(s, 2H), 4.45(s, 2H), 4.18(s, 2H), 3.56(m, 6H), 2.75(br s, 1H), 2.11 (s, 3H), 1.68(m, 4H). MS($ES^-$) M-H= 564.93. HPLC(C-18, 3μm) 1%MeOH/0-90% $CH_3CN$/Water (0.1% TFA)/(50mM $Et_3N$/TFA) 4min run $R_t$=2.56 min

**[2-Methyl-4-({[2-(4-{trifluoromethyl}phenyl)-4-(4-morpholinylmethyl)-1,3-thiazol-5-yl]methyl}sulfanyl) phenoxy]acetic acid**

$^1H$ NMR ($CD_3OD$) 300MHz δ 8.11(d, 2H, J=8.23 Hz), 7.79(d, 2H, J=8.23 Hz), 7.25(br s, 1H), 7.17(dd, 1H, J=8.23, 2.39 Hz), 6.74(d, 1H, J=8.23 Hz), 4.46(s, 2H), 4.32(s, 2H), 3.69(br s, 4H), 3.47(s, 2H), 2.50(br s, 4H), 2.23(s, 3H). MS($ES^-$) M-H= 536.43. TLC(20% MeOH/$CH_2Cl_2$) $R_f$= 0.39

**[4-({[4-[(Cyclohexylamino)methyl]-2-(4-{trifluoromethyl}phenyl)-1,3-thiazol-5-yl]methyl}sulfanyl)-2-methylphenoxy]acetic acid**

$^1H$ NMR ($CDCl_3$) 400MHz δ 8.01(d, 2H, J=8.20 Hz), 7.66(d, 2H, J=8.20 Hz), 7.04(m, 2H), 6.61 (d, 1H, J=8.20 Hz), 4.64(s, 2H), 4.14(s, 2H), 3.39(s, 2H), 2.86(m, 1H), 2.14(s, 3H), 2.01 (m, 2H), 1.73(m, 2H), 1.48(m, 4H), 1.08(m, 2H). MS($ES^-$) M-H= 548.7-. HPLC(C-18, 3μm) 1%MeOH/0-90% $CH_3CN$/Water (0.1% TFA)/(50mM $Et_3N$/TFA) 4min run $R_t$=2.75 min

**[2-Methyl-4-({[4-{[(2-methylcyclohexyl)amino]methyl}-2-(4-{trifluoromethyl}phenyl)-1,3-thiazol-5-yl]methyl} sulfanyl)phenoxy]acetic acid**

$^1H$ NMR 400MHz δ 7.98(d, 2H, J=8.20 Hz), 7.68(d, 2H, J=8.20 Hz), 7.09(dd, 1H, J=8.37, 2.39 Hz), 6.98(d,

1H, J=2.39 Hz), 6.65(d, 1H, J=8.37 Hz), 4.66(s, 2H), 4.15(d, 1H, J .70 Hz), 4.00(d, 1H, J .70 Hz), 3.53(d, 1H, J .04 Hz), 3.33(d, 1H, J .04 Hz), 2.53(m, 1H), 2.10(s, 3H), 1.74(m, 7H), 1.37(m, 2H), 1.03(d, 3H, J=6.32 Hz). MS(ES$^-$) M-H= 562.80. HPLC(C-18, 3μm) 1%MeOH/0-90% $CH_3CN$/Water (0.1 % TFA)/(50mM $Et_3N$/TFA) 4min run $R_t$=2.87 min

**[2-Methyl-4-({[4-{[(3-methylcyclohexyl)amino]methyl}-2-(4-{trifluoromethyl}phenyl)-1,3-thiazol-5-yl]methyl} sulfanyl)phenoxy]acetic acid**

$^1$H NMR 400MHz δ 8.01(d, 2H, J=8.20 Hz), 7.68(d, 2H, J=8.20 Hz), 7.05(m, 2H), 6.62(d, 1H, J=8.37 Hz), 4.68 (s, 2H), 4.29(s, 2H), 3.32(s, 2H), 2.90(m, 1H), 2.15(s, 3H), 2.00(m, 5H), 1.56(m, 4H), 0.89(d, 3H, J=6.32 Hz). MS(ES$^-$) M-H= 562.9. HPLC(C-18, 3μm) 1%MeOH/0-90% $CH_3CN$/Water (0.1% TFA)/(50mM $Et_3N$/TFA) 4min run $R_t$=2.85 min

**[2-Methyl-4-({[4-{[(4-methylcyclohexyl)amino]methyl}-2-(4-{trifluoromethyl}phenyl)-1,3-thiazol-5-yl]methyl} sulfanyl)phenoxy]acetic acid**

$^1$H NMR 400MHz δ 7.99(d, 2H, J=8.20 Hz), 7.64(d, 2H, J=8.20 Hz), 7.02(m, 2H), 6.59(d, 1H, J=8.03 Hz), 4.58 (s, 2H), 4.16(s, 2H), 3.44(s, 2H), 2.90(br s, 1H), 2.12(s, 3H), 2.01(m, 3H), 1.62(m, 6H), 0.90(d, 3H, J=6.84 Hz). MS (ES$^-$) M-H= 562.90. HPLC(C-18, 3μm) 1%MeOH/0-90% $CH_3CN$/Water (0.1% TFA)/(50mM $Et_3N$/TFA) 4min run $R_t$=2.85 min

**[2-Methyl-4-({[4-[(2-methylphenoxy)methyl]-2-(4-{trifluoromethyl}phenyl)-1,3-thiazol-5-yl]methyl}sulfanyl) phenoxy]acetic acid**

$^1$H ($CDCl_3$) 300MHz δ 8.03(d, 2H, J=8.23 Hz), 7.72(d, 2H, J=8.23 Hz), 7.17(m, 4H), 6.91 (m, 2H). 6.59(d, 1H, J=8.49 Hz), 4.96(s, 2H), 4.67(s, 2H), 2.25{s, 3H), 2.21(s, 3H). MS(ES$^-$) M-H= 557.8

**[2-Methyl-4-({[4-[(3-methylphenoxy)methyl]-2-(4-{trifluoromethyl}phenyl)-1,3-thiazol-5-yl]methyl}sulfanyl) phenoxy]acetic acid**

$^1$H ($CDCl_3$) 300MHz δ 8.06(d, 2H, J=8.23 Hz), 7.73(d, 2H, J=8.23 Hz), 7.26(dd. 1H. J=2.39, 0.53 Hz), 7.20(t, 1H, J=7.83 Hz), 7.12(ddd, 1H, J=8.49, 2.39, 0.53 Hz), 6.80(m, 3H), 6.61(d, 1H, J=8.49 Hz), 4.86(s, 2H), 4.67(s, 2H), 4.32(s, 2H), 2.36(s, 3H), 2.23(s, 3H). MS(ES$^-$) M-H= 557.83

**[2-Methyll-4-({[4-[(4-Methyllphenoxy)methyl]-2-(4-{trifluoromethyl}phenyl)-1,3-thiazol-5-yl]methyl}sulfanyl) phenoxy]acetic acid**

MS(ES$^-$) M-H= 557.8

CHN Analysis: Theory $1.5H_20$ (C, 57.33%; H, 4.64%; N, 2.39%) Found (C, 57.34%; H, 4.24%; N, 2.37%)

**[4-({[4-[(3-Cyanophenoxy)methyl]-2-(4-{trifluoromethyl}phenyl)-1,3-thiazol-5-yl]methyl}sulfanyl)-2-Methyllphenoxy]acetic acid**

$^1$H ($CDCl_3$) 300MHz δ 8.05(d, 2H, J=8.23 Hz), 7.74(d, 2H, J=8.23 Hz), 7.38(m, 2H), 7.17(m, 4H), 6.67(d, 1H, J=8.23 Hz), 4.76(s, 2H), 4.72(s, 2H), 4.25(s, 2H), 2.23(s. 3H). MS(ES$^-$) M-H= 569.2

**[4-({[4-[(4-Cyanophenoxy)methyl]-2-(4-{trifluoromethyl}phenyl)-1,3-thiazol-5-yl]methyl}sulfanyl)-2-methylphenoxy]acetic acid**

$^1$H ($CDCl_3$) 300MHz δ 9.94(s, 1H), 8.03(d, 2H, J=8.23 Hz), 7.73(d, 2H, J=8.23 Hz), 7.60(d, 2H, J=9.03 Hz), 7.27(d, 1H, J=2.12 Hz), 7.10(dd, 1H, J=8.49, 2.12 Hz), 7.00(d, 2H, J=9.03 Hz), 6.61 (d, 1H, J=8.49 Hz), 4.85(s, 2H), 4.69(s, 2H), 4.25(s, 2H), 2.21 (s, 3H). MS(ES$^-$) M-H= 569.2

**(2-Methyl-4-{[(4-{[4-(5-methyl-1,2,4-oxadiazol-3-yl)phenoxy]methyl}-2-phenyl-1,3-thiazol-5-yl)methyl]sulfanyl} phenoxyacetic acid**

$^1$H ($CDCl_3$) 400MHz 7.95(d, 2H, J=9.06 Hz), 7.87(m, 2H), 7.43(m, 3H), 7.20(d, 1 H, J=2.39 Hz), 7.05(dd, 1H, J=8.55, 2.39 Hz), 6.95(d, 2H, J=9.06 Hz), 6.52(d, 1H, J=8.55 Hz), 4.80(s, 2H), 4.61 (s, 2H), 4.24(s, 2H), 2.63(s, 3H), 2.17(s, 3H). MS(ES$^-$) M-H= 558.40

**2-(2-Methyl-4-{[(4-{[4-(5-methyl-1,2,4-oxadiazol-3-yl)phenoxy]methyl}-2-phenyl-1,3-thiazol-5-yl)methyl] sulfanyl}phenoxy)propanoic acid**

$^1H$ ($CDCl_3$) 400MHz δ 7.93(d, 2H, J=9.06 Hz), 7.85(m, 2H), 7.40(m, 3H), 7.19(d, 1H, J=2.22 Hz), 7.02(dd, 1H, J=8.37, 2.22 Hz), 6.94(d, 2H, J=9.06 Hz), 6.52(d, 1H, J=8.37 Hz), 4.81 (d, 1H, J .79 Hz), 4.74(d, 1H, J .79 Hz), 4.68(q, 1H, J=6.78 Hz), 4.21 (s, 2H), 2.62(m, 3H), 2.16(s, 3H), 1.61(d, 3H, J=6.78 Hz). MS($ES^-$) M-H= 571.50

**2-{2-Methyl-4-[({4-{[3-(5-methyl-1,2,4-oxadiazol-3-yl)phenoxy]methyl}-2-[4-(trifluoromethyl)phenyl]-1,3-thiazol-5-yl}methyl)sulfanyl]phenoxy}propanoic acid**

$^1H$ NMR ($CDCl_3$) 400MHz δ 7.99(d, 2H, J=8.20 Hz), 7.67(m, 3H), 7.47(m, 1H), 7.36(t, 1H, J=8.03 Hz), 7.10 (dd, 1H, J=8.37, 2.39 Hz), 7.04(dd, 1H, J=8.37, 2.39 Hz), 6.99(m, 1H), 6.61(d, 1H, J=8.37 Hz), 4.75(q, 1H, J=6.84 Hz), 4.62(d, 1H, J .45 Hz), 4.43(d, 1H, J .45 Hz), 4.23(d, 1H, J .70 Hz), 4.16(d, 1H, J .70 Hz), 2.70(s, 3H), 2.12(s, 3H), 1.68 (d, 3H, J=6.84 Hz). MS($ES^+$) M+H= 642.00

**2-(2-Methyl-4-{[(4-{[3-(5-methyl-1,2,4-oxadiazol-3-yl)phenoxy]methyl}-2-phenyl-1,3-thiazol-5-yl)methyl] sulfanyl}phenoxy)propanoic acid**

$^1H$ NMR ($CDCl_3$) 400MHz δ 7.90(m, 2H), 7.67(t, 1H, J=7.52Hz), 7.46(m, 1H), 7.42(m, 3H), 7.35(t, 1H, J=7.52 Hz), 7.08(dd, 1H, J=8.37, 2.39 Hz), 7.04(d, 1H, J=8.37 Hz), 7.00(d, 1H, J=2.39 Hz), 6.61 (d, 1H, J=8.37 Hz), 4.73(q, 1H, J=6.84 Hz), 4.58(d, 1H, J .45 Hz), 4.43(d, 1H, J .45 Hz), 4.20(d, 1H, J .70 Hz), 4.15(d, 1H, J .70 Hz), 2.69(s, 3H), 2.12(s, 3H), 1.66(d, 3H, J=6.84 Hz). MS($ES^+$) M+H= 573.80

**[2-Methyl-4-({[2-(4-{trifluoromethyl}phenyl)-4-(phenoxymethyl)-1,3-thiazol-5-yl]methyl}sulfanyl)phenoxy] acetic acid**

$^1H$ ($CDCl_3$) 300MHz δ 8.02(d, 2H, J=8.23 Hz), 7.70(d, 2H, J=8.23 Hz), 7.33(m, 2H), 7.22(s, 1H), 7.12(d, 1H, J=9.03 Hz), 6.98(m, 3H), 6.58(d, 1H, J=8.49 Hz), 4.87(s, 2H), 4.63(s, 2H), 4.30(s, 2H), 2.22(s, 3H). TLC(5% MeOH/ $CH_2Cl_2$) $R_f$ = 0.17

**(2-Methyl-4-{[(4-{[3-(5-methyl-1,2,4-oxadiazol-3-yl)phenoxy]methyl}-2-phenyl-1,3-thiazol-5-yl)methyl]sulfanyl} phenoxy)acetic acid**

$^1H$ NMR ($CDCl_3$) 400MHz δ 7.91 (m, 2H), 7.95(d, 1H, J=7.69 Hz), 7.47(m, 1H), 7.42(m, 3H), 7.35(t, 1H, J=7.95 Hz), 7.13(dd, 1H, J=8.37, 2.39 Hz), 7.04(s, 2H), 6.60(d, 1 H, J=8.37 Hz), 4.67(s, 2H), 4.57(s, 2H), 4.20(s, 2H), 2.69(s, 3H), 2.12(s, 3H),

MS($ES^+$) M+H= 560.30

**2-{2-Methyl-4-[({4-{[4-(5-methyl-1,2,4-oxadiazol-3-yl)phenoxy]methyl}-2-[4-(trifluoromethyl)phenyl]-1,3-thiazol-5-yl}methyl)sulfanyl]phenoxy}propanoic acid**

$^1H$ ($CDCl_3$) 400MHz δ 7.94(m, 4H), 7.66(d, 2H, J=8.20 Hz), 7.18(d, 1H, J=2.22 Hz), 7.03(dd, 1H, J=8.20, 2.22 Hz), 6.94(d, 2H, J=8.89 Hz), 6.53(d, 1H, J=8.20 Hz), 4.85(d, 1H, J .79 Hz), 4.80(d, 1H, J .79 Hz), 4.69(q, 1H, J=6.84 Hz), 4.26(d, 1H, J .70 Hz), 4.21 (d, 1H, J .70 Hz), 2.63(m, 3H), 2.18(s, 3H), 1.62(d, 3H, J=6.84 Hz),

MS($ES^-$) M-H= 640.00

**{2-Ethyl-4-[({4-{[4-(4-fluorophenyl)-1-piperazinyl]methyl}-2-[4-(trifluoromethyl)phenyl]-1,3-thiazol-5-yl}methyl) sulfanyl]phenoxy}acetic acid**

$^1H$ NMR ($CDCl_3$) 400MHz δ 7.98(d, 2H, J=8.06 Hz), 7.67(d, 2H, J=8.06 Hz), 7.11(dd, 1H, J=8.61, 2.20 Hz), 7.02(d, 1H, J=2.20 Hz), 6.93(m, 2H), 6.82(m, 2H), 6.68(d, 1H, J=8.61 Hz), 4.62(s, 2H), 4.12(s, 2H), 3.44(s, 2H), 3.25 (m, 4H), 3.02(br s, 4H), 2.58(q, 2H, J=7.51 Hz), 1.10(t, 3H, J=7.51 Hz),

MS($ES^-$) M-H= 644.5

**{4-[({4-[(4-Acetyl-1-piperazinyl)methyl]-2-[4-(trifluoromethyl)phenyl]-1,3-thiazol-5-yl}methyl)sulfanyl]-2-ethylphenoxy}acetic acid**

$^1H$ NMR ($CDCl_3$) 300MHz δ 8.04(d, 2H, J=8.28 Hz), 7.75(d, 2H, J=8.28 Hz), 7.22(dd, 1 H, J=8.55, 2 21 Hz),

7.03(s, 1 H), 6.74(d, 1H, J=8.55 Hz), 4.74(s, 2H), 4.13(s, 2H), 3.76(br s, 4H), 3.36(s, 2H), 2.99(br s, 2H), 2.72(br s, 2H), 2.61 (q, 2H, J=7.45 Hz), 2.09(s, 3H), 1.12(t, 3H, J=7.45 Hz),

MS($ES^+$) M+H= 594.1

**{4-[({4-{[4-(4-Acetylphenyl)-1-piperazinyl]methyl}-2-[4-(trifluoromethyl)phenyl]-1,3-thiazol-5-yl}methyl) sulfanyl]-2-ethylphenoxy}acetic acid**

$^1H$ NMR ($CDCl_3$) 400MHz δ 7.95(d, 2H, J=8.42 Hz), 7.84(d, 2H, J=8.97 Hz), 7.67(d, 2H, J=8.42 Hz), 7.14(dd, 1H, J=8.42, 2.20 Hz), 6.95(s, 1H), 6.80(d, 2H, J=8.97 Hz), 6.70(d, 1H, J=8.42 Hz), 4.66(s, 2H), 4.08(s, 2H), 3.54(br s, 4H), 3.38(s, 2H), 3.06(br s, 4H), 2.56(q, 2H, J=7.60 Hz), 2.49(s, 3H), 1.08(t, 3H, J=7.60 Hz),

**{2-Ethyl-4-[({4-{[4-(4-methoxyphenyl)-1-piperazinyl]methyl}-2-[4-(trifluoromethyl)phenyl]-1,3-thiazol-5-yl} methyl)sulfanyl]phenoxy}acetic acid**

$^1H$ NMR ($CDCl_3$) 400MHz δ 7.97(d, 2H, J=8.28 Hz), 7.68(d, 2H, J=8.28 Hz), 7.15(dd, 1H, J=8.45, 2.24 Hz), 6.94(d, 1H, J=2.24 Hz), 6.88(d, 2H, J=9.14 Hz), 6.79(d, 2H, J=9.14 Hz), 6.72(d, 1H, J=8.45 Hz), 4.66(s, 2H). 4.08(s, 2H). 3.72(s, 3H), 3.32(m, 6H), 3.09(br s, 4H), 2.56(q, 2H, J=7.50 Hz), 1.08(t, 3H, J=7.50 Hz),
MS($ES^-$) M-H= 656.2

**2-(4-{[(2-(4-Fluorophenyl)-4-{[4-(phenoxycarbonyl)-1-piperazinyl]methyl}-1,3-thiazol-5-yl)methyl]sulfanyl}-2-methylphenoxy)propanoic acid**

$^1H$ NMR ($CDCl_3$) 400MHz δ 7.91(m, 2H), 7.35(m, 3H), 7.19(m, 3H), 7.12(br s, 1H), 7.07(d, 2H, J=8.79 Hz), 6.67(br s, 1H), 4.58(br s, 1 H), 4.27(s, 2H), 3.59(m, 4H), 3.41 (s, 2H), 2.51 (br s, 4H), 2.19(s, 3H), 1.54(d, 1H, J=6.59 Hz),

MS($ES^-$) M-H= 620.4

**2-(4-{[(2-(4-Fluorophenyl)-4-{(4-(isopropoxycarbonyl)-1-piperazinyl]methyl}-1,3-thiazol-5-yl)methyl]sulfanyl}-2-methylphenoxy)propanoic acid**

$^1H$ NMR ($CDCl_3$) 400MHz δ 7.94(m, 2H}, 7.19(m, 3H), 7.05(br s, 1H), 6.64(d, 1H, J=8.42 Hz), 4.69(br s, 1H), 4.47(br s, 1H), 4.21 (s, 2H), 3.50(br s, 4H), 3.36(s, 2H), 2.64(br s, 4H), 2.18(s, 3H), 1.57(d, 3H, J=5.68 Hz), 1.22(d, 6H, J=6.23 Hz),

MS($ES^-$) M-H= 586.2

**2-[4-({[4-{(4-(Ethoxycarbonyl)-1-piperazinyl]methyl}-2-(4-fluorophenyl)-1,3-thiazol-5-yl]methyl}sulfanyl)-2-methylphenoxy]propanoic acid**

$^1H$ NMR ($CD_3OD$) 400MHz δ 7.93(m, 2H), 7.19(m, 3H), 7.09(br s, 1H), 6.67(br s, 1H), 4.70(br s, 1H), 4.21 (s, 2H), 4.10(q, 2H, J=7.14 Hz), 3.49(m, 4H), 3.37(s, 2H). 2.60(br s, 4H), 2.18(s, 3H), 1.58(br s, 3H), 1.23(t, 3H, J=7.14 Hz),

MS($ES^-$) M-H= 572.2

**2-(4-{[(2-(4-Fluorophenyl)-4-{[4-(3-methoxyphenyl)-1-plperazinyl]methyl}-1,3-thiazol-5-yl)methyl]sulfanyl}-2-methylphenoxy)propanoic acid**

$^1H$ NMR ($CDCl_3$) 400MHz δ 7.84(m, 2H), 7.13(m, 4H), 6.92(br s, 1H), 6.72(br s, 1H), 6.44(m, 3H), 4.38(br s, 1H), 4.00(s, 2H), 3.74(s, 3H), 3.40(m, 6H), 3.03(m, 4H), 2.17(s, 3H), 1.61(m, 3H),

MS($ES^-$) M-H= 606.2

**2-[4-({[4-{[4-(4-Acetylphenyl)-1-piperazinyl]methyl}-2-(4-fluorophenyl)-1,3-thiazol-5-yl]methyl}sulfanyl)-2-methylphenoxy]propanoic acid**

$^1H$ NMR ($CD_3OD$) 400MHz δ 7.94(m, 2H), 7.85(d, 2H, J=8.97 Hz), 7.18(m, 3H), 7.03(br s, 1H), 6.92(d, 2H, J=8.97 Hz), 6.67(br s, 1H). 4.61(br s, 1H), 4.19(s, 2H), 3.41(m, 6H), 2.73(br s, 4H), 2.48(s, 3H), 2.17(s, 3H), 1.61(br s, 3H),

MS($ES^-$) M-H= 618.2

**2-(4-{[(2-(4-Fluorophenyl)-4-{[4-(4-methoxyphenyl)-1-piperazinyl]methyl)-1,3-thiazol-5-yl)methyl]sulfanyl}-2-methylphenoxy)propanoic acid**

$^1$H NMR ($CD_3OD$) 400MHz δ 7.97(m, 2H), 7.18(m, 3H), 7.02(br s, 1H), 6.91(d, 2H, J=8.79 Hz), 6.81 (d, 2H, J=8.79 Hz), 6.62(br s, 1H), 4.66(br s, 1H), 4.17(s, 2H), 3.72(s, 3H), 3.41 (s, 2H), 3.15(br s, 4H), 2.92(br s, 4H), 2.18 (s, 3H), 1.59(br s, 3H),

MS($ES^-$) M-H= 606.2

**{4-[({4-[(4-Acetyl-1-piperazinyl)methyl]-2-[4-(trifluoromethyl)phenyl]-1,3-thiazol-5-yl}methyl)sulfanyl]-2-propylphenoxy}acetic acid**

$^1$H NMR ($CD_3OD$) 400MHz δ 8.07(d, 2H, J=8.28 Hz), 7.75(d, 2H, J=8.28 Hz), 7.21(dd, 1H, J=8.45, 2.41 Hz), 7.09(d, 1H, J=2.41 Hz), 6.74(d, 1H, J=8.45 Hz), 4.65(s, 2H), 4.26(s, 2H), 3.60(br s, 4H), 3.53(s, 2H), 2.75(t, 2H, J=4.74 Hz), 2.69(t, 2H, J=4.74 Hz), 2.52(t, 2H, J=7.41 Hz), 2.07(s, 3H), 1.50(m, 2H), 0.80(t, 3H, J=7.41 Hz),

MS($ES^-$) M-H= 606.3

**{4-[({4-{[4-(3-Methoxyphenyl)-1-piperazinyl]methyl}-2-[4-(trifluoromethyl)phenyl]-1,3-thiazol-5-yl}methyl) sulfanyl]-2-propylphenoxy}acetic acid**

$^1$H NMR ($CD_3OD$) 400MHz δ 8.11(d, 2H, J=8.28 Hz), 7.77(d, 2H, J=8.28 Hz), 7.19(dd, 1H, J=8.28, 2.41 Hz), 7.13(t, 1H, J=8.45 Hz), 7.08(d, 1H, J=2.41 Hz), 6.73(t, 1H, J=8.45 Hz), 6.54(dd, 1H, J=8.28, 2.41 Hz), 6.49(t, 1H, J=2.33 Hz), 6.45(dd, 1H, J=8.28, 2.41 Hz), 4.58(s, 2H), 4.26(s, 2H), 3.73(s, 3H), 3.69(s, 2H), 3.31 (m, 4H), 3.11 (t, 4H, J=4.66 Hz), 2.52(t, 2H, J=7.33 Hz), 1.49(s, 2H), 0.80(t, 3H, J=7.33 Hz),

MS($ES^-$) M-H= 670.3

**{4-[({4-{[4-(4-Acetylphenyl)-1-piperazinyl]methyl}-2-[4-(trifluoromethyl)phenyl]-1,3-thiazol-5-yl}methyl) sulfanyl]-2-propylphenoxy}acetic acid**

$^1$H NMR ($CDCl_3$) 400MHz δ 7.93(d, 2H, J=8.45 Hz), 7.82(d, 2H, J=8.97 Hz), 7.68(d, 2H, J=8.45 Hz), 7.19(dd, 1 H, J=8.45, 2.41 Hz), 6.78(m, 4H), 4.73(s, 2H), 4.03(s, 2H), 3.71 (t, 4H, J=5.09 Hz), 3.28(m, 6H), 2.47(m, 5H), 1.46 (m, 2H), 0.86(t, 3H, J=7.24 Hz),

MS($ES^-$) M-H= 682.1

**{4-[({4-{[4-(4-Methoxyphenyl)-1-piperazinyl]methyl}-2-[4-(trifluoromethyl)phenyl]-1,3-thiazol-5-yl}methyl) sulfanyl]-2-propylphenoxy}acetic acid**

$^1$H NMR ($CD_3OD$) 400MHz δ 8.11 (d, 2H, J=8.10 Hz), 7.77(d, 2H, J=8.10 Hz), 7.19(dd, 1H, J=8.62, 2.24 Hz), 7.08(d, 1H, J=2.24 Hz), 6.93(d, 2H, J=9.14 Hz), 6.82(d, 2H, J=9.14 Hz), 6.74(d, 1H, J=8.62 Hz), 4.59(s, 2H), 4.26(s, 2H), 3.73(s, 2H), 3.71 (s, 3H), 3.18(m, 8H), 2.52(t, 2H, J=7.33 Hz), 1.48(m, 2H), 0.80(t, 3H, J=7.33 Hz),

MS($ES^+$) M+H= 672.2

**2-{2-Ethyl-4-[({4-{[4-(3-methoxyphenyl)-1-piperazinyl]methyl}-2-[4-(trifluoromethyl)phenyl]-1,3-thiazol-5-yl} methyl)sulfanyl]phenoxy}propanoic acid**

$^1$H NMR ($CDCl_3$) 400MHz δ 7.95(d, 2H, J=8.28 Hz), 7.66(d, 2H, J=8.28 Hz), 7.12(m, 2H), 6.90(s, 1H), 6.76 (d, 1H, J=8.28 Hz), 6.45(m, 3H), 4.80(q, 1H, J=6.90 Hz), 4.02(s, 2H), 3.73(s, 3H), 3.35(m, 4H), 3.21 (d, 1H, J .66 Hz), 3.15(d, 1H, J .66 Hz), 2.95(br s, 4H), 2.55(s, 2H), 1.62(d, 3H, J=6.90 Hz), 1.07(t, 3H, J=7.50 Hz),

MS($ES^-$) M-H= 670.0

**2-{4-[({4-{[4-(4-Acetylphenyl)-1-piperazinyl]methyl}-2-[4-(trifluoromethyl)phenyl]-1,3-thiazol-5-yl}methyl) sulfanyl]-2-ethylphenoxy}propanoic acid**

$^1$H NMR ($CDCl_3$) 400MHz δ 7.93(d, 2H, J=8.28 Hz), 7.82(d, 2H, J=8.97 Hz), 7.65(d, 2H, J=8.28 Hz), 7.08(dd, 1H, J=8.62, 2.41 Hz), 6.87(d, 1H, J=2.41 Hz). 6.79(d, 2H, J=8.97 Hz), 6.72(d, 1H, J=8.62 Hz), 4.80(q, 1H, J=6.72 Hz), 4.04(d, 1H, J .66 Hz), 3.98(d, 1H, J .66 Hz), 3.49(br s, 4H), 3.28(d, 1H, J .83 Hz), 3.14(d, 1H, J .83 Hz), 3.00(br s, 4H), 2.54(m, 5H), 1.63(d, 3H, J=6.72 Hz), 1.06(t, 3H, J=7.50 Hz),

MS($ES^-$) M-H=682.2

**2-{2-Ethyl-4-[({4-{[4-(4-methoxyphenyl)-1-piperazinyl]methyl}-2-[4-(trifluoromethyl)phenyl]-1,3-thiazol-5-yl}methyl)sulfanyl]phenoxy}propanoic acid**

$^1H$ NMR ($CDCl_3$) 400MHz δ 7.97(d, 2H, J=8.45 Hz), 7.66(d. 2H, J=8.45 Hz), 7.10(dd, 1H, J=8.45, 2.24 Hz), 6.94(d, 1H, J=2.24 Hz), 6.89(d, 2H, J=9.14 Hz), 6.80(d, 2H, J=9.14 Hz), 6.75(d, 1H, J=8.45 Hz), 4.77(q, 1 H, J=6.72 Hz), 4.04(s, 2H), 3.73(s, 3H), 3.25(m, 6H), 2.96(br s, 4H), 2.57(s, 2H), 1.61(d, 3H, J=6.72 Hz), 1.09(t, 3H, J=7.50 Hz),

MS($ES^-$) M-H= 670.3

**2-{2-Ethyl-4-[({4-(4-morpholinylmethyl)-2-[4-(trifluoromethyl)phenyl]-1,3-thiazol-5-yl}methyl)sulfanyl]phenoxy}propanoic acid**

$^1H$ NMR ($CDCl_3$) 400MHz δ 8.01(d, 2H, J=8.42 Hz), 7.70(d, 2H, J=8.42 Hz), 7.13(dd, 1H, J=8.42, 2.20 Hz), 6.86(s, 1H), 6.76(d, 1H, J=8.42 Hz), 4.84(q, 1H. J=6.65 Hz), 4.04(d, 1H, J .47 Hz), 3.98(d, 1H, J .47 Hz), 3.87(br s, 4H), 3.21(d, 1H, J .83 Hz), 3.08(d, 1H, J .83 Hz), 2.95(br s, 4H), 2.55(s, 2H), 1.64(d, 3H, J=6.65 Hz), 1.07(t, 3H, J=7.51 Hz),

MS($ES^-$) M-H=565.0

**2-{2-Ethyl-4-[({4-{[4-(4-fluorophenyl)-1-piperazinyl]methyl}-2-[4-(trifluoromethyl)phenyl]-1,3-thiazol-5-yl}methyl)sulfanyl]phenoxy}propanoic acid**

$^1H$ NMR ($CD_3OD$) 400MHz δ 8.12(d, 2H, J=8.24 Hz), 7.78(d, 2H, J=8.24 Hz), 7.17(dd, 1H, J=8.61, 2.20 Hz), 7.10(d, 1H, J=2.20 Hz), 6.98(m, 4H), 6.71 (d, 1H, J=8.61 Hz), 4.71 (q, 1H, J=6.90 Hz), 4.27(s, 2H), 3.66(s, 2H), 3.20 (m, 8H), 2.59(q, 2H, J=7.51 Hz), 1.57(d, 3H, J=6.90 Hz), 1.09(t, 3H, J=7.51 Hz),

MS($ES^-$) M-H= 658.0

**2-{4-[({4-[(4-Acetyl-1-piperazinyl)methyl]-2-[4-(trifluoromethyl)phenyl]-1,3-thiazol-5-yl}methyl)sulfanyl]-2-ethylphenoxy}propanoic acid**

$^1H$ NMR ($CD_3OD$) 400MHz δ 8.10(d, 2H, J=8.24 Hz), 7.77(d, 2H, J=824 Hz), 7.19(t, 1H, J=2.38 Hz), 7.09(d, 1H, J=2.38 Hz), 6.71 (d, 1H, J=8.24 Hz), 4.80(q, 1H, J=6.78 Hz), 4.26(s, 2H), 3.65(m, 6H), 3.56(d, 1H, J .92 Hz), 3.51 (d, 1H, J .92 Hz), 2.83(m, 4H), 2.58(q, 2H, J=7.60 Hz), 2.09(s, 3H), 1.60(d, 3H, J=6.78 Hz), 1.09(t, 3H, J=7.60 Hz),

MS($ES^-$) M-H= 606.0

**{2-Ethyl-4-[({4-(4-morpholinylmethyl)-2-[4-(trifluoromethyl)phenyl]-1,3-thiazol-5-yl}methyl)sulfanyl]phenoxy}acetic acid**

$^1H$ NMR ($CDCl_3$) 300MHz δ 8.05(d, 2H, J=8.28 Hz), 7.75(d, 2H, J=8.28 Hz), 7.19(dd, 1 H, J=8.55, 2.21 Hz), 6.98(s, 1 H), 6.76(d, 1 H, J=8.55 Hz), 4.74(s, 2H), 4.12(s, 2H), 3.95(br s, 4H), 3.32(s, 2H), 3.06(br s, 4H), 2.61 (q, 2H, J=7.54 Hz), 1.14(t, 3H, J=7.54 Hz),

MS($ES^-$) M-H= 551.3

**2-{2-Isopropyl-4-[({4-(4-morpholinylmethyl)-2-[4-(trifluoromethyl)phenyl]-1,3-thiazol-5-yl}methyl)sulfanyl]phenoxy}propanoic acid**

$^1H$ NMR ($CD_3OD$) 400MHz δ 8.11(d, 2H, J=8.24 Hz), 7.78(d, 2H, J=8.24 Hz), 7.25(dd, 1H, J=8.42, 2.38 Hz), 7.00(d, 1H, J=2.38 Hz), 6.74(d, 1H, J=8.42 Hz), 4.88(q, 1H, J=6.78 Hz), 4.25(s, 2H), 3.84(m, 5H), 3.66(d, 1H, J .28 Hz), 3.22(m, 5H), 1.60(d, 3H, J=6.78 Hz), 1.05(m, 6H),

MS($ES^-$) M-H= 579.0

**2-{4-[({4-{[4-(4-Fluorophenyl)-1-piperazinyl]methyl}-2-[4-(trifluoromethyl)phenyl]-1,3-thiazol-5-yl}methyl)sulfanyl]-2-isopropylphenoxy}propanoic acid**

$^1H$ NMR ($CD_3OD$) 400MHz δ 8.13(d, 2H, J=8.06 Hz), 7.79(d, 2H, J=8.06 Hz), 7.26(d, 1H, J=8.42 Hz), 7.06 (s, 1H), 6.99(m, 4H), 6.75(d, 1H, J=8.42 Hz), 4.88(q, 1H, J=6.78 Hz), 4.29(s, 2H), 3.91 (d, 1H, J .10 Hz), 3.80(d, 1H, J .10 Hz), 3.33(m, 9H), 1.60(d, 3H, J=6.78 Hz), 1.08(m, 6H),

MS($ES^-$) M-H= 672.0

**2-{4-[({4-[(4-Acetyl-1-piperazinyl)methyl]-2-[4-(trifluoromethyl)phenyl]-1,3-thiazol-5-yl}methyl)sulfanyl]-2-isopropylphenoxy}propanoic acid**

MS(ES$^-$) M-H= 620.0

**2-{2-Isopropyl-4-[({4-{[4-(3-methoxyphenyl)-1-piperazinyl]methyl}-2-[4-(trifluoromethyl)phenyl]-1,3-thiazol-5-yl}methyl)sulfanyl]phenoxy}propanoic acid**

$^1$H NMR ($CD_3OD$) 400MHz δ 8.13(d, 2H, J=8.06 Hz), 7.79(d, 2H, J=8.06 Hz), 7.26(d, 1H, J=8.42 Hz), 7.16 (t, 1H, J=8.42 Hz), 7.06(s, 1H), 6.74(d, 1H, J=8.42 Hz), 6.56(d, 1H, J=8.42 Hz), 6.50(br s, 2H), 4.90(q, 1H, J=6.78 Hz), 4.27(s, 2H), 3.89(d, 1H, J .10 Hz), 3.79(d, 1H, J .10 Hz), 3.74(s, 3H), 3.34(m, 9H), 1.60(d, 3H, J=6.78 Hz), 1.07(m, 6H),
**[0699]** MS(ES$^-$) M-H= 684.1

**2-{4-[({4-{[4-(4-Acetylphenyl)-1-piperazinyl]methyl}-2-[4-(trifluoromethyl)phenyl]-1,3-thiazol-5-yl}methyl) sulfanyl]-2-isopropylphenoxy}propanoic acid**

$^1$H NMR ($CD_3OD$) 400MHz δ 8.13(d, 2H, J=8.24 Hz), 7.91(d, 2H, J=8.97 Hz), 7.78(d, 2H, J=8.24 Hz), 7.25 (d, 1H, J=8.97 Hz), 7.04(m, 3H), 6.74(d, 1H, J=8.24 Hz), 4.89(q, 1H, J=6.78Hz), 4.28(s, 2H), 3.90(d, 1H, J .55 Hz), 3.79(d, 1H, J .55 Hz), 3.60(br s, 4H), 3.32(m, 5H), 2.50(s, 3H), 1.61(d, 3H, J=6.78 Hz), 1.07(d, 6H, J=7.51 Hz),
**[0701]** MS(ES$^-$) M-H= 696.2

**2-{2-Isopropyl-4-[({4-{[4-(4-methoxyphenyl)-1-piperazinyl]methyl}-2-[4-(trifluoromethyl)phenyl]-1,3-thiazol-5-yl}methyl)sulfanyl]phenoxy}propanoic acid**

$^1$H NMR ($CD_3OD$) 400MHz δ 8.13(d, 2H, J=8.24 Hz), 7.79(d, 2H, J=8.24 Hz), 7.27(d, 1H, J=8.61 Hz), 7.05 (s, 1 H), 6.95(d, 2H, J=8.79 Hz), 6.84(d, 2H, J=8.79 Hz), 6.75(d, 1H, J=8.61 Hz), 4.88(m, 1H) buried under MeOH signal, 4.28(s, 2H), 3.90(d, 1H, J .28 Hz), 3.80(d, 1H, J .28 Hz), 3.71 (s, 3H), 3.56(br s, 4H), 3.28(m, 1 H) buried under MeOH signal, 2.96(br s, 4H), 1.58(d, 3H, J=6.59 Hz), 1.07(m, 6H),
**[0703]** MS(ES$^-$) M-H= 684.1

**{2-Isopropyl-4-[({4-(4-morpholinylmethyl)-2-[4-(trifluoromethyl)phenyl]-1,3-thiazol-5-yl}methyl)sulfanyl] phenoxy}acetic acid**

$^1$H NMR ($CD_3OD$) 400MHz δ 8.12(d, 2H, J=8.06 Hz), 7.79(d, 2H, J=8.06 Hz), 7.27(d, 1H, J=8.42 Hz), 7.04 (s, 1H), 6.80(d, 1H, J=8.42 Hz), 4.76(s, 2H), 4.27(s, 2H), 3.87(m, 6H), 3.22(m, 5H), 1.07(d, 6H, J=6.78 Hz),
**[0705]** MS(ES$^-$) M-H= 565.0

**{4-[({4-{[4-(4-Fluorophenyl)-1-piperazinyl]methyl}-2-[4-(trifluoromethyl)phenyl]-1,3-thiazol-5-yl}methyl) sulfanyl]-2-isopropylphenoxy}acetic acid**

$^1$H NMR ($CD_3OD$) 400MHz δ 8.13(d, 2H, J=8.06 Hz), 7.79(d, 2H, J=8.06 Hz), 7.28(d, 1H, J=8.42 Hz), 7.09 (s, 1 H), 6.98(m, 4H), 6.81(d, 1H, J=8.42 Hz), 4.74(s, 2H), 4.28(s, 2H), 3.89(s, 2H), 3.61 (br s, 4H), 3.29(m, 1 H) buried under MeOH signal, 3.02(br s, 4H), 1.07(d, 6H, J=6.78 Hz),
**[0707]** MS(ES$^-$) M-H= 658.0

**{4-[({4-[(4-Acetyl-1-piperazinyl)methyl]-2-[4-(trifluoromethyl)phenyl]-1,3-thiazol-5-yl}methyl)sulfanyl]-2-isopropylphenoxy}acetic acid**

$^1$H NMR ($CD_3OD$) 400MHz δ 8.13(d, 2H, J=8.06 Hz), 7.79(d, 2H, J=8.06 Hz), 7.28(d, 1H, J=8.42 Hz), 7.03 (br s, 1H), 6.80(d, 1H, J=8.42 Hz), 4.76(s, 2H), 4.27(s, 2H), 3.80(m, 6H), 3.21 (m, 5H), 2.11 (s, 3H), 1.06(d, 6H, J=6.78 Hz),
**[0709]** MS(ES$^-$) M-H= 606.2

**2-{4-[({4-{[4-(4-Methoxyphenyl)-1-piperazinyl]methyl}-2-[4-(trifluoromethyl)phenyl]-1,3-thiazol-5-yl}methyl) sulfanyl]-2-propylphenoxy}propanoic acid**

$^1$H NMR ($CD_3OD$) 400MHz δ 8.12(d, 2H, J=8.28 Hz), 7.79(d, 2H, J=8.28 Hz), 7.23(dd, 1H, J=8.45, 2.24 Hz), 7.09(d, 1H, J=2.24 Hz), 6.95(d, 2H, J=9.14 Hz), 6.84(d, 2H, J=9.14 Hz), 6.71 (d, 1H, J=8.45 Hz), 4.81(q, 1H, J=6.72

Hz), 4.29(s, 2H), 3.98(d, 1H, J .14 Hz), 3.90(d, 1H, J .14 Hz), 3.71(s, 3H), 3.50(br s, 4H), 3.21 (m, 4H), 2.50(t, 2H, J=7.33 Hz), 1.58(d, 3H, J=6.72 Hz), 1.48(m, 2H), 0.79(t, 3H, J=7.33 Hz),

MS($ES^-$) M-H= 684.0

**{4-[({4-(4-Morpholinylmethyl)-2-[4-(trifluoromethyl)phenyl]-1,3-thiazol-5-yl}methyl)sulfanyl]-2-propylphenoxy} acetic acid**

$^1$H NMR ($CD_3OD$) 400MHz δ 8.10(d, 2H, J=8.79 Hz), 7.78(d, 2H, J=8.79 Hz), 7.20(dd, 1H, J=8.42, 2.20 Hz), 7.08(d, 1H, J=2.20 Hz), 6.75(d, 1H, J=8.42 Hz), 4.63(s, 2H), 4.26(s, 2H), 3.79(t, 4H. J=4.21 Hz), 3.64(s, 2H), 2.97(t, 4H, J=4.21 Hz), 2.53(t, 2H, J=7.42 Hz), 1.50(s, 2H), 0.82(t. 3H, J=7.42 Hz),

MS($ES^-$) M-H= 658.0

**{4-[({4-{[4-(4-Fluorophenyl)-1-piperazinyl]methyl}-2-[4-(trifluoromethyl)phenyl]-1,3-thiazol-5-yl}methyl) sulfanyl]-2-propylphenoxy}acetic acid**

$^1$H NMR ($CDCl_3$) 400MHz δ 7.97(d, 2H, J=8.24 Hz), 7.67(d, 2H, J=8.24 Hz), 7.12(dd, 1H, J=8.42, 2.20 Hz), 7.01 (d, 1H, J=2.20 Hz), 6.93(m, 2H), 6.83(m, 2H), 6.69(d, 1H, J=8.42 Hz), 4.62(s, 2H), 4.12(s, 2H), 3.45(s, 2H), 3.26 (t, 4H, J=4.85 Hz), 3.04(t, 4H, J=4.85 Hz), 2.52(t, 2H, J=7.33 Hz), 1.51(s, 2H), 0.83(t, 3H, J=7.33 Hz),

**2-{4-[({4-[(3,5-Dimethyl-1-piperazinyl)methyl]-2-[4-(trifluoromethyl)phenyl]-1,3-thiazol-5-yl}methyl)sulfanyl]-2-methylphenoxy}propanoic acid**

$^1$H NMR ($CDCl_3$) 400MHz δ 8.03(d, 2H, J=8.23 Hz), 7.71(d, 2H, J=8.23 Hz), 7.20(m, 2H), 6.66(d, 1H, J=8.55 Hz), 4.72(q, 1H, J=6.64 Hz), 4.26(d, 1H, J .87 Hz), 4.18(d, 1H, J 8.7 Hz), 3.34(m, 2H), 3.05(m, 2H), 2.71(m, 2H), 2.21 (s, 3H), 1.97(m, 2H), 1.63(d, 3H, J=6.64 Hz), 1.35(m, 6H),

MS($ES^+$) M+H= 580.1

HPLC(C-18 3μm) 1 %MeOH/ 0-99% Acetonitrile/Water (0.1%TFA) 5min run $R_t$=3.98

**2-{4-[({4-{[4-(4-Chlorophenyl)-1-piperazinyl]methyl}-2-[4-(trifluoromethyl)phenyl]-1,3-thiazol-5-yl}methyl) sulfanyl]-2-methylphenoxy}propanoic acid**

$^1$H NMR ($CDCl_3$) 400MHz δ 10.42(s, 1H), 7.92(d, 2H, J=8.20 Hz), 7.64(d, 2H, J=8.20 Hz), 7.15(d, 2H, J=9.06 Hz), 7.01 (d, 1H, J=2.20 Hz), 6.96(d, 1H, J=8.37 Hz), 6.72(d, 2H, J=9.06 Hz), 6.59(d, 1H. J=8.37 Hz), 4.64(q, 1H, J=6.78 Hz), 4.09(s, 2H), 3.58(d, 1H, J.18 Hz), 3.49(d, 1H, J. 18 Hz), 3.26(m, 4H), 3.05(m, 4H), 2.13(s, 3H), 1.56(d, 3H, J=6.78 Hz),

MS($ES^+$) M+H= 662.0

HPLC(C-18 3μm) 1%MeOH/ 0-99% Acetonitrile/Water (0.1%TFA) 5min run $R_t$=4.13

**2-{4-[({4-{[4-(*tert*-Butoxycarbonyl)-1-piperazinyl]methyl}-2-[4-(trifluoromethyl)phenyl]-1,3-thiazol-5-yl}methyl) sulfanyl]-2-methylphenoxy}propanoic acid**

$^1$H NMR ($CDCl_3$) 400MHz δ 10.07(s, 1 H), 7.93(d, 2H, J=8.23 Hz), 7.63(d, 2H, J=8.23 Hz), 7.04(s, 1H), 6.98 (d, 1H, J=8.37 Hz), 6.58(d, 1H, J=8.37 Hz), 4.65(q, 1H, J=6.78 Hz), 4.12(d, 1 H, J .70 Hz), 4.05(d, 1H, J .70 Hz), 3.47 (m, 6H), 2.73(m, 4H), 2.14(s, 3H), 1.57(d, 3H, J=6.78 Hz), 1.38(s, 9H),

MS($ES^+$) M+H= 652.0

HPLC(C-18 3μm) 1%MeOH/ 0-99% Acetonitrile/Water (0.1 %TFA) 5min run $R_t$= 4.16

**2-{2-Methyl-4-[({4-(1-piperazinylmethyl)-2-[4-(trifluoromethyl)phenyl]-1,3-thiazol-5-yl}methyl)sulfanyl] phenoxy}propanoic acid**

$^1$H NMR ($CDCl_3$) 400MHz δ 9.26(br s, 1 H), 7.97(br s, 2H), 7.63(br s, 2H), 7.10(br s, 2H), 6.67(br s, 1H), 4.56 (br s, 1H), 4.11 (br s, 2H), 3.39(br s, 2H), 2.98(br s, 4H), 2.41 (br s, 4H), 2.07(br s, 3H), 1.44(br s, 3H),

MS($ES^+$) M+H= 552

HPLC(C-18 3μm) 1%MeOH/ 0-99% Acetonitrile/Water (0.1%TFA) 5min run $R_t$= 3.80

**{2-Isopropyl-4-[({4-{[4-(3-methoxyphenyl)-1-piperazinyl]methyl}-2-[4-(trifluoromethyl)phenyl]-1,3-thiazol-5-yl}methyl)sulfanyl]phenoxy}acetic acid**

$^{1}H$ NMR ($CD_3OD$) 400MHz δ 8.13(d, 2H, J=8.06 Hz), 7.79(d, 2H, J=8.06 Hz), 7.28(d, 1 H, J=8.24 Hz), 7.15 (m, 1H), 7.09(s, 1H), 6.80(d, 1H, J=8.24 Hz), 6.52(m, 3H), 4.74(s, 2H), 4.28(s, 2H), 3.88(s, 2H), 3.73(m, 3H), 3.48(br s, 4H), 3.29(m, 1 H) buried under MeOH signal, 3.05(s, 4H), 1.06(d, 6H, J=6.59 Hz),

MS($ES^-$) M-H= 670.0

**{4-[({4-{[4-(4-Acetylphenyl)-1-piperazinyl]methyl}-2-[4-(trifluoromethyl)phenyl]-1,3-thiazol-5-yl}methyl)sulfanyl]-2-isopropylphenoxy}acetic acid**

$^{1}H$ NMR ($CD_3OD$) 400MHz δ 8.13(d, 2H, J=7.87 Hz), 7.91(d, 2H, J=8.79 Hz), 7.78(d, 2H, J=7.87 Hz), 7.27 (d, 1H, J=8.24 Hz), 7.09(br s, 1 H), 7.02(d, 2H, J=8.24 Hz), 6.80(d, 1H, J=8.79 Hz), 4.74(s, 2H), 4.29(s, 2H), 3.89(s, 2H), 3.62(br s, 4H), 3.30(m, 5H), 2.51 (s, 3H), 1.07(d, 6H, J=6.78 Hz),

MS($ES^-$) M-H= 682.0

**{2-Isopropyl-4-[({4-{[4-(4-methoxyphenyl)-1-piperazinyl]methyl}-2-[4-(trifluoromethyl)phenyl]-1,3-thiazol-5-yl}methyl)sulfanyl]phenoxy}acetic acid**

$^{1}H$ NMR ($CD_3OD$) 400MHz δ 8.13(d, 2H, J=8.06 Hz), 7.79(d, 2H, J=8.06 Hz), 7.29(d, 1H, J=8.45 Hz), 7.09 (s, 1H), 6.98(d, 2H, J=8.45 Hz), 6.83(m, 3H), 4.73(s, 2H), 4.30(s, 2H), 3.90(s, 3H), 3.35(m, 11 H), 1.07(d, 6H, J=6.59 Hz),

MS($ES^-$) M-H= 670.0

**2-{4-[({4-(4-Morpholinylmethyl)-2-[4-(trifluoromethyl)phenyl]-1,3-thiazol-5-yl}methyl)sulfanyl]-2-propylphenoxy}propanoic acid**

$^{1}H$ NMR ($CD_3OD$) 400MHz δ 8.18(d, 2H, J=8.00 Hz), 7.84(d, 2H, J=8.00 Hz), 7.30(dd, 1H, J=8.55, 2.48 Hz), 7.11(d, 1H, J=2.48 Hz), 6.78(d, 1H, J=8.55 Hz), 4.91 (s, 1H) buried under MeOH signal, 4.33(s, 2H), 3.94(m, 6H), 3.24 (br s, 4H), 2.56(t, 2H, J=7.45 Hz), 1.59(m, 5H), 0.86(t, 3H, J=7.45 Hz),

MS(ES-) M-H= 579.0

**2-{4-[({4-{[4-(4-Fluorophenyl)-1-piperazinyl]methyl}-2-[4-(trifluoromethyl)phenyl]-1,3-thiazol-5-yl}methyl)sulfanyl]-2-propylphenoxy}propanoic acid**

$^{1}H$ NMR ($CD_3OD$) 400MHz δ 8.18(d, 2H, J=8.28 Hz), 7.85(d, 2H, J=8.28 Hz), 7.30(dd, 1H, J=8.55, 2.21 Hz), 7.16(d, 1 H, J=2.21 Hz), 7.06(m. 4H), 6:78(d, 1H, J=8.55 Hz), 4.89(br s, 1 H) hidden under MeOH signal, 4.35(s, 2H), 4.06(d, 1H, J.35 Hz), 3.98(d, 1H, J .35 Hz), 3.68(br s, 4H), 3.08(br s, 4H), 2.56(t, 2H, J=7.45 Hz), 1.57(m, 5H), 0.86(t, 3H, J=7.45 Hz),

MS($ES^-$) M-H= 672.0

**2-[4-[({4-[(4-Acetyl-1-piperazinyl)methyl]-2-[4-(trifluoromethyl)phenyl]-1,3-thiazol-5-yl}methyl)sulfanyl]-2-propyiphenoxy}propanoic acid**

$^{1}H$ NMR ($CD_3OD$) 400MHz δ 8.17(d, 2H. J=8.28 Hz), 7.84(d, 2H, J=8.28 Hz), 7.29(dd, 1 H, J=8.55, 2.21 Hz), 7.10(d, 1H, J=2.21 Hz), 6.77(d, 1H. J=8.55 Hz), 4.93(q, 1H, J=6.78 Hz), 4.32(s, 2H), 3.86(m, 6H), 3.27(m, 4H), 2.56 (m, 2H), 2.18(s, 3H), 1.66(d, 3H, J=6.78 Hz), 1.54(m, 2H), 0.85(t, 3H, J=7.31 Hz),

MS($ES^-$) M-H= 620.0

**2-{4-[({4-{[4-(3-Methoxyphenyl)-1-piperazinyl]methyl}-2-[4-(trifluoromethyl)phenyl]-1,3-thiazol-5-yl}methyl)sulfanyl]-2-propylphenoxy}propanoic acid**

$^{1}H$ NMR ($CD_3OD$) 400MHz δ 8.18(d, 2H, J=8.55 Hz), 7.85(d, 2H, J=8.55 Hz), 7.30(dd, 1H, J=8.55, 2.21 Hz), 7.22(t, 1H, J=8.55 Hz), 7.16(d, 1H, J=2.21 Hz), 6.77(d, 1H, J=8.55 Hz), 6.58(m, 3H), 4.80(m, 1H), 4.34(s, 2H), 4.06 (d, 1H, J .07 Hz), 3.97(d, 1H, J .07 Hz), 3.79(s, 3H), 3.60(br s, 4H), 3.08(br s, 4H), 2.56(t, 2H, J=7.17 Hz), 1.58(m, 5H), 0.85(t, 3H, J=7.17 Hz),

MS($ES^-$) M-H= 684.1

**2-{4-[({4-{[4-{4-Acetylphenyl)-1-piperazinyl]methyl}-2-[4-{trifluoromethyl)phenyl]-1,3-thiazol-5-yl}methyl) sulfanyl]-2-propylphenoxy}propanoic acid**

$^1$H NMR ($CD_3OD$) 400MHz δ 8.12(d, 2H,J=8.28 Hz), 7.91(d, 2H, J=9.14 Hz), 7.78(d, 2H, J=8.28 Hz), 7.22 (dd, 1H. J=8.28, 2.24 Hz), 7.10(d. 1H, J=2.24 Hz), 7.03(d, 2H, J=9.14 Hz), 6.71(d, 1H, J=8.28 Hz), 4.81 (q, 1H, J=6.72 Hz), 4.29(s, 2H), 3.99(d, 1H, J=.14 Hz), 3.91 (d, 1H, J .14 Hz), 3.60(br s, 4H), 3.33(m, 4H), 2.48(m, 5H), 1.59(d, 3H, J=6.72 Hz), 1.48(m, 2H), 0.78(t, 3H, J=7.41 Hz),

MS($ES^-$) M-H= 696.1

**2-{2-Methyl-4-[({4-{[4-(2-pyrimidinyl)-1-piperazinyl]methyl}-2-[4-(trifluoromethyl)phenyl]-1,3-thiazol-5-yl} methyl)sulfanyl]phenoxy}propanoic acid**

$^1$H NMR ($CD_3OD$) 300MHz δ 8.35(d, 2H. J=4.69 Hz), 8.13(d, 2H, J=8.28 Hz), 7.80(d, 2H, J=8.28 Hz), 7.21 (s, 1H), 7.13(d, 1H, J=8.28 Hz), 6.71 (d, 1H, J=8.28 Hz), 6.63(t, 1H, J=4.69 Hz), 4.59(m, 1H), 4.31(s, 2H), 3.86(t, 4H, J=4.69 Hz), 3.50(s. 2H), 2.69(t, 4H, J=4.69 Hz), 2.22(s. 3H), 1.59(d, 3H, J=6.78 Hz),

MS($ES^-$) M-H= 628.5

**2-{4-[({4-{[4-(2,4-Dimethoxyphenyl)-1-piperazinyl]methyl}-2-[4-{trifluoromethyl)phenyl]-1,3-thiazol-5-yl} methyl)sulfanyl]-2-methylphenoxy}propanoic acid**

$^1$H NMR ($CD_3OD$) 300MHz δ 8.17(d, 2H, J=8.00 Hz), 7.80(d, 2H, J=8.00 Hz), 7.20(br s, 1 H), 7.04(br s, 1H), 6.92(d, 1H, J=8.55 Hz), 6.67(br s, 1H), 6.56(m, 1H), 6.48(m, 1H), 4.59(br s, 1H), 4.27(s, 2H), 3.84(s, 3H), 3.78(s, 3H), 3.55(s, 2H), 3.07(m 8H), 2.21 (s, 3H), 1.56(br s, 3H),

MS($ES^-$) M-H= 685.6

**2-{2-Methyl-4-[({4-({4-[2-oxo-2-(1-pyrrolidinyl)ethyl]-1-piperazinyl}methyl)-2-[4-(trifluoromethyl)phenyl]-1,3-thiazol-5-yl}methyl)sulfanyl]phenoxy}propanoic acid**

MS($ES^-$) M-H= 660.7

CHN Analysis 0.3 $H_2O$ (Theoretical %C=53.62, %H=6.05, %N=7.82; Found %C=53.33, %H=6.01, %N=7.95)

**2-[2-Methyl-4-({[2-[4-(trifluoromethyl)phenyl]-4-({4-[3-(trifluoromethyl)phenyl]-1-piperazinyl}methyl)-1,3-thiazol-5-yl]methyl}sulfanyl)phenoxy]propanoic acid**

$^1$H NMR ($CD_3OD$) 300MHz δ 8.09(d, 2H, J=8.28 Hz), 7.77(d, 2H, J=8.28 Hz), 7.40(s, 1H), 7.19(m, 4H), 7.07 (d, 1H, J=7.73 Hz), 6.71(d, 1H, J=8.28 Hz), 4.47(m, 1H), 4.33(s, 2H), 3.53(s, 2H), 3.23(m, 4H), 2.64(m, 4H), 2.22(s, 3H), 1.57(d, 3H, J=6.78 Hz),

MS($ES^-$) M-H= 694.5

**2-{4-[({4-{[4-(2-Methoxyphenyl)-1-piperazinyl]methyl}-2-[4-(trifluoromethyl)phenyl]-1,3-thiazol-5-yl}methyl) sulfanyl]-2-methylphenoxy}propanoic acid**

$^1$H NMR ($CD_3OD$) 300MHz δ 8.09(d, 2H, J=8.28 Hz), 7.77(d, 2H, J=8.28 Hz), 7.25(s, 1H), 7.17(s, 1H), 6.96 (m, 4H), 6.70(s, 1H), 4.51 (m, 1H), 4.34(s, 2H), 3.86(s, 3H), 3.57(s, 2H), 3.07(br s, 4H), 2.76(br s, 4H), 2.23(br s, 3H), 1.54(br s, 3H),

MS($ES^-$) M-H= 656.5

**2-{4-[({4-[(4-Acetyl-1-piperazinyl)methyl]-2-[4-(trifluoromethyl)phenyl]-1,3-thiazol-5-yl}methyl)sulfanyl]-2-methylphenoxy}propanoic acid**

$^1$H NMR ($CDCl_3$) 400MHz δ 7.93(d, 2H, J=8.20 Hz), 7.63(d, 2H, J=8.20 Hz), 7.02(m, 2H), 6.57(d, 1H, J=8.20 Hz), 4.65(q, 1H, J=6.78 Hz), 4.16(d, 1H, J .87 Hz), 4.09(d, 1H, J .87 Hz), 3.55(m, 6H), 2.74(m, 4H), 2.11(s, 3H), 1.98 (s, 3H), 1.55(d, 3H, J=6.78 Hz),

MS($ES^+$) M+H= 594.0

HPLC(C-18 3μm) 1%MeOH/ 0-99% Acetonitrile/Water (0.1%TFA) 5min run $R_t$= 3.79

**2-{2-Methyl-4-[({4-{[4-(4-pyridinyl)-1-piperazinyl]methyl}-2-[4-(trifluoromethyl)phenyl]-1,3-thiazol-5-yl}methyl) sulfanyl]phenoxy}propanoic acid**

$^1$H NMR ($CD_3OD$) 400MHz δ 8.01(d, 2H, J=8.20 Hz), 7.95(d, 2H, J=8.20 Hz), 7.64(d, 2H, J=8.20 Hz), 7.16 (d, 1H, J=2.22 Hz), 7.09(dd, 1H, J=8.37, 2.22 Hz), 6.97(d, 2H, J=8.20 Hz), 6.63(d, 1H, J=8.37 Hz), 4.48(q, 1H, J=6.78 Hz), 4.19(s, 2H), 3.57(t, 4H, J=5.10Hz), 3.48(s, 2H), 2.46(t, 4H, J=5.10 Hz), 2.14(s, 3H), 1.54(d, 3H, J=6.78 Hz),

MS($ES^+$) M+H= 629.0

HPLC(C-18 3μm) 1%MeOH/ 0-99% Acetonitrile/Water (0.1%TFA) 5min run $R_t$= 4.22

**2-{4-[({4-{[4-(3-Methoxyphenyl)-1-piperazinyl]methyl}-2-[4-(trifluoromethyl)phenyl]-1,3-thiazol-5-yl}methyl) sulfanyl]-2-methylphenoxy}propanoic acid**

$^1$H NMR ($CDCl_3$) 400MHz δ 10.57(s, 1H), 7.91 (d, 2H, J=8.20 Hz), 7.63(d, 2H, J=8.20 Hz), 7.11(t, 1H, J=8.20 Hz), 6.98(m, 2H), 6.60(d, 1H, J=8.20 Hz), 6.41 (dd, 2H, J=8.20, 2.22 Hz), 6.35(t, 1H, J=2.22 Hz), 4.65(q, 1H, J=6.84 Hz), 4.10(s, 2H), 3.72(s, 3H), 3.59(d, 1H, J .18 Hz), 3.49(d, 1H, J .18 Hz), 3.35(m, 4H), 3.10(m, 4H), 2.12(s, 3H), 1.55 (d, 3H, J=6.84 Hz),

MS($ES^+$) M+H= 658.0

HPLC(C-18 3μm) 1%MeOH/ 0-99% Acetonitrile/Water (0.1%TFA) 5min run $R_t$= 4.09

**2-{2-Methyl-4-[({4-(4-morpholinylmethyl)-2-[4-(trifluoromethyl)phenyl]-1,3-thiazol-5-yl}methyl)sulfanyl] phenoxy}propanoic acid**

$^1$H NMR ($CDCl_3$) 400MHz δ 11.61 (s, 1 H), 8.00(d, 2H, J=8.23 Hz), 7.69(d, 2H, J=8.23 Hz), 7.10(dd, 1H, J=8.37, 2.20 Hz), 6.83(d, 1H, J=2.20 Hz), 6.71 (d, 1H, J=8.37 Hz), 4.84(q, 1H, J=6.72 Hz), 4.12(m, 4H), 3.84(m, 2H), 3.43(m, 3H), 3.19(m, 2H), 2.88(m, 1H), 2.10(s, 3H), 1.61(d, 3H, J=6.72 Hz),

MS($ES^+$) M+H= 553.0

HPLC(C-18 3μm) 1%MeOH/ 0-99% Acetonitrile/Water (0.1%TFA) 5min run $R_t$= 3.89

**2-{4-[({4-{[4-(Ethoxycarbonyl)-1-piperazinyl]methyl}-2-[4-(trifluoromethyl)phenyl]-1,3-thiazol-5-yl}methyl) sulfanyl]-2-methylphenoxy}propanoic acid**

$^1$H NMR ($CDCl_3$) 400MHz δ 10.39(s, 1H), 7.93(d, 2H, J=8.23 Hz), 7.64(d, 2H, J=8.23 Hz), 7.05(d, 1H, J=2.39 Hz), 6.97(d, 1H, J=8.37 Hz), 6.57(d, 1H, J=8.37 Hz), 4.65(q, 1H, J=6.78 Hz), 4.09(q, 4H, J=7.06 Hz), 3.58(m, 4H), 3.39 (m, 2H), 2.74(m, 4H), 2.14(s, 3H), 1.57(d, 3H, J=6.78 Hz), 1.21(t, 3H, J=7.06 Hz),

MS($ES^+$) M+H= 624.0

HPLC(C-18 3μm) 1%MeOH/ 0-99% Acetonitrile/Water (0.1%TFA) 5min run $R_t$= 3.93

**2-{4-[({4-{[4-(4-Acetylphenyl)-1-piperazinyl]methyl}-2-[4-(trifluoromethyl)phenyl]-1,3-thiazol-5-yl}methyl) sulfanyl]-2-methylphenoxy}propanoic acid**

$^1$H NMR ($CDCl_3$) 400MHz δ 9.84(s, 1H), 7.91 (d, 2H, J=8.20 Hz), 7.81 (d, 2H, J=8.89 Hz), 7.63(d, 2H, J=8.20 Hz), 7.00(d, 1H, J=2.20 Hz), 6.93(dd, 1H, J=8.37, 2.20 Hz), 6.76(d, 2H, J=8.89 Hz), 6.58(d, 1H, J=8.37 Hz), 4.66(q, 1H, J=6.78 Hz), 4.08(s, 2H), 3.45(m, 6H), 2.96(m, 4H), 2.47(s, 3H), 2.13(s, 3H), 1.59(d, 3H, J=6.78 Hz),

MS($ES^+$) M+H= 670.0

HPLC(C-18 3μm) 1%MeOH/ 0-99% Acetonitrile/Water (0.1%TFA) 5min run $R_t$= 4.03

**2-{4-[({4-{[4-(4-Fluorophenyl)-1-piperazinyl]methyl}-2-[4-(trifluoromethyl)phenyl]-1,3-thiazol-5-yl}methyl) sulfanyl]-2-methylphenoxy}propanoic acid**

$^1$H NMR ($CDCl_3$) 400MHz δ 7.92(d, 2H, J=8.23 Hz), 7.62(d, 2H, J=8.23 Hz), 7.05(s, 1H), 6.89(m, 2H), 6.75 (m, 2H), 6.55(d, 1H, J=8.23 Hz), 4.59(m, 1H), 4.17(m, 2H), 3.53(m, 2H), 3.21(m, 4H), 2.97(m, 4H), 2.12(s, 3H), 1.51 (d, 3H, J=6.78 Hz),

MS($ES^+$) M+H= 646.0

HPLC(C-18 3μm) 1 %MeOH/ 0-99% Acetonitrile/Water (0.1 %TFA) 5min run $R_t$= 4.11

**2-{4-[({4-({4-[(4-Fluorophenyl)sulfonyl]-1-piperazinyl}methyl)-2-[4-(trifluoromethyl)phenyl]-1,3-thiazol-5-yl}methyl)sulfanyl]-2-methylphenoxy}propanoic acid**

$^1$H NMR ($CD_3OD$) 400MHz δ 8.03(d, 2H, J=8.20 Hz), 7.83(t, 2H, J=7.69 Hz), 7.73(d, 2H, J=8.20 Hz), 7.33(t, 2H, J=7.69 Hz), 7.17(s, 1H), 7.08(d, 1H, J=8.20 Hz), 6.64(d, 1H, J=8.20 Hz), 4.67(br s, 1H), 4.22(s, 2H), 3.37(s, 2H), 2.99(br s, 4H), 2.50(br s, 4H), 2.16(s, 3H), 1.57(d, 3H, J=6.84 Hz),

MS($ES^-$) M-H= 708.0

**2-{2-Methyl-4-[({4-{[4-(3-methylbutanoyl)-1-piperazinyl]methyl}-2-[4-(trifluoromethyl)phenyl]-1,3-thiazol-5-yl}methyl)sulfanyl]phenoxy}propanoic acid**

$^1$H NMR ($CD_3OD$) 400MHz δ 8.12(d, 2H, J=8.28 Hz), 7.80(d, 2H, J=8.28 Hz), 7.23(d, 1H, J=2.21 Hz), 7.17 (dd, 1H, J=8.28, 2.21 Hz), 6.72(d, 1H, J=8.28 Hz), 4.72(q, 1 H, J=6.44 Hz), 4.32(s, 2H), 3.63(br s, 4H), 3.44(s, 2H), 2.59(br s, 4H), 2.31 (d, 2H, J=6.90 Hz), 2.21 (s, 3H), 2.06(m, 1 H), 1.62(d, 3H, J=6.44 Hz), 0.98(d, 6H, J=6.90 Hz),

MS($ES^-$) M-H= 634.0

**2-{4-[({4-{[4-(Cyclohexylcarbonyl)-1-piperazinyl]methyl}-2-[4-(trifluoromethyl)phenyl]-1,3-thiazol-5-yl}methyl)sulfanyl]-2-methylphenoxy}propanoic acid**

$^1$H NMR ($CD_3OD$) 400MHz δ 8.06(d, 2H, J=8.10 Hz), 7.74(d, 2H, J=8.10 Hz), 7.16(d, 1H, J=2.24 Hz), 7.09 (dd, 1H, J=8.45, 2.24 Hz), 6.64(d, 1H, J=8.45 Hz), 4.68(q, 1H, J=6.78 Hz), 4.25(s, 2H), 3.60(br s, 4H), 3.42(s, 2H), 2.62(br s, 4H), 2.16(s, 3H), 1.72(m, 5H), 1.56(d, 3H, J=6.72 Hz), 1.31 (m, 6H),

MS($ES^-$) M-H= 661.0

**2-{2-Methyl-4-[({4-{[4-(2-pyrazinyl)-1-piperazinyl]methyl}-2-[4-(trifluoromethyl)phenyl]-1,3-thiazol-5-yl}methyl)sulfanyl]phenoxy}propanoic acid**

$^1$H NMR ($CD_3OD$) 300MHz δ

8.17(m, 4H), 7.81 (m, 3H), 7.26(br s, 1 H), 7.13(br s, 1H), 6.75(br s, 1H), 4.68(br s, 1H), 4.32(s, 2H), 3.65(br s, 4H), 3.48(s, 2H), 2.64(br s, 4H), 2.20(s, 3H), 1.60(br s, 3H),

MS($ES^-$) M-H= 628.3

**2-{4-[({4-({4-[4-(dimethylamino)benzoyl]-1-piperazinyl}methyl)-2-[4-(trifluoromethyl)phenyl]-1,3-thiazol-5-yl}methyl)sulfanyl]-2-methylphenoxy}propanoic acid**

$^1$H NMR ($CD_3OD$) 400MHz δ,

8.12(d, 2H, J=8.28 Hz), 7.80(d, 2H, J=8.28 Hz), 7.35(d, 2H, J=9.11 Hz), 7.21 (d, 1 H, J=2.21 Hz), 7.14(d, 1 H, J=8.55 Hz), 6.78(d, 2H, J=9.11 Hz), 6.70(d, 1H, J=8.55 Hz), 4.68(q, 1H, J=6.62 Hz), 4.31(s, 2H), 3.70(br s, 4H), 3.45(s, 2H), 3.02(s, 6H), 2.63(br s, 4H), 2.19(s, 3H), 1.59(d, 3H, J=6.62 Hz),

MS($ES^-$) M-H= 697.0

**2-{4-[({4-{[4-(2-Furoyl)-1-piperazinyl]methyl}-2-[4-(trifluoromethyl)phenyl]-1,3-thiazol-5-yl}methyl)sulfanyl]-2-methylphenoxy}propanoic acid**

$^1$H NMR ($CD_3OD$) 400MHz δ 8.06(d, 2H, J=8.28 Hz), 7.73(d, 2H, J=8.23 Hz), 7.65(m, 1H), 7.16(d, 1H, J=2.20 Hz), 7.07(d, 1H, J=8.55 Hz), 7.01 (d, 1H, J=3.62 Hz), 6.63(d, 1H, J=8.45 Hz), 6.55(m, 1H), 4.66(q, 1 H, J=6.55 Hz), 4.25(s, 2H), 3.77(br s, 4H), 3.39(s, 2H), 2.59(br s, 4H), 2.14(s, 3H), 1.54(d, 3H, J=6.55 Hz),

MS($ES^-$) M-H= 644.1

**2-{4-[({4-{[4-(Cyclopentylcarbonyl)-1-piperazinyl]methyl}-2-[4-(trifluoromethyl)phenyl]-1,3-thiazol-5-yl}methyl)sulfanyl]-2-methylphenoxy}propanoic acid**

$^1$H NMR ($CD_3OD$) 400MHz δ 8.13(d, 2H, J=8.28 Hz), 7.80(d, 2H, J=8.28 Hz), 7.23(d, 1H, J=2.39 Hz), 7.15 (d, 1H, J=8.28 Hz), 6.71 (br s, 1H), 4.73(q, 1H, J=6.78 Hz), 4.31 (s, 2H), 3.67(br s, 4H), 3.45(s, 2H), 3.06(m, 1 H), 2.62 (br s, 4H), 2.22(s, 3H), 1.75(m, 14H),

MS($ES^-$) M-H= 646.2

**2-{4-[({4-{[4-(Cyclobutylcarbonyl)-1-piperazinyl]methyl}-2-[4-(trifluoromethyl)phenyl]-1,3-thiazol-5-yl}methyl) sulfanyl]-2-methylphenoxy}propanoic acid**

$^1$H NMR ($CD_3OD$) 400MHz δ 8.09(d, 2H, J=8.20 Hz), 7.77(d, 2H, J=8.20 Hz), 7.18(d, 1 H, J=2.22 Hz), 7.13 (dd, 1H, J=8.55, 2.22 Hz), 6.68(d, 1H, J=8.55 Hz), 4.71 (q, 1H, J=6.75 Hz), 4.28(s, 2H), 3.60(br s, 2H), 3.46(br s, 2H), 3.41(s, 2H), 2.57(t, 4H, J=4.44 Hz), 2.22(m, 6H), 2.00(m, 2H), 1.83(m, 2H), 1.60(d, 3H, J=6.75 Hz),

MS($ES^-$) M-H= 633.1

**2-{4-[({4-{[4-(Cyclopropylcarbonyl)-1-piperazinyl]methyl}-2-[4-(trifluoromethyl)phenyl]-1,3-thiazol-5-yl} methyl)sulfanyl]-2-methylphenoxy}propanoic acid**

$^1$H NMR ($CD_3OD$) 400MHz δ 8.10(d, 2H, J=8.23 Hz), 7.76(d, 2H, J=8.23 Hz), 7.21(d, 1H, J=2.20 Hz), 7.11 (d, 1 H, J=8.20 Hz), 6.67(s, 1 H), 4.68(q, 1 H, J=6.84 Hz), 4.28(s, 2H), 3.68(br s, 4H), 3.42(s, 2H), 2.59(br s, 4H), 2.19 (s, 3H), 1.95(m, 1H), 1.57(d, 3H, J=6.84 Hz), 0.84(m, 4H),

MS($ES^-$) M-H= 619.1

**2-{2-Methyl-4-[({4-{[4-(2-thienylcarbonyl)-1-piperazinyl]methyl}-2-[4-(trifluoromethyl)phenyl]-1,3-thiazol-5-yl} methyl)sulfanyl]phenoxy}propanoic acid**

$^1$H NMR ($CD_3OD$) 300MHz δ 8.10(d, 2H, J=8.20 Hz), 7.76(d, 2H, J=8.20 Hz), 7.63(d, 1H, J=5.13 Hz), 7.37 (d, 1H, J=5.13 Hz), 7.22(br s, 1H), 7.10(br s, 1 H), 7.02(br s, 1H), 6.64(br s, 1H), 4.67(br s, 1H), 4.27(s, 2H), 3.74(br s, 4H), 3.40(s, 2H), 2.53(br s, 4H), 2.16(br s, 3H), 1.57(br s, 3H),

MS($ES^-$) M-H= 660.1

**2-{4-[({4-{[4-(2,4-Difluorophenyl)-1-piperazinyl]methyl}-2-[4-(trifluoromethyl)phenyl]-1,3-thiazol-5-yl}methyl) sulfanyl]-2-methylphenoxy}propanoic acid**

$^1$H NMR ($CD_3OD$) 300MHz δ 8.10(d, 2H, J=8.28 Hz), 7.73(d, 2H, J=8.28 Hz), 7.20(br s, 1H), 6.92(m, 4H), 6.60(d, 1H, J=8.55 Hz), 4.59(br s, 1H), 4.23(s, 2H), 3.44(s, 2H), 3.06(br s, 4H), 2.80(br s, 4H), 2.17(s, 3H), 1.53(d, 3H, J=6.35 Hz),

MS($ES^-$) M-H= 661.2

**2-[2-Methyl-4-({[2-[4-(trifluoromethyl)phenyl]-4-({4-[4-(trifluoromethyl)phenyl]-1-piperazinyl}methyl)-1,3-thiazol-5-yl]methyl}sulfanyl)phenoxy]propanoic acid**

$^1$H NMR ($CD_3OD$) 300MHz δ 8.11 (d, 2H, J=8.28 Hz), 7.78(d, 2H, J=8.28 Hz), 7.49(d, 2H, J=8.55 Hz), 7.24 (d, 1H, J=2.39 Hz), 7.15(d, 1H, J=8.55 Hz), 7.04(d, 2H, J=8.55 Hz), 6.71 (d, 1H, J=8.55 Hz), 4.55(br s, 1 H), 4.32(s, 2H), 3.51 (s, 2H), 3.31 (m, 4H), 2.68(t, 4H, J=4.97 Hz), 2.22(s, 3H), 1.59(d, 3H, J=6.07 Hz),

MS($ES^-$) M-H= 694.5

**2-{4-[({4-{[4-(Isobutoxycarbonyl)-1-piperazinyl]methyl}-2-[4-(trifluoromethyl)phenyl]-1,3-thiazol-5-yl}methyl) sulfanyl]-2-methylphenoxy}propanoic acid**

$^1$H NMR ($CD_3OD$) 400MHz δ 8.13(d, 2H, J=8.28 Hz), 7.80(d, 2H, J=8.28 Hz), 7.22(d, 1H, J=2.21 Hz), 7.15 (dd, 1H, J=8.28, 2.21 Hz), 6.71(d, 1H, J=8.28 Hz), 4.75(q, 1H, J=6.90 Hz), 4.31(s, 2H), 3.89(d, 2H, J=6.90 Hz), 3.57 (br s, 4H), 2.68(t, 4H, J=4.69 Hz), 2.22(s, 3H), 1.96(m, 1 H), 1.62(d, 3H, J=6.90 Hz), 0.96(d, 6H, J=6.90 Hz),

MS($ES^-$) M-H= 650

**2-{4-[({4-({4-[(Benzyloxy)carbonyl]-1-piperazinyl}methyl)-2-[4-(trifluoromethyl)phenyl]-1,3-thiazol-5-yl}methyl) sulfanyl]-2-methylphenoxy}propanoic acid**

$^1$H NMR ($CD_3OD$) 400MHz δ 8.06(d, 2H, J=8.03 Hz), 7.73(d, 2H, J=8.03 Hz), 7.30(m, 5H), 7.15(br s, 1 H), 7.08(dd, 1 H, J=8.20, 2.22 Hz), 6.64(d, 1H, J=8.20 Hz), 5.08(s, 2H), 4.65(q, 1 H, J=6.72 Hz), 4.23(s, 2H), 3.51 (br s, 4H), 3.37(s, 2H), 2.57(br s, 4H), 2.15(s, 3H), 1.55(d, 3H, J=6.72 Hz),

MS($ES^-$) M-H= 684.0

**2-{4-[({4-{[4-(Methoxycarbonyl)-1-piperazinyl]methyl}-2-[4-(trifluoromethyl)phenyl]-1,3-thiazol-5-yl}methyl) sulfanyl]-2-methylphenoxy}propanoic acid**

$^1H$ NMR ($CD_3OD$) 400MHz δ 8.06(d, 2H, J=8.37 Hz), 7.74(d, 2H, J=8.37 Hz), 7.16(d, 1H, J=2.21 Hz), 7.10 (dd, 1H, J=8.55, 2.39 Hz), 6.66(d, 1 H, J=8.55 Hz), 4.59(br s, 1H), 4.25(s, 2H), 3.65(s, 3H), 3.45(t, 4H, J=4.79 Hz), 3.38(s, 2H), 2.49(br s, 4H), 2.17(s, 3H), 1.55(d, 3H, J=6.32 Hz),

MS($ES^-$) M-H= 608.0

**2-{2-Methyl-4-[({4-{[4-(phenoxycarbonyl)-1-piperazinyl]methyl}-2-[4-(trifluoromethyl)phenyl]-1,3-thiazol-5-yl} methyl)sulfanyl]phenoxy}propanoic acid**

$^1H$ NMR ($CD_3OD$) 400MHz δ 8.08(d, 2H, J=8.20 Hz), 7.75(d, 2H, J=8.20 Hz), 7.34(m, 2H), 7.19(m, 2H), 7.13 (dd, 1H, J=8.20, 2.22 Hz), 7.06(m, 2H), 6.66(d, 1H, J=8.20 Hz), 4.69(q, 1H, J=6.78 Hz), 4.27(s, 2H), 3.69(br s, 2H), 3.54(br s, 2H), 3.43(s, 2H), 2.62(br s, 4H), 2.17(s, 3H), 1.54(d, 3H, J=6.78 Hz),

MS($ES^-$) M-H= 670.0

**2-{2-Methyl-4-[({4-([4-(phenylsulfonyl)-1-piperazinyl]methyl}-2-[4-(trifluoromethyl)phenyl]-1,3-thiazol-5-yl} methyl)sulfanyl]phenoxy}propanoic acid**

$^1H$ NMR ($CD_3OD$) 400MHz δ 8.01(d, 2H, J=8.20 Hz), 7.72(m, 4H), 7.63(d, 1H, J=8.20 Hz), 7.56(M, 2H), 7.13 (d, 1H, J=2.22Hz), 7.05(dd, 1H, J=8.20, 2.22 Hz), 6.62(d, 1H, J=8.20 Hz), 4.70(q, 1H, J=6.61 Hz), 4.19(s, 2H), 3.34 (s, 2H), 2.97(br s, 4H), 2.51 (br s, 4H), 2.13(s, 3H), 1.57(d, 3H, J=6.61 Hz),

MS($ES^-$) M-H= 690.0

**2-{2-Methyl-4-[({2-[4-(trifluoromethyl)phenyl]-4-[(4-{[4-(trifluoromethyl)phenyl]sulfonyl}-1-piperazinyl)methyl]-1,3-thiazol-5-yl}methyl)sulfanyl]phenoxy}propanoic acid**

$^1H$ NMR ($CD_3OD$) 400MHz δ 8.01 (d, 2H, J=8.20 Hz), 7.91 (m, 4H), 7.71 (d, 2H, J=8.20 Hz), 7.15(d, 1H, J=2.22 Hz), 7.08(dd, 1H, J=8.20, 2.22 Hz), 6.62(d, 1H, J=8.20 Hz), 4.71 (q, 1H, J=6.58Hz), 4.20(s, 2H), 3.33(s, 2H), 3.01 (br s, 4H), 2.49(br s, 4H), 2.14(s, 3H), 1.57(d, 3H, J=6.58 Hz),

MS($ES^-$) M-H= 758.0

**2-{4-[({4-({4-[(4-Methoxyphenyl)sulfonyl]-1-piperazinyl}methyl)-2-[4-(trifluoromethyl)phenyl]-1,3-thiazol-5-yl} methyl)sulfanyl]-2-methylphenoxy}propanoic acid**

$^1H$ NMR ($CD_3OD$) 400MHz δ 8.02(d, 2H, J=8.37 Hz), 7.72(d, 2H, J=8.37 Hz), 7.66(d, 2H, J=8.72 Hz), 7.14 (d, 1H, J=2.21 Hz), 7.07(m, 3H), 6.63(d, 1H, J=8.37 Hz), 4.71 (q, 1H, J=6.72 Hz), 4.20(s, 2H), 3.84(s, 3H), 3.35(s, 2H), 2.97(br s, 4H), 2.53(t, 4H, J=4.61 Hz), 2.14(s, 3H), 1.58(d, 3H, J=6.72 Hz),

MS($ES^-$) M-H= 720.0

**2-{2-Methyl-4-[({4-{[4-(propylsulfonyl)-1-piperazinyl]methyl}-2-[4-(trifluoromethyl)phenyl]-1,3-thiazol-5-yl} methyl)sulfanyl]phenoxy}propanoic acid**

$^1H$ NMR ($CD_3OD$) 400MHz δ 8.07(d, 2H, J=8.20 Hz), 7.75(d, 2H, J=8.20 Hz), 7.19(s, 1H), 7.13(d, 1H, J=8.20 Hz), 6.66(d, 1H, J=8.20 Hz), 4.70(q, 1H, J=6.67 Hz), 4.26(s, 2H), 3.40(s, 2H), 3.22(br s, 4H), 2.95(t, 2H, J=7.43 Hz), 2.54(br s, 4H), 2.17(s, 3H), 1.76(m, 2H), 1.57(d, 3H, J=6.67 Hz), 1.02(t, 3H, J=7.43 Hz),

MS($ES^-$) M-H= 656.0

**2-{4-[({4-{[4-(Ethylsulfonyl)-1-piperazinyl]methyl}-2-[4-(trifluoromethyl)phenyl]-1,3-thiazol-5-yl}methyl) sulfanyl]-2-methylphenoxy}propanoic acid**

$^1H$ NMR ($CD_3OD$) 400MHz δ 8.07(d, 2H, J=8.03 Hz), 7.74(d, 2H, J=8.03 Hz), 7.19(s, 1H), 7.11(d, 1H, J=8.03 Hz), 6.65(d, 1H, J=8.03 Hz), 4.64(q, 1H, J=6.49 Hz), 4.26(s, 2H), 3.39(s, 2H), 3.23(br s, 4H), 2.99(q, 2H, J=7.41 Hz), 2.51 (br s, 4H), 2.16(s, 3H), 1.55(d, 3H, J=6.49 Hz), 1.27(t, 3H, J=7.41 Hz),

MS($ES^-$) M-H= 642.0

**2-{2-Methyl-4-[({4-{[4-(methylsulfonyl)-1-piperazinyl]methyl}-2-[4-(trifluoromethyl)phenyl]-1,3-thiazol-5-yl}methyl)sulfanyl]phenoxy}propanoic acid**

$^1$H NMR ($CD_3OD$) 400MHz δ 8.06(d, 2H, J=8.03 Hz), 7.74(d, 2H, J=8.03 Hz), 7.19(s, 1H), 7.13(dd, 1H, J=8.03, 2.22 Hz), 6.66(d, 1H, J=8.03 Hz), 4.65(q, 1H, J=6.84 Hz), 4.27(s, 2H), 3.40(s, 2H), 3.17(t, 4H, J=4.19 Hz), 2.80(s, 3H), 2.53(t, 4H, J=4.19 Hz), 2.17(s, 3H), 1.56(d, 3H, J=6.84 Hz),
**[0819]** MS($ES^-$) M-H= 628.0

**2-{4-[({4-{[4-(4-Fluorobenzoyl)-1-piperazinyl]methyl}-2-[4-(trifluoromethyl)phenyl]-1,3-thiazol-5-yl}methyl)sulfanyl]-2-methylphenoxy}propanoic acid**

$^1$H NMR ($CD_3OD$) 300MHz δ 8.09(d, 2H, J=8.28 Hz), 7.76(d, 2H, J=8.28 Hz), 7.52(M, 2H), 7.22(M, 3H), 7.13 (dd, 1H, J=8.28, 2.20 Hz), 6.68(d, 1H, J=8.28 Hz), 4.67(q, 1H, J=6.81 Hz), 4.32(s, 2H), 3.79(br s, 4H), 3.66(s, 2H), 2.90(br s, 4H), 2.17(s, 3H), 1.59(d, 3H, J=6.81 Hz),
**[0821]** MS($ES^-$) M-H= 671.9

**2-{4-[({4-[(4-{[4-(Acetylamino)phenyl]sulfonyl}-1-piperazinyl)methyl]-2-[4-(trifluoromethyl)phenyl]-1,3-thiazol-5-yl}methyl)sulfanyl]-2-methylphenoxy}propanoic acid**

$^1$H NMR ($CD_3OD$) 400MHz δ 8.07(d, 2H, J=8.28 Hz), 7.83(d, 2H, J=8.83 Hz), 7.77(d, 2H, J=8.28 Hz), 7.71 (d, 2H, J=8.83 Hz), 7.18(d, 1H, J=2.20 Hz), 7.10(dd, 1H, J=8.28, 2.20 Hz), 6.68(d, 1H, J=8.28 Hz), 4.71 (q, 1H, J=6.53 Hz), 4.26(s, 2H), 3.42(s, 2H), 3.03(br s, 4H), 2.56(t, 4H, J=4.83 Hz), 2.20(m, 6H), 1.63(d, 3H, J=6.53 Hz),
**[0823]** MS($ES^-$) M-H= 747.0

**2-{4-[({4-({4-[(4-Fluoroanilino)carbonyl]-1-piperazinyl}methyl)-2-[4-(trifluoromethyl)phenyl]-1,3-thiazol-5-yl}methyl)sulfanyl]-2-methylphenoxy}propanoic acid**

MS($ES^-$) M-H= 687.5

**2-{4-[({4-{[4-(4-Methoxybenzoyl)-1-piperazinyl]methyl}-2-[4-(trifluoromethyl)phenyl]-1,3-thiazol-5-yl}methyl)sulfanyl]-2-methylphenoxy}propanoic acid**

$^1$H NMR ($CD_3OD$) 300MHz δ 8.02(d, 2H, J=8.20 Hz), 7.69(d, 2H, J=8.20 Hz), 7.38(d, 2H, J=8.79 Hz), 7.12 (d, 1H, J=2.24 Hz), 7.06(dd, 1H, J=8.28, 2.24 Hz), 6.95(d, 2H, J=8.79 Hz), 6.61(d, 1H, J=8.28 Hz), 4.58(q, 1H, J=6.78 Hz), 4.25(s, 2H), 3.78(s, 3H), 3.71(br s, 4H), 3.64(s, 2H), 2.88(br s, 4H), 2.10(s, 3H), 1.52(d, 3H, J=6.78 Hz),
**[0826]** MS($ES^-$) M-H= 683.6

**2-4-[({4-({4-[(3-Methoxyanilino)carbonyl]-1-piperazinyl}methyl)-2-[4-(trifluoromethyl)phenyl]1,3-thiazol-5-yl}methyl)sulfanyl]-2-methylphenoxy}propanoic acid**

$^1$H NMR ($CDCl_3$) 300MHz δ 8.04(d, 2H, J=8.28 Hz), 7.69(d, 2H, J=8.28 Hz), 7.31 (d, 1H, J=2.21 Hz), 7.16 (m, 2H), 6.89(m, 2H), 6.59(dd, 1H, J=8.28, 2.21 Hz), 6.53(d, 1H, J=8.28 Hz), 4.73(q, 1H, J=6.90 Hz), 4.33(d, 1H, J . 63 Hz), 4.23(d, 1H, J .63 Hz), 3.79(s, 3H), 3.45(m, 6H), 2.36(t, 4H, J=4.69 Hz), 2.24(s, 3H), 1.64(d, 3H, J=6.90 Hz),
**[0828]** MS($ES^-$) M-H= 699.6

**2-{4-[({4-{[4-(Aminocarbonyl)-1-piperazinyl]methyl}-2-[4-(trifluoromethyl)phenyl]-1,3-thiazol-5-yl}methyl)sulfanyl]-2-methylphenoxy}propanoic acid**

$^1$H NMR ($CD_3OD$) 400MHz δ 8.15(d, 2H, J=8.28 Hz), 7.83(d, 2H, J=8.28 Hz), 7.27(d, 1H, J=2.48 Hz), 7.19 (dd, 1H, J=8.55, 2.48 Hz), 6.74(d, 1H, J=8.55 Hz), 4.65(br s, 1H), 4.36(s, 2H), 3.57(s, 2H), 3.48(br s, 4H), 2.64(br s, 4H), 2.24(s, 3H), 1.62(d, 3H, J=6.62 Hz),
**[0830]** MS($ES^-$) M-H= 593.1

**2-{4-[({4-({4-[(Cyclohexylamino)carbonyl]-1-piperazinyl}methyl)-2-[4-(trifluoromethyl)phenyl]1,3-thiazol-5-yl}methyl)sulfanyl]-2-methylphenoxy}propanoic acid**

$^1$H NMR ($CD_3OD$) 400MHz δ 8.15(d, 2H, J=8.28 Hz), 7.81 (d, 2H, J=8.28 Hz), 7.24(br s, 1H), 7.13(br s, 1H), 6.73(br s, 1H), 4.75(br s, 1H), 4.30(s, 2H), 3.52(m, 7H), 2.68(br s, 4H), 2.24(s, 3H), 1.75(m, 7H), 1.26(m, 6H),

MS(ES⁻) M-H= 675.0

**2-{2-Methyl-4-[({4-({4-[(propylamino)carbonyl]-1-piperazinyl)methyl)-2-[4-(triftuoromethyl)phenyl]-1,3-thiazol-5-yl}methyl)sulfanyl]phenoxy}propanoic acid**

$^1$H NMR ($CD_3OD$) 400MHz δ 8.15(d, 2H, J=8.00 Hz), 7.81(d, 2H, J=8.00 Hz), 7.25(d, 1H, J=2.21 Hz), 7.15 (dd, 1H. J=8.55, 2.21 Hz), 6.70(d, 1H, J=8.55 Hz), 4.68(q, 1H, J=6.53 Hz), 4.30(s, 2H), 3.60(s, 2H), 3.48(br s, 4H), 3.14(t, 2H, J=7.45 Hz), 2.73(t, 4H, J=5.10 Hz), 2.22(s, 3H), 1.63(d, 3H, J=6.53 Hz), 1.52(s, 2H), 0.93(t, 3H, J=7.45 Hz),
**[0834]** MS($ES^-$) M-H= 635.3

**2-{4-[({4-({4-[(Ethylamino)carbonyl]-1-piperazinyl}methyl)-2-[4-(trifluoromethyl)phenyl]-1,3-thiazol-5-yl}methyl)sulfanyl]-2-methylphenoxy}propanoic acid**

$^1$H NMR ($CD_3OD$) 400MHz δ 8.15(d, 2H, J=8.28 Hz), 7.81(d, 2H, J=8.28 Hz), 7.25(d, 1H, J=2.48 Hz), 7.14 (dd, 1H, J=8.28, 2.48 Hz), 6.70(d, 1H, J=8.28 Hz), 4.67(br s, 1H), 4.29(s, 2H), 3.56(s, 2H), 3.46(br s, 4H), 3.22(q, 2H, J=7.17 Hz), 2.68(t, 4H, J=4.92 Hz), 2.21(s, 3H), 1.61(d, 3H, J=6.35 Hz), 1.14(t, 3H, J=7.17 Hz),
**[0836]** MS($ES^-$) M-H= 621.1

**2-{2-Methyl-4-[({4-({4-[(methylamino)carbonyl]-1-piperazinyl}methyl)-2-[4-(trifluoromethyl)phenyl]-1,3-thiazol-5-yl}methyl)sulfanyl]phenoxy}propanoic acid**

$^1$H NMR ($CD_3OD$) 400MHz δ 8.05(d, 2H, J=8.20 Hz), 7.72(d, 2H, J=8.20 Hz), 7.17(d, 1H, J=2.22 Hz), 7.09 (dd, 1H, J=8.37, 2.22 Hz), 6.61(d, 1H, J=8.37 Hz), 4.66(q, 1H, J=6.75 Hz), 4.20(s, 2H), 3.56(s, 2H), 3.42(br s, 4H), 2.69(m, 7H), 2.15(s, 3H), 1.58(d, 3H, J=6.75 Hz),
**[0838]** MS($ES^-$) M-H= 607.0

**2-{4-[({4-({4-[(Isopropylamino)carbonyl]-1-piperazinyl}methyl)-2-[4-(trifluoromethyl)phenyl]-1,3-thiazol-5-yl}methyl)sulfanyl]-2-methylphenoxy}propanoic acid**

$^1$H NMR ($CD_3OD$) 400MHz δ 8.09(d, 2H, J=8.20 Hz), 7.75(d, 2H, J=8.20 Hz), 7.17(br s, 1H), 7.08(d, 1H, J=8.20 Hz), 6.64(d, 1H, J=8.20 Hz), 4.63(q, 1H, J=6.49 Hz), 4.23(s, 2H), 3.84(m, 1H), 3.46(m, 6H), 2.68(br s, 4H), 2.16(s, 3H), 1.57(d, 3H, J=6.49 Hz), 1.10(d, 6H, J=6.32 Hz),
**[0840]** MS($ES^-$) M-H= 635.0

**2-{4-[({4-({4-[(*tert*-Butylamino)carbonyl]-1-piperazinyl}methyl)-2-[4-(trifluoromethyl)phenyl]-1,3-thiazol-5-yl}methyl)sulfanyl]-2-methylphenoxy}propanoic acid**

$^1$H NMR ($CD_3OD$) 400MHz δ 8.08(d, 2H, J=8.20 Hz), 7.75(d, 2H, J=8.20 Hz), 7.16(d, 1H, J=2.22 Hz), 7.07 (dd, 1H, J=8.37, 2.22 Hz), 6.64(d, 1H, J=8.37 Hz), 4.61 (q, 1H, J=6.75 Hz), 4.21 (s, 2H), 3.44(m, 6H), 2.71(br s, 4H), 2.16(s, 3H), 1.55(d, 3H, J=6.75 Hz), 1.27(s, 9H),
**[0842]** MS(ES-) M-H= 649.0

**2-{2-Methyl-4-[({4-[(4-{[(2-phenylethyl)amino]carbonyl}-1-piperazinyl)methyl]-2-[4-(trifluoromethyl)phenyl]-1,3-thiazol-5-yl}methyl)sulfanyl]phenoxy}propanoic acid**

$^1$H NMR ($CD_3OD$) 400MHz δ 8.08(d, 2H, J=8.03 Hz), 7.75(d, 2H, J=8.03 Hz), 7.17(s, 7H), 6.64(d, 1H, J=8.55 Hz), 4.61 (q, 1H, J=6.84 Hz), 4.24(s, 2H), 3.43(m, 9H), 2.76(t, 2H, J=7.52 Hz), 2.62(br s, 4H), 2.16(s, 3H), 1.56(d, 3H, J=6.67 Hz),
**[0844]** MS($ES^-$) M-H= 697.0

**2-{4-[({4-[(4-Benzoyl-1-piperazinyl)methyl]-2-[4-(trifluoromethyl)phenyl]-1,3-thiazol-5-yl}methyl)sulfanyl]-2-methylphenoxy}propanoic acid**

$^1$H NMR ($CD_3OD$) 300MHz δ 8.03(d, 2H, J=8.28 Hz), 7.70(d, 2H, J=8.28 Hz), 7.42(m, 5H), 7.13(d, 1H, J=2.24 Hz), 7.07(dd, 1H, J=8.45, 2.24 Hz), 6.62(d, 1H, J=8.45 Hz), 4.61 (q, 1H, J=6.78 Hz), 4.26(s, 2H), 3.83(br s, 4H), 3.62 (s, 2H), 2.86(br s, 4H), 2.11(s, 3H), 1.53(d, 3H, J=6.78 Hz),
**[0846]** MS($ES^-$) M-H= 653.7

**2-{2-Methyl-4-[({4-{[4-(4-propoxyphenyl)-1-piperazinyl]methyl}-2-[4-(trifluoromethyl)phenyl]-1,3-thiazol-5-yl}methyl)sulfanyl]phenoxy}propanoic acid**

$^1H$ NMR ($CD_3OD$) 400MHz δ 8.11(d, 2H, J=7.69 Hz), 7.77(d, 2H, J=7.69 Hz), 7.15(s, 1H), 7.08(dd, 1H, J=8.61, 2.20 Hz), 6.93(d, 2H, J=8.97 Hz), 6.82(d, 2H, J=8.97 Hz), 8.67(d, 1H, J=8.61 Hz), 4.57(q, 1H, J=6.78 Hz), 4.24(s, 2H), 3.85(t, 2H, J=7.01 Hz), 3.55(s, 2H), 3.18(br s, 4H), 3.03(br s, 4H), 2.16(s, 3H), 1.73(m, 2H), 1.54(d, 3H, J=6.78 Hz), 1.00(t, 3H, J=7.01 Hz),

MS($ES^-$) M-H= 684.0

**2-{4-[({4-{[4-(4-Ethoxyphenyl)-1-piperazinyl]methyl}-2-[4-(trifluoromethyl)phenyl]-1,3-thiazol-5-yl}methyl)sulfanyl]-2-methylphenoxy}propanoic acid**

$^1H$ NMR ($CD_3OD$) 400MHz δ 8.11 (d, 2H, J=8.06 Hz), 7.77(d, 2H, J=8.06 Hz), 7.15(s, 1H), 7.08(dd, 1H, J=8.42, 2.20 Hz), 6.92(d, 2H, J=8.97 Hz), 6.81 (d, 2H, J=8.97 Hz), 6.67(d, 1H, J=8.42 Hz), 4.59(q, 1H, J=6.78 Hz), 4.24(s, 2H), 3.95(q, 2H, J=6.78 Hz), 3.54(s, 2H), 3.17(br s, 4H), 3.04(br s, 4H), 2.17(s, 3H), 1.55(d, 3H, J=6.78 Hz), 1.32(t, 3H, J=6.78 Hz),

MS($ES^-$) M-H= 671.0

**2-{2-Methyl-4-[({4-({4-[4-(trifluoromethoxy)phenyl]-1-piperazinyl}methyl)-2-[4-(trifluoromethyl)phenyl]-1,3-thiazol-5-yl}methyl)sulfanyl]phenoxy}propanoic acid**

$^1H$ NMR ($CD_3OD$) 400MHz δ 8.10(d, 2H, J=8.28 Hz), 7.75(d, 2H, J=8.28 Hz), 7.15(d, 1H, J=2.24 Hz), 7.12 (d, 2H, J=9.14 Hz), 7.08(dd, 1H, J=8.45, 2.24 Hz), 7.00(d, 2H, J=9.31 Hz), 6.66(d, 1H, J=8.45 Hz), 4.59(q, 1H, J=6.72 Hz), 4.24(s, 2H), 3.54(s, 2H), 3.27(m, 4H), 2.97(t, 4H, J=4.83 Hz), 2.16(s, 3H), 1.54(d, 3H, J=6.72 Hz),

MS($ES^-$) M-H= 710.0

**2-{4-[({4-{[4-(3,4-Dimethoxyphenyl)-1-piperazinyl]methyl}-2-[4-(trifluoromethyl)phenyl]-1,3-thiazol-5-yl}methyl)sulfanyl]-2-methylphenoxy}propanoic acid**

$^1H$ NMR ($CD_3OD$) 300MHz δ 8.17(d, 2H, J=8.28 Hz), 7.82(d, 2H, J=8.28 Hz), 7.20(br s, 1H), 7.12(br s, 1H), 6.89(d, 1H, J=8.83 Hz), 6.72(m, 2H), 6.55(dd, 1H, J=8.83, 2.76 Hz), 4.66(br s, 1H), 4.29(s, 2H), 3.84(s, 3H), 3.80(s, 3H), 3.57(s, 2H), 3.25(br s, 4H), 3.07(br s, 4H), 2.23(s, 3H), 1.61(br s, 3H),

MS($ES^-$) M-H= 686.0

**2-{4-[({4-{[4-(4-Hydroxyphenyl)-1-piperazinyl]methyl}-2-[4-(trifluoromethyl)phenyl)-1,3-thiazol-5-yl}methyl)sulfanyl]-2-methylphenoxy}propanoic acid**

$^1H$ NMR ($CD_3OD$) 300MHz δ 8.14(br s, 2H), 7.80(br s, 2H), 7.24(br s, 1H), 7.12(br s, 1H), 6.92(br s, 2H), 6.76 (br s, 2H), 6.63(sbr , 1 H), 4.54(br s, 1 H), 4.31 (br s, 2H), 3.67(br s, 2H), 3.06(br s, 8H), 2.23(br s, 3H), 1.60(br s, 3H),

MS($ES^-$) M-H= 642.3

**2-{4-[({4-{[4-(3-Hydroxyphenyl)-1-piperazinyl]methyl}-2-[4-(trifluoromethyl)phenyl]-1,3-thiazol-5-yl}methyl)sulfanyl]-2-methylphenoxy}propanoic acid**

$^1H$ NMR ($CD_3OD$) 300MHz δ 8.15(d, 2H, J=8.28 Hz), 7.81(d, 2H, J=8.28 Hz), 7.22(d, 1H, J=2.21 Hz), 7.15 (dd, 1H, J=8.28, 2.21 Hz), 7.08(t, 1H, J=8.14 Hz), 6.71 (d, 1H, J=8.28 Hz), 6.49(dd, 1H, J□4, 2.21 Hz), 6.45(t, 1H, J=2.21 Hz), 6.39(dd, 1H, J=8.14, 2.21 Hz), 4.74(q, 1H, J=6.81 Hz), 4.30(s, 2H), 3.85(s, 2H), 3.36(m, 4H), 3.24(m, 4H), 2.21 (s, 3H), 1.61(d, 3H, J=6.81 Hz),

MS($ES^-$) M-H= 642.0

**2-[4-[({4-{[4-(2-Hydroxyphenyl)-1-piperazinyl]methyl}-2-[4-(trifluoromethyl)phenyl]-1,3-thiazol-5-yl}methyl)sulfanyl]-2-methylphenoxy}propanoic acid**

$^1H$ NMR ($CD_3OD$) 300MHz δ 8.20(d, 2H, J=8.00 Hz), 7.80(d, 2H, J=8.00 Hz), 7.23(br s, 1H), 7.01 (m, 3H), 6.82(m, 2H), 6.66(br s, 1H), 4.74(br s, 1H), 4.26(s, 2H), 3.56(s, 2H), 3.12(m, 8H), 2.19(s, 3H), 1.58(br s, 3H),

MS($ES^-$) M-H= 642.1

**2-{4-[({4-[(4-Butyryl-1-piperazinyl)methyl]-2-[4-(trifluoromethyl)phenyl]-1,3-thiazol-5-yl}methyl)sulfanyl]-2-methylphenoxy}propanoic acid**

$^1$H NMR ($CD_3OD$) 400MHz δ 8.05(d, 2H, J=8.55 Hz), 7.72(d, 2H, J=8.55 Hz), 7.17(d, 1H, J=2.22 Hz), 7.08 (d, 1H, J=8.55 Hz), 6.64(s, 1H), 4.56(q, 1H, J=6.55 Hz), 4.26(s, 2H), 3.54(br s, 4H), 3.38(s, 2H), 2.46(br s, 4H), 2.33 (t, 2H, J=7.43 Hz), 2.16(s, 3H), 1.58(m, 5H), 0.93(t, 3H, J=7.43 Hz),

MS($ES^-$) M-H= 620.0

**2-{2-Methyl-4-[({4-[(4-pentanoyl-1-piperazinyl)methyl]-2-[4-(trifluoromethyl)phenyl]-1,3-thiazol-5-yl}methyl) sulfanyl]phenoxy}propanoic acid**

$^1$H NMR ($CD_3OD$) 400MHz δ 8.06(d, 2H, J=8.20 Hz), 7.74(d, 2H, J=8.20 Hz), 7.17(d, 1H, J=2.22 Hz), 7.10 (dd, 1H, J=8.20, 2.22 Hz), 6.65(d, 1H, J=8.20 Hz), 4.68(q, 1H, J=6.75 Hz), 4.25(s, 2H), 3.56(br s, 4H), 3.40(s, 2H), 2.56(br s, 4H), 2.36(t, 2H, J=7.35 Hz), 2.16(s, 3H), 1.54(m, 5H), 1.34(m, 2H), 0.90(t, 3H, J=7.35 Hz),

MS($ES^-$) M-H= 634.0

**2-{4-[({4-{[4-(Methoxyacetyl}-1-piperazinyl]methyl}-2-[4-(trifluoromethyl)phenyl]-1,3-thiazol-5-yl}methyl) sulfanyl]-2-methylphenoxy}propanoic acid**

$^1$H NMR ($CD_3OD$) 300MHz δ 8.07(d, 2H, J=8.37 Hz), 7.75(d, 2H, J=8.37 Hz), 7.18(d, 1H, J=2.20 Hz), 7.11 (d, 1H, J=8.37 Hz), 6.65(d, 1H, J=8.37 Hz), 4.68(q, 1H, J=6.72 Hz), 4.26(s, 2H), 4.12(s, 2H), 3.57(br s, 2H), 3.46(br s, 2H), 3.39(s, 2H), 3.35(s, 3H), 2.53(t, 4H, J=4.79 Hz), 2.16(s, 3H), 1.56(d, 3H, J=6.72 Hz),

MS($ES^-$) M-H= 622.0

**2-{4-[({4-[(4-Isobutyryl-1-piperazinyl)methyl]-2-[4-(trifluoromethyl)phenyl]-1,3-thiazol-5-yl}methyl)sulfanyl]-2-methylphenoxy}propanoic acid**

$^1$H NMR ($CD_3OD$) 300MHz δ 8.10(d, 2H, J=8.28 Hz), 7.76(d, 2H, J=8.28 Hz), 7.20(d, 1H, J=2.21 Hz), 7.13 (dd, 1H, J=8.55, 2.21 Hz), 6.69(d, 1H, J=8.55 Hz), 4.67(q, 1H, J=6.81 Hz), 4.31(s, 2H), 3.76(br s, 4H), 3.69(s, 2H), 2.92(m, 5H), 2.20(s, 3H), 1.59(d, 3H, J=6.81 Hz), 1.10(d, 6H, J=6.62 Hz),

MS($ES^-$) M-H= 620.4

**2-{4-[({4-{[4-(2,2-Dimethylpropanoyl)-1-piperazinyl]methyl}-2-[4-{trifluoromethyl)phenyl]-1,3-thiazol-5-yl} methyl)sulfanyl]-2-methylphenoxy}propanoic acid**

$^1$H NMR ($CD_3OD$) 300MHz δ 8.10(d, 2H, J=8.28 Hz), 7.76(d, 2H, J=8.28 Hz), 7.19(d, 1H, J=2.21 Hz), 7.13 (dd, 1H, J=8.28, 2.21 Hz), 6.69(d, 1H, J=8.28 Hz), 4.68(q, 1H, J=6.71 Hz), 4.32(s, 2H), 3.83(br s, 4H), 3.71(s, 2H), 2.98(t, 4H, J=4.83 Hz), 2.20(s, 3H), 1.60(d, 3H, J=6.71 Hz), 1.28(s, 9H),

MS($ES^-$) M-H= 634.2

**2-Methyl-2-[4-({[2-[4-(trifluoromethyl)phenyl]-4-({4-[3-(trifluoromethyl)phenyl]-1-piperazinyl}methyl)-1,3-thiazol-5-yl]methyl}sulfanyl)phenoxy]propanoic acid**

$^1$H NMR ($CD_3OD$) 400MHz δ 8.10(d, 2H, J=8.06 Hz), 7.76(d, 2H, J=8.06 Hz), 7.40(t, 1H, J=7.69 Hz), 7.28(d, 2H, J=8.79 Hz), 7.18(s, 2H), 7.09(d, 1H, J=7.69 Hz), 6.81 (d, 2H, J=8.79 Hz), 4.31 (s, 2H), 3.59(s, 2H), 3.31 (t, 4H, J=4.94 Hz), 2.88(t, 4H, J=4.94 Hz), 1.54(s, 6H),

MS($ES^-$)M-H=694.5

CHN Analysis (Theoretical %C=56.97, %H=4.49, %N=6.04; Found %C=56.69, %H=4.66, %N=5.77)

**{4-[({4-{[4-(*tert*-Butoxycarbonyl)-1-piperazinyl]methyl}-2-[4-(trifluoromethyl)phenyl]-1,3-thiazol-5-yl}methyl) sulfanyl]-2-methylphenoxy}acetic acid**

$^1$H NMR ($CD_3OD$) 400MHz δ 8.05(d, 2H, J=8.28 Hz), 7.73(d, 2H, J=8.28 Hz), 7.18(s, 1H), 7.11 (br s, 1 H), 6.66(br s, 1 H), 4.54(s, 2H), 4.26(s, 2H), 3.42(m, 6H), 2.50(br s, 4H), 2.19(s, 3H), 1.43(s, 9H),

MS($ES^-$) M-H= 636.5

**{2-Methyl-4-[({4-{[4-(2-pyrazinyl)-1-piperazinyl]methyl}-2-[4-(trifluoromethyl)phenyl]-1,3-thiazol-5-yl}methyl) sulfanyl]phenoxy}acetic acid**

$^1$H NMR ($CD_3OD$) 400MHz δ 8.21(s, 1H), 8.09(d, 3H, J=8.10 Hz), 7.80(s, 1H), 7.75(d, 2H, J=8.28 Hz), 7.19 (d, 1H, J=2.07 Hz), 7.13(dd, 1H, J=8.45, 2.24 Hz), 6.70(d, 1H, J=8.45 Hz), 4.57(s, 2H), 4.27(s, 2H), 3.66(br s, 4H), 3.53(s, 2H), 2.77(br s, 4H), 2.17(s, 3H),

MS($ES^-$) M-H= 612.4

**{4-[({4-{[4-(2-Methoxyphenyl)-1-piperazinyl]methyl}-2-[4-(trifluoromethyl)phenyl]-1,3-thiazol-5-yl}methyl) sulfanyl]-2-methylphenoxy}acetic acid**

$^1$H NMR ($CD_3OD$) 400MHz δ 8.10(d, 2H, J=8.28 Hz), 7.75(d, 2H, J=8.28 Hz), 7.18(d, 1H, J=2.20 Hz), 7.02 (s, 2H), 6.92(dd, 2H, J=8.10, 2.20 Hz), 6.86(s, 1 H), 6.62(d, 1H, J=8.45 Hz), 4.48(s, 2H), 4.25(s, 2H), 3.81 (s, 3H), 3.55 (s, 2H), 3.11 (br s, 4H), 2.96(br s, 4H), 2.17(s, 3H),

MS($ES^-$) M-H= 640.5

**{4-[({4-{[4-(3-Methoxyphenyl)-1-piperazinyl]methyl}-2-[4-(trifluoromethyl)phenyl]-1,3-thiazol-5-yl}methyl) sulfanyl]-2-methylphenoxy}acetic acid**

$^1$H NMR ($CD_3OD$) 400MHz δ 8.10(d, 2H, J=8.28 Hz), 7.75(d, 2H, J=8.28 Hz), 7.18(d, 1H, J=2.24 Hz), 7.10 (s, 2H), 6.67(d, 1H, J=8.23 Hz), 6.53(dd, 1 H, J=8.28, 2.24 Hz), 6.47(t, 1H, J=2.24 Hz), 6.43(dd, 1H, J=8.28, 2.24 Hz), 4.52(s, 2H), 425(s, 2H), 3.72(s, 3H), 3.58(s, 2H), 3.24(t, 4H, J=5.09 Hz), 2.98(t, 4H, J=5.09 Hz), 2.17(s, 3H),

MS($ES^-$) M-H= 642.0

**2-Methyl-2-{4-[({4-{[4-(phenoxycarbonyl)-1-piperazinyl]methyl}-2-[4-(trifluoromethyl)phenyl]-1,3-thiazol-5-yl} methyl)sulfanyl]phenoxy}propanoic acid**

$^1$H NMR ($CD_3OD$) 400MHz δ 8.07(d, 2H, J=8.28 Hz), 7.73(d, 2H, J=8.28 Hz), 7.34(t, 2H, J=7.59 Hz), 7.27(d, 2H, J=8.45 Hz), 7.18(t, 1H, J=7.59 Hz), 7.06(d, 2H, J=7.59 Hz), 6.80(d, 2H, J=8.45 Hz), 4.33(s, 2H), 3.68(br s, 2H), 3.53(br s, 2H), 3.44(s, 2H), 2.56(br s, 4H), 1.52(s, 6H),

CHN Analysis 1 MeOH(Theoretical %C=58.02, %H=5.16, %N=5.97; Found %C=58.33, %H=5.09, %N=5.72)

**2-{4-[({4-{[4-*tert*-Butoxycarbonyl)-1-piperazinyl]methyl}-2-[4-(trifluoromethyl)phenyl]-1,3-thiazol-5-yl}methyl) sulfanyl]phenoxy}-2-methylpropanoic acid**

$^1$H NMR ($CD_3OD$) 400MHz δ 8.04(d, 2H, J=8.28 Hz), 7.71 (d, 2H, J=8.28 Hz), 7.22(d, 2H, J=8.10 Hz), 6.78 (d, 2H, J=8.10 Hz), 4.27(s, 2H), 3.40(m, 6H), 2.49(br s, 4H), 1.50(s, 6H), 1.41 (s, 9H),

MS($ES^-$) M-H= 650.5

**2-Methyl-2-{4-[({4-{[4-(2-pyrazinyl)-1-piperazinyl]methyl}-2-[4-(trifluoromethyl)phenyl]-1,3-thiazol-5-yl}methyl) sulfanyl]phenoxy}propanoic acid**

$^1$H NMR ($CD_3OD$) 400MHz δ 8.21 (s, 1H), 8.07(m, 3H), 7.79(s, 1H), 7.73(d, 2H, J=8.28 Hz), 7.25(d, 2H, J=8.10 Hz), 6.79(d, 2H, J=8.10 Hz), 4.30(s, 2H), 3.65(br s, 4H), 3.53(s, 2H), 2.72(br s, 4H), 1.53(s, 6H),

MS($ES^-$) M-H= 627.6

**2-{4-[({4-{[4-(2-Methoxyphenyl)-1-piperazinyl]methyl}-2-[4-(trifluoromethyl)phenyl]-1,3-thiazol-5-yl}methyl) sulfanyl]phenoxy}-2-methylpropanoic acid**

$^1$H NMR ($CD_3OD$) 400MHz δ 8.10(d, 2H, J=8.28 Hz), 7.74(d, 2H, J=8.28 Hz), 7.21(d, 2H, J=8.42 Hz), 7.00 (m, 1 H), 6.92(m, 2H), 6.86(m, 1H), 6.78(d, 2H, J=8.42 Hz), 4.27(s, 2H), 3.81 (s, 3H), 3.59(s, 2H), 3.14(br s, 4H), 3.01 (br s, 4H), 1.51 (s, 6H),

MS($ES^-$) M-H= 656.0

**2-{4-[({4-{[4-(Ethoxycarbonyl)-1-piperazinyl]methyl}-2-[4-(trifluoromethyl)phenyl]-1,3-thiazol-5-yl}methyl) sulfanyl]phenoxy}-2-methylpropanoic acid**

$^1$H NMR ($CD_3OD$) 400MHz δ 8.05(d, 2H, J=8.10 Hz), 7.72(d, 2H, J=8.10 Hz), 7.24(d, 2H, J=8.42 Hz), 6.79(d, 2H, J=8.42 Hz), 4.30(s, 2H), 4.09(q, 2H, J=7.16 Hz), 3.44(m, 6H), 2.50(s, 4H), 1.52(s, 6H), 1.21(t, 3H, J=7.16 Hz),

MS($ES^-$) M-H= 621.7

**2-{4-[({4-{[4-(4-Isopropoxyphenyl)-1-piperazinyl]methyl}-2-[4-(trifluoromethyl)phenyl]-1,3-thiazol-5-yl}methyl) sulfanyl]-2-methylphenoxy}propanoic acid**

$^1$H NMR ($CD_3OD$) 400MHz δ 8.13(d, 2H, J=8.06 Hz), 7.79(d, 2H, J=8.06 Hz), 7.13(m, 2H), 6.92(d, 2H, J=8.97 Hz), 6.81 (d, 2H, J=8.97 Hz), 6.67(d, 1H, J=8.42 Hz), 4.61 (q, 1H, J=6.78 Hz), 4.46(m, 1H), 4.25(s, 2H), 3.56(s, 2H), 3.19(br s, 4H), 3.06(br s, 4H), 2.17(s, 3H), 1.55(d, 3H, J=6.78 Hz), 1.24(d, 6H, J=6.87 Hz),

MS($ES^-$) M-H= 685.0

**[4-({[4-([1,1'-Biphenyl]-4-ylmethyl)-2-(4-{trifluoromethyl}phenyl)-1,3-thiazol-5-yl]methyl}sulfanyl)-2-methylphenoxy]acetic acid**

TLC(5% MeOH/$CH_2Cl_2$) $R_f$= 0.16

MS($ES^-$) M-H= 603

**{2-Methyl-4-[({2-{4-{trifluoromethyl}phenyl)-4-[4-(3-thienyl)benzyl]-1,3-thiazol-5-yl}methyl)sulfanyl]phenoxy} acetic acid**

$^1$H NMR ($CDCl_3$) 300MHz δ 7.93(d, 2H, J=8.23 Hz), 7.61(d, 2H, J=8.23 Hz), 7.44(d, 2H, J=8.23 Hz), 7.36(s, 1H), 7.29(m, 2H), 7.08(m, 3H), 6.54(d, 1H, J=8.23 Hz), 4.52(s, 2H), 4.06(s, 2H), 3.90(s, 2H), 2.15(s, 3H),

TLC(5% MeOH/$CH_2Cl_2$) $R_f$= 0.18

MS($ES^-$) M-H= 609

**[4-({[4-Benzyl-2-(4-{trifluoromethyl}phenyl)-1,3-thiazol-5-yl]methyl}sulfanyl)phenoxy]acetic acid**

$^1$H NMR ($CD_3OD$) 300MHz δ 8.04(d, 2H, J=8.23 Hz), 7.75(d, 2H, J=8.23 Hz), 7.34(d, 2H, J=8.76 Hz), 7.20 (m, 5H), 6.88(d, 2H, J=9.76 Hz), 4.66(s, 2H), 4.25(s, 2H), 3.93(s, 2H),

MS($ES^-$) M-H= 513.86

TLC(20% MeOH/$CH_2Cl_2$) $R_f$= 0.37

**2-[4-({[4-Benzyl-2-(4-{trifluoromethyl}phenyl)-1,3-thiazol-5-yl]methyl}sulfanyl)phenoxy]propanoic acid**

$^1$H NMR ($CDCl_3$) 300MHz δ 8.02(d, 2H. J=8.23 Hz), 7.69(d, 2H, J=8.23 Hz), 7.26(m, 7H), 6.83(d, 2H, J=8.76 Hz), 4.80(q, 1H, J=6.72 Hz), 4.14(s, 2H), 3.90(m, 2H), 1.68(d, 3H, J=6.72 Hz),

MS($ES^-$) M-H= 528.43

TLC(20% MeOH/$CH_2Cl_2$) $R_f$= 0.60

**[2-Methyl-4-({[2-{4-{trifluoromethyl}phenyl)-4-(2-phenylethyl)-1,3-thiazol-5-yl]methyl}sulfanyl)phenoxy]acetic acid**

$^1$H ($CDCl_3$) 300MHz δ 7.99(d, 2H, J=8.79 Hz), 7.67(d, 2H, J=8.93 Hz), 7.18(m, 8H), 6.60(d, 1 H, J=8.51 Hz), 4.64(s, 2H), 3.85(s, 2H), 2.90(m, 2H), 2.80(m. 2H), 2.23(s, 3H),

**[4-({[4-[(Benzyloxy)methyl]-2-(4-{trifluoromethyl}phenyl)-1,3-thiazol-5-yl]methyl}sulfanyl)-2-methylphenoxy] acetic acid**

$^1$H ($CDCl_3$) 300MHz δ 7.99(d, 2H, J=8.79 Hz), 7.67(d, 2H, J=8.79 Hz), 7.33(m, 4H), 7.28(s, 2H), 7.18(dd, 1H, J=2.33, 0.55 Hz), 7.08(ddd, 1H, J=8.38, 2.33, 0.55 Hz), 6.56(d, 1H, J=8.38 Hz), 4.63(s, 2H), 4.53(s, 2H), 4.39(s, 2H), 4.19(s, 2H), 2.21 (s, 3H),

**[2-Methyl-4-({[2-(4-{trifluoromethyl}phenyl)-4-(3-phenylpropyl)-1,3-thiazol-5-yl]methyl}sulfanyl)phenoxy] acetic acid**

$^1$H NMR ($CDCl_3$) 300MHz δ 7.82(m, 2H), 7.50(m, 2H), 6.94(m, 8H), 3.95(s, 2H), 2.55(m, 4H), 1.99(m, 7H),

**{2-Methyl-4-[({2-(4-{trifluoromethyl}phenyl)-4-[(2-phenylethoxy)methyl]-1,3-thiazol-5-yl}methyl)sulfanyl] phenoxy}acetic acid**

$^1$H NMR ($CDCl_3$) 300MHz δ 7.92(m, 2H), 7.62(m, 2H), 7.20(m, 7H), 7.05(br s, 1H), 4.55(s, 2H), 4.38(s, 2H), 4.09(s, 2H), 3.66(br s, 2H), 2.87(br s, 2H), 2.17(s, 3H),

TLC(5% MeOH/Dichloromethane) $R_f$= 0.65

**[4-({[4-(4-Bromobenzyl)-2-(4-{trifluoromethyl}phenyl)-1,3-thiazol-5-yl]methyl}sulfanyl)-2-methylphenoxy] acetic acid**

$^1$H NMR ($CDCl_3$) 400MHz δ 7.82(d, 2H, J=8.20 Hz), 7.53(d, 2H, J=8.20 Hz), 7.22(d, 2H, J=8.55 Hz), 7.05(m, 1H), 6.97(dd, 1H, J=8.37, 2.39 Hz), 6.88(d, 2H, J=8.55 Hz), 6.47(d, 1 H, J=8.37 Hz), 4.47(s, 2H), 3.72(s, 2H), 3.36(s, 2H), 2.08(s, 3H),

TLC(5% MeOH/$CH_2Cl_2$) $R_f$= 0.16

**[4-({[4-Benzyl-2-(4-{triflurormethyl}phenyl)-1,3-thiazol-5-yl]methyl}sulfanyl)-2-methylphenoxy]acetic acid**

$^1$H ($CDCl_3$) 300MHz δ 7.97(d, 2H, J=8.79 Hz), 7.64(d, 2H, J=9.48 Hz), 7.21(m, 8H), 6.58(d, 1H, J=8.38 Hz), 4.65(s, 2H), 4.11(s, 2H), 3.93(s, 2H), 2.22(s, 3H),

MS($ES^+$) M+H= 529.99

**2-{4-[({4-{[3-(5-Methyl-1,2,4-oxadiazol-3-yl)phenoxy]methyl}-2-[4-(trifluoromethyl)phenyl]-1,3-thiazol-5-yl} methyl)sulfanyl]phenoxy}propanoic acid**

$^1$H NMR ($CDCl_3$) 400MHz δ 8.01 (d, 2H, J=8.03 Hz), 7.68(m, 3H), 7.43(m, 1H), 7.36(t, 1H, J=8.03 Hz), 7.20 (d, 2H, J=8.89 Hz), 7.05(dd, 1H, J=8.20, 2.39 Hz), 6.79(d, 2H, J=8.89 Hz), 4.76(q, 1H, J=6.78 Hz), 4.66(d, 1H, J .28 Hz), 4.36(d, 1H, J .28 Hz), 4.24(d, 1H, J .70 Hz), 4.15(d, 1H, J .70 Hz), 2.71(s, 3H), 1.67(m, 3H),

MS($ES^+$) M+H= 628.0

**2-{4-[({4-{[4-(4-Methoxyphenyl)-1-piperazinyl]methyl}-2-[4-(trifluoromethyl)phenyl]-1,3-thiazol-5-yl}methyl] sulfanyl]-2-methylphenoxy}propanoic acid**

$^1$H NMR ($CDCl_3$) 400MHz δ 9.03(br s, 1H), 7.96(d, 2H, J=8.20 Hz), 7.67(d, 2H, J=8.20 Hz), 7.15(d, 2H, J=8.72 Hz), 6.81 (m, 6H), 4.12(s, 2H), 3.73(s, 3H), 3.50(s, 2H), 3.27(br s, 4H), 3.15(br s, 4H), 1.63(s, 6H),

HPLC(C-18, 3μm) 1%MeOH/0-90% $CH_3CN$/Water (0.1% TFA)/(50mM $Et_3N$/TFA) 4min run $R_t$=2.89 min

**2-(4-{[(4-{[4-(4-Methoxyphenyl)-1-piperazinyl]methyl}-2-phenyl-1,3-thiazol-5-yl)methyl]sulfanyl}phenoxy)-2-methylpropanoic acid**

$^1$H NMR ($CDCl_3$) 400MHz δ 7.87(m, 2H), 7.44(m, 3H), 7.15(d, 2H, J=8.55 Hz), 6.82(m, 6H), 4.08(s, 2H), 3.73 (s, 3H), 3.46(s, 2H), 3.31(m, 4H), 3.18(m, 4H), 1.65(s, 6H),

HPLC(C-18, 3μm) 1%MeOH/0-90% $CH_3CN$/Water (0.1% TFA)/(50mM $Et_3N$/TFA) 4min run $R_t$=2.74 min

**{4-[({4-{[4-(4-Methoxyphenyl)-1-piperazinyl]methyl}-2-[4-(trifluoromethyl)phenyl]-1,3-thiazol-5-yl}methyl) sulfanyl]phenoxy}acetic acid**

$^1$H NMR ($CDCl_3$) 400MHz δ 10.00(s, 1H), 7.96(d, 2H, J=8.20 Hz), 7.66(d, 2H, J=8.20 Hz), 7.27(d, 2H, J=8.72 Hz), 6.82(m, 6H), 4.51 (s, 2H), 4.22(s, 2H), 3.80(s, 2H), 3.72(s, 3H), 3.21 (m, 8H),

HPLC(C-18, 3μm) 1%MeOH/0-90% $CH_3CN$/Water (0.1% TFA)/(50mM $Et_3N$/TFA) 4min run $R_t$=2.74 min

**(4-{[(4-{[4-(4-Methoxyphenyl)-1-piperazinyl]methyl}-2-phenyl-1,3-thiazol-5-yl)methyl]sulfanyl}phenoxy)acetic acid**

$^1$H NMR ($CDCl_3$) 400MHz δ 9.49(br s, 1 H), 7.86(m, 2H), 7.42(m, 3H), 7.24(d, 2H, J=8.55 Hz), 6.80(m, 6H), 4.50(s, 2H), 4.22(s, 2H), 3.81 (s, 2H), 3.71 (s, 3H), 3.24(m, 8H),

HPLC(C-18, 3μm) 1%MeOH/0-90% $CH_3CN$/Water (0.1% TFA)/(50mM $Et_3N$/TFA) 4min run $R_t$=2.55 min

**2-{4-[({4-{[4-(4-Methoxyphenyl)-1-piperazinyl]methyl}-2-[4-trifluoromethyl)phenyl]-1,3-thiazol-5-yl}methyl) sulfanyl]phenoxy}propanoic acid**

$^1$H NMR ($CDCl_3$) 400MHz δ9.31(s, 1 H), 7.96(d, 2H, J=8.20 Hz), 7.68(d, 2H, J=8.20 Hz), 7.18(d, 2H, J=8.55 Hz), 6.82(m, 6H), 4.73(q, 1H, J=6.67 Hz), 4.16(d, 1H, J .87 Hz), 4.10(d, 1H, J .87 Hz), 3.72(s, 3H), 3.58(d, 1H, J .53 Hz), 3.51 (d, 1H, J .53 Hz), 3.24(m, 8H), 1.59(d, 3H, J=6.67 Hz),

HPLC(C-18, 3μm) 1%MeOH/0-90% $CH_3CN$/Water (0.1% TFA)/(50mM $Et_3N$/TFA) 4min run $R_t$=2.80 min

**2-(4-{[(4-{[4-(4-Methoxyphenyl)-1-piperazinyl]methyl}-2-phenyl-1,3-thiazol-5-yl)methyl]sulfanyl}phenoxy) propanoic acid**

$^1$H NMR ($CDCl_3$) 400MHz δ8.42(s, 1H), 7.84(m, 2H), 7.40(m, 3H), 7.17(d, 2H, J=8.72 Hz), 6.81(m, 6H), 4.69 (q, 1H, J=6.67 Hz), 4.11(d, 1H, J .18 Hz), 4.07(d, 1H, J .18 Hz), 3.73(s, 3H), 3.57(d, 1H, J .87 Hz), 3.49(d, 1H, J.87 Hz), 3.18(m, 8H), 1.59(d, 3H, J=6.67 Hz),

HPLC(C-18, 3μm) 1%MeOH/0-90% $CH_3CN$/Water (0.1% TFA)/(50mM $Et_3N$/TFA) 4min run $R_t$=2.63 min

**{4-[({4-{[3-(5-Methyl-1,2,4-oxadiazol-3-yl)phenoxy]methyl}-2-[4-(trifluoromethyl)phenyl]-1,3-thiazol-5-yl} methyl)sulfanyl]phenoxy}acetic acid**

$^1$H NMR ($CDCl_3$) 400MHz δ 10.17(s, 1 H), 8.02(d, 2H, J=8.20 Hz), 7.67(m, 3H), 7.46(m, 1 H), 7.36(t, 1H, J=7.95 Hz), 7.22(d, 2H, J=8.72 Hz), 7.06(dd, 1H, J=8.37, 2.39 Hz), 6.79(d, 2H, J=8.72 Hz), 4.69(s, 2H), 4.58(s, 2H), 4.22(s, 2H), 2.73(s, 3H),

MS($ES^+$) M+H= 614.00

**2-Methyl-2-{4-[({4-{[4-(5-methyl-1,2,4-oxadiazol-3-yl)phenoxy]methyl}-2-[4-(trifluoromethyl)phenyl]-1,3-thiazol-5-yl}methyl)sulfanyl]phenoxy}propanoic acid**

$^1$H ($CDCl_3$) 400MHz δ 7.98(d, 2H, J=8.03 Hz), 7.92(d, 2H, J=9.06 Hz), 7.67(d, 2H, J=8.03 Hz), 7.18(d, 2H, J=9.06 Hz), 6.96(d, 2H, J=8.75 Hz), 6.74(d, 2H, J=8.75 Hz), 4.98(s, 2H), 4.29(s, 2H), 2.66(s, 3H), 1.57(s, 6H)

MS($ES^-$) M-H= 640.00

**2-Methyl-2-(4-{[(4-{[4-(5-methyl-1,2,4-oxadiazol-3-yl)phenoxy]methyl}-2-phenyl-1,3-thiazol-5-yl)methyl] sulfanyl}phonoxy)propanoic acid**

$^1$H NMR ($CDCl_3$) 400MHz δ7.93(d, 2H, J=9.06 Hz), 7.86(m, 2H), 7.42(m, 3H), 7.17(d, 2H, J=8.72 Hz), 6.96 (d, 2H, J=9.06 Hz), 6.73(d, 2H, J=8.72 Hz), 4.92(s, 2H), 4.27(s, 2H), 2.66(s, 3H), 1.57(s, 6H),

MS($ES^-$) M-H= 571.50

**{4-[({4-{[4-(5-Methyl-1,2,4-oxadiazol-3-yl)phenoxy]methyl}-2-[4-(trifluoromethyl)phenyl]-1,3-thiazol-5-yl} methyl)sulfanyl]phenoxy}acetic acid**

$^1$H NMR ($CDCl_3$) 400MHz δ 7.98(d, 2H, J=8.20 Hz), 7.93(d, 2H, J=9.06 Hz), 7.66(d, 2H, J=8.20 Hz), 7.28(d, 2H, J=8.89 Hz), 6.96(d, 2H, J=9.06 Hz), 6.76(d, 2H, J=8.89 Hz), 4.86(s, 2H), 4.60(s, 2H), 4.25(s, 2H), 2.62(s, 3H),

MS($ES^-$) M-H= 611.80

**(4-{[(4-{[4-(5-Methyl-1,2,4-oxadiazol-3-yl)phenoxy]methyl}-2-phenyl-1,3-thiazol-5-yl)methyl]sulfanyl}phenoxy) acetic acid**

$^1$H NMR ($CDCl_3$) 400MHz 7.92(d, 2H, J=9.06 Hz), 7.83(m, 2H), 7.39(m, 3H), 7.23(d, 2H, J=8.90 Hz), 6.95(d, 2H, J=9.06 Hz), 6.76(d, 2H, J=8.90 Hz), 4.70(s, 2H), 4.54(s, 2H), 4.18(s, 2H), 2.60(s, 3H),

MS($ES^+$) M+H= 546.20

**2-{4-[({4-{[4-(5-Methyl-1,2,4-oxadiazol-3-yl)phenoxy]methyl}-2-[4-(trifluoromethyl)phenyl]-1,3-thiazol-5-yl} methyl)sulfanyl]phenoxy}propanoic acid**

$^1$H NMR ($CDCl_3$) 400MHz δ7.97(d, 2H, J=8.20 Hz), 7.92(d, 2H, J=8.89 Hz), 7.65(d, 2H, J=8.20 Hz), 7.22(d, 2H, J=8.89 Hz), 6.94(d, 2H, J=8.89 Hz), 6.73(d, 2H, J=8.89 Hz), 4.86(d, 1H, J .79 Hz), 4.80(d, 1H, J .96 Hz), 4.66(q, 1H, J=6.89 Hz), 4.26(d, 1H, J .87 Hz), 420(d, 1H, J .87 Hz), 2.62(s, 3H), 1.58(d, 3H, J=6.89 Hz),

MS($ES^-$) M-H= 626.00

**2-(4-{[(4-{[4-(5-Methyl-1,2,4-oxadiazol-3-yl)phenoxy]methyl}-2-phenyl-1,3-thiazol-5-yl)methyl]sulfanyl}phenoxy)propanoic acid**

$^1$H NMR ($CDCl_3$) 400MHzδ7.93(d, 2H, J=9.06 Hz), 7.85(m, 2H), 7.41(m, 3H), 7.24(d, 2H, J=8.89 Hz), 6.95 (d, 2H, J=9.06 Hz), 6.74(d, 2H, J=8.89 Hz), 4.82(s, 2H), 4.68(q, 1H, J=6.89 Hz), 4.25(d, 1H, J .87 Hz), 4.19(d, 1H, J . 87 Hz), 2.64(s, 3H), 1.61(d, 3H, J=6.89 Hz),

MS($ES^-$) M-H= 558.30

**2-{4-[({4-{[4-(4-Acetylphenyl)-1-piperazinyl]methyl}-2-[4-(trifluoromethyl)phenyl]-1,3-thiazol-5-yl}methyl)sulfanyl]phenoxy}-2-methylpropanoic acid**

$^1$H NMR ($CD_3OD$) 400MHz δ 8.04(d, 2H, J=8.10 Hz), 7.85(d, 2H, J=9.14 Hz), 7.72(d, 2H, J=8.10 Hz), 7.25 (d, 2H, J=8.79 Hz), 6.93(d, 2H, J=9.14 Hz), 6.81(d, 2H, J=8.79 Hz), 4.32(s, 2H), 3.47(s, 2H), 3.35(t, 4H, J=4.91 Hz), 2.59(t, 4H, J=4.91 Hz), 2.47(s, 3H), 1.47(s, 6H),

MS($ES^-$) M-H= 668.1

**2-{4-[({4-{[4-(4-Chlorophenyl)-1-piperazinyl]methyl}-2-[4-(trifluoromethyl)phenyl]-1,3-thiazol-5-yl}methyl)sulfanyl]phenoxy}-2-methylpropanoic acid**

$^1$H NMR ($CD_3OD$) 400MHz δ 8.05(d, 2H, J=8.10 Hz), 7.73(d, 2H, J=8.10 Hz), 7.24(d, 2H, J=8.79 Hz), 7.15 (d, 2H, J=8.97 Hz), 6.90(d, 2H, J=8.97 Hz), 6.80(d, 2H, J=8.79 Hz), 4.30(s, 2H), 3.57(s, 2H), 3.18(t, 4H, J=5.00 Hz), 2.77(t, 4H, J=5.00 Hz), 1.49(s, 6H),

CHN Analysis: Theory (C, 58.04%; H, 4.72%; N, 6.35%) Found (C, 57.65%; H, 4.80%; N, 6.13%)

**2-{4-[({4-(3-Methoxyphenyl)-1-piperazinyl]methyl}-2-[4-(trifluoromethyl)phenyl]-1,3-thiazol-5-yl}methyl)sulfanyl]phenoxy}-2-methylpropanoic acid**

$^1$H NMR ($CD_3OD$) 400MHz δ 7.98(d. 2H, J=7.93 Hz), 7.63(d, 2H, J=7.93 Hz), 7.12(m, 3H), 6.73(m, 2H), 6.47 (m, 1 H), 6.38(m, 2H), 4.18(s, 2H), 3.70(s, 3H), 3.50(s, 2H). 3.14(br s, 4H), 2.76(sbr , 4H), 1.49(s, 6H),

CHN Analysis: Theory (C, 60.26%; H, 5.21%; N, 6.39%) Found (C, 59.83%; H, 5.29%; N, 6.32%)

**2-(4-{[(2-(4-Fluorophenyl)-4-{[4-{phenoxycarbonyl)-1-piperazinyl]methyl}-1,3-thiazol-5-yl)methyl]sulfanyl}-2-methylphenoxy)-2-methylpropanoic acid**

$^1$H NMR ($CDCl_3$) 400MHz δ 7.93(m, 2H); 7.35(m, 3H), 7.19(m, 4H), 7.08(m, 2H), 6.69(br s, 1H), 4.27(s, 2H), 3.60(br s, 4H), 3.39(s, 2H), 2.54(br s, 4H), 2.14(s, 3H), 1.55(s, 6H),

MS($ES^-$) M-H= 634.1

**2-{4-[({4-{[4-(4-Methoxyphenyl)-1-piperazinyl]methyl}-2-[4-{trifluoromethyl)phenyl-1,3-thiazol-5-yl}methyl)sulfanyl]-2-methylphenoxy}-2-methylpropanoic acid**

$^1$H NMR ($CD_3OD$) 400MHz δ 8.05(br s, 2H), 7.66(d, 2H, J=8.28 Hz), 7.15(s, 1H), 6.84(m, 6H), 4.19(s, 2H), 3.44(s, 2H) 3.69(s, 3H), 3.10(m. 4H), 2.82(br s, 4H), 2.10(s, 3H), 1.52(s, 6H),

MS($ES^+$) M+H= 672.2

**2-{4-[({4-{[4-(4-Acetylphenyl)-1-piperazinyl]methyl}-2-[4-(trifluoromethyl)phenyl]-1,3-thiazol-5-yl}methyl)sulfanyl]-2-methylphenoxy}-2-methylpropanoic acid**

$^1$H NMR ($CD_3OD$) 400MHz δ 7.97(d, 2H, J=8.1 0 Hz), 7.80(d, 2H, J=8.42 Hz), 7.65(d, 2H, J=8.10 Hz), 7.16 (br s, 1H), 7.01 (br s, 1H), 6.84(d, 2H, J=8.42 Hz), 6.60(br s, 1H), 4.23(s, 2H), 3.44(s, 2H), 3.27(br s, 4H), 2.55(br s, 4H), 2.44(s, 3H), 2.11 (s, 3H), 1.52(s, 6H),

MS($ES^+$) M+H= 684.2

**2-{4-[({4-{[4-(3-Methoxyphenyl)-1-piperazinyl]methyl}-2-[4-(trifluoromethyl)phenyl]-1,3-thiazol-5-yl}methyl) sulfanyl]-2-methylphenoxy}-2-methylpropanoic acid**

$^1H$ NMR ($CD_3OD$) 400MHz δ 7.96(d, 2H, J=8.10 Hz), 7.61(d, 2H, J=8.10 Hz), 7.03(m, 3H), 6.38(m, 4H), 4.18 (s, 2H), 3.69(s, 3H), 3.33(s, 2H), 3.11(m, 4H), 2.66(br s, 4H), 2.09(s, 3H), 1.50(s, 6H),
**[0955]** MS($ES^-$) M-H= 670.0

**2-{4-[({4-{[4-(4-Fluorophenyl)-1-piperazinyl]methyl}-2-[4-(trifluoromethyl)phenyl]-1,3-thiazol-5-yl}methyl) sulfanyl]-2-methylphenoxy}-2-methylpropanoic acid**

$^1H$ NMR ($CD_3OD$) 400MHz δ 8.08(d, 2H, J=8.24 Hz), 7.73(d, 2H, J=8.24 Hz), 7.18(br s, 1 H), 7.04(br s, 1H), 6.92(m, 4H), 6.72(br s, 1H), 4.26(s, 2H), 3.58(s, 2H), 3.14(br s, 4H), 2.84(br s, 4H), 2.10(s, 3H), 1.60(s, 6H),
**[0957]** MS($ES^-$) M-H= 658.4

**2-Methyl-2-{2-methyl-4-[({4-{[4-(phenoxycarbonyl)-1-piperazinyl]methyl}-2-[4-(trifluoromethyl)phenyl]-1,3-thiazol-5-yl}methyl)sulfanyl]phenoxy}propanoic acid**

$^1H$ NMR ($CD_3OD$) 400MHz δ 8.04(br s, 2H), 7.71 (br s, 2H), 7.34(m, 2H), 7.19(m, 3H), 7.04(m, 3H), 4.28(s, 2H), 3.65(s, 2H), 3.45(br s, 4H), 2.47(br s, 4H), 2.12(s, 3H), 1.61(s, 6H),
**[0959]** MS($ES^-$) M-H= 684.0

**2-[4-({[4-{[4-(4-Acetylphenyl)-1-piperazinyl]methyl}-2-(4-fluorophenyl)-1,3-thiazol-5-yl]methyl}sulfanyl)-2-methylphenoxy]-2-methylpropanoic acid**

$^1H$ NMR ($CD_3OD$) 400MHz δ 7.93(m, 2H), 7.86(d, 2H, J=9.16 Hz), 7.18(m, 3H), 7.07(br s, 1H), 6.95(d, 2H, J=9.16 Hz), 6.69(br s, 1H), 4.23(s, 2H), 3.42(m, 6H), 2.69(br s, 4H), 2.49(s, 3H), 2.13(s, 3H), 1.56(s, 6H),
**[0961]** MS($ES^-$) M-H= 632.3

**2-(4-{[(2-(4-Fluorophenyl)-4-{[4-(3-methoxyphenyl)-1-piperazinyl]methyl}-1,3-thiazol-5-yl)methyl]sulfanyl}-2-methylphenoxy)-2-methylpropanoic acid**

$^1H$ NMR ($CD_3OD$) 400MHz δ 7.96(m, 2H), 7.19(m, 3H), 7.12(t, 1H, J=8.24 Hz), 7.01(br s, 1H), 6.66(br s, 1H), 6.54(dd, 1H, J=8.24, 2.20 Hz), 6.47(t, 1H, J=2.20 Hz), 6.43(dd, 1H, J=8.24, 2.20 Hz), 4.20(s, 2H), 3.73(s, 3H), 3.55(s, 2H), 3.24(br s, 4H), 2.91 (br s, 4H), 2.13(s, 3H), 1.56(s, 6H),
**[0963]** MS($ES^-$) M-H= 620.0

**2-[4-({[4-{[4-(Ethoxycarbonyl)-1-piperazinyl]methyl}-2-(4-fluorophenyl)-1,3-thiazol-5-yl]methyl}sulfanyl)-2-methylphenoxy]-2-methylpropanoic acid**

$^1H$ NMR ($CD_3OD$) 400MHz δ 7.94(m, 2H), 7.19(m, 3H), 7.00(br s, 1H), 6.66(br s, 1H), 4.23(s, 2H), 4.09(q, 2H, J=7.05 Hz), 3.48(m, 6H), 2.49(br s, 4H), 2.13(s, 3H), 1.56(s, 6H), 1.23(t, 3H, J=7.05 Hz),
**[0965]** MS($ES^-$) M-H= 586.2

**2-(4-{[(2-(4-Fluorophenyl)-4-{[4-(isopropoxycarbonyl)-1-piperazinyl]methyl}-1,3-thiazol-5-yl)methyl]sulfanyl}-2-methylphenoxy)-2-methylpropanoic acid**

$^1H$ NMR ($CDCl_3$) 400MHz δ 7.90(m, 2H), 7.18(m, 3H), 7.07(br s, 1H), 6.74(br s, 1H), 4.64(m, 1H), 4.26(s, 2H), 3.44(t, 4H, J=4.58 Hz), 3.36(s, 2H), 2.43(br s, 4H), 2.13(s, 3H), 1.55(s, 6H), 1.22(d, 6H, J=6.23 Hz),
**[0967]** MS($ES^-$) M-H= 600.0

**2-{2-methyl-4-[({4-[4-(trifluoromethoxy)benzyl]-2-[4-(trifluoromethyl)phenyl]-1,3-thiazol-5-yl}methyl)sulfanyl] phenoxy}propanoic acid**

From ethyl 2-{2-methyl-4-[({4-[4-(trifluoromethoxy)benzyl]-2-[4-(trifluoromethyl)phenyl]-1,3-thiazol-5-yl}methyl)sulfanyl]phenoxy}propanoate (0.167g, 0.25 mmol), 2-{2-methyl-4-[({4-[4-(trifluoromethoxy)benzyl]-2-[4-(trifluoromethyl)phenyl]-1,3-thiazol-5-yl}methyl)sulfanyl]phenoxy}propanoic acid (0.066g, 41%) was obtained as a white solid.
$^1H$ NMR ($CD_3OD$): δ 8.05 (d, 2 H), 7.77 (d, 2 H), 7.20 (m, 6 H), 6.71 (d, 1 H), 4.80 (q, 1H), 4.25 (s, 2 H), 3.93 (s, 2 H), 2.20 (s, 3 H), 1.60 (d, 3 H); $^{19}F$ NMR ($CD_3OD$): δ -59.87 (s) -64.72 (s); MS *m/z* 628 (M+1); Anal. Calcd. for

$C_{29}H_{23}FNOS_2$: C, 55.5; H, 3.69; N, 2.23%; found: C, 55.27; H, 3.80; N, 2.21%.

**{2-methyl-4-[({4-[4-(trifluoromethoxy)benzyl]-2-[4-(trifluoromethyl)phenyl]-1,3-thiazol-5-yl}methyl)sulfanyl] phenoxy}acetic acid**

From methyl {2-methyl-4-[({4-[4-(trifluoromethoxy)benzyl]-2-[4-(trifluoromethyl)phenyl]-1,3-thiazol-5-yl}methyl)sulfanyl]phenoxy}acetate (0.15g, 0.24 mmol), {2-methyl-4-[({4-[4-(trifluoromethoxy)benzyl]-2-[4-(trifluoromethyl) phenyl]-1,3-thiazol-5-yl}methyl)sulfanyl]phenoxy}acetic acid ( 0.053g, 36%) was obtained as a white solid.

$^1H$ NMR ($CD_3OD$): δ 8.05 (d, 2 H), 7.77 (d, 2 H), 7.20 (m, 6 H), 6.71 (d, 1 H), 4.70 (s, 2 H), 4.27 (s, 2 H), 3.94 (s, 2 H), 2.20 (s, 3 H); $^{19}F$ NMR ($CD_3OD$): δ -59.88 (s) -64.72 (s); MS *m/z* 614 (M+1); Anal. Calcd. for $C_{28}H_{21}F_6NO_4S_2$: C, 54.81; H, 3.45; N, 2.28%; found: C, 54.64; H, 3.46; N, 2.23%.

**2-{2-methyl-4-[({4-(3-thienylmethyl)-2-[4-(trifluoromethyl)phenyl]-1,3-thiazol-5-yl}methyl)sulfanyl]phenoxy} propanoic acid**

From ethyl 2-{2-methyl-4-[({4-(3-thienylmethyl)-2-[4-(trifluoromethyl)phenyl]-1,3-thiazol-5-yl}methyl)sulfanyl] phenoxy}propanoate (0.255g, 0.44 mmol), 2-{2-methyl-4-[({4-(3-thienylmethyl)-2-[4-(trifluoromethyl)phenyl]-1,3-thiazol-5-yl}methyl)sulfanyl]phenoxy}propanoic acid (0.058g, 24%) was obtained as a white solid.

$^1H$ NMR ($CD_3OD$): δ 8.05 (d, 2 H), 7.77 (d, 2 H), 7.33 (t, 1 H), 7.18 (m, 2 H), 6.95 (m, 2 H), 6.69 (d, 1 H), 4.80 (q, 1 H), 4.22 (s, 2 H), 3.95 (s, 2 H), 2.20 (s, 3 H), 1.61 (d, 3 H); MS *m/z* 550 (M+1); HPLC RT 4.056 (C18 4.2x100mm, 0-100% ACN/$H_2O$ (0.1% TFA), 6min @ 2ml/min @254/220nm). Anal. Calcd. for $C_{26}H_{22}F_3NO_3S_3$: C, 56.82; H, 4.03; N, 2.55%; found: C, 56.84; H, 4.16; N, 2.53%.

**{2-methyl-4-[({4-(3-thienylmethyl)-2-[4-(trifluoromethyl)phenyl]-1,3-thiazol-5-yl}methyl)sulfanyl]phenoxy} acetic acid**

From methyl {2-methyl-4-[({4-(3-thienylmethyl)-2-[4-(trifluoromethyl)phenyl]-1,3-thiazol-5-yl}methyl)sulfanyl] phenoxy}acetate (0.259g, 0.47 mmol), {2-methyl-4-[({4-(3-thienylmethyl)-2-[4-(trifluoromethyl)phenyl]-1,3-thiazol-5-yl} methyl)sulfanyl]phenoxy}acetic acid (0.138g, 55%) was obtained as a white solid.

$^1H$ NMR ($CD_3OD$): δ 8.05 (d, 2 H), 7.77 (d, 2 H), 7.33 (t, 1 H), 7.18 (m, 2 H), 6.95 (m, 2 H), 6.69 (d, 1 H), 4.70 (s, 2 H), 4.24 (s, 2 H), 3.95 (s, 2 H), 2.21 (s, 3 H); MS *m/z* 536 (M+1); HPLC RT 3.979 (C18 4.2x100mm, 0-100% ACN/ $H_2O$ (0.1% TFA), 6min @ 2ml/min @254/220nm). Anal. Calcd. for $C_{25}H_{20}F_3NO_3S_3$: C, 56.06; H, 3.76; N, 2.61%; found: C, 55.90; H, 3.88; N, 2.62%.

**2-{4-[({4-(2-furylmethyl)-2-[4-(trifluoromethyl)phenyl]-1,3-thiazol-5-yl}methyl)sulfanyl]-2-methylphenoxy} propanoic acid**

From ethyl 2-{4-[({4-(2-furylmethyl)-2-[4-(trifluoromethyl)phenyl]-1,3-thiazol-5-yl}methyl)sulfanyl]-2-methylphenoxy}propanoate (0.091g, 0.16 mmol), 2-{4-[({4-(2-furylmethyl)-2-[4-(trifluoromethyl)phenyl]-1,3-thiazol-5-yl}methyl)sulfanyl]-2-methylphenoxy}propanoic acid (0.019g, 22%) was obtained as a white solid.

$^1H$ NMR ($CD_3OD$): δ 8.05 (d, 2 H), 7.77 (d, 2 H), 7.37 (s, 1 H), 7.21 (s, 1 H), 7.17 (d, 1 H), 6.72 (d, 1 H), 6.31 (s, 1 H), 5.99 (s, 1 H), 4.80 (q, 1 H), 4.22 (s, 2 H), 3.97 (s, 2 H), 2.22 (s, 3 H), 1.63 (d, 3 H); MS *m/z* 534 (M+1); HPLC RT 3.929 (C18 4.2x100mm, 0-100% ACN/$H_2O$ (0.1% TFA), 6min @ 2ml/min @254/220nm). Anal. Calcd. for $C_{26}H_{22}F_3NO_4S_2$: C, 58.53; H, 4.16; N, 2.62%; found: C, 58.04; H, 4.76; N, 2.47%

**2-{4-[({4-(3-furylmethyl)-2-[4-(trifluoromethyl)phenyl]-1,3-thiazol-5-yl}methyl)sulfanyl]-2-methylphenoxy} propanoic acid**

From ethyl 2-{4-[({4-(3-furylmethyl)-2-[4-(trifluoromethyl)phenyl]-1,3-thiazol-5-yl}methyl)sulfanyl]-2-methylphenoxy}propanoate (0.177g, 0.32 mmol), 2-{4-[({4-(3-furylmethyl)-2-[4-(trifluoromethyl)phenyl]-1,3-thiazol-5-yl}methyl)sulfanyl]-2-methylphenoxy}propanoic acid (0.030g, 18%) was obtained as a white solid.

$^1H$ NMR ($CD_3OD$): δ 8.05 (d, 2 H), 7.77 (d, 2 H), 7.39 (s, 1 H), 7.20 (m, 3 H), 6.70 (d, 1 H), 6.29 (s, 1 H), 4.80 (q, 1H), 4.22 (s, 2 H), 3.70 (s, 2 H), 2.20 (s, 3 H), 1.62 (d, 3 H); MS *mlz* 534 (M+1); HPLC RT 3.966 (C18 4.2x100mm, 0-100% ACN/$H_2O$ (0.1% TFA), 6min @ 2ml/min @254/220nm). Anal. Calcd. for $C_{26}H_{22}F_3NO_4S_2$: C, 58.53; H, 4.16; N, 2.62%; found: C, 58.38; H, 4.30; N, 2.54%

**2-{2-methyl-4-[({4-(2-thienylmethyl)-2-[4-(trifluoromethyl)phenyl]-1,3-thiazol-5-yl}methyl)sulfanyl]phenoxy} propanoic acid**

From ethyl 2-{4-[({4-(2-thienylmethyl)-2-[4-(trifluoromethyl)phenyl]-1,3-thiazol-5-yl}methyl)sulfanyl]-2-methylphenoxy}propanoate (0.21g, 0.36 mmol), 2-{2-methyl-4-[({4-(2-thienylmethyl)-2-[4-(trifluoromethyl)phenyl]-1,3-thiazol-5-yl}methyl)sulfanyl]phenoxy}propanoic acid (0.019g, 10%) was obtained as a white solid.
$^1H$ NMR ($CD_3OD$): δ 8.05 (d, 2 H), 7.77 (d, 2 H), 7.20 (m, 3 H), 6.91 (t, 1 H), 6.79 (s, 1 H), 6.69 (d, 1 H), 4.80 (q, 1 H), 4.24 (s, 2 H), 4.09 (s, 2 H), 2.20 (s, 3 H), 1.62 (d, 3 H); MS *m/z* 550 (M+1); HPLC RT 4.074 (C18 4.2x100mm. 0-100% ACN/$H_2O$ (0.1% TFA), 6min @ 2ml/min @254/220nm).

**2-methyl-2-{4-[({4-[4-(trifluoromethoxy)benzyl]-2-[4-(trifluoromethyl)phenyl]-1,3-thiazol-5-yl}methyl)sulfanyl] phenoxy}propanoic acid**

From ethyl 2-methyl-2-{4-[({4-[4-(trifluoromethoxy)benzyl]-2-[4-(trifluoromethyl)phenyl]-1,3-thiazol-5-yl}methyl)sulfanyl]phenoxy}propanoate (0.210g, 0.32 mmol), 2-methyl-2-{4-[({4-[4-(trifluoromethoxy)benzyl]-2-[4-(trifluoromethyl)phenyl]-1,3-thiazol-5-yl}methyl)sulfanyl]phenoxy}propanoic acid (0.035g, 17%) was obtained as a cream solid.
$^1H$ NMR ($CD_3Cl_3$): δ 8.05 (d, 2 H), 7.77 (d, 2 H), 7.28 (d, 2 H), 7.22 (d, 2 H), 7.13 (d, 2 H), 6.86 (d, 2 H), 4.19 (s, 2 H), 3.96 (s, 2 H), 1.63 (s, 6 H); $^{19}F$ NMR ($CD_3Cl_3$): δ -58.26 (s) -63.16 (s); MS *m/z* 628 (M+1); HPLC RT 4.526 (C18 4.2x100mm, 0-100% ACN/$H_2O$ (0.1 % TFA), 6min @ 2ml/min @254/220nm). Anal. Calcd. for $C_{29}H_{23}F_6NO_4S_2$: C, 55.5; H, 3.69; N, 2.23%; found: C, 55.78; H, 3.83; N, 2.10%

**{2-Methyll-4-[({4-[(4-methyl-2-thienyl)methyl]-2-[4-(trifluoromethyl)phenyl]-1,3-thiazol-5-yl}methyl)sulfanyl] phenoxy}acetic acid**

From ethyl {2-methyl-4-[({4-[(4-methyl-2-thienyl)methyl]-2-[4-(trifluoromethyl)phenyl]-1,3-thiazol-5-yl}methyl) sulfanyl]phenoxy}acetate (0.13g, 0.23 mmol), {2-methyl-4-[({4-[(4-methyl-2-thienyl)methyl]-2-[4-(trifluoromethyl)phenyl]-1,3-thiazol-5-yl}methyl)sulfanyl]phenoxy}acetic acid (0.011g, 9%) was obtained as a cream solid.
$^1H$ NMR ($CD_3Cl_3$): δ 8.01 (d, 2 H), 7.68 (d. 2 H), 7.24 (s, 1 H), 7.15 (d, 2 H), 6.72 (s, 1 H), 6.64 (d, 1 H), 4.75 (s, 2 H), 4.19 (s, 2 H), 4.05 (s, 2 H), 2.20 (s, 3 H), 2.29 (s, 3 H); MS *m/z* 550 (M+1); HPLC RT 4.366 (C18 4.2x100mm, 0-100% ACN/$H_2O$ (0.1 % TFA), 6min @ 2ml/min @254/220nm).

**{4-[({4-(2,4-difluorobenzyl)-2-[4-(trifluoromethyl)phenyl]-1,3-thiazol-5-yl}methyl)sulfanyl]-2-methylphenoxy} acetic acid**

From ethyl {4-[({4-(2,4-difluorobenzyl)-2-[4-(trifluoromethyl)phenyl]-1,3-thiazol-5-yl}methyl)sulfanyl]-2-methylphenoxy}acetate, (0.1g, 0.17 mmol), {4-[({4-(2,4-difluorobenzyl)-2-[4-(trifluoromethyl)phenyl]-1,3-thiazol-5-yl}methyl) sulfanyl]-2-methylphenoxy}acetic acid (0.027g, 28%) was obtained as a cream solid.
$^1H$ NMR ($CD_3Cl_3$): δ 7.99 (d, 2 H), 7.68 (d, 2 H), 7.22 (s, 1 H), 7.13 (m, 2 H), 6.79 (m, 2 H), 6.62 (d, 1 H), 4.70 (s, 2 H), 4.20 (s, 2 H), 3.86 (s, 2 H), 2.23 (s, 3 H); $^{19}F$ NMR ($CD_3Cl_3$): δ -63.15 (s) - 114.03 (s) -114.06 (s); MS *m/z* 566 (M+1); HPLC RT 4.356 (C18 4.2x100mm, 0-100% ACN/$H_2O$ (0.1 % TFA), 6min @ 2ml/min @254/220nm). Anal. Calcd. for $C_{27}H_{20}F_5NO_3S_2.0.5H_2O$: C, 56.44; H, 3.68; N, 2.44%; found: C, 56.40; H, 3.79; N, 2.20%

**{4-[({4-(4-methoxybenzyl)-2-[4-(trifluoromethyl)phenyl]-1,3-thiazol-5-yl}methyl)sulfanyl]-2-methylphenoxy} acetic acid**

From ethyl {4-[({4-(4-methoxybenzyl]-2-[4-(trifluoromethyl)phenyl]-1,3-thiazol-5-yl}methyl)sulfanyl]-2-methylphenoxy}acetate (0.160g 0.27 mmol), {4-[({4-(4-methoxybenzyl)-2-[4-(trifluoromethyl)phenyl]-1,3-thiazol-5-yl}methyl) sulfanyl]-2-methylphenoxy}acetic acid (0.005g, 3%) was obtained as a cream solid.
$^1H$ NMR ($CD_3Cl_3$): δ 8.01 (d, 2 H), 7.68 (d, 2 H). 7.23 (s, 1 H), 7.11 (m, 3 H), 6.82 (d, 2 H), 6.62 (d, 1 H), 4.90 (s, 2.H), 4.17 (s, 2 H), 3.90 (s, 2 H), 3.80 (s, 3 H), 2.25 (s, 3 H); MS *m/z* 560.

**2-Methyll-2-{4-[({4-[(4-methyl-2-thienyl)methyl]-2-[4-(trifluoromethyl)phenyl]-1,3-thiazol-5-yl}methyl)sulfanyl] phenoxy}propanoic acid**

From ethyl 2-methyl-2-{4-[({4-[(4-methyl-2-thienyl)methyl]-2-[4-(trifluoromethyl)phenyl]-1,3-thiazol-5-yl}methyl)sulfanyl]phenoxy}propanoate (0.17g 0.29 mmol), 2-methyl-2-{4-[({4-[(4-methyl-2-thienyl)methyl]-2-[4-(trifluoromethyl)phenyl]-1,3-thiazol-5-yl}methyl)sulfanyl]phenoxy}propanoic acid (0.002g, 1.2%) was obtained as a cream solid.

$^1H$ NMR ($CD_3Cl_3$): δ 8.01 (d, 2 H), 7.78 (d, 2 H), 7.28 (d, 2 H), 6.86 (d 2 H), 6.73 (s, 1 H), 6.63 (s, 1 H), 4.18 (s, 2 H), 3.99 (s, 2 H), 2.21 (s, 3 H), 1.63 (s, 6 H); MS *m/z* 564 (M+1); HPLC RT 4.413 (C18 4.2x100mm, 0-100% ACN/$H_2O$ (0.1% TFA), 6min @ 2m)/min @254/220nm).

The following is an alternative procedure for the synthesis of Ethyl 2-{4-[({4-(hydroxymethyl)-2-[4-(trifluoromethyl)phenyl]-1,3-thiazol-5-yl}methyl)sulfanyl}phenoxy}-2-methylpropanoate

**Ethyl 2-[4-(chlorosulfonyl)phenoxy]-2-methylpropanoate**

Cool a solution of the ethyl 2-methyl-2-phenoxypropanoate, (1.0 wt, 1.0 eq), in dichloromethane (7.5 vols) to 0°C with stirring under a nitrogen atmosphere. Slowly add neat chlorosulfonic acid (0.78 wt, 1.4 eq) to the reaction mixture at a rate such that the reaction temperature never rises above 5.0 °C. The addition typically takes 30 minutes to complete. Following the completion of the addition, stir the reaction mixture at 0-1°C. Follow the course of the reaction by HPLC. The reaction is typically complete after 30 minutes. At this point, slowly treat the reaction mixture with DMF (1.75 L) (1.40 wt, 4.0 eq). The addition of DMF to the reaction mixture is very exothermic. Adjust the rate of addition so that the reaction temperature never rises above 10.0 °C. The addition of DMF to the reaction mixture takes approximately 30 minutes. Following the completion of the DMF addition, re-cool the reaction mixture to 0.5 to 1°C. Treat the cooled reaction mixture with neat thionyl chloride (619 mL, 1.01 kg) (0.86 wt, 1.5 eq). Adjust the rate of addition so that the process temperature never reaches 5°C. The addition of thionyl chloride to the reaction mixture is not very exothermic at all. Hence, the addition of thionyl chloride is typically complete in 5 minutes. Following the completion of the DMF addition, warm the reaction mixture to 20°C with stirring. Follow the course of the reaction via HPLC. After 2.0 h, the reaction is typically complete. At this point. cool the reaction mixture to 0-1 °C and carefully treat the reaction mixture with water (8.8 L) (7.5 vols). [Note: The addition of water may be somewhat exothermic depending upon how much unreacted thionyl chloride is left in the reaction mixture.] Separate the organic layer and wash the organic layer with aqueous 0.1 N HCl solution (2 X 7.5 vols). Separate the organic layer, concentrate the organic layer to a minimum stir volume, treat the organic layer with isopropyl acetate (1 X 5.0 vols) and then concentrate the resulting solution via vacuum distillation to afford the titled compound as a translucent bronze colored oil.

Yield (% theory): 85-98%.

$^1H$ NMR (400 MHz, $CDCl_3$) δ 7.90 (2H, bd), 6.90 (2H, bd), 4.22 (2H, q, J=7.0 Hz), 1.67 (6H, s), 1.20 (3H, t, J=7.0 Hz)

**Diethyl 2-[4-(trifluoromethyl)phenyl]-1,3-thiazole-4,5-dicarboxylate**

Heat a solution of the 4-fluorobenzenecarbothioamide, (1.0 wt, 1.0 eq), in absolute ethanol (3 vols) to 50 °C with stirring under a nitrogen atmosphere. Add diethyl 2-chloro-3-oxosuccinate (1.2 wt, 1.1 eq), in one portion. Some warming is seen during the addition which is typically complete in less then 30 minutes. After the addition is complete, heat the reaction mixture to about 68°C. Hold the reaction mixture at 67-69°C for 6 h and then cool the reaction mixture to ambient temperature overnight. Dilute the resulting yellow hazy solution slowly with aqueous 50% ethanol solution (3 vols), stir at ambient temperature for 4h, and then cool the reaction mixture to <5°C. Filter the solids. Wash the wet cake with aqueous 50% ethanol solution (3 vols) and dry at 45 °C to constant weight to afford the title compound as an off-white to white colored solid.

Yield (% theory): 78-83%.

$^1H$ NMR (300 MHz, $CDCl_3$) δ 8.14 (2H, d, J=8.2Hz), 7.76 (2H, d, J=8.2Hz), 4.52 (2H, q, J=7.1 Hz), 4.43 (2H, q, J=7.1 Hz), 1.47 (3H, t, J=7.1Hz), 1.42 (3H, t, J=7.1Hz).

**{5-Hydroxymethyl-2-[4-(trifluoromethyl)phenyl]-1,3-thiazol-4-yl}methanol**

To a suspension of lithium aluminum hydride (0.14 wt) in THF (3.4 vols), add a solution of the diethyl 2-[4-(trifluoromethyl)phenyl]-1,3-thiazole-4,5-dicarboxylate (1.0 wt, 1.0 eq), dissolved in THF (2 vols) at a rate such that the temperature of the reaction mixture is maintained at below -10°C. The addition time is 1.5-3.0 hr. After the addition is complete, stir the reaction mixture at ambient temperature for 18 h. Quench the reaction by adding aqueous 16% sulfuric acid (2.4 vols). Charge ethyl acetate (5 vols) with stirring to the reaction mixture followed with water (5 vols). Filter the resulting two phase mixture through celite (0.4 wt). Separate the layers and wash the organic layer with water (4 X 4 vols) and with brine (2 X 4 vol). Reduce the total volume of the reaction mixture via vacuum distillation to leave the solid suspended in ethyl acetate (1-1.5 vols). Dilute the slurry with dichloromethane (5 vols) and stir the suspension for at least 6 h. Filter the tan-colored solid. Wash the wet cake with dichloromethane ( 2 vols) and dry the wet cake at 45°C under mild vacuum to afford the title compound as an off-white solid.

Yield (% theory): 65-85%.

$^1H$ NMR (300 MHz, $CD_3OD$) δ 8.15(2H, d, J=8.3Hz), 7.79(2H, d, J=8.3Hz), 4.92 (2H, s), 4.90 (2H, s), 4.77(2H, s).

**Ethyl 2-{4-[({4-(hydroxymethyl)-2-[4-(trifluoromethyl)phenyl]-1,3-thiazol-5-yl}methyl)-sulfanyl]phenoxy}-2-methylpropanoate.**

To a stirred suspension of zinc dust (0.75 wt, 3.5 eq) in isopropyl acetate (5 vols), add a solution of DME (0.5 vol) and water (0.5 eq). Heat the resulting solution from room temperature to 40°C. Treat the reaction mixture with a solution of ethyl 2-[4-(chlorosulfonyl)phenoxy]-2-methylpropanoate (1.0 wt, 1.0 eq) and dichlorodimethylsilane (0.32 wt, 0.75 eq) in isopropyl acetate (3 vols) over a period of 2 h as this addition is mildly exothermic. After the addition is complete, increase the process temperature to 60°C. Treat the suspension at 60°C slowly with neat dichlorodimethylsilane (0.95 wt, 2.3 eq) over a period of 1 h. When the reduction. of the sulfonylchloride is deemed complete (by HPLC), treat the reaction mixture with (5-Hydroxymethyl-2-[4-(trifluoromethyl)phenyl]-1,3-thiazol-4-yl}methanol (1.04 wt, 1.1 eq) in one portion at 60°C. After the addition is complete, increase the process temperature to 89°C and stir the reaction mixture at this temperature for 3 to 5 h then cool to ambient temperature. Filter the reaction mixture to remove unreacted zinc residue, wash the filtrate with water (2 X 8 vols) and concentrate the organic layer to about 3.5 volumes via vacuum distillation at 40-45°C. Dissolve the resultant, somewhat syrupy, residue in ethanol (2 vols) and treat the resulting solution with iso-octane (2vols). Cool the clear yellow-tinted solution to ambient temperature to induce crystallization of the product. Collect the solid via filtration. Wash the wet cake with iso-octane/EtOH (9:1, 1 vol) and dry under vacuum (~21 Torr) at 60 °C for 12 h to afford the title compound as an off-white solid.

Yield (% theory): 45-55%.

$^1H$ NMR (400 MHz, $CDCl_3$) δ 7.96 (2H, d, J=8.5 Hz), 7.66 (2H, d, J=8.5 Hz), 7.24 (2H, d, J=8.8 Hz), 6.74 (2H, d, J=8.8 Hz), 4.45 (2H, d, J=3.5 Hz), 4.19 (2H, q, J=7.2 Hz), 4.16 (2H, s), 2.30 (1 H, br s), 1.57 (6H, s), 1.20 (3H, t, J=7.2 Hz).

The following intermediates and ligands were prepared for the binding and transfection assays described below:

**i) 2-{2-methyl-4-[({4-methyl-2-[4-(trifluoromethyl)phenyl]-1,3-thiazol-5-yl}methyl)sulfanyl]phenoxy}acetic acid**

This compound was used as a PPARdelta reference in the transfection assays described below and was prepared according to the following method:

**Intermediate A**

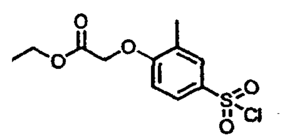

Chlorosulfonic acid (15mL) was cooled to 0°C. then 10.0 g (0.05M) of ethyl (2-methylphenoxyacetate was added over 10 m. The reaction mixture was stirred at 0-5°C for 30m, the bath was removed and stirring continued for 2 h. The reaction mixture was poured into ice, forming a white solid which was washed with ice water and dried under high vacuum affording the title compound (12.846 g ,86%).

**Intermediate B**

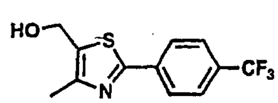

To a well stirred solution of $LiAlH_4$ (1.52 g, 40 mmol) in dry THF (50 mL) at 0 °C, was slowly added a solution of ethyl 4-methyl-2-[4-(trifluoromethyl)phenyl]-thiazole-5-carboxylate (12.6 g, 40 mmol) in dry THF (50 mL). The mixture

was stirred at room temperature for 2 hs. The reaction was quenched by slow addition at 0 °C of water (2 mL), 5N NaOH (2 mL) and water (6 mL). The precipitate was filtered, washed with EtOAc, MeOH, $CH_2Cl_2$ and THF. After evaporation, a yellow solid was obtained, that was crystallyzed from MeOH-water to afford intermediate 1 depicted above (9.90 g, 36 mmol, 90%) as a yellow solid mp 120-122 °C.

**Intermediate C:**

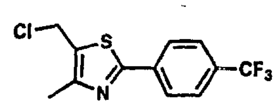

To a cold (0°C) stirred solution of intermediate 1 (8.2g, 30 mmol) and $Et_3N$ (6.07 g, 8.36 mL, 60 mmol), in dry $CH_2Cl_2$ (120 mL) was slowly added $MeSO_2Cl$ (5.49 g, 3.71 mL, 48 mmol). After 2 hs at 0°C more $Et_3N$ (6 mmol) and $MeSO_2Cl$ (4.8 mmol) were added. After 2 more h a tlc (hexane:EtOAc, 1:1) showed complete reaction. The reaction mixture was diluted with $CH_2Cl_2$ (120 mL) and washed with $NaHCO_3$ (sat.) (2 x 240 mL) and water (2 x 240 mL), dried, filtered and evaporated to afford intermediate 2 (8.0 g, 27 mmol, 90%) as a yellow solid.

**2-{2-methyl-4-[({4-methyl-2-[4-(trifluoromethyl)phenyl]-1,3-thiazol-5-yl}methyl)sulfanyl]phenoxy}acetic acid:**

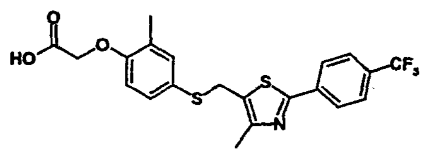

Intermediate A (4.68g, 16mM) was refluxed with 9.6 g of tin powder in ethanol (20mL) and dioxane/HCl (20 mL). After 3 h the reaction mixture was poured into ice and $CH_2Cl_2$ (200mL) and filtered. The phases were separated and the aqueous layer was extracted 2X 50 mL $CH_2Cl_2$. The combined organic layers were dried ($MgSO_4$), filtered and evaporated to yield 3.5g (97%). This material readily forms disulfides and therefore was used immediately. It was dissolved in acetonitrile (50mL) with intermediate C (4.0g, 14.0mM) and $Cs_2CO_3$ (10.1g. 31.0 mM) and stirred for 1 h then diluted with ether (200mL) and water (200mL). The phases were separated and the organic phase was washed 2X NaOH 0.1 N (50mL), dried ($MgSO_4$) filtered and evaporated to afford crude product (6.57 g, ) which was slurried in hexane:ether (1:1) and filtered to yield pure intermediate D (5.0g, 74%). This material was hydrolyzed as described below to prepare the title compound. A solution of the corresponding ester (Intermediate D) (1 mmol) in THE (10 mL) (in some cases few drops of MeOH were added to help solubility), was treated with 1 N LiOH in water (2mL, 2 mmol), and stirred 16 h at room temperature (when reactions were slow, the temperature was elevated to 50°C). The solution was neutralized with 1 N HCl (2 mL, 2 mmol) and the organic solvent evaporated to afford an aqueous solution with an insoluble product. If the insoluble was a solid, it was filtered and dried to afford the final product. If the insoluble was an oil, it was extracted with EtOAc (30 mL). The organic solution was separated, washed with water (2 x 30 mL), dried, filtered, and evaporated to afford the final product.

**Binding Assay:**

Compounds were tested for their ability to bind to hPPAR gamma hPPARalpha or PPARdelta using a Scintillation Proximity Assay (SPA). The PPAR ligand binding domain (LBD) was expressed in E. coli as polyHis tagged fusion proteins and purified. The LBD was then labeled with biotin and immobilized on streptavidin-modified scintillation proximity beads. The beads were then incubated with a constant amount of the appropriate radioligand (3H-BRL 49653 for PPARgamma, radiolabelled 2-(4-(2 -(2,3-Ditritio-1-heptyl-3-(2,4-difluorophenyl)ureido)ethyl)phenoxy)-2-methylbutanoic acid for hPPAR alpha (see WO 00/08002) and labelled GW 2433 (see Brown, P. J et al. *Chem. Biol.,* 4, 909-918 (1997). For the structure and synthesis of this ligand) for PPAR delta) and variable concentrations of test compound, and after equilibration the radioactivity bound to the beads was measured by a scintillation counter. The amount of

nonspecific binding, as assessed by control wells containing 50 μM of the corresponding unlabeled ligand, was subtracted from each data point. For each compound tested, plots of ligand concentration vs. CPM of radioligand bound were constructed and apparent Ki values were estimated from nonlinear least squares fit of the data assuming simple competitive binding. The details of this assay have been reported elsewhere (see, Blanchard, S. G. et. al. Development of a Scintillation Proximity Assay for Peroxisome Proliferator-Activated Receptor gamma Ligand Binding Domain. *Anal. Biochem.*, 257, 112-119 (1998)).

**Transfection assay:**

Compounds were screened for functional potency in transient transfection assays in CV-1 cells for their ability to activate the PPAR subtypes (transactivation assay). A previously established chimeric receptor system was utilized to allow comparison of the relative transcriptional activity of the receptor subtypes on the same target gene and to prevent endogenous receptor activation from complicating the interpretation of results. See, for example, Lehmann, J. M.; Moore, L. B.; Smith-Oliver, T. A.; Wilkison, W. O.; Willson, T. M.; Kliewer, S. A., An antidiabetic thiazolidinedione is a high affinity ligand for peroxisome proliferator-activated receptor gamma (PPARgamma), *J. Biol. Chem.*, 270, 12953-6 (1995). The ligand binding domains for murine and human PPAR alpha, PPAR gamma, and PPAR delta were each fused to the yeast transcription factor GAL4 DNA binding domain. CV-1 cells were transiently transfected with expression vectors for the respective PPAR chimera along with a reporter construct containing five copies of the GAL4 DNA binding site driving expression of secreted placental alkaline phosphatase (SPAP) and beta-galactosidase. After 16 h, the medium was exchanged to DME medium supplemented with 10% delipidated fetal calf serum and the test compound at the appropriate concentration. After an additional 24h, cell extracts were prepared and assayed for alkaline phosphatase and □-galactosidase activity. Alkaline phosphatase activity was corrected for transfection efficiency using the beta-galactosidase activity as an internal standard (see, for example, Kliewer, S. A., et, al. Cell 83, 813-819 (1995)). Rosiglitazone (BRL 49653) was used as a positive control in the hPPAR gamma assay. The positive control for PPAR delta assays was 2-{2-methyl-4-[({4-methyl-2-{trifluoromethyl)phenyl]-1,3-thiazol-5-yl}methyl)sulfanyl]phenoxy}acetic acid.

The positive control in the hPPARalpha transfection assay was 2-[4-(2-(3-(4-fluorophenyl)-1-heptylureido) ethyl)-phenoxy]-2-methylpropionic acid, which can be prepared as described in Brown, Peter J., et. al. *Synthesis* Issue 7, 778-782 (1997), or patent publication WO 9736579.

All of the above examples of this invention were agonists of at least one hPPAR subtype.

## Claims

**1.** A compound of formula (1) or a pharmaceutically acceptable salt, solvate, or hydrolyzable ester thereof wherein:

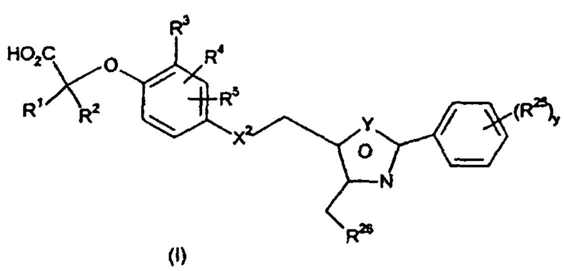

(I)

$R^1$ and $R^2$ are independently hydrogen or $C_{1-3}$ alkyl;
$X^2$ is O, S, or $CH_2$;
$R^3$, $R^4$, and $R^5$ are independently H, $C_{1-3}$alkyl, $OCH_3$, $CF_3$, $OCF_3$, CN, allyl, or halogen; Y is S or O;
each $R^{25}$ is independently $CH_3$, $OCH_3$, $CF_3$, or halogen;
y is 0, 1, 2, 3, 4 or 5; and
$R^{26}$ is selected from the group consisting of the moieties **A** through **K** depicted below:

A

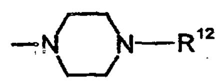

wherein $R^{12}$ is selected from the group consisting of $C_{1-6}$alkyl, $C_{1-6}$alkylenearyl, and the moieties depicted below in Group II,

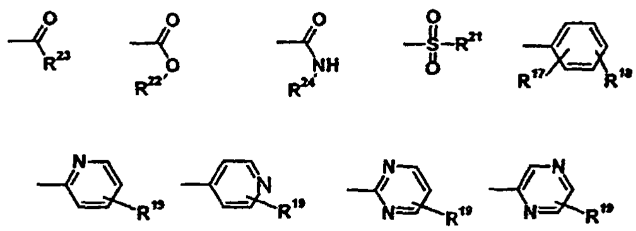

Group II

wherein $R^{17}$ and $R^{18}$ are independently hydrogen, halogen, hydroxy, -CN, $C_{1-6}$alkyl, $C_{1-6}$ perfluoroalkyl, $C_{1-6}$acyl, -O$C_{1-6}$alkyl, perfluoroO$C_{1-6}$alkyl, or $C_{1-6}$hydroxyalkyl;

$R^{19}$ is hydrogen or $C_{1-6}$alkyl;

$R^{21}$ is $C_{1-6}$alkyl, -$C_{1-6}$alkylenearyl, aryl, or -aryl-heteroaryl;

$R^{22}$ is $C_{1-6}$alkyl, aryl, or -$C_{1-6}$alkylenearyl;

$R^{23}$ is $C_{1-6}$alkyl, $C_{3-6}$cycloalkyl, or aryl;

$R^{24}$ is $C_{1-6}$alkyl, -$C_{1-6}$alkylenearyl, $C_{3-6}$cycloalkyl, or aryl;

B

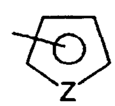

wherein Z is O, N or S (note that when Z is N, the depicted bond can be attached to the nitrogen in the ring as well as any of the carbons in the ring);

C

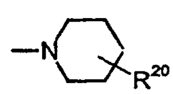

wherein $R^{20}$ is $C_{1-6}$alkyl, aryl, -O$C_{1-6}$alkyl, hydroxy, $C_{1-6}$hydroxyalkyl, or 1-alkoxy$C_{1-6}$alkyl;

D

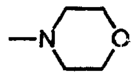

E

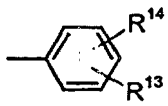

wherein $R^{13}$ and $R^{14}$ are independently hydrogen, halogen, CN, perfluoro$C_{1-6}$alkyl, perfluro$OC_{1-6}$alkyl, $C_{1-6}$alkyl, -$OC_{1-6}$alkyl, -$C_{1-6}$alkylene$OC_{1-6}$alkyl, -$SC_{1-6}$alkyl, or aryl;

F

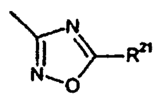

wherein $R^{21}$ is independently as defined above;

G

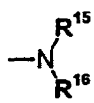

wherein $R^{15}$ and $R^{16}$ are independently hydrogen, $C_{1-6}$alkyl, $C_{3-6}$cycloalkyl optionally substituted with 1 or 2 $C_{1-3}$alkyl groups, or $R^{12}$ as defined above;

H

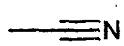

I

$—(CH_2)nPh$

wherein n is 1-3

J

$—O—R^{21}$

wherein $R^{21}$ is independently as defined above; and

**K**

$$-S-R^{21}$$

wherein $R^{21}$ is independently as defined above.

2. A compound according to claim 1 wherein $R^1$ and $R^2$ are independently H or $CH_3$.

3. A compound according to claim 2 wherein $R^1$ and $R^2$ are either both H or both $CH_3$.

4. A compound according to any of claims 1-3 wherein $X^2$ is O or S.

5. A compound according to any of claims 1-4 wherein $R^3$ is $CH_3$ or H.

6. A compound according to any of claims 1-5 wherein $R^4$ and $R^5$ are H.

7. A compound according to any preceding claim wherein Y is S.

8. A compound according to any of claims 1-8 wherein y is 1 or 2.

9. A compound according to claim 8 wherein each $R^{25}$ is independently halogen or $CF_3$.

10. A compound according to any preceding claim wherein $R^{26}$ is selected from the group consisting of

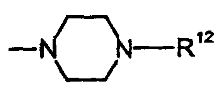 , 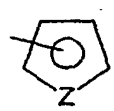 , and 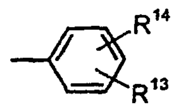

wherein $R^{12}$, Z, $R^{13}$, and $R^{14}$ are as defined in Claim 1.

11. A compound according to any preceeding claim wherein $R^{13}$ and $R^{14}$ are independently fluorine, bronime, phenyl, thienyl, $CF_3$, $OCF_3$, $OCH_3$, $SCH_3$, or t-butyl, $R^{17}$ and $R^{18}$ are independently hydrogen, OH, CN, $OC_{1-3}$alkyl, halogen, $CF_3$, $COCH_3$, $CH(OH)CH_3$, or $OCF_3$, $R^{21}$ is phenyl optionally substituted by methyl or CN, -$C_{1-3}$alkylenephenyl, or phenyl-5-methyl-1,2,4-oxadiazol-3-yl, $R^{22}$ is $C_{1-6}$ alkyl, phenyl, or benzyl, $R^{23}$ is $C_{1-6}$alkyl, furanyl, thienyl, phenyl optionally substituted by a halogen a methoxy or a dimethylamino group, methoxymethylcyclopropyl, or $C_{3-6}$cyclalkyl, and $R^{24}$ is H, $C_{1-6}$alkyl, cyclohexyl, m-methoxyphenyl, p-fluorophenyl, or -$CH_2CH_2$phenyl.

12. A compound accordina to Claim 11 wherein $R^{26}$ is

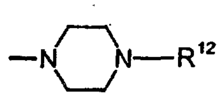

and $R^{12}$ is selected from the moieties shown in Group IV.

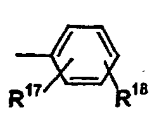 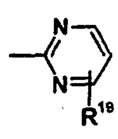 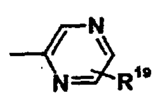

Group IV

**13.** A compound according to Claim 12 wherein $R^{17}$ is fluorine, chlorine, $OC_{1-3}$alkyl or $COCH_3$ and $R^{18}$ is $OCH_3$ or hydrogen, and $R^{19}$ is hydrogen.

**14.** A compound according to Claim 10 wherein $R^{26}$ is

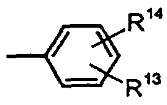

**15.** A compound according to Claim 14 wherein $R^{14}$ is thienyl, $OCH_3$, $OCF_3$, $CF_3$, or fluorine, and $R^{13}$ is hydrogen or fluorine.

**16.** A compound of formula (I) selected from:

2-[4-({[4-{[4-(4-acetylphenyl)-1-piperazinyl]methyl}-2-(4-fluorophenyl)-1,3-thiazol-5-yl]methyl}sulfanyl)-2-methylphenoxy]-2-methylpropanoic acid,
2-methyl-2-{2-methyl-4-[({4-[4-(methylsulfanyl)benzyl]-2-[4-(trifluoromethyl)phenyl]-1,3-thiazol-5-yl}methyl) sulfanyl]phenoxy}propanoic acid,
{2-methyl-4-[({4-(3-thienylmethyl)-2-[4-(trifluoromethyl)phenyl]-1,3-thiazol-5-yl}methyl)sulfanyl]phenoxy}acetic acid,
{4-[({4-benzyl-2-[4-(trifluoromethyl)phenyl]-1,3-thiazol-5-yl}methyl)sulfanyl]-2,5-dimethylphenoxy}acetic acid,
2-{4-[({4-{[4-(4-acetylphenyl)-1-piperazinyl]methyl}-2-[4-(trifluoromethyl)phenyl]-1,3-thiazol-5-yl}methyl)sulfanyl]-2-methylphenoxy}propanoic acid,
2-{4-[({4-{[4-(4-acetylphenyl)-1-piperazinyl]methyl}-2-[4-(trifluoromethyl)phenyl]-1,3-thiazol-5-yl}methyl)sulfanyl]-2-ethylphenoxy}propanoic acid,
2-{2-methyl-4-[({4-(2-thienylmethyl)-2-[4-(trifluoromethyl)phenyl]-1,3-thiazol-5-yl}methyl)sulfanyl]phenoxy} propanoic acid,
2-{4-[({4-{[4-(4-methoxyphenyl)-1-piperazinyl]methyl}-2-[4-(trifluoromethyl)phenyl]-1,3-thiazol-5-yl}methyl) sulfanyl]-2-methylphenoxy}propanoic acid,
2-{4-[({4-{[4-(4-ethoxyphenyl)-1-piperazinyl]methyl}-2-[4-(trifluoromethyl)phenyl]-1,3-thiazol-5-yl}methyl)sulfanyl]-2-methylphenoxy}propanoic acid,
2-methyl-2-{2-methyl-4-[({4-{[4-(phenoxycarbonyl)-1-piperazinyl]methyl}-2-[4-(trifluoromethyl)phenyl]-1,3-thiazol-5-yl}methyl)sulfanyl]phenoxy}propanoic acid,
2-{4-[({4-{[4-(4-acetylphenyl)-1-piperazinyl]methyl}-2-[4-(trifluoromethyl)phenyl]-1,3-thiazol-5-yl}methyl)sulfanyl]-2-propylphenoxy}propanoic acid,
{2-methyl-4-[({4-[4-(3-thienyl)benzyl]-2-[4-(trifluoromethyl)phenyl]-1,3-thiazol-5-yl}methyl)sulfanyl]phenoxy} acetic acid,
2-(4-{[(2-(4-fluorophenyl)-4-{[4-(4-methoxyphenyl)-1-piperazinyl]methyl}-1,3-thiazol-5-yl)methyl]sulfanyl}-2-methylphenoxy)-2-methylpropanoic acid,
2-{4-[({4-{[4-(4-acetylphenyl)-1-piperazinyl]methyl}-2-[4-(trifluoromethyl)phenyl]-1,3-thiazol-5-yl}methyl)sulfanyl]phenoxy}-2-methylpropanoic acid,
2-{4-[({4-{[4-(2,4-dimethoxyphenyl)-1-piperazinyl]methyl}-2-[4-(trifluoromethyl)phenyl]-1,3-thiazol-5-yl}methyl)sulfanyl]-2-methylphenoxy}propanoic acid,
{2-isopropyl-4-[({4-{[4-(4-methoxyphenyl)-1-piperazinyl]methyl}-2-[4-(trifluoromethyl)phenyl]-1,3-thiazol-5-yl} methyl)sulfanyl]phenoxy}acetic acid,
2-{4-[({4-{[4-(4-methoxyphenyl)-1-piperazinyl]methyl}-2-[4-(trifluoromethyl)phenyl]-1,3-thiazol-5-yl}methyl) sulfanyl]-2-propylphenoxy}propanoic acid,
2-{4-[({4-benzyl-2-[4-(trifluoromethyl)phenyl]-1,3-thiazol-5-yl}methyl)sulfanyl]-2-methylphenoxy}propanoic acid,
2-{2-ethyl-4-[({4-{[4-(4-methoxyphenyl)-1-piperazinyl]methyl}-2-[4-(trifluoromethyl)phenyl]-1,3-thiazol-5-yl} methyl)sulfanyl]phenoxy}propanoic acid,
2-methyl-2-{2-methyl-4-[({4-[4-(trifluoromethyl)benzyl]-2-[4-(trifluoromethyl)phenyl]-1,3-thiazol-5-yl}methyl) sulfanyl]phenoxy}propanoic acid,
2-{4-[({4-{[4-(4-fluorophenyl)-1-piperazinyl]methyl}-2-[4-(trifluoromethyl)phenyl]-1,3-thiazol-5-yl}methyl)sulfanyl]-2-methylphenoxy}-2-methylpropanoic acid,
{4-[({4-{[4-(4-acetylphenyl)-1-piperazinyl]methyl}-2-[4-(trifluoromethyl)phenyl]-1,3-thiazol-5-yl}methyl)sulfanyl]-2-propylphenoxy}acetic acid,
{4-[({4-([1,1'-biphenyl]-4-ylmethyl)-2-[4-(trifluoromethyl)phenyl]-1,3-thiazol-5-yl}methyl)sulfanyl]-2-methylphenoxy}acetic acid,
2-{4-[({4-{[4-(4-fluorophenyl)-1-piperazinyl]methyl}-2-[4-(trifluoromethyl)phenyl]-1,3-thiazol-5-yl}methyl)sulfanyl]-2-methylphenoxy}propanoic acid,
{4-[({4-{[4-(3-methoxyphenyl)-1-piperazinyl]methyl}-2-[4-(trifluoromethyl)phenyl]-1,3-thiazol-5-yl}methyl)sulfanyl]-2-methylphenoxy}acetic acid,
2-{2-methyl-4-[({4-(3-thienylmethyl)-2-[4-(trifluoromethyl)phenyl]-1,3-thiazol-5-yl}methyl)sulfanyl]phenoxy} propanoic acid,
{4-[({4-{[4-(2-methoxyphenyl)-1-piperazinyl]methyl}-2-[4-(trifluoromethyl)phenyl]-1,3-thiazol-5-yl}methyl)sulfanyl]-2-methylphenoxy}acetic acid,
2-{2-isopropyl-4-[({4-{[4-(4-methoxyphenyl)-1-piperazinyl]methyl}-2-[4-(trifluoromethyl)phenyl]-1,3-thiazol-5-yl}methyl)sulfanyl]phenoxy}propanoic acid,
2-{4-[({4-(4-tert-butylbenzyl)-2-[4-(trifluoromethyl)phenyl]-1,3-thiazol-5-yl}methyl)sulfanyl]-2-methylphenoxy}-2-methylpropanoic acid,
2-{4-[({4-{[4-(3-methoxyphenyl)-1-piperazinyl]methyl}-2-[4-(trifluoromethyl)phenyl]-1,3-thiazol-5-yl}methyl) sulfanyl]-2-methylphenoxy}propanoic acid,
2-{4-[({4-benzyl-2-[4-(trifluoromethyl)phenyl]-1,3-thiazol-5-yl}methyl)sulfanyl]-2,3-dimethylphenoxy}propanoic acid,
2-{4-[({4-{[4-(4-chlorophenyl)-1-piperazinyl]methyl}-2-[4-(trifluoromethyl)phenyl]-1,3-thiazol-5-yl}methyl)sulfanyl]-2-methylphenoxy}propanoic acid,
2-{4-[({4-benzyl-2-[4-(trifluoromethyl)phenyl]-1,3-thiazol-5-yl}methyl)sulfanyl]-2-fluorophenoxy}propanoic acid,
2-{4-[({4-{[4-(2,4-difluorophenyl)-1-piperazinyl]methyl}-2-[4-(trifluoromethyl)phenyl]-1,3-thiazol-5-yl}methyl) sulfanyl]-2-methylphenoxy}propanoic acid,
{4-[({4-(2,4-difluorobenzyl)-2-[4-(trifluoromethyl)phenyl]-1,3-thiazol-5-yl}methyl)sulfanyl]-2-methylphenoxy} acetic acid,
2-{4-[({4-{[4-(4-acetylphenyl)-1-piperazinyl]methyl}-2-[4-(trifluoromethyl)phenyl]-1,3-thiazol-5-yl}methyl)sulfanyl]-2-methylphenoxy}-2-methylpropanoic acid,
2-methyl-2-{2-methyl-4-[({4-(3-thienylmethyl)-2-[4-(trifluoromethyl)phenyl]-1,3-thiazol-5-yl}methyl)sulfanyl] phenoxy}propanoic acid,
2-{4-[({4-{[4-(4-methoxyphenyl)-1-piperazinyl]methyl}-2-[4-(trifluoromethyl)phenyl]-1,3-thiazol-5-yl}methyl) sulfanyl]-2-methylphenoxy}propanoic acid,
{2-ethyl-4-[({4-{[4-(4-methoxyphenyl)-1-piperazinyl]methyl}-2-[4-(trifluoromethyl)phenyl]-1,3-thiazol-5-yl}methyl)sulfanyl]phenoxy}acetic acid,
2-{4-[({4-(4-methoxybenzyl)-2-[4-(trifluoromethyl)phenyl]-1,3-thiazol-5-yl}methyl)sulfanyl]-2-methylphenoxy}-2-methylpropanoic acid,
2-methyl-2-{4-[({4-{[4-(2-pyrazinyl)-1-piperazinyl]methyl}-2-[4-(trifluoromethyl)phenyl]-1,3-thiazol-5-yl}methyl)sulfanyl]phenoxy}propanoic acid,
2-{4-[({4-{[4-(4-methoxyphenyl)-1-piperazinyl]methyl}-2-[4-(trifluoromethyl)phenyl]-1,3-thiazol-5-yl}methyl) sulfanyl]-2-methylphenoxy}-2-methylpropanoic acid,
2-{4-[({4-{[4-(4-methoxyphenyl)-1-piperazinyl]methyl}-2-[4-(trifluoromethyl)phenyl]-1,3-thiazol-5-yl}methyl) sulfanyl]phenoxy}-2-methylpropanoic acid,
2-methyl-2-{2-methyl-4-[({4-[4-(trifluoromethoxy)benzyl]-2-[4-(trifluoromethyl)phenyl]-1,3-thiazol-5-yl}methyl) sulfanyl]phenoxy}propanoic acid,
2-{4-[({4-{[4-(4-isopropoxyphenyl)-1-piperazinyl]methyl}-2-[4-(trifluoromethyl)phenyl]-1,3-thiazol-5-yl}methyl) sulfanyl]-2-methylphenoxy}propanoic acid,
2-{2-methyl-4-[({4-{[4-(2-pyrimidinyl)-1-piperazinyl]methyl}-2-[4-(trifluoromethyl)phenyl]-1,3-thiazol-5-yl}methyl)sulfanyl]phenoxy}propanoic acid.
{2-methyl-4-[({4-(3-phenylpropyl)-2-[4-(trifluoromethyl)phenyl]-1,3-thiazol-5-yl}methyl)sulfanyl]phenoxy}acetic acid,
[4-[({4-benzyl-2-[4-(trifluoromethyl)phenyl]-1,3-thiazol-5-yl}methyl)sulfanyl]-2-(trifluoromethyl)phenoxy]acetic acid,
{2-methyl-4-[({4-{[4-(5-methyl-1,2,4-oxadiazol-3-yl)phenoxy]methyl}-2-[4-(trifluoromethyl)phenyl]-1,3-thiazol-5-yl}methyl)sulfanyl]phenoxy}acetic acid,
{4-[({4-benzyl-2-[4-(trifluoromethyl)phenyl]-1,3-thiazol-5-yl}methyl)sulfanyl]-5-chloro-2-methylphenoxy}acetic acid,
{4-[({4-benzyl-2-[4-(trifluoromethyl)phenyl]-1,3-thiazol-5-yl}methyl)sulfanyl]-2-methylphenoxy}acetic acid,

{4-[({4-(4-methoxybenzyl)-2-[4-(trifluoromethyl)phenyl]-1,3-thiazol-5-yl}methyl)sulfanyl]-2-methylphenoxy} acetic acid,
{2,5-dimethyl-4-[({4-(3-thienylmethyl)-2-[4-(trifluoromethyl)phenyl]-1,3-thiazol-5-yl}methyl)sulfanyl]phenoxy} acetic acid,
{2-methyl-4-[({4-{[4-(2-pyrazinyl)-1-piperazinyl]methyl}-2-[4-(trifluoromethyl)phenyl]-1,3-thiazol-5-yl}methyl) sulfanyl]phenoxy}acetic acid,
{4-[({4-benzyl-2-[4-(trifluoromethyl)phenyl]-1,3-thiazol-5-yl}methyl)sulfanyl]-2,3-dimethylphenoxy}acetic acid,
[4-({[2-(4-chlorophenyl)-4-methyl-1,3-thiazol-5-yl]methyl}sulfanyl)-2-methylphenoxy]acetic acid,
{2-methyl-4-[({4-[(4-methyl-2-thienyl)methyl]-2-[4-(trifluoromethyl)phenyl]-1,3-thiazol-5-yl}methyl)sulfanyl] phenoxy}acetic acid,
{4-[({4-benzyl-2-[4-(trifluoromethyl)phenyl]-1,3-thiazol-5-yl}methyl)sulfanyl]-2-bromophenoxy}acetic acid,
{2-methyl-4-[({4-[(2-phenylethoxy)methyl]-2-[4-(trifluoromethyl)phenyl]-1,3-thiazol-5-yl}methyl)sulfanyl]phenoxy}acetic acid,
{2-methyl-4-[({4-(2-phenylethyl)-2-[4-(trifluoromethyl)phenyl]-1,3-thiazol-5-yl}methyl)sulfanyl]phenoxy}acetic acid, and

pharmaceutically acceptable salts, solvates, and hydrolyzable esters thereof.

**17.** A compound of formula (I) selected from:

2-methyl-2-{2-methyl-4-[({4-(3-thienylmethyl)-2-[4-(trifluoromethyl)phenyl]-1,3-thiazol-5-yl}methyl)sulfanyl] phenoxy}propanoic acid,
2-{4-[({4-{[4-(4-methoxyphenyl)-1-piperazinyl]methyl}-2-[4-(trifluoromethyl)phenyl]-1,3-thiazol-5-yl}methyl) sulfanyl]-2-methylphenoxy}propanoic acid,
{2-ethyl-4-[({4-{[4-(4-methoxyphenyl)-1-piperazinyl]methyl}-2-[4-(trifluoromethyl)phenyl]-1,3-thiazol-5-yl}methyl)sulfanyl]phenoxy}acetic acid,
2-{4-[({4-(4-methoxybenzyl)-2-[4-(trifluoromethyl)phenyl]-1,3-thiazol-5-yl}methyl)sulfanyl]-2-methylphenoxy}-2-methylpropanoic acid,
2-methyl-2-{4-[({4-{[4-(2-pyrazinyl)-1-piperazinyl]methyl}-2-[4-(trifluoromethyl)phenyl]-1,3-thiazol-5-yl}methyl)sulfanyl]phenoxy}propanoic acid,
2-{4-[({4-{[4-(4-methoxyphenyl)-1-piperazinyl]methyl}-2-[4-(trifluoromethyl)phenyl]-1,3-thiazol-5-yl}methyl) sulfanyl]-2-methylphenoxy}-2-methylpropanoic acid,
2-{4-[({4-{[4-(4-methoxyphenyl)-1-piperazinyl]methyl}-2-[4-(trifluoromethyl)phenyl]-1,3-thiazol-5-yl}methyl) sulfanyl]phenoxy}-2-methylpropanoic acid,
2-methyl-2-{2-methyl-4-[({4-[4-(trifluoromethoxy)benzyl]-2-[4-(trifluoromethyl)phenyl]-1,3-thiazol-5-yl}methyl) sulfanyl]phenoxy}propanoic acid,
2-{4-[({4-{[4-(4-isopropoxyphenyl)-1-piperazinyl]methyl}-2-[4-(trifluoromethyl)phenyl]-1,3-thiazol-5-yl}methyl) sulfanyl]-2-methylphenoxy}propanoic acid,
2-{2-methyl-4-[({4-{[4-(2-pyrimidinyl)-1-piperazinyl]methyl}-2-[4-(trifluoromethyl)phenyl]-1,3-thiazol-5-yl}methyl)sulfanyl]phenoxy}propanoic acid, and

pharmaceutically acceptable salts, solvates, or hydrolyzable esters thereof

**18.** A compound according to any preceding claim which is a hPPARδ agonist.

**19.** A compound according to Claim 18 which is also a hPPARα or hPPARgamma agonist.

**20.** A compound according to any preceding claim which is a hPPAR pan agonist.

**21.** A compound according to any of claims 1-20 for use in therapy.

**22.** A pharmaceutical composition comprising a compound according to any of claims 1-20.

**23.** A pharmaceutical composition according to claim 22 further comprising a pharmaceutically acceptable diluent or carrier.

**24.** Use of a compound according to any of claims 1-20 for the manufacture of a medicament for the treatment of a hPPAR disease or condition.

25. Use according to claim 24 wherein the hPPAR mediated disease or condition is dyslipidemia, syndrome X, heart failure, hypercholesteremia, cardiovascular disease, type II diabetes mellitus, type I diabetes, insulin resistance, hyperlipidemia, obesity, anorexia bulimia and anorexia nervosa

**Patentansprüche**

1. Verbindung der Formel (I) oder ein pharmazeutisch akzeptables (akzeptabler) Salz, Solvat oder hydrolysierbarer Ester davon:

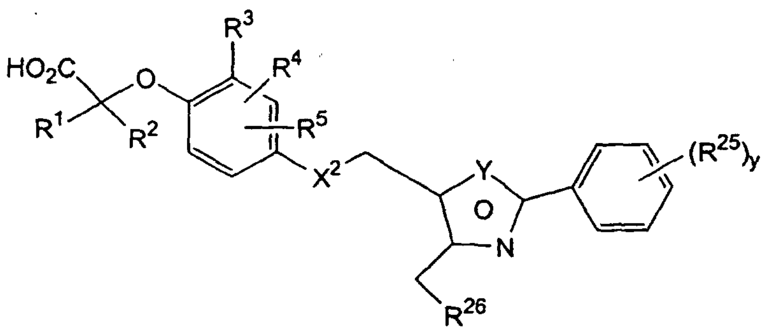

(I)

worin
$R^1$ und $R^2$ unabhängig Wasserstoff oder $C_{1-3}$-Alkyl sind;
$X^2$ O, S oder $CH_2$ ist;
$R^3$, $R^4$ und $R^5$ unabhängig H, $C_{1-3}$-Alkyl, $OCH_3$, $CF_3$, $OCF_3$, CN, Allyl oder Halogen sind;
Y S oder O ist;
$R^{25}$ jeweils unabhängig $CH_3$, $OCH_3$, $CF_3$ oder Halogen ist;
y 0, 1, 2, 3, 4 oder 5 ist; und
$R^{26}$ aus der Gruppe ausgewählt ist, die aus den nachfolgend dargestellten Einheiten A bis K besteht:

A 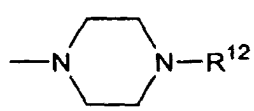

worin $R^{12}$ aus der Gruppe ausgewählt ist, die aus $C_{1-6}$-Alkyl, $C_{1-6}$-Alkylenaryl und den nachfolgend in Gruppe II dargestellten Einheiten besteht:

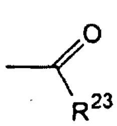 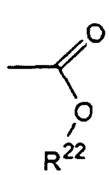 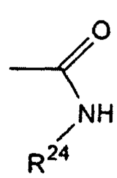 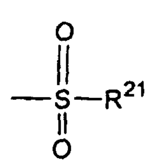 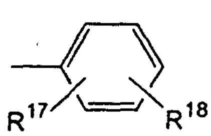

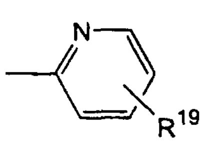 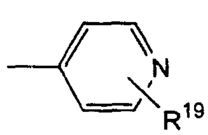 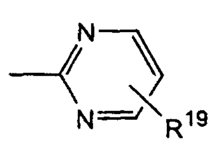 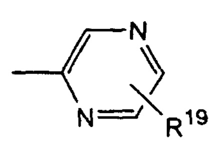

Gruppe II

worin $R^{17}$ und $R^{18}$ unabhängig Wasserstoff, Halogen, Hydroxy, -CN, $C_{1-6}$-Alkyl, $C_{1-6}$-Perfluoralkyl, $C_{1-6}$-Acyl, -$OC_{1-6}$-Alkyl, Perfluor-$OC_{1-6}$-alkyl oder $C_{1-6}$-Hydroxyalkyl sind;
$R^{19}$ Wasserstoff oder $C_{1-6}$-Alkyl ist;
$R^{21}$ $C_{1-6}$-Alkyl, -$C_{1-6}$-Alkylenaryl, Aryl oder -Arylheteroaryl ist;
$R^{22}$ $C_{1-6}$-Alkyl, Aryl oder -$C_{1-6}$-Alkylenaryl ist;
$R^{23}$ $C_{1-6}$-Alkyl, $C_{3-6}$-Cycloalkyl oder Aryl ist;
$R^{24}$ $C_{1-6}$-Alkyl, -$C_{1-6}$-Alkylenaryl, $C_{3-6}$-Cycloalkyl oder Aryl ist;

B 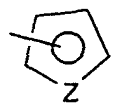

worin Z O, N oder S ist (man bemerke, daß dann, wenn Z N ist, die angegebene Bindung an den Stickstoff im Ring sowie an jeden der Kohlenstoffe im Ring gebunden sein kann);

C 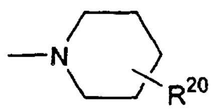

worin $R^{20}$ $C_{1-6}$-Alkyl, Aryl, -$OC_{1-6}$-Alkyl, Hydroxy, $C_{1-6}$-Hydroxyalkyl oder 1-Alkoxy-$C_{1-6}$-alkyl ist;

D 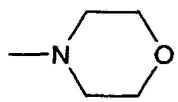

E 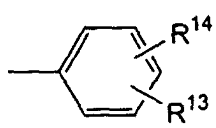

worin $R^{13}$ und $R^{14}$ unabhängig Wasserstoff, Halogen, CN, Perfluor-$C_{1-6}$-alkyl, Perfluor-$OC_{1-6}$-alkyl, $C_{1-6}$-Alkyl, -$OC_{1-6}$-Alkyl, -$C_{1-6}$-Alkylen-$OC_{1-6}$-alkyl, -$SC_{1-6}$-Alkyl oder Aryl sind;

F 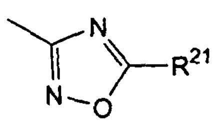

worin $R^{21}$ unabhängig wie oben definiert ist;

G 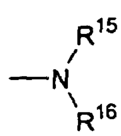

worin $R^{15}$ und $R^{16}$ unabhängig Wasserstoff, $C_{1-6}$-Alkyl, gegebenenfalls mit 1 oder 2 $C_{1-3}$-Alkylgruppen substituiertes $C_{3-6}$-Cycloalkyl oder $R^{12}$ wie oben definiert sind;

H 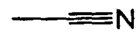

I $-(CH_2)_nPh$

worin n 1-3 ist

J $-O-R^{21}$

worin $R^{21}$ unabhängig wie oben definiert ist; und

K $-S-R^{21}$

worin $R^{21}$ unabhängig wie oben definiert ist.

**2.** Verbindung gemäß Anspruch 1, worin $R^1$ und $R^2$ unabhängig H oder $CH_3$ sind.

**3.** Verbindung gemäß Anspruch 2, worin $R^1$ und $R^2$ entweder beide H oder beide $CH_3$ sind.

**4.** Verbindung gemäß einem der Ansprüche 1 bis 3, worin $X^2$ O oder S ist.

**5.** Verbindung gemäß einem der Ansprüche 1 bis 4, worin $R^3$ $CH_3$ oder H ist.

**6.** Verbindung gemäß einem der Ansprüche 1 bis 5, worin $R^4$ und $R^5$ H sind.

**7.** Verbindung gemäß jedem vorhergehenden Anspruch, worin Y S ist.

**8.** Verbindung gemäß einem der Ansprüche 1 bis 8, worin y 1 oder 2 ist.

**9.** Verbindung gemäß Anspruch 8, worin $R^{25}$ jeweils unabhängig Halogen oder $CF_3$ ist.

**10.** Verbindung gemäß jedem vorhergehenden Anspruch, worin $R^{26}$ aus der Gruppe ausgewählt ist, die aus

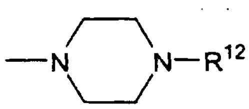 , 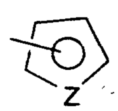 und 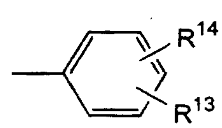

besteht, worin $R^{12}$, Z, $R^{13}$ und $R^{14}$ wie in Anspruch 1 definiert sind.

**11.** Verbindung gemäß jedem vorhergehenden Anspruch, worin $R^{13}$ und $R^{14}$ unabhängig Fluor, Brom, Phenyl, Thienyl, $CF_3$, $OCF_3$, $OCH_3$, $SCH_3$ oder t-Butyl sind, $R^{17}$ und $R^{18}$ unabhängig Wasserstoff, OH, CN, $OC_{1-3}$-Alkyl, Halogen, $CF_3$, $COCH_3$, $CH(OH)CH_3$ oder $OCF_3$ sind, $R^{21}$ gegebenenfalls mit Methyl oder CN substituiertes Phenyl, $-C_{1-3}$-Alkylenphenyl oder Phenyl-5-methyl-1,2,4-oxadiazol-3-yl ist, $R^{22}$ $C_{1-6}$-Alkyl, Phenyl oder Benzyl ist, $R^{23}$ $C_{1-6}$-Alkyl, Furanyl, Thienyl, gegebenenfalls mit einem Halogen, einer Methoxy- oder einer Dimethylaminogruppe substituiertes Phenyl, Methoxymethylcyclopropyl oder $C_{3-6}$-Cycloalkyl ist und $R^{24}$ H, $C_{1-6}$-Alkyl, Cyclohexyl, m-Methoxyphenyl, p-Fluorphenyl oder $-CH_2CH_2$-Phenyl ist.

**12.** Verbindung gemäß Anspruch 11, worin $R^{26}$

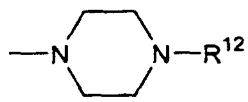

ist und $R^{12}$ aus den in Gruppe IV gezeigten Einheiten ausgewählt ist:

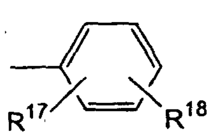

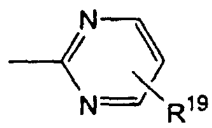

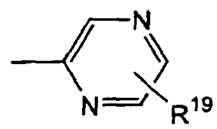

Gruppe IV

**13.** Verbindung gemäß Anspruch 12, worin $R^{17}$ Fluor, Chlor, $OC_{1-3}$-Alkyl oder $COCH_3$ ist und $R^{18}$ $OCH_3$ oder Wasserstoff ist und $R^{19}$ Wasserstoff ist.

**14.** Verbindung gemäß Anspruch 10, worin $R^{26}$

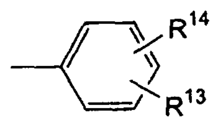

ist.

**15.** Verbindung gemäß Anspruch 14, worin $R^{14}$ Thienyl, $OCH_3$, $OCF_3$, $CF_3$ oder Fluor ist und $R^{13}$ Wasserstoff oder Fluor ist.

**16.** Verbindung der Formel (I), ausgewählt aus:

2-[4-({[4-{[4-(4-Acetylphenyl)-1-piperazinyl]methyl}-2-(4-fluorphenyl)-1,3-thiazol-5-yl]methyl}sulfanyl)-2-methylphenoxy]-2-methylpropansäure,
2-Methyl-2-{2-methyl-4-[({4-[4-(methylsulfanyl)benzyl]-2-[4-(trifluormethyl)phenyl]-1,3-thiazol-5-yl}methyl) sulfanyl]phenoxy}propansäure,
{2-Methyl-4-[({4-(3-thienylmethyl)-2-[4-(trifluormethyl)phenyl]-1,3-thiazol-5-yl}methyl)sulfanyl]phenoxy}essigsäure,
{4-[({4-Benzyl-2-[4-(trifluormethyl)phenyl]-1,3-thiazol-5-yl}methyl)sulfanyl]-2,5-dimethylphenoxy}essigsäure,
2-{4-[({4-{[4-(4-Acetylphenyl)-1-piperazinyl]methyl}-2-[4-(trifluormethyl)phenyl]-1,3-thiazol-5-yl}methyl)sulfanyl]-2-methylphenoxy}propansäure,
2-{4-[({4-{[4-(4-Acetylphenyl)-1-piperazinyl]methyl}-2-[4-trifluormethyl)phenyl]-1,3-thiazol-5-yl}methyl)sulfanyl]-2-ethylphenoxy}propansäure,
2-{2-Methyl-4-[({4-(2-thienylmethyl)-2-[4-(trifluormethyl)phenyl]-1,3-thiazol-5-yl}methyl)sulfanyl]phenoxy}propansäure,
2-{4-[({4-{[4-(4-Methoxyphenyl)-1-piperazinyl]methyl}-2-[4-(trifluormethyl)phenyl]-1,3-thiazol-5-yl}methyl)sulfanyl]-2-methylphenoxy}propansäure,
2-{4-[({4-{[4-(4-Ethoxyphenyl)-1-piperazinyl]methyl}-2-[4-(trifluormethyl)phenyl]-1,3-thiazol-5-yl}methyl)sulfanyl]-2-methylphenoxy}propansäure,
2-Methyl-2-{2-methyl-4-[({4-{[4-(phenoxycarbonyl)-1-piperazinyl]methyl}-2-[4-(trifluormethyl)phenyl]-1,3-thiazol-5-yl}methyl)sulfanyl]phenoxy}propansäure,
2-{4-[({4-{[4-(4-Acetylphenyl)-1-piperazinyl]methyl}-2-[4-(trifluormethyl)phenyl]-1,3-thiazol-5-yl}methyl)sulfanyl]-2-propylphenoxy}propansäure,
{2-Methyl-4-[({4-[4-(3-thienyl)benzyl]-2-[4-(trifluormethyl)phenyl]-1,3-thiazol-5-yl}methyl)sulfanyl]phenoxy} essigsäure,

2-(4-{[(2-(4-Fluorphenyl)-4-{[4-(4-methoxyphenyl)-1-piperazinyl]methyl}-1,3-thiazol-5-yl)methyl]sulfanyl}-2-methylphenoxy)-2-methylpropansäure,
2-{4-[({4-{[4-(4-Acetylphenyl)-1-piperazinyl]methyl}-2-[4-(trifluormethyl)phenyl]-1,3-thiazol-5-yl}methyl)sulfanyl]phenoxy}-2-methylpropansäure,
2-{4-[({4-{[4-(2,4-Dimethoxyphenyl)-1-piperazinyl]methyl}-2-[4-(trifluormethyl)phenyl]-1,3-thiazol-5-yl}methyl) sulfanyl]-2-methylphenoxy}propansäure,
{2-Isopropyl-4-[({4-{[4-(4-methoxyphenyl)-1-piperazinyl]methyl}-2-[4-(trifluormethyl)phenyl]-1,3-thiazol5-yl} methyl)sulfanyl]phenoxy}essigsäure,
2-{4-[({4-{[4-(4-Methoxyphenyl)-1-piperazinyl]methyl}-2-[4-(trifluormethyl)phenyl]-1,3-thiazol-5-yl}methyl)sulfanyl]-2-propylphenoxy}propansäure,
2-{4-[({4-Benzyl-2-[4-(trifluormethyl)phenyl]-1,3-thiazol-5-yl}methyl)sulfanyl]-2-methylphenoxy}propansäure,
2-{2-Ethyl-4-[({4-{[4-(4-methoxyphenyl)-1-piperazinyl]methyl}-2-[4-(trifluormethyl)phenyl]-1,3-thiazol-5-yl} methyl)sulfanyl]phenoxy}propansäure,
2-Methyl-2-{2-methyl-4-[({4-[4-(trifluormethyl)benzyl]-2-[4-(trifluormethyl)phenyl]-1,3-thiazol-5-yl}methyl)sulfanyl]phenoxy}propansäure,
2-{4-[({4-{[4-(4-Fluorphenyl)-1-piperazinyl]methyl}-2-[4-(trifluormethyl)phenyl]-1,3-thiazol-5-yl}methyl)sulfanyl]-2-methoxyphenoxy}-2-methylpropansäure,
{4-[({4-{[4-(4-Acetylphenyl)-1-piperazanyl]methyl}-2-[4-(trifluormethyl)phenyl]-1,3-thiazol-5-yl}methyl)sulfanyl]-2-propylphenoxy}essigsäure,
{4-[({4-([1,1'-Biphenyl]-4-ylmethyl)-2-[4-(trifluormethyl)phenyl]-1,3-thiazol-5-yl}methyl)sulfanyl]-2-methylphenoxy}essigsäure,
2-{4-[({4-{[4-(4-Fluorphenyl)-1-piperazinyl]methyl}-2-[4-(trifluormethyl)phenyl]-1,3-thiazol-5-yl}methyl)sulfanyl]-2-methylphenoxy}propansäure,
{4-[({4-{[4-(3-Methoxyphenyl)-1-piperazinyl]methyl}-2-[4-(trifluormethyl)phenyl]-1,3-thiazol-5-yl}methyl)sulfanyl]-2-methylphenoxy}essigsäure,
2-{2-Methyl-4-[({4-(3-thienylmethyl)2-[4-(trifluormethyl)phenyl]-1,3-thiazol-5-yl}methyl)sulfanyl]phenoxy}propansäure,
{4-[({4-{[4-(2-Methoxyphenyl)-1-piperazinyl]methyl}-2-[4-(trifluormethyl)phenyl]-1,3-thiazol-5-yl}methyl)sulfanyl]-2-methylphenoxy}essigsäure,
2-{2-Isopropyl-4-[({4-{[4-(4-methoxyphenyl)-1-piperazinyl]methyl}-2-[4-(trifluormethyl)phenyl]-1,3-thiazol-5-yl}methyl)sulfanyl]phenoxy}propansäure,
2-{4-[({4-(4-tert-butylbenzyl)2-[4-(trifluormethyl)phenyl]-1,3-thiazol-5-yl}methyl)sulfanyl]-2-methylphenoxy}-2-methylpropansäure,
2-{4-[({4-{[4-(3-Methoxyphenyl)-1-piperazinyl]methyl}-2-[4-(trifluormethyl)phenyl]-1,3-thiazol-5-yl}methyl)sulfanyl]-2-methylphenoxy}propansäure,
2-{4-[({4-Benzyl-2-[4-(trifluormethyl)phenyl]-1,3-thiazol-5-yl}methyl)sulfanyl]-2,3-dimethylphenoxy}propansäure,
2-{4-[({4-{[4-(4-Chlorphenyl)-1-piperazinyl]methyl}-2-[4-(trifluormethyl)phenyl]-1,3-thiazol-5-yl}methyl)sulfanyl]-2-methylphenoxy}propansäure,
2-{4-[({4-Benzyl-2-[4-(trifluormethyl)phenyl]-1,3-thiazol-5-yl}methyl)sulfanyl]-2-fluorphenoxy)propansäure,
2-{4-[({4-{[4-(2,4-Difluorphenyl)1-piperazinyl]methyl}-2-[4-(trifluormethyl)phenyl]-1,3-thiazol-5-yl}methyl)sulfanyl]-2-methylphenoxy}propansäure,
{4-[({4-(2,4-Difluorbenzyl)-2-[4-(trifluormethyl)phenyl]-1,3-thiazol-5-yl}methyl)sulfanyl]-2-methylphenoxy}essigsäure,
2-{4-[({4-{[4-(4-Acetylphenyl)-1-piperazinyl]methyl}-2-[4-(trifluormethyl)phenyl]-1,3-thiazol-5-yl}methyl)sulfanyl]-2-methylphenoxy}-2-methylpropansäure,
2-Methyl-2-{2-methyl-4-[({4-(3-thienylmethyl)-2-[4-(trifluormethyl)phenyl]-1,3-thiazol-5-yl}methyl)sulfanyl] phenoxy}propansäure,
2-{4-[({4-{(4-(4-Methoxyphenyl)-1-piperazinyl)methyl}-2-[4-(trifluormethyl)phenyl]-1,3-thiazol-5-yl}methyl)sulfanyl]-2-methylphenoxy}propansäure,
{2-Ethyl-4-[({4-{[4-(4-methoxyphenyl)-1-piperazinyl]methyl}-2-[4-(trifluormethyl)phenyl]-1,3-thiazol-5-yl}methyl)sulfanyl]phenoxy}essigsäure,
2-{4-[({4-(4-Methoxybenzyl)-2-[4-(trifluormethyl)phenyl]-1,3-thiazol-5-yl}methyl)sulfanyl]-2-methylphenoxy}-2-methylpropansäure,
2-Methyl-2-{4-[({4-{[4-(2-pyrazinyl)-1-piperazinyl]methyl}-2-[4-(trifluormethyl)phenyl]-1,3-thiazol-5-yl}methyl) sulfanyl]phenoxy}propansäure,
2-{4-[({4-{[4-(4-Methoxyphenyl)-1-piperazinyl]methyl}-2-[4-(trifluormethyl)phenyl]-1,3-thiazol-5-yl}methyl)sulfanyl]-2-methylphenoxy}-2-methylpropansäure,

2-{4-[({4-{[4-(4-Methoxyphenyl)-1-piperazinyl]methyl}-2-[4-(trifluormethyl)phenyl]-1,3-thiazol-5-yl}methyl)sulfanyl]phenoxy}-2-methylpropansäure,
2-Methyl-2-{2-methyl-4-[({4-[4-(trifluormethoxy)benzyl]-2-[4-(trifluormethyl)phenyl]-1,3-thiazol-5-yl}methyl) sulfanyl]phenoxy}propansäure,
2-{4-[({4-{[4-(4-Isopropoxyphenyl)-1-piperazinyl]methyl}-2-[4-(trifluormethyl)phenyl]-1,3-thiazol-5-yl}methyl) sulfanyl]-2-methylphenoxy}propansäure,
2-{2-Methyl-4-[({4-{[4-(2-Pyrimidinyl)-1-piperazinyl]methyl}-2-[4-(trifluormethyl)phenyl]-1,3-thiazol-5-yl}methyl)sulfanyl]phenoxy}propansäure,
{2-Methyl-4-[({4-(3-phenylpropyl)-2-[4-(trifluormethyl)phenyl)-1,3-thiazol-5-yl}methyl)sulfanyl]phenoxy}essigsäure,
[4-[({4-Benzyl-2-[4-(trifluormethyl)phenyl]-1,3-thiazol-5-yl}methyl)sulfanyl]-2-(trifluormethyl)phenoxy]essigsäure,
{2-Methyl-4-[({4-{[4-(5-methyl-1,2,4-oxadiazol-3-yl)phenoxy]methyl}-2-[4-(trifluormethyl)phenyl]-1,3-thiazol-5-yl}methyl)sulfanyl]phenoxy}essigsäure,
{4-[({4-Benzyl-2-[4-(trifluormethyl)phenyl]-1,3-thiazol-5-yl}methyl)sulfanyl]-5-chloro-2-methylphenoxy}essigsäure,
{4-[({4-Benzyl-2-[4-(trifluormethyl)phenyl]-1,3-thiazol-5-yl}methyl)sulfanyl]-2-methylphenoxy}essigsäure,
{4-[({4-(4-Methoxybenzyl)-2-[4-(trifluormethyl)phenyl]-1,3-thiazol-5-yl}methyl)sulfanyl]-2-methylphenoxy}essigsäure,
{2,5-Dimethyl-4-[({4-(3-thienylmethyl)-2-[4-(trifluormethyl)phenyl]-1,3-thiazol-5-yl}methyl)sulfanyl]phenoxy} essigsäure,
{2-Methyl-4-[({4-{[4-(2-pyrazinyl)-1-piperazinyl]methyl}-2-[4-(trifluormethyl)phenyl]-1,3-thiazol-5-yl}methyl) sulfanyl]phenoxy}essigsäure,
{4-[({4-Benzyl-2-[4-(trifluormethyl)phenyl]-1,3-thiazol-5-yl}methyl)sulfanyl]-2,3-dimethylphenoxy}essigsäure,
[4-({[2-(4-Chlorphenyl)-4-methyl-1,3-thiazol-5-yl]methyl}sulfanyl)-2-methylphenoxy]essigsäure,
{2-Methyl-4-[({4-[(4-methyl-2-thienyl)methyl]-2-[4-(trifluormethyl)phenyl]-1,3-thiazol-5-yl}methyl)sulfanyl] phenoxy}essigsäure,
{4-[({4-Benzyl-2-[4-(trifluormethyl)phenyl]-1,3-thiazol-5-yl}methyl)sulfanyl]-2-bromphenoxy}essigsäure,
{2-Methyl-4-[({4-[(2-phenylethoxy)methyl]-2-[4-(trifluormethyl)phenyl]-1,3-thiazol-5-yl}methyl)sulfanyl]phenoxy}essigsäure,
{2-Methyl-4-[({4-(2-phenylethyl)-2-[4-(trifluormethyl)phenyl]-1,3-thiazol-5-yl}methyl)sulfanyl]phenoxy}essigsäure und pharmazeutisch akzeptablen Salzen, Solvaten und hydrolysierbaren Estern davon.

**17.** Verbindung der Formel (I), ausgewählt aus:

2-Methyl-2-{2-methyl-4-[({4-(3-thienylmethyl)-2-[4-(trifluormethyl)phenyl]-1,3-thiazol-5-yl}methyl)sulfanyl] phenoxy}propansäure,
2-{4-[({4-{[4-(4-Methoxyphenyl)-1-piperazinyl]methyl}-2-[4-(trifluormethyl)phenyl]-1,3-thiazol-5-yl}methyl)sulfanyl]-2-methylphenoxy}propansäure,
{2-Ethyl-4-[({4-(4-methoxyphenyl)-1-piperazinyl]methyl}-2-[4-(trifluormethyl)phenyl]-1,3-thiazol-5-yl}methyl) sulfanyl]phenoxy}essigsäure,
2-{4-[({4-(4-Methoxybenzyl)-2-[4-(trifluormethyl)phenyl]-1,3-thiazol-5-yl}methyl)sulfanyl]-2-methylphenoxy}-2-methylpropansäure,
2-Methyl-2-{4-[({4-{[4-(2-pyrazinyl)-1-piperazinyl]methyl}-2-[4-(trifluormethyl)phenyl]-1,3-thiazol-5-yl}methyl) sulfanyl]phenoxy}propansäure,
2-{4-[({4-{[4-(4-Methoxyphenyl)-1-piperazinyl]methyl}-2-[4-(trifluormethyl)phenyl]-1,3-thiazol-5-yl}methyl)sulfanyl]-2-methylphenoxy}-2-methylpropansäure,
2-{4-[({4-{[4-(4-Methoxyphenyl)-1-piperazinyl]methyl}-2-[4-(trifluormethyl)phenyl]-1,3-thiazol-5-yl}methyl)sulfanyl]phenoxy}-2-methylpropansäure,
2-Methyl-2-{2-methyl-4-[({4-[4-(trifluormethoxy)benzyl]-2-[4-(trifluormethyl)phenyl]-1,3-thiazol-5-yl}methyl) sulfanyl]phenoxy}propansäure,
2-{4-[({4-{[4-(4-Isopropoxyphenyl)-1-piperazinyl]methyl}-2-[4-(trifluormethyl)phenyl]-1,3-thiazol-5-yl}methyl) sulfanyl]-2-methylphenoxy}propansäure,
2-{2-Methyl-4-[({4-{[4-(2-pyrimidinyl)-1-piperazinyl]methyl}-2-[4-(trifluormethyl)phenyl]-1,3-thiazol-5-yl}methyl)sulfanyl]phenoxy}propansäure und pharmazeutisch akzeptablen Salzen, Solvaten und hydrolysierbaren Estern davon.

**18.** Verbindung gemäß jedem vorhergehenden Anspruch, die ein hPPARδ-Agonist ist.

**19.** Verbindung gemäß Anspruch 18, die ebenfalls ein hPPARα-oder hPPARγ-Agonist ist.

**20.** Verbindung gemäß jedem vorhergehenden Anspruch, die ein hPPAR-Panagonist ist.

**21.** Verbindung gemäß einem der Ansprüche 1 bis 20 zur Verwendung in der Therapie.

**22.** Pharmazeutische Zusammensetzung, die eine Verbindung gemäß einem der Ansprüche 1 bis 20 umfaßt.

**23.** Pharmazeutische Zusammensetzung gemäß Anspruch 22, die ferner einen pharmazeutisch akzeptablen Verdünnungsstoff oder Träger umfaßt.

**24.** Verwendung einer Verbindung gemäß einem der Ansprüche 1 bis 20 zur Herstellung eines Medikaments zur Behandlung einer hPPAR-Krankheit oder eines hPPAR-Zustands.

**25.** Verwendung gemäß Anspruch 24, worin die/der hPPARvermittelte Krankheit oder Zustand Dyslipidämie, Syndrom X, Herzversagen, Hypercholesterinämie, kardiovaskuläre Krankheit, Typ II-Diabetes mellitus, Typ I-Diabetes, Insulinresistenz, Hyperlipidämie, Fettsucht, Anorexia bulimia und Anorexia nervosa ist.

## Revendications

**1.** Composé de formule (I) ou sel, solvate ou ester hydrolysable de celui-ci acceptable du point de vue pharmaceutique, dans laquelle :

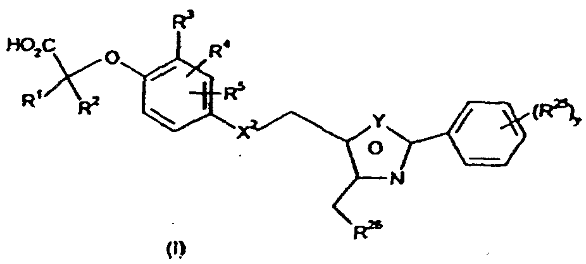

(I)

| | |
|---|---|
| $R^1$ et $R^2$ | sont indépendamment un atome d'hydrogène ou un groupe alkyle en $C_{1-3}$ ; |
| $X^2$ | est O, S ou $CH_2$ ; |
| $R^3$, $R^4$ et $R^5$ | sont indépendamment un atome d'hydrogène, un groupe alkyle en $C_{1-3}$, $OCH_3$, $CF_3$, $OCF_3$, CN, allyle ou un atome d'halogène ; |
| Y | est S ou O ; |
| chaque $R^{25}$ | est indépendamment un groupe $CH_3$, $OCH_3$, $CF_3$ ou un atome d'halogène ; |
| y | est 0, 1, 2, 3, 4 ou 5 ; et |
| $R^{26}$ | est choisi dans le groupe constitué des parties A à K illustrées ci-dessous : |

A

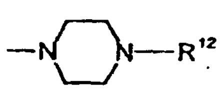

où $R^{12}$ est choisi dans le groupe consistant en groupe alkyle en $C_{1-6}$, alkylène(en $C_{1-6}$)-aryle, et les parties illustrées ci-dessous dans le Groupe II :

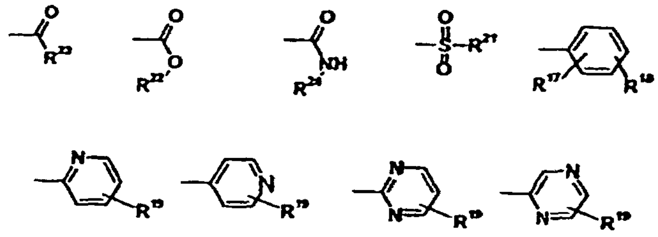

Groupe II

où $R^{17}$ et $R^{18}$ sont indépendamment un atome d'hydrogène, d'halogène, un groupe hydroxy, -CN, alkyle en $C_{1-6}$, perfluoroalkyle en $C_{1-6}$, acyle en $C_{1-6}$, -O-alkyle en $C_{1-6}$, perfluoro-O-alkyle en $C_{1-6}$, ou hydroxyalkyle en $C_{1-6}$, ;

$R^{19}$ est un atome d'hydrogène ou un groupe alkyle en $C_{1-6}$ ;

$R^{21}$ est un groupe alkyle en $C_{1-6}$, -alkylène-(en $C_{1-6}$,)-aryle, aryle ou -aryl-hétéroaryle ;

$R^{22}$ est un groupe alkyle en $C_{1-6}$, aryle ou -alkylène-(en $C_{1-6}$)-aryle ;

$R^{23}$ est un groupe alkyle en $C_{1-6}$, cycloalkyle en $C_{3-6}$, ou aryle ;

$R^{24}$ est un groupe alkyle en $C_{1-6}$, -alkylène-(en $C_{1-6}$)-aryle, cycloalkyle en $C_{3-6}$, ou aryle ;

B

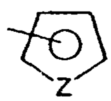

où Z est O, N ou S (à noter que lorsque Z est N, la liaison indiquée peut être attachée à l'atome d'azote dans le cycle ainsi qu'à l'un quelconque des atomes de carbone dans le cycle) ;

C

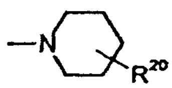

où $R^{20}$ est un groupe alkyle en $C_{1-6}$, aryle, -O-alkyle en $C_{1-6}$, hydroxy, hydroxyalkyle en $C_{1-6}$, ou 1-alcoxyalkyle en $C_{1-6}$ ;

D

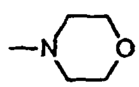

E

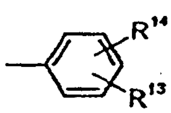

où $R^{13}$ et $R^{14}$ sont indépendamment un atome d'hydrogène, d'halogène, un groupe CN, perfluo-

roalkyle en $C_{1-6}$, perfluoro-O-alkyle en $C_{1-6}$, alkyle en $C_{1-6}$, -O-alkyle en $C_{1-6}$, -alkylène-(en $C_{1-6}$) -O-alkyle en $C_{1-6}$, -S-alkyle en $C_{1-6}$, ou aryle ;

F

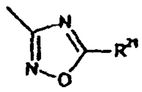

où $R^{21}$ est indépendamment tel que défini plus haut ;

G

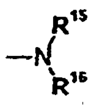

où $R^{15}$ et $R^{16}$ sont indépendamment un atome d'hydrogène, un groupe alkyle en $C_{1-6}$, cycloalkyle en $C_{3-6}$, éventuellement substitué avec 1 ou 2 groupes alkyle en $C_{1-3}$, ou $R^{12}$ tel que défini plus haut ;

H

$— \equiv N$

I

$—(CH_2)_nPh$

où n est égal à 1 à 3 ;

J

$—O—R^{21}$

où $R^{21}$ est indépendamment tel que défini plus haut ; et

K

$—S—R^{21}$

où $R^{21}$ est indépendamment tel que défini plus haut.

2. Composé selon la revendication 1, dans lequel $R^1$ et $R^2$ sont indépendamment un atome d'hydrogène ou un groupe $CH_3$.

3. Composé selon la revendication 2, dans lequel $R^1$ et $R^2$ sont soit tous deux un atome d'hydrogène, soit tous deux un groupe $CH_3$.

4. Composé selon l'une quelconque des revendications 1 à 3, dans lequel $X^2$ est un atome d'oxygène ou de soufre

5. Composé selon l'une quelconque des revendications 1 à 4, dans lequel $R^3$ est un groupe $CH_3$ ou un atome d'hydrogène.

**6.** Composé selon l'une quelconque des revendications 1 à 5, dans lequel $R^4$ et $R^5$ sont un atome d'hydrogène.

**7.** Composé selon l'une quelconque des revendications précédentes, dans lequel Y est un atome de soufre.

**8.** Composé selon l'une quelconque des revendications 1 à 8, dans lequel y est 1 ou 2.

**9.** Composé selon la revendication 8, dans lequel chaque $R^{25}$ est indépendamment un atome d'halogène ou un groupe $CF_3$.

**10.** Composé selon l'une quelconque des revendications précédentes, dans lequel $R^{26}$ est choisi dans le groupe consistant en

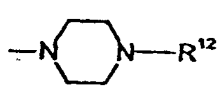
,
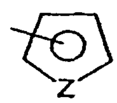
et
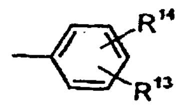

où $R^{12}$, Z, $R^{13}$ et $R^{14}$ sont tels que définis dans la revendication 1.

**11.** Composé selon l'une quelconque des revendications précédentes, dans lequel $R^{13}$ et $R^{14}$ sont indépendamment un atome de fluor, de brome, un groupe phényle, thiényle, $CF_3$, $OCF_3$, $OCH_3$, $SCH_3$ ou t-butyle, $R^{17}$ et $R^{18}$ sont indépendamment un atome d'hydrogène, un groupe OH, CN, O-alkyle en $C_{1-3}$, un atome d'halogène, un groupe $CF_3$, $COCH_3$, $CH(OH)CH_3$ ou $OCF_3$, $R^{21}$ est un groupe phényle éventuellement substitué par un groupe méthyle ou CN, -alkylène(en $C_{1-3}$)phényle, ou phényle-5-méthyl-1,2,4-oxadiazol-3-yle, $R^{22}$ est un groupe alkyle en $C_{1-6}$, phényle ou benzyle, $R^{23}$ est un groupe alkyle en $C_{1-6}$, furanyle, thiényle, phényle éventuellement substitué par un atome d'halogène, un groupe méthoxy ou diméthylamino, méthoxyméthylcyclopropyle ou cycloalkyle en $C_{3-6}$, et $R^{24}$ est un atome d'hydrogène, un groupe alkyle en $C_{1-6}$, cyclohexyle, m-méthoxyphényle, p-fluorophényle ou -$CH_2CH_2$phényle.

**12.** Composé selon la revendication 11, dans lequel $R^{26}$ est

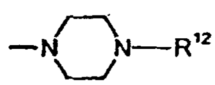

et $R^{12}$ est choisi parmi les parties montrées dans le Groupe IV.

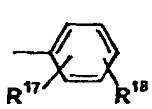
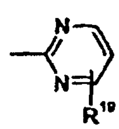
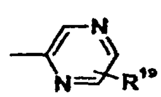

Groupe IV

**13.** Composé selon la revendication 12, dans lequel $R^{17}$ est un atome de fluor, de chlore, un groupe O-alkyle en $C_{1-3}$, ou $COCH_3$ et $R^{18}$ est un groupe $OCH_3$ ou un atome d'hydrogène, et $R^{19}$ est un atome d'hydrogène.

**14.** Composé selon la revendication 10, dans lequel $R^{26}$ est

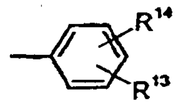

**15.** Composé selon la revendication 14, dans lequel $R^{14}$ est un groupe thiényle, $OCH_3$, $OCF_3$, $CF_3$ ou un atome de fluor, et $R^{13}$ est un atome d'hydrogène ou de fluor.

**16.** Composé de formule (I) choisi parmi :

l'acide 2-[4-({[4{[4-(4-acétylphényl)-1-pipérazinyl]méthyl}-2-(4-fluorophényl)-1,3-thiazol-5- yl]méthyl}sulfanyl)-2-méthylphénoxy]-2-méthylpropanoïque,
l'acide 2-méthyl-2-{2-méthyl-4-[({4-[4-(méthylsulfanyl)benzyl]-2-[4-(trifluorométhyl)phényl]-1,3-thiazol-5-yl}-méthyl)sulfanyl]phénoxy}propanoïque,
l'acide {2-méthyl-4-[({4-(3-thiénylméthyl)-2-[4-(trifluorométhyl)phényl]-1,3-thiazol-5-yl}-méthyl)sulfanyl]phénoxy}acétique,
l'acide {4-[({4-benzyl-2-(4-(trifluorométhyl)phényl]-1,3-thiazol-5-yl}méthyl)sulfanyl]-2,5-diméthylphénoxy}acétique,
l'acide 2-{4-[({4-{[4-(4-acétylphényl)-1-pipérazinyl]méthyl}-2-[4-(trifluorométhyl)phényl]-1,3-thiazol-5-yl}méthyl)sulfanyl]-2-méthylphénoxy}propanoïque,
l'acide 2-{4-[({4{[4-(4-acétylphényl)-1-pipérazinyl]méthyl}-2-[4-(trifluorométhyl)phényl]-1,3-thiazol-5-yl}méthyl)sulfanyl]-2-éthylphénoxy}propanoïque,
l'acide 2-(2-méthyl-4-[({4-(2-thiénylméthyl)-2-[4-(trifluorométhyl)phényl]-1,3-thiazol-5 yl}-méthyl)sulfanyl]phénoxy}propanoïque,
l'acide 2-{4-[({4-{[4-(4-méthoxyphényl)-1-pipérazinyl]méthyl}-2-[4-(trifluorométhyl)phényl]-1,3-thiazol-5-yl} méthyl)sulfanyl]-2-méthylphénoxy}propanoïque,
l'acide 2-{4-[({4-{[4-(4-éthoxyphényl)-1-pipérazinyl]méthyl}-2-[4-(trifluorométhyl)phényl]-1,3-thiazol-5 yl}méthyl)sulfanyl]-2-méthylphénoxy}propanoïque,
l'acide 2-méthyl-2-{2-méthyl-4-[({4-{[4-(phénoxycarbonyl)-1-pipérazinyl]méthyl}-2-[4-(trifluorométhyl)phényl]-1,3-thiazol-5-yl}méthyl)sulfanyl]phénoxy}propanoïque,
l'acide 2-{4-[({4-{[4-(4-acétylphényl)-1-pipérazinyl]méthyl}-2-[4-(trifluorométhyl)phényl]-1,3-thiazol-5-yl}méthyl)sulfanyl]-2-propylphénoxy}propanoïque,
l'acide {2-méthyl-4-[({4-[4-(3-thiényl)benzyl]-2-[4-(trifluorométhyl)phényl]-1,3-thiazol-5-yl}-méthyl)sulfanyl] phénoxy}acétique,
l'acide 2-(4-{[(2-(4-fluorophényl)-4-{[4-(4-méthoxyphényl)-1-pipérazinyl]méthyl}-1,3-thiazol-5-yl)méthyl]sulfanyl}-2-méthylphénoxy}-2-méthylpropanoïque,
l'acide 2-{4-[({4-{[4-(4-acétylphényl)-1-pipérazinyl]méthyl}-2-[4-(trifluorométhyl)phényl]-1,3-thiazol-5-yl}-méthyl)sulfanyl]phénoxy}-2-méthylpropanoïque,
l'acide 2-{4-[({4-{[4-(2,4-diméthoxyphényl)-1-pipérazinyl]méthyl}-2-[4-(trifluorométhyl)phényl]-1,3 thiazol-5-yl} méthyl)sulfanyl]-2-méthylphénoxy}propanoïque,
l'acide {2-isopropyl-4-[({4-{[4-(4-méthoxyphényl)-1-pipérazinyl]méthyl}2-[4-(trifluorométhyl)phényl]-1,3-thiazol-5-yl}méthyl)sulfanyl]phénoxy}acétique,
l'acide 2-{4-[({4-{[4-(4-méthoxyphényl)-1-pipérazinyl]méthyl}-2-[4-(trifluorométhyl)phényl]-1,3-thiazol-5-yl} méthyl)sulfanyl]-2-propylphénoxy}propanoïque,
l'acide 2-{4-[({4-benzyl-2-[4-(trifluorométhyl)phényl]-1,3-thiazol-5-yl}méthyl)sulfanyl]-2 méthylphénoxy}propanoïque,
l'acide 2-{2-éthyl-4-[({4-{[4-(4-méthoxyphényl)-1-pipérazinyl]méthyl}-2-[4-(trifluorométhyl)phényl]-1,3-thiazol-5-yl}méthyl)sulfanyl]phénoxy}propanoïque,
l'acide 2-méthyl-2-{2-méthyl-4-[({4-[4-(trifluorométhyl)benzyl]-2-(4-(trifluorométhyl)phényl]-1,3-thiazol-5-yl}-méthyl)sulfanyl]phénoxy}propanoïque,
l'acide 2-{4-[({4-{[4-(4-fluorophényl)-1-pipérazinyl]méthyl}-2-[4-(trifluorométhyl)phényl]-1,3-thiazol-5-yl}méthyl)sulfanyl]-2-méthylphénoxy}-2-méthylpropanoïque,
l'acide {4-[({4-{[4-(4-acétylphényl)-1-pipérazinyl]méthyl}-2-[4-(trifluorométhyl)phényl]-1,3-thiazol-5-yl}méthyl) sulfanyl]-2-propylphénoxy}acétique,
l'acide {4-[({4-([1,1'-biphényl]-4-ylméthyl)-2-[4-(trifluorométhyl)phényl]-1,3-thiazol-5-yl}méthyl)sulfanyl]-2-méthylphénoxy}acétique,

l'acide 2-{4-[({4-{[4-(4-fluorophényl)-1-pipérazinyl)méthyl}-2-[4-(trifluorométhyl)phényl]-1,3-thiazol-5-yl}méthyl)sulfanyl]-2-méthylphénoxy}propanoïque,
l'acide {4-[({4-{[4-(3-méthoxyphényl)-1-pipérazinyl]méthyl}-2-[4-(trifluorométhyl)phényl)-1,3-thiazol-5-yl}méthyl)sulfanyl]-2-méthylphénoxy}acétique,
l'acide 2-{2-méthyl-4-[({4-(3-thiénylméthyl)-2-[4-(trifluorométhyl)phényl]-1,3-thiazol-5-yl}-méthyl)sulfanyl]phénoxy}propanoïque,
l'acide {4-[({4-{[4-(2-méthoxyphényl)-1-pipérazinyl]méthyl}-2-[4-(trifluorométhyl)phényl]-1,3-thiazol-5-yl}méthyl)sulfanyl]-2-méthylphénoxy}acétique,
l'acide 2-{2-isopropyl-4-[({4-{[4-(4-méthoxyphényl)-1-pipérazinyl]méthyl}-2-[4-(trifluorométhyl)phényl]-1,3-thiazol-5-yl}méthyl)sulfanyl]phénoxy}propanoïque,
l'acide 2-{4-[({4-(4-t-butylhenzyl)-2-[4-(trifluorométhyl)phényl]-1,3-thiazol-5-yl}méthyl)sulfanyl]-2 méthylphénoxy}-2-méthylpropanoïque,
l'acide 2-{4-[({4-{[4-(3-méthoxyphényl)-1-pipérazinyl]méthyl}-2-[4-(trifluorométhyl)phényl]-1,3-thiazol-5-yl}méthyl)sulfanyl]-2-méthylphénoxy}propanoïque,
l'acide 2-{4-[({4-benzyl-2-[4-(trifluorométhyl)phényl]-1,3-thiazol-5-yl}méthyl)sulfanyl]-2,3-diméthylphénoxy}propanoïque,
l'acide 2-{4-[({4-{[4-(4-chlorophényl)-1-pipérazinyl]méthyl}-2-[4-(trifluorométhyl)phényl]-1,3-thiazol-5-yl}méthyl)sulfanyl]-2-méthylphénoxy}propanoïque,
l'acide 2-{4-[({4-benzyl-2-[4-(trifluorométhyl)phényl]-1,3-thiazol-5-yl}méthyl)sulfanyl]-2 fluorophénoxy}propanoïque,
l'acide 2-{4-[({4-{[4-(2,4-difluorophényl)-1-pipérazinyl]méthyl}-2-[4-(trifluorométhyl)phényl]-1,3-thiazol-5-yl}méthyl)sulfanyl]-2-méthylphénoxy}propanoïque,
l'acide {4-[({4-(2,4-difluorobenzyl)-2-[4-(trifluorométhyl)phényl]- 1,3-thiazol-5-yl}méthyl)sulfanyl]-2-méthylphénoxy}acétique,
l'acide 2-{4-[({4-{[4-(4-acétylphényl)-1-pipérazinyl]méthyl}-2-[4-(trifluorométhyl)phényl]-1,3-thiazol-5 yl}méthyl)sulfanyl]-2-méthylphénoxy}-2-méthylpropanoïque,
l'acide 2-méthyl-2-{2-méthyl-4-[({4-(3-thiénylméthyl)-2-[4-(trifluorométhyl)phényl]-1,3-thiazol-5-yl}-méthyl)sulfanyl]phénoxy}propanoïque,
l'acide 2-{4-[({4-{[4-(4-méthoxyphényl)-1-pipérazinyl]méthyl}-2-[4-(trifluorométhyl)phényl]-1,3-thiazol-5-yl}méthyl)sulfanyl]-2-méthylphénoxy}propanoïque,
l'acide {2-éthyl-4-[({4-{[4-(4-méthoxyphényl)-1-pipérazinyl]méthyl}-2-[4-(trifluorométhyl)phényl]-1,3-thiazol-5-yl}méthyl)sulfanyl]phénoxy}acétique,
l'acide-2-{4-[({4-(4-méthoxybenzyl)-2-[4-(trifluorométhyl)phényl]-1,3-thiazol-5-yl}méthyl)sulfanyl]-2-méthylphénoxy}-2-méthylpropanoïque,
l'acide 2-méthyl-2-{4-[({4-{[4-(2-pyrazinyl)-1-pipérazinyl]méthyl}-2-[4-(trifluorométhyl)phényl]-1,3-thiazol-5-yl}-méthyl)sulfanyl]phénoxy}propanoïque,
l'acide 2-{4-[({4-{[4-(4-méthoxyphényl)-1-pipérazinyl]méthyl}-2-[4-(trifluorométhyl)phényl]-1,3-thiazol-5-yl}méthyl)sulfanyl]-2-méthylphénoxyl]-2-méthylpropanoïque,
l'acide 2-{4-[({4-{[4-{4-méthoxyphényl)-1-pipérazinyl]méthyl}-2-[4-(trifluorométhyl)phényl]-1,3-thiazol-5-yl}-méthyl)sulfanyl]phénoxy}-2-méthylpropanoïque,
l'acide 2-méthyl-2-{2-méthyl-4-[({4-[4-(trifluorométhoxy)benzyl]-2-[4-(trifluorométhyl)phényl]-1,3-thiazol-5-yl}-méthyl)sulfanyl]phénoxy}propanoïque,
l'acide 2-{4-[({4-{[4-(4-isopropoxyphényl)-1-pipérazinyl]méthyl)}-2-[4-(trifluorométhyl)phényl]-1,3-thiazol-5-yl}méthyl)sulfanyl]-2-méthylphénoxy}propanoïque,
l'acide 2-{2-méthyl-4-[({4-{[4-(2-pyrimidinyl)-1-pipérazinyl]méthyl}-2-[4-(trifluorométhyl)phényl]-1,3-thiazol-5-yl}méthyl)sutfanyl]phénoxy}propanoïque,
l'acide {2-méthyl-4-[({4-{3-phénylpropyl)-2-[4-(trifluorométhyl)phényl]-1,3-thiazol-5-yl}-méthyl)sulfanyl]phénoxy}acétique,
l'acide [4-[({4-benzyl-2-[4-(trifluorométhyl)phényl]-1,3-thiazol-5-yl}méthyl)sulfanyl]-2-(trifluorométhyl)phénoxy]acétique,
l'acide {2-méthyl-4-[({4-{[4-(5-méthyl-1,2,4-oxadiazol-3-yl)-phénoxy]méthyl}-2-[4-(trifluorométhyl)phényl]-1,3-thiazol-5-yl}méthyl)sulfanyl]phénoxy)acétique,
l'acide {4-[({4-benzyl-2-[4-(trifluorométhyl)phényl]-1,3-thiazol-5-yl}méthyl)sulfanyl]-5-chloro-2-méthylphénoxy}acétique,
l'acide {4-[({4-benzyl-2-[4-(trifluorométhyl)phényl]-1,3-thiazol-5-yl}méthyl)sulfanyl]-2-méthylphénoxy}acétique,
l'acide {4-[({4-(4-méthoxybenzyl)-2-[4-(trifluorométhyl)phényl]-1,3-thiazol-5-yl}méthyl)sulfanyl]-2-méthylphénoxy}acétique,

l'acide {2,5-diméthyl-4-[({4-(3-thiénylméthyl)-2-[4-(trifluorométhyl)phényl]-1,3-thiazol-5-yl}-méthyl)sulfanyl]phénoxy}acétique,
l'acide {2-méthyl-4-[({4-{[4-(2-pyrazinyl)-1-pipérazinyl]méthyl}-2-[4-(trifluorométhyl)phényl]-1,3-thiazol-5-yl}-méthyl)sulfanyl]phénoxy}acétique,
l'acide {4-[({4-benzyl-2-[4-(trifluorométhyl)phényl]-1,3-thiazol-5-yl}méthyl)sulfanyl]-2,3-diméthylphénoxy}acétique,
l'acide [4-({[2-(4-chlorophényl)-4-méthyl-1,3-thiazol-5-yl]méthyl}sulfanyl)-2-méthylphénoxy]acétique,
l'acide {2-méthyl-4-[({4-[(4-méthyl-2-thiényl)méthyl]-2-[4-(trifluorométhyl)phényl]-1,3-thiazol-5-yl}-méthyl)sulfanyl]phénoxy}acétique,
l'acide {4-[({4-benzyl-2-[4-(trifluorométhyl)phényl]-1,3-thiazol-5-yl}méthyl)sulfanyl]-2 bromophénoxy}acétique,
l'acide {2-méthyl-4-[({4-[(2-phényléthoxy)méthyl]-2-[4-(trifluorométhyl)phényl]-1,3-thiazol-5-yl}-méthyl)sulfanyl]phénoxy}acétique,
l'acide (2-méthyl-4-[({4-(2-phényléthyl)-2-[4-(trifluorométhyl)phényl]-1,3-thiazol-5-yl}-méthyl)sulfanyl]phénoxy}acétique,

et leurs sels, solvates et esters hydrolysables acceptables du point de vue pharmaceutique.

**17.** Composé de formule (I) choisi parmi :

l'acide 2-méthyl-2-{2-méthyl-4-[({4-(3-thiénylméthyl)-2-[4-(trifluorométhyl)phényl]-1,3-thiazol-5-yl}-méthyl)sulfanyl]phénoxy}propanoïque,
l'acide 2-{4-[({4-{[4-(4-méthoxyphényl)-1-pipérazinyl]méthyl}-2-[4-(trifluorométhyl)phényl]-1,3-thiazol-5-yl}méthyl)sulfanyl]-2-méthylphénoxy}propanoïque,
l'acide {2-éthyl-4-[({4-{[4-(4-méthoxyphényl)-1-pipérazinyl]méthyl}-2-[4-(trifluorométhyl)phényl]-1,3-thiazol-5-yl}méthyl)sulfanyl]phénoxy}acétique,
l'acide 2-{4-[({4-(4-méthoxybenzyl)-2-[4-(trifluorométhyl)phényl]-1,3-thiazol-5-yl}méthyl)sulfanyl]-2-méthylphénoxy}-2-méthylpropanoïque,
l'acide 2-méthyl-2-{4-[({4-{[4-(2-pyrazinyl)-1-pipérazinyl]méthyl}-2-[4-(trifluorométhyl)phényl]-1,3-thiazol-5-yl}-méthyl)sulfanyl]phénoxy}propanoïque,
l'acide 2-{4-[({4-{[4-(4-méthoxyphényl)-1-pipérazinyl]méthyl}-2-[4-(trifluorométhyl)phényl]-1,3-thiazol-5-yl}méthyl)sulfanyl]-2-méthylphénoxy}-2-méthylpropanoïque,
l'acide 2-{4-[({4-{[4-(4-méthoxyphényl)-1-pipérazinyl]méthyl}-2-[4-(trifluorométhyl)phényl]-1,3-thiazol-5-yl}-méthyl)sulfanyl]phénoxy}-2-méthylpropanoïque,
l'acide 2-méthyl-2-{2-méthyl-4-[({4-[4-(trifluorométhoxy)benzyl]-2-[4-(trifluorométhyl)phényl]-1,3-thiazol-5-yl}-méthyl)sulfanyl]phénoxy}propanoïque,
l'acide 2-{4-[({4-{[4-(4-isopropoxyphényl)-1-pipérazinyl]méthyl}-2-[4-(trifluorométhyl)phényl]-1,3-thiazol-5-yl}méthyl)sulfanyl]-2-méthylphénoxy}propanoïque,
l'acide 2-{2-méthyl-4-[({4-{[4-(2-pyrimidinyl)-1-pipérazinyl]méthyl}-2-[4-(trifluorométhyl)phényl]-1,3-thiazol-5-yl}méthyl)sulfanyl]phénoxy}propanoïque,

et leurs sels, solvates ou esters hydrolysables acceptables du point de vue pharmaceutique.

**18.** Composé selon l'une quelconque des revendications précédentes, qui est un agoniste de hPPAR$\delta$.

**19.** Composé selon la revendication 18, qui est aussi un agoniste de hPPAR$\alpha$ ou hPPAR$\gamma$.

**20.** Composé selon l'une quelconque des revendications précédentes, qui est un pan agoniste de hPPAR.

**21.** Composé selon l'une quelconque des revendications 1 à 20 utile en thérapie.

**22.** Composition pharmaceutique comprenant un composé selon l'une quelconque des revendications 1 à 20.

**23.** Composition pharmaceutique selon la revendication 22 comprenant de plus un support ou un diluant acceptable du point de vue pharmaceutique.

**24.** Utilisation d'un composé selon l'une quelconque des revendications 1 à 20 pour la fabrication d'un médicament destiné au traitement d'un état ou d'une maladie à hPPAR.

**25.** Utilisation selon la revendication 24, dans laquelle l'état ou la maladie médié par hPPAR est une dyslipidémie, un syndrome X, une insuffisance cardiaque, une hypercholestérolémie, une affection cardiovasculaire, un diabète sucré de type II, un diabète de type I, une résistance à l'insuline, une hyperlipidémie, l'obésité, l'anorexie avec boulimie et l'anorexie mentale.